# EUROPEAN PATENT APPLICATION

(11) **EP 4 063 347 A1**
(43) Date of publication of application: **28.09.2022**
(21) Application number: 19953515.4
(22) Date of filing: 20.11.2019
(51) Int. Cl.: C07C 263/04, C07C 265/04, C07C 265/14, C07C 323/57

(54) **METHOD FOR PRODUCING ISOCYANATE**

(71) Applicant: Asahi Kasei Kabushiki Kaisha, Tokyo 1000006 (JP)
(72) Inventor: TAKAGAKI Kazuhiro, Tokyo 100-0006 (JP); IWATA Yusuke, Tokyo 100-0006 (JP); SHINOHATA Masaaki, Tokyo 100-0006 (JP); NAKAOKA Koichi, Tokyo 100-0006 (JP); SAKURAI Yusuke, Tokyo 100-0006 (JP)
(74) Representative: D Young & Co LLP
(86) International application number: PCT/JP2019/045351
(87) International publication number: WO 2021/100131

(57) **Abstract**

This isocyanate production method, for continuously producing an isocyanate while suppressing side reactions, is a method for producing an isocyanate through the thermal decomposition of carbamate, and comprises: a thermal decomposition step in which a mixed solution containing carbamate and a compound (A) having a specific structure is continuously put into a pyrolysis reactor and carry out a pyrolysis reaction of carbamate; a low-boiling-point decomposition product recovery step in which a low-boiling-point decomposition product having a lower standard boiling point than the compound (A) is continuously extracted in a gaseous form from the pyrolysis reactor, and a high-boiling-point component recovery step in which a liquid phase component, which is not recovered in a gaseous form in the low-boiling-point decomposition product recovery step, is continuously extracted as a high-boiling-point component from the pyrolysis reactor.

## Description

### TECHNICAL FIELD

The present invention relates to a preparation method of an isocyanate.

### BACKGROUND OF THE INVENTION

Isocyanates are widely used as raw materials of polyurethane foam, coating materials, or adhesives. Industrial preparation of isocyanates mainly uses a reaction of an amine compound and phosgene (phosgene method), and almost the entire production of isocyanates worldwide is by the phosgene method. However, the phosgene method has numerous problems.

Firstly, a large amount of phosgene is used as a raw material. Phosgene is an extremely highly toxic substance, its handling requires special precautions to prevent handlers from being exposed, and a special device is required to remove waste.

Secondly, since a large amount of highly corrosive hydrogen chloride is produced as a by-product in the phosgene method, a process for removing hydrogen chloride is required. In addition, a hydrolyzable chlorine is often contained in the resultant isocyanates. Accordingly, there is a case in which the use of isocyanates produced by the phosgene method has a detrimental effect on the weather resistance or the heat resistance of polyurethane products.

In consideration of these factors, there is a need for a preparation method of isocyanate compounds without using phosgene. As one of such preparation methods of isocyanate compounds without using phosgene, a method in which a carbamic acid ester is subjected to thermal decomposition has been proposed. It is known that an isocyanate and a hydroxy compound are obtained by the thermal decomposition of a carbamic acid ester (see, for example, Non-Patent Document 1). The basic reaction thereof is indicated by the following general formula (1):

R(NHCOOR')a → R(NCO)a+a R'OH (1)

In the general formula (1), R is an a-valent organic remaining group. R' is a monovalent organic remaining group. a is an integer of 1 or more.

Patent Document 1 discloses a method for preparing an isocyanate by subjecting a carbamate to thermal decomposition in a flask in the presence of an inert solvent. Patent Document 2 discloses a method for preparing an isocyanate by subjecting a carbamate to thermal decomposition in the presence of both an aromatic hydroxy compound and a carbonic acid derivative.

On the other hand, the thermal decomposition reaction of a carbamic acid ester tends to be accompanied by various irreversible side reactions such as an unfavorable thermal denaturation reaction of the carbamic acid ester or condensation reaction of an isocyanate produced by the thermal decomposition reaction (see, for example, Non-Patent Documents 1 and 2).

These side reactions not only cause a decrease in yield or selectivity of a target isocyanate, but also cause a case in which the long-term operation becomes difficult in the preparation of polyisocyanate particularly due to deposition of polymeric solid materials, thereby causing blockage of a reactor.

### DOCUMENTS OF RELATED ART

### Patent Documents

Patent Document 1: Japanese Unexamined Patent Application Publication No. 2003-252846
Patent Document 2: Japanese Unexamined Patent Application Publication No. 2012-233014

### Non-Patent Documents

Non-Patent Document 1: Berchte der Deutechen Chemischengesellschaft, Vol. 3, pp. 653, 1870.
Non-Patent Document 2: Journal of American Chemical Society, Vol. 81, pp. 2138, 1959.

### SUMMARY OF THE INVENTION

### PROBLEMS TO BE SOLVED BY THE INVENTION

Although Patent Document 1 discloses a method in which thermal decomposition is conducted by supplying carbamates into a reactor while extracting the resultant isocyanates, it is difficult to prepare isocyanates continuously over a long period due to the absence of any structure configured to extract high-boiling-point components produced by side reactions.

Although isocyanates produced by thermal decomposition of carbamates are extracted continuously as low-boiling-point decomposition products in a method disclosed in Patent Document 2, carbamates produced by reaction of the resultant isocyanates and hydroxy compounds fall to the bottom of a reactor, and high-boiling-point components are produced by side reactions in the bottom of the reactor, thereby tending to decrease the yield of isocyanates.

The present invention has been obtained in view of the above-mentioned circumstances, and provides a preparation method of an isocyanate in which side reactions are suppressed and an isocyanate is prepared continuously.

### MEANS TO SOLVE THE PROBLEMS

The present intention encompasses the following aspects. (1) A preparation method of an isocyanate in which the isocyanate is prepared by thermal decomposition of a carbamate, the preparation method including:
a thermal decomposition step in which a mixture liquid containing a carbamate and at least one compound (A) is introduced continuously into a thermal decomposition reactor to allow a thermal decomposition reaction of the carbamate to proceed;
a low-boiling-point decomposition product collecting step in which a low-boiling-point decomposition product having a standard boiling point lower than a standard boiling point of the compound (A) is extracted continuously from the thermal decomposition reactor in a gaseous state; and
a high-boiling-point component collecting step in which a liquid-phase component which is not collected in a gaseous state in the low-boiling-point decomposition product collecting step is extracted continuously from the thermal decomposition reactor as a high-boiling-point component,
wherein the compound (A) is selected from the group consisting of polymers having a repeating unit of the following general formula (4), compounds of the following general formula (5), compounds of the following general formula (6), compounds of the following general formula (7), compounds of the following general formula (S1), compounds of the following general formula (S2), compounds of the following general formula (S3), compounds of the following general formula (9), compounds of the following general formula (10) and C9-35 chained or cyclic aliphatic hydrocarbons.

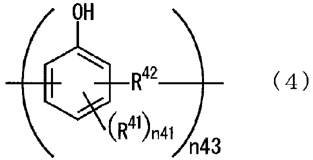

In the general formula (4), R⁴¹ is a monovalent hydrocarbon group. The hydrocarbon group may have either an ether bond or an ester bond. n41 is 0 or an integer of 1 to 3. R⁴² is a divalent organic group. n43 is an integer of 2 to 50.

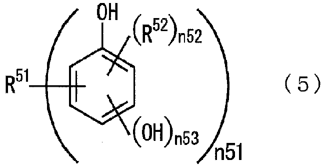

In the general formula (5), n51 is an integer of 1 to 4. R⁵¹ is a hydrogen atom or an n51-valent organic group. R⁵² is a monovalent hydrocarbon group. The hydrocarbon group may have either an ether bond or an ester bond. n52 is 0 or an integer of 1 to 4. n53 is 0 or 1.

R⁶¹-(COO-R⁶²)ₙ₆₁ (6)

In the general formula (6), n61 is an integer of 1 to 3. R⁶¹ is an n61-valent C1-60 hydrocarbon group. The C1-60 hydrocarbon group may have either an ether bond or an ester bond. R⁶² is a C1-20 aliphatic hydrocarbon group or a C6-20 aromatic hydrocarbon group.

R⁷¹-(OCO-R⁷²)ₙ₇₁ (7)

In the general formula (7), n71 is 2 or 3. R⁷¹ is an n71-valent C1-60 hydrocarbon group. The C1-60 hydrocarbon group may have either an ether bond or an ester bond. R⁷² is a C1-20 aliphatic hydrocarbon group or a C6-20 aromatic hydrocarbon group.

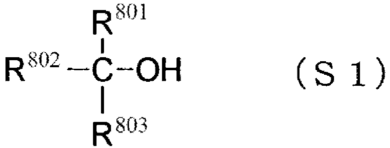

In the general formula (S1), R⁸⁰¹, R⁸⁰² and R⁸⁰³ are each independently a C1-60 saturated or unsaturated linear or branched hydrocarbon group, when R⁸⁰¹, R⁸⁰² or R⁸⁰³ has a methylene group, the methylene group may be substituted with an oxygen atom, an arylene group, a cycloalkylene group or an NH group at least one CH group constituting R⁸⁰¹, R⁸⁰² or R⁸⁰³ may be substituted with a nitrogen atom, at least one hydrogen atom constituting R⁸⁰¹, R⁸⁰² or R⁸⁰³ may be substituted with a halogen atom or a hydroxy group, and R⁸⁰¹, R⁸⁰² or R⁸⁰³ may be bonded together to form a monocycle or a polycycle.

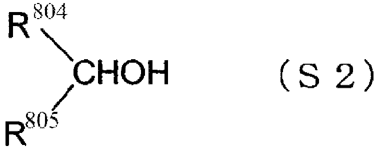

In the general formula (S2), R⁸⁰⁴ and R⁸⁰⁵ are each independently a C1-60 saturated or unsaturated linear or branched hydrocarbon group, when R⁸⁰⁴ or R⁸⁰⁵ has a methylene group, the methylene group may be substituted with an oxygen atom, an arylene group, a cycloalkylene group or an NH group, at least one CH group constituting R⁸⁰⁴ or R⁸⁰⁵ may be substituted with a nitrogen atom, at least one hydrogen atom constituting R⁸⁰⁴ or R⁸⁰⁵ may be substituted with a halogen atom or a hydroxy group, and R⁸⁰⁴ or R⁸⁰⁵ may be bonded together to form a monocycle or a polycycle.

R⁸⁰⁶-CH₂OH (S3)

In the general formula (S3), R⁸⁰⁶ is a C1-60 saturated or unsaturated linear or branched hydrocarbon group, when R⁸⁰⁶ has a methylene group, the methylene group may be substituted with an oxygen atom, an arylene group, a cycloalkylene group or an NH group, at least one CH group constituting R⁸⁰⁶ may be substituted with a nitrogen atom, at least one hydrogen atom constituting R⁸⁰⁶ may be substituted with a halogen atom or a hydroxy group, and branched chains may be bonded together to form a cycle.

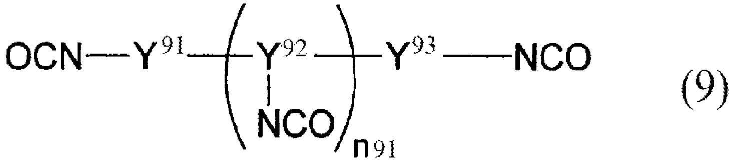

In the general formula (9), Y⁹¹ and Y⁹³ are each independently a C4-10 divalent hydrocarbon group having either an alicyclic hydrocarbon group or an aromatic hydrocarbon group. Y⁹² is a C4-10 trivalent hydrocarbon group having either an alicyclic hydrocarbon group or an aromatic hydrocarbon group, at least one CH group constituting an aromatic hydrocarbon group may be substituted with a nitrogen atom or a carbonyl group. n91 is an integer of 0 to 5.

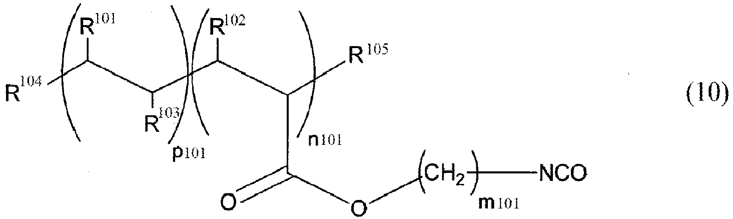

In the general formula (10), p101 is an integer of 0 to 90. n101 is an integer of 1 to 100. The sum of p101 and n101 is an integer of 10 to 100. m101 is an integer of 1 to 5. R¹⁰¹ and R¹⁰² are each independently a hydrogen atom or a C1-5 monovalent hydrocarbon group. R¹⁰³ is a C1-5 alkoxycarbonyl group or a C1-12 monovalent hydrocarbon group. R¹⁰⁴ and R¹⁰⁵ are each independently a monovalent organic group.

(2) The preparation method of an isocyanate according to (1) mentioned above, wherein the compound (A) is selected from the group consisting of the polymers having a repeating unit of the general formula (4) and the compounds of the general formula (5).

(3) The preparation method of an isocyanate according to (2) mentioned above, wherein the compound (A) is selected from the group consisting of polymers having either a repeating unit of the following general formula (4-1) or a repeating unit of the following general formula (4-2), compounds of the following general formula (5-1) and compounds of the following general formula (5-2).

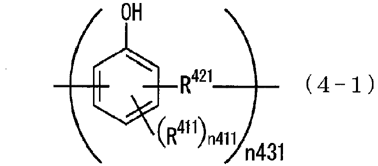

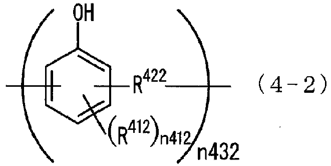

In the general formula (4-1), R⁴¹¹ is a monovalent hydrocarbon group. The monovalent hydrocarbon group may have either an ether bond or an ester bond, and may be substituted with a hydroxy group. n411 is 0 or an integer of 1 to 3. When n411 is 2 or 3, R⁴¹¹ may be identical to or different from each other. R⁴²¹ is a divalent aliphatic hydrocarbon group. The divalent aliphatic hydrocarbon group may have either an ether bond or an ester bond. n431 is an integer of 2 to 50.

In the general formula (4-2), R⁴¹² is a monovalent hydrocarbon group. The monovalent hydrocarbon group may have either an ether bond or an ester bond. n412 is 0 or an integer of 1 to 3. R⁴²² is a divalent aromatic hydrocarbon group or a divalent group formed by bonding an aliphatic hydrocarbon group and an aromatic hydrocarbon group. The aliphatic hydrocarbon group may have either an ether bond or an ester bond. n432 is an integer of 2 to 50.

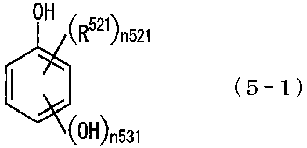

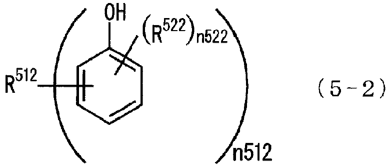

In the general formula (5-1), R⁵²¹ is a C1-20 alkyl group which may be substituted with a C6-12 aryl group or a C1-20 alkoxycarbonyl group which may be substituted with a C6-12 aryl group. n521 is 0 or an integer of 1 to 4. n531 is 0 or 1.

In the general formula (5-2), n512 is an integer of 2 to 4. R⁵¹² is an n512-valent hydrocarbon group. The n512-valent hydrocarbon group may have an ether bond, an ester bond, a carbonyl group or a hetero ring. R⁵²² is a monovalent hydrocarbon group. The monovalent hydrocarbon group may have either an ether bond or an ester bond. n522 is 0 or an integer of 1 to 4.

(4) The preparation method of an isocyanate according to (1) mentioned above, wherein the compound (A) is selected from the group consisting of the compounds of the general formula (6) and the compounds of the general formula (7).

(5) The preparation method of an isocyanate according to (4) mentioned above, wherein the compound (A) is selected from the group consisting of compounds of the following general formula (6-1), compounds of the following general formula (6-2) and compounds of the following general formula (7-1).

R⁶¹¹-(COO-R⁶¹²)ₙ₆₁₁ (6-1)

R⁶²¹-(COO-R⁶²²)ₙ₆₂₁ (6-2)

In the general formula (6-1), n611 is 2 or 3. R⁶¹¹ is an n611-valent C1-60 aliphatic hydrocarbon group. The C1-60 aliphatic hydrocarbon group may have either an ether bond or an ester bond. R⁶¹² is a C1-20 aliphatic hydrocarbon group or a C6-20 aromatic hydrocarbon group.

In the general formula (6-2), n621 is 2 or 3. R⁶²¹ is an n621-valent C6-60 aromatic hydrocarbon group. The C6-60 aromatic hydrocarbon group may have either an ether bond or an ester bond. R⁶²² is a C1-20 aliphatic hydrocarbon group or a C6-20 aromatic hydrocarbon group.

R⁷¹¹-(OCO-R⁷¹²)ₙ₇₁₁ (7-1)

In the general formula (7-1), n711 is 2 or 3. R⁷¹¹ is an n711-valent C1-60 aliphatic hydrocarbon group. The C1-60 aliphatic hydrocarbon group may have either an ether bond or an ester bond. R⁷¹² is a CI-20 aliphatic hydrocarbon group or a C6-20 aromatic hydrocarbon group.

(6) The preparation method of an isocyanate according to (5) mentioned above, wherein the compound (A) is selected from the group consisting of compounds of the following general formula (6-1-1), compounds of the following general formula (6-2-1) and compounds of the following general formula (7-1-1).

R⁶¹³-OOC-Y⁶¹¹-COO-R⁶¹⁴ (6-1-1)

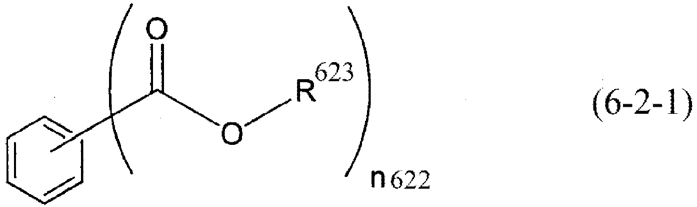

In the general formula (6-1-1), R⁶¹³ and R⁶¹⁴ are each independently a C1-20 aliphatic hydrocarbon group or a C6-20 aromatic hydrocarbon group. Y⁶¹¹ is a divalent C1-60 aliphatic hydrocarbon group. The C1-60 aliphatic hydrocarbon group may have either an ether bond or an ester bond.

In the general formula (6-2-1), R⁶²³ is a C1-20 aliphatic hydrocarbon group or a C6-20 aromatic hydrocarbon group. n622 is 2 or 3.

R⁷¹³-OCO-Y⁷¹¹-OCO-R⁷¹⁴ (7-1-1)

In the general formula (7-1-1), R⁷¹³ and R⁷¹⁴ are each independently a Cl-20 aliphatic hydrocarbon group or a C6-20 aromatic hydrocarbon group. Y⁷¹¹ is a divalent C1-60 aliphatic hydrocarbon group. The C1-20 aliphatic hydrocarbon group may have either an ether bond or an ester bond.

(7) The preparation method of an isocyanate according to (1) mentioned above, wherein the compound (A) is selected from the group consisting of the compounds of the general formula (S1), the compounds of the general formula (S2), and the compounds of the general formula (S3).

(8) The preparation method of an isocyanate according to (7) mentioned above, wherein the compound (A) is the compound of the general formula (S1).

(9) The preparation method of an isocyanate according to (1) mentioned above, wherein the compound (A) is selected from the group consisting of the compounds of the general formula (9) and the compounds of the general formula (10).

(10) The preparation method of an isocyanate according to (9) mentioned above, wherein the compound (A) is selected from the group consisting of compounds of the following general formula (9-1) and compounds of the following general formula (10-1).

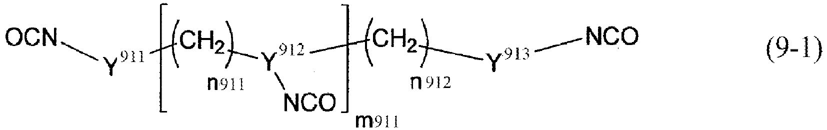

In the general formula (9-1), Y⁹¹¹ and Y⁹¹³ are each independently a C4-10 divalent alicyclic hydrocarbon group or a C6-10 divalent aromatic hydrocarbon group, Y⁹¹² is a C4-10 trivalent alicyclic hydrocarbon group or a C6-10 divalent aromatic hydrocarbon group, n911 and n912 are each independently an integer of 1 to 5, and m911 is an integer of 0 to 5.

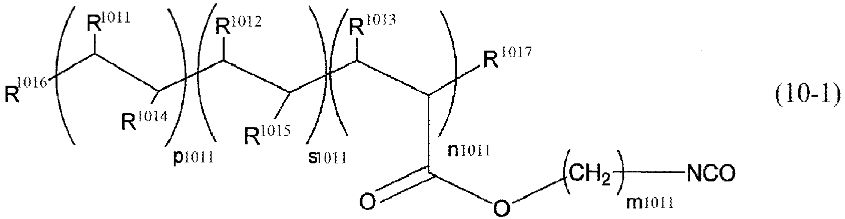

In the general formula (10-1), p1011 is an integer of 0 to 50, s1011 is an integer of 0 to 50, n1011 is an integer of 1 to 100, the sum of p1011, s1011 and n1011 is an integer of 10 to 100, m1011 is an integer of 1 to 5, R¹⁰¹¹, R¹⁰¹² and R¹⁰¹³ are each independently a hydrogen atom or a C1-5 monovalent hydrocarbon group, R¹⁰¹⁴ and R¹⁰¹⁵ are each independently a C1-5 alkoxycarbonyl group or a C1-12 monovalent hydrocarbon group, and R¹⁰¹⁶ and R¹⁰¹⁷ are each independently a monovalent organic group.

(11) The preparation method of an isocyanate according to (9) mentioned above, wherein the compound (A) is a compound of the following formula (9-2).

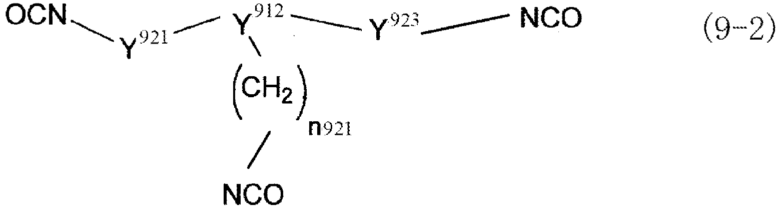

In the general formula (9-2), Y⁹²¹ and Y⁹²³ are each independently a C4-10 alkylene group, Y⁹¹² is a 2,4,6-trioxohexahydro-1,3,5-triazine-1,3,5-triynyl group, and n921 is an integer of 1 to 6.

(12) The preparation method of an isocyanate according to (1) mentioned above, wherein the compound (A) is selected from the group consisting of the C9-35 chained or cyclic aliphatic hydrocarbons.

(13) The preparation method of an isocyanate according to (12) mentioned above, wherein the chained aliphatic hydrocarbons are chained aliphatic hydrocarbons having a branched chain composed of a C1-3 linear aliphatic hydrocarbon group.

(14) The preparation method of an isocyanate according to (12) or (13) mentioned above, wherein the carbon number of the chained aliphatic hydrocarbon is 12 to 30. (15) The preparation method of an isocyanate according to any one of (1) to (14) mentioned above, wherein the mixture liquid further contains an inert solvent,
the low-boiling-point decomposition product and the inert solvent are extracted continuously in a gaseous state from the thermal decomposition reactor in the low-boiling-point decomposition product collecting step, and
the inert solvent is substantially inert under thermal decomposition reaction conditions, and has a standard boiling point lower than the standard boiling point of the compound (A) but between standard boiling points of an isocyanate and a hydroxy compound that are produced by thermal decomposition.

(16) The preparation method of an isocyanate according to any one of (1) to (15) mentioned above, wherein the carbamate is a compound of the following general formula (2).

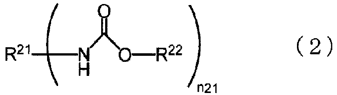

In the general formula (2), n21 is an integer of 1 or more. R²¹ is an n21-valent organic group. R²² is a remaining group obtained by removing one hydroxy group from a hydroxy compound.

(17) The preparation method of an isocyanate according to (16) mentioned above, wherein in the general formula (2), n21 is 2 or 3, and R²² is a C6-20 aromatic group.

(18) The preparation method of an isocyanate according to any one of (1) to (17) mentioned above, wherein the thermal decomposition reactor is a tubular reactor.

(19) The preparation method of an isocyanate according to any one of (1) to (18) mentioned above, wherein the low-boiling-point decomposition product contains the isocyanate, and the preparation method further includes a separation step in which the low-boiling-point decomposition product is supplied in a gaseous state to a distillation column to separate the isocyanate in the distillation column.

(20) The preparation method of an isocyanate according to any one of (1) to (19) mentioned above, wherein a carrier agent which is substantially inert in a gaseous state under thermal decomposition reaction conditions is introduced into the thermal decomposition reactor to discharge a gaseous component from the thermal decomposition reactor.

### EFFECTS OF THE INVENTION

The preparation method of an isocyanate according to the above-mentioned aspects makes it possible to suppress side reactions and to produce the isocyanate continuously.

### BRIEF DESCRIPTION OF DRAWINGS

Fig. 1 is a schematic diagram illustrating the constitution of a preparation device of isocyanate used in Example 1-1 and the like.
Fig. 2 is a schematic diagram illustrating the constitution of a preparation device of isocyanate used in Example 1-2 and the like.

### EMBODIMENTS FOR CARRYING OUT THE INVENTION

An embodiment in which the present invention (hereinafter, referred to as "present embodiment") is carried out will be explained in detail below. The following present embodiment is an example to explain the present invention, although the present invention is not limited to the following present embodiment. The present invention may be appropriately modified within the scope of the summary thereof to be carried out.

### <<Preparation method of isocyanate>>

The preparation method of an isocyanate according to the present embodiment is a method for preparing an isocyanate by subjecting a carbamate to thermal decomposition.

The preparation method of an isocyanate according to the present embodiment is a method including: a thermal decomposition step; a low-boiling-point decomposition product collecting step; and a high-boiling-point component collecting step.

In the thermal decomposition step, a mixture liquid containing a carbamate and at least one compound (A) mentioned below is introduced continuously into a thermal decomposition reactor to allow a thermal decomposition reaction of the carbamate to proceed.

In the low-boiling-point decomposition product collecting step, a low-boiling-point decomposition product having a standard boiling point lower than that of the compound (A) is extracted continuously from the thermal decomposition reactor in a gaseous state.

In the high-boiling-point component collecting step, a liquid-phase component which is not collected in a gaseous state in the low-boiling-point decomposition product collecting step is extracted continuously from the thermal decomposition reactor as a high-boiling-point component.

The preparation method according to the present embodiment makes it possible to suppress side reaction and to produce continuously an isocyanate.

Each step will be explained below.

### [Thermal decomposition step]

In the step, a mixture liquid containing a carbamate and the compound (A) is introduced continuously into a thermal decomposition reactor to allow a thermal decomposition reaction to proceed, thereby obtaining an isocyanate. In the thermal decomposition reaction, an isocyanate and a hydroxy compound (preferably an aromatic hydroxy compound) are produced from the carbamate. The step is preferably conducted in a liquid-phase.

The mixture liquid may further contain an inert solvent. The inert solvent is substantially inert under thermal decomposition reaction conditions, and has a standard boiling point lower than the standard boiling point of the compound (A) and between standard boiling points of the isocyanate and the hydroxy compound that are produced by thermal decomposition. Namely, the standard boiling point of each component in the mixture liquid becomes high in the order of the hydroxy compound, the inert solvent, the isocyanate, and the compound (A).

In the present specification, the phrase "substantially inert" means that the carbamate and thermally decomposed products, that is, the isocyanate and the hydroxy compound, do not react, or even if a reaction is caused, there are no significant effects on the thermal decomposition of the carbamate.

The carbamate used in the present step is preferably a carbamate obtained by the preparation method described later.

The inert solvent and the compound (A) used in the present step will also be described later.

The amount of the carbamate in the mixture liquid is generally 1% by mass to 50% by mass, preferably 3% by mass to 40% by mass, and more preferably 5% by mass to 30% by mass, relative to the total mass of the mixture liquid.

When the amount of the carbamate is the lower limit or more, the space-time yield of the isocyanate is further improved, which tends to be advantageous in an industrial operation. When the amount of the carbamate is the upper limit or less, side reactions tend to be further suppressed during thermal decomposition.

The reaction temperature is generally 100°C to 300°C. Although a high temperature is preferable in order to increase the reaction rate, the reaction temperature is preferably 120°C to 270°C, and more preferably 150°C to 250°C from the viewpoint of further suppression of side reactions caused by at least either the carbamate or the resultant isocyanate.

In order to keep the reaction temperature constant, a conventionally-known cooling device and heating device may be installed in the thermal decomposition reactor.

Although the reaction pressure varies depending on the type of compounds used and the reaction temperature, the reaction pressure may be reduced pressure, ordinary pressure or pressurization, and is generally 1 Pa to 1×10⁶ Pa.

The reaction time (residence time) is not particularly limited, and is usually preferably 0.001 hours to 100 hours, more preferably 0.005 hours to 50 hours, and even more preferably 0.01 hours to 10 hours.

Although the type of the thermal decomposition reactor is not particularly limited, a conventionally-known distillation device is preferably used, and the thermal decomposition reactor is more preferably constituted by at least one reactor selected from the group consisting of an evaporator, a continuous multi-stage distillation column, a packed column, a thin film evaporator and a falling film evaporator, in order to efficiently collect the gaseous phase components.

In addition, various known methods such as a method in which a reactor including any of a distillation column, a multi-stage distillation column, a multitubular reactor, a reactor having an internal support, a forced circulation reactor, a falling film evaporator and a falling drop evaporator is used and a method in which these are combined are used.

From the viewpoint of quickly removing the low-boiling-point decomposition product having a standard boiling point lower than that of the compound (A) from the reaction system, a packed column or a tubular reactor is preferably used, a tubular reactor is more preferably used, and a tubular reactor such as a tubular thin-film evaporator or a tubular falling film evaporator is even more preferably used. As the internal structure of these reactors, a structure having a large gas-liquid contact area that allows the resultant low-boiling-point decomposition product to be quickly transferred to the gaseous phase is preferable.

When a packed column is used, a filler generally used in a distillation column or an absorption column may be appropriately used as a solid filler provided in the packed column. Specific examples of the preferable solid filler include a Raschig ring, Lessing ring, Spiral ring, Pall ring, Intalox saddle, Stedman packing, McMahon packing, Dixon packing, helix packing, coil packing, and heat pipe packing.

A material of the solid filler is not particularly limited, and may be porcelain, metallic, or the like. Among these, a material having a high thermal conductivity is preferable as the solid filler.

Although the thermal decomposition reactor or lines may be formed by any of conventionally known materials, unless the materials exert harmful effects on the carbamate or the resultant hydroxy compound or isocyanate, SUS 304, SUS 316, or SUS 316L is preferably used because of the low cost thereof.

In the present step, a catalyst is not always required, but a catalyst may be used so as to decrease the reaction temperature or terminate the reaction promptly.

The amount of the catalyst to be used is preferably 0.01% by mass to 30% by mass, and more preferably 0.5% by mass to 20% by mass, relative to the mass of the carbamate.

Examples of the catalyst include: Lewis acids; transition metal compounds that generate Lewis acids; organic tin compounds; compounds containing a copper group metal; compounds containing lead; compounds containing zinc; compounds containing an iron group metal; and amines.

Specific examples of Lewis acids and transition metal compounds that generate Lewis acids include AlX₃, TiX₃, TiX₄, VOX₃, VX₅, ZnX₂, FeX₃, and SnX₄. In the formulae, "X" is a halogen, an acetoxy group, an alkoxy group, or an aryloxy group.

Specific examples of organic tin compounds include (CH₃)₃SnOCOCH₃, (C₂H₅)SnOCOC₆H₅, Bu₃SnOCOCH₃, Ph₃SnOCOCH₃, Bu₂Sn(OCOCH₃)₂, Bu₂Sn(OCOC₁₁H₂₃)₂ (dibutyltin dilaurate), Ph₃SnOCH₃, (C₂H₅)₃SnOPh, Bu₂Sn(OCH₃)₂, Bu₂Sn(OC₂H₅)₂, Bu₂Sn(OPh)₂, Ph₂Sn(CH₃)₂, (C₂H₅)₃SnOH, PhSnOH, Bu₂SnO, (C₈H₁₇)₂SnO, Bu₂SnCl₂, BuSnO(OH) and tin octylate. In the formulae, "Bu" indicates a butyl group and "Ph" indicates a phenyl group.

Specific examples of compounds containing a copper group metal include CuCl, CuCl₂, CuBr, CuBr₂, CuI, CuI₂, Cu(OAc)₂, Cu(acac)₂, copper olefinate, Bu₂Cu, (CH₃O)₂Cu, AgNO₃, AgBr, silver picrate, and AgC₆H₆ClO₄. In the formula, "acac" indicates an acetylacetone chelate ligand.

Specific examples of compounds containing lead include lead octylate.

Specific examples of compounds containing zinc include Zn(acac)₂.

Specific examples of compounds containing an iron group metal include Fe(C₁₀H₈)(CO)₅, Fe(CO)₅, Fe(C₄H₆)(CO)₃, Co(mesitylene)₂(PEt₂Ph₂), CoC₅F₅(CO)₇, and ferrocene.

Specific examples of amines include 1,4-diazabicyclo[2,2,2]octane, triethylene diamine, and triethyl amine.

Among these, dibutyltin dilaurate, lead octylate, or tin octylate is preferable. One of these catalysts may be used alone or at least two thereof may be used in combination.

### [Low-boiling-point decomposition product collecting step]

In the present step, a low-boiling-point decomposition product produced by the thermal decomposition reaction of the carbamate is extracted continuously from the thermal decomposition reactor in a gaseous state. The term "low-boiling-point decomposition product" refers to a compound having a standard boiling point lower than the standard boiling point of the compound (A) among the isocyanate and the hydroxy compound that are produced by the thermal decomposition reaction of the carbamate. As the low-boiling-point decomposition product, at least either the hydroxy compound or the isocyanate is preferable, and both the hydroxy compound and the isocyanate are preferable. When the mixture liquid contains an inert solvent, the low-boiling-point decomposition product and the inert solvent are extracted continuously from the thermal decomposition reactor in a gaseous state in the present step.

In order to collect these components in a gaseous state, it is preferable that the temperature, the pressure, and other conditions under which the step is conducted be determined depending on used compounds or compounds produced by thermal decomposition of a carbamate.

In order to collect the low-boiling-point decomposition product promptly, a carrier agent may be introduced into the thermal decomposition reactor to discharge a gaseous component containing the carrier agent from the thermal decomposition reactor. The term "carrier agent" used herein refers to an agent which is substantially inert in a gaseous state under thermal decomposition reaction conditions.

Specific examples of such a carrier agent include inert gases and hydrocarbon gases. Examples of the inert gases include nitrogen, argon, helium, carbon dioxide, methane, ethane, and propane. Among these, inert gases such as nitrogen are preferable.

Examples of an agent that exhibits a similar effect include low-boiling-point organic solvents. Examples of the low-boiling-point organic solvents include halogenated hydrocarbons, lower hydrocarbons and ethers. Examples of the halogenated hydrocarbons include dichloromethane, chloroform, and carbon tetrachloride. Examples of the lower hydrocarbons include pentane, hexane, heptane, and benzene. Examples of the ethers include tetrahydrofuran and dioxane.

One of these carrier agents may be used alone, or at least two thereof may be mixed to be used. These carrier agents are preferably heated in advance to be used.

The low-boiling-point decomposition product, or both the low-boiling-point decomposition product and the inert solvent, which are collected from the thermal decomposition reactor in a gaseous state, may be directly introduced into a cooler and then collected partially or entirely in a liquid state. The purification and separation may be conducted by supplying, to a distillation column, the low-boiling-point decomposition product or both the low-boiling-point decomposition product and the inert solvent in a gaseous state, or in a liquid state after being introduced into the cooler.

### [High-boiling-point component collecting step]

In the present step, a liquid-phase component which is not collected in a gaseous state in the low-boiling-point decomposition product collecting step is extracted continuously from the reactor to be collected as a high-boiling-point component. The low-boiling-point decomposition product having a standard boiling point lower than that of the compound (A) supplied to the thermal decomposition reactor or both the low-boiling-point decomposition product and the inert solvent are collected in a gaseous state in the low-boiling-point decomposition product collecting step. Thus, it is understood that the high-boiling-point component collected in the present step is a liquid phase component that cannot be collected in a gaseous state in the low-boiling-point decomposition product collecting step, and that has a standard boiling point equal to or higher than the standard boiling point of the compound (A). The high-boiling-point component often contains side reaction products caused by an isocyanate produced by thermal decomposition of a carbamate and the carbamate, side reaction products caused by the isocyanate, side reaction products caused by the carbamate, or compounds caused by an additional reaction of these side reaction products. There are many cases in which these compounds are not collected in a gaseous state in the low-boiling-point decomposition product collecting step. In contrast, there are many cases in which these compounds adhere to the reactor surface, which causes blockage. Thus, the continuous collection of the liquid phase component from the thermal decomposition reactor with the compound (A) supplied to the thermal decomposition reaction suppresses adhesion to the reactor surface.

The thermal decomposition step, the low-boiling-point decomposition product collecting step and the high-boiling-point component collecting step may be conducted separately using plural devices, or conducted simultaneously using one device.

### [Other steps]

The preparation method of an isocyanate according to the present embodiment may further include a separation step and a carbamate preparing step, for example, in addition to the above-mentioned thermal decomposition step, the low-boiling-point decomposition product collecting step and the high-boiling-point component collecting step.

### (Separation step)

In the separation step, the isocyanate which is contained in the low-boiling-point decomposition product collected in the low-boiling-point decomposition product collecting step is separated and purified. Specifically, the low-boiling-point decomposition product collected in the low-boiling-point decomposition product collecting step is supplied in a gaseous state to a distillation column to separate an isocyanate from a hydroxy compound, thereby obtaining a highly purified isocyanate. The distillation condition, the distillation device, or the like, may be appropriately selected from conventionally-known conditions or devices depending on the types of the isocyanate and the hydroxy compound or the like.

### (Carbamate preparing step)

It is preferable that a carbamate used in the thermal decomposition step be prepared using the method mentioned below. In addition, from the viewpoint of the quality and the yield of the obtained isocyanate, it is preferable that the carbamate be derived from an amino acid ester that produces a hydroxyl compound as the low-boiling-point decomposition product and an isocyanate as the high-boiling-point decomposition product.

In the present step, a carbonic acid ester and an amine compound are reacted to obtain a reaction mixture containing a carbamate which is a reaction product of the carbonic acid ester and the amine compound, a hydroxy compound which is a reaction by-product of the carbonic acid ester, and the carbonic acid ester.

The reaction of the carbonic acid ester and the amine compound may be conducted in a reaction solvent. The carbonic acid ester used in an excess amount relative to the molar amount of amino group of the amine compound is preferably used as a solvent in the reaction.

Although the reaction conditions of the carbonic acid ester and the amine compound depend on the compounds to be reacted, the stoichiometric ratio of the molar amount of the carbonic acid ester to the molar amount of an amino group of the amine compound may be 1 time or more. From the viewpoint of increase in the reaction rate and prompt termination of the reaction, the molar amount of the carbonic acid ester relative to the molar amount of an amino group of the amine compound is preferably in excess, and more preferably 1 time to 1000 times, and, in view of the size of the reactor, even more preferably 1.1 times to 50 times, and particularly preferably 1.5 times to 10 times.

The reaction temperature may be generally 0°C to 150°C. Although a high reaction temperature is preferable so as to increase the reaction rate, there is a case in which an unfavorable reaction is caused at a high temperature. Thus, the reaction temperature is preferably 10°C to 100°C. In order to keep the reaction temperature constant, a conventionally-known cooling device and heating device may be installed in the reactor.

Although the reaction pressure depends on the type of compounds to be used or the reaction temperature, the reaction pressure may be reduced pressure, ordinary pressure or pressurization, and is generally 20 Pa to 1×10⁶ Pa. The reaction time (residence time in a case of a continuous method) is not particularly limited, generally preferably 0.001 hours to 50 hours, more preferably 0.01 hours to 20 hours, and even more preferably 0.1 hours to 10 hours. The reaction may be terminated after confirming that a predetermined amount of a carbamate is produced by subjecting a collected reaction liquid to liquid chromatography, for example.

In the reaction of the carbonic acid ester and the amine compound, a catalyst may or may not be used. When no catalyst is used, the thermal denaturation of a carbamate due to a metal component derived from the catalyst can be inhibited.

When a catalyst is used, the reaction can be completed in a short time, and the reaction temperature can be lowered.

In particular, when a used compound forms a salt with an inorganic acid or an organic acid, a basic compound may be used.

The basic compound may be an inorganic base or an organic base. Examples of the inorganic base include alkali metal hydroxides, alkaline earth metal hydroxides, and ammonia. Examples of the organic base include amines and phosphazene. Among them, the basic compound is preferably an amine, more preferably an aliphatic amine, and even more preferably a secondary aliphatic amine or a tertiary aliphatic amine.

Although the amount of the basic compound to be used is appropriately determined depending on the compound to be used, the stoichiometric ratio of the molar amount of the basic compound to the molar amount of an amino group of the amine compound forming the salt is preferably 0.001 times or more, and more preferably 0.01 times to 100 times.

As the reactor available in the reaction of the carbonic acid ester and the amine compound, a conventionally-known tank reactor, column reactor, or distillation column may be used. Although materials of the reactor and the lines may be appropriately selected to be used from conventionally-known materials, unless starting materials or reaction materials are adversely affected, SUS 304, SUS 316, SUS 316L or the like is inexpensive and preferably used.

### <Each raw material and reaction product>

Each raw material used in the preparation method according to the present embodiment and the reaction product will be explained below.

### [Carbamate]

A carbamate used in the preparation method according to the present embodiment is preferably a carbamate of the following general formula (2) (hereinafter, may be referred to as "carbamate (2)"). The term "carbamate" used herein is not limited to the carbamate obtained in the above-mentioned "carbamate preparing step", and encompasses any carbamates available in the preparation method according to the present embodiment.

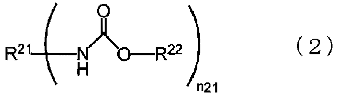

In the general formula (2), n21 is an integer of 1 or more. R²¹ is an n21-valent organic group. R²² is a remaining group formed by removing one hydroxy group from a hydroxy compound.

### (n21)

In the general formula (2), n21 is preferably an integer of 1 to 5, more preferably 2 or more, and even more preferably 3 or more, in view of ease of preparation or ease of operation.

### (R²¹)

In the general formula (2), R²¹ is preferably a C3-85 organic group, and more preferably a C3-30 organic group. The organic group as R²¹ is an aliphatic hydrocarbon group, an aromatic hydrocarbon group or a group formed by binding an aliphatic hydrocarbon group and an aromatic hydrocarbon group. Specific examples of R²¹ include cyclic hydrocarbon groups, noncyclic hydrocarbon groups, groups formed by binding a noncyclic hydrocarbon group with at least one cyclic group, and groups formed by binding these groups with specific nonmetallic atoms via covalent bonds. Examples of the cyclic group include cyclic hydrocarbon groups, hetero ring groups, hetero ring-type spiro groups, and hetero cross-linked cyclic groups. Examples of the cyclic hydrocarbon groups include monocyclic hydrocarbon groups, condensed polycyclic hydrocarbon groups, cross-linked cyclic hydrocarbon groups, spiro hydrocarbon groups, ring-assembly hydrocarbon groups, and side chain-containing cyclic hydrocarbon groups.

Examples of the nonmetallic atoms include carbon, oxygen, nitrogen, sulfur, and silicon.

Preferable examples of noncyclic aliphatic hydrocarbon groups as R²¹ include C3-15, and more preferably C5-10 alkylene groups and alkenylene groups.

Preferable examples of cyclic aliphatic hydrocarbon groups as R²¹ include C3-15 cycloalkylene groups, and more preferably include a cyclohexylene group.

Additional preferable examples of aliphatic hydrocarbon groups as R²¹ include groups formed by binding a noncyclic aliphatic hydrocarbon group (such as C1-10 alkylene group) with at least one (preferably one or two) C3-15 cyclic aliphatic hydrocarbon group (preferably cyclohexylene group).

The noncyclic aliphatic hydrocarbon group as R²¹ may have one to four (preferably one or two) ester groups.

A hydrogen atom constituting the noncyclic aliphatic hydrocarbon group as R²¹ may be substituted with a C1-3 alkylthio group, an imidazolyl group or an indolyl group.

As the aromatic hydrocarbon group as R²¹, a C6-20, more preferably C6-12 arylene group is preferable, and a phenylene group is more preferable. The phenylene group may be substituted with a C1-6 alkyl group.

As the group formed by bonding an aliphatic hydrocarbon group and an aromatic hydrocarbon group as R²¹, a group formed by bonding a C1-6 alkylene group with one or two phenylene groups is preferable.

### (R²²)

In the general formula (2), R²² is a remaining group formed by removing one hydroxy group from a hydroxy compound, preferably a C1-20 monovalent aliphatic hydrocarbon group or a C6-20 monovalent aromatic hydrocarbon group, and more preferably a C6-20 monovalent aromatic hydrocarbon group. The C1-20 monovalent aliphatic hydrocarbon group or the C6-20 monovalent aromatic hydrocarbon group may have a substituent.

The C1-20 monovalent aliphatic hydrocarbon group as R²² may be chained or cyclic.

Examples of the chained aliphatic hydrocarbon group include linear alkyl groups and branched alkyl groups. The carbon number of the linear alkyl group is preferably 1 to 5, more preferably 1 to 4, and even more preferably 1or 2. Specific examples of the linear alkyl groups include a methyl group, an ethyl group, a n-propyl group, a n-butyl group, and a n-pentyl group. The carbon number of the branched alkyl group is preferably 3 to 10, and more preferably 3 to 5. Specific examples of the branched alkyl groups include an isopropyl group, an isobutyl group, a tert-butyl group, an isopentyl group, a neopentyl group, a 1,1-diethylpropyl group, and a 2,2-dimethylbutyl group.

The cyclic aliphatic hydrocarbon group (that is, alicyclic hydrocarbon group) may be monocyclic or polycyclic. Specific examples of the monocyclic alicyclic hydrocarbon groups include cyclopentane and cyclohexane. Specific examples of the polycyclic alicyclic hydrocarbon groups include adamantane, norbornane, isobornane, tricyclodecane, and tetracyclododecane.

The carbon number of the aromatic hydrocarbon group as R²² is preferably 6 to 20, and more preferably 6 to 12. Although R²² may be an aromatic hydrocarbon group having a carbon number of 21 or more, the carbon number of R²² is preferably 20 or less from the viewpoint of facilitating separation from an isocyanate produced by the thermal decomposition reaction of a carbamate.

Examples of the aromatic hydrocarbon group as R²² include a phenyl group, a methylphenyl group (each isomer), an ethylphenyl group (each isomer), a propylphenyl group (each isomer), a butylphenyl group (each isomer), a pentylphenyl group (each isomer), a hexylphenyl group (each isomer), a dimethylphenyl group (each isomer), a methylethylphenyl group (each isomer), a methylpropylphenyl group (each isomer), a methylbutylphenyl group (each isomer), a methylpentylphenyl group (each isomer), a diethylphenyl group (each isomer), an ethylpropylphenyl group (each isomer), an ethylbutylphenyl group (each isomer), a dipropylphenyl group (each isomer), a trimethylphenyl group (each isomer), a triethylphenyl group (each isomer), and a naphthyl group (each isomer).

Among these, R²² is preferably a phenyl group or a C1-5 alkyl group.

### 1. Monofunctional carbamate

In the case of a monofunctional carbamate in which n21 in the carbamate (2) is 1 (that is, a compound having one carbamate group in one molecule), preferable examples of the carbamate (2) include carbamates of the following general formula (2-1a) (hereinafter, may be referred to as "carbamates (2-1a)") and carbamates of the following general formula (2-1b) (hereinafter, may be referred to as "carbamates (2-1b)").

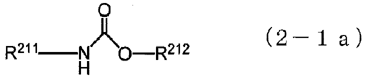

In the general formula (2-1a), R²¹¹ is a C3-85 hydrocarbon group, preferably a C5-10 alkylene group or alkenylene group which may have one ester bond, and more preferably a C5-10 alkenylene group which has one ester bond. Although R²¹² is the same group as R²², a phenyl group is preferable.

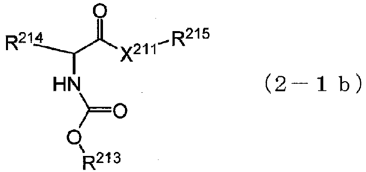

In the general formula (2-1b), X²¹¹ is an oxygen atom or a secondary amino group (-NH-). R²¹³ is the same group as R²². R²¹⁴ is a hydrogen atom, a C1-10 aliphatic hydrocarbon group or a C6-10 aromatic hydrocarbon group. The C1-10 aliphatic hydrocarbon group and the C6-10 aromatic hydrocarbon group may have at least one selected from the group consisting of a sulfur atom, an oxygen atom and halogen atoms. R²¹⁵ is a C1-10 monovalent aliphatic hydrocarbon group or a C6-10 monovalent aromatic hydrocarbon group.

The carbamate (2-1b) is a carbamate having an α-amino acid skeleton. α-Amino acids have two possible sterically bonding modes of an amino group or a carboxyl group to an α carbon, and are respectively distinguished as D-type or L-type photoisomer. An amino acid (and a compound having an amino acid skeleton) available to prepare the carbamate (3-1b) may be D-type, L-type, a mixture thereof, or a racemic body. Many industrially inexpensively available amino acids are amino acids produced by fermentation, and are almost all L-type, which are preferably used. Although the steric configuration is not shown in the present specification, the steric configuration is either D-type or L-type.

(R²¹¹) R²¹¹ is a C3-85 hydrocarbon group. The hydrocarbon group as R²¹¹ may be an aliphatic hydrocarbon group or a C6-60 aromatic hydrocarbon group. Examples of the hydrocarbon group as R²¹¹ include the same hydrocarbon groups as R²¹. Among these, R²¹¹ is preferably a C5-10 alkylene group or alkenylene group which may have one ester bond, and more preferably a C5-10 alkenylene group which has one ester bond.

### (R²¹⁴ and R²¹⁵)

Specific examples of the C1-10 monovalent aliphatic hydrocarbon group as R²¹⁴ and R²¹⁵ include a methyl group, an ethyl group, a propyl group, a butyl group, a pentyl group, a hexyl group, and a decyl group. Specific examples of the C6-10 monovalent aromatic hydrocarbon group as R²¹⁴ and R²¹⁵ include a phenyl group, a methylphenyl group, an ethylphenyl group, a butylphenyl group, a dimethylphenyl group, and a diethylphenyl group. The C1-10 aliphatic hydrocarbon group and C6-10 aromatic hydrocarbon group as R²¹⁴ may have at least one selected from the group consisting of a sulfur atom, an oxygen atom and halogen atoms. When a sulfur atom or an oxygen atom is contained, a carbon atom constituting the C1-10 aliphatic hydrocarbon group or the C6-10 aromatic hydrocarbon group is substituted with a sulfur atom or an oxygen atom.

### (X²¹¹)

X²¹¹ is an oxygen atom or a secondary amino group (-NH-). When X²¹¹ is an oxygen atom, X²¹¹ forms an ester bond with an adjacent carbonyl group. When X²¹¹ is a secondary amino group (-NH-), X²¹¹ forms an amido bond with an adjacent carbonyl group.

Among these, the monofunctional carbamate is preferably the carbamate (2-1b).

Preferable examples of the carbamate (2-1b) include a compound of the following formula (2-1b-1), a compound of the following formula (2-1b-2), a compound of the following formula (2-1b-3), and a compound of the following formula (2-lb-4).

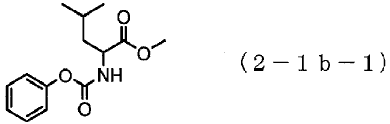

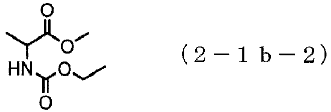

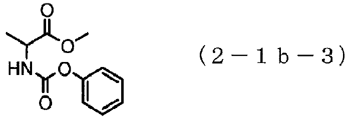

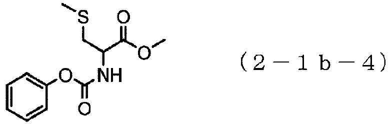

### 2. Difunctional carbamate

In the case of a difunctional carbamate in which n21 in the carbamate (2) is 2 (that is, a compound having two carbamate groups in one molecule), preferable examples of the carbamate (2) include carbamates of the following general formula (2-2a) (hereinafter, may be referred to as "carbamates (2-2a)"), carbamates of the following general formula (2-2b) (hereinafter, may be referred to as "carbamates (2-2b)"), carbamates of the following general formula (2-2c) (hereinafter, may be referred to as "carbamates (2-2c)"), carbamates of the following general formula (2-2d) (hereinafter, may be referred to as "carbamates (2-2d)"), and carbamates of the following general formula (2-2e) (hereinafter, may be referred to as "carbamates (2-2e)").

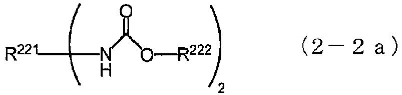

In the general formula (2-2a), although R²²¹ is the same as R²¹ mentioned above, R²²¹ is preferably a C3-10 alkylene group, a C3-10 cycloalkylene group (more preferably a cyclohexylene group), a group formed by bonding a C3-10 cycloalkylene group (preferably a cyclohexylene group) which may be substituted with a C1-3 alkyl group with a C1-6 alkylene group, a phenylene group which may be substituted with a C1-6 alkyl group, or a group formed by connecting a C1-6 alkylene group with one or two phenylene group.

Although R²²² is the same as R²², R²²² is preferably a phenyl group or a C1-5 alkyl group.

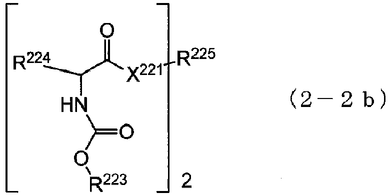

In the general formula (2-2b), X²²¹ is the same as X²¹¹ mentioned above. R²²³ is the same as R²². R²²⁴ is the same as R²¹⁴. R²²⁵ is a C1-10 divalent aliphatic hydrocarbon group or a C6-10 divalent aromatic hydrocarbon group.

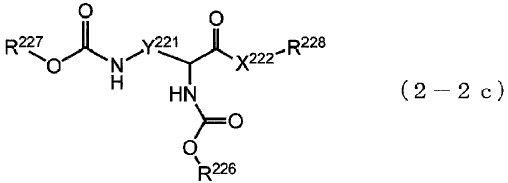

In the general formula (2-2c), although X²²² is the same as X²¹¹, X²²² is preferably an oxygen atom. Although R²²⁶ and R²²⁷ are each independently the same as R²², R²²⁶ and R²²⁷ are preferably phenyl groups. Y²²¹ is a C1-5 alkylene chain. Although R²²⁸ is the same as R²¹⁵ mentioned above, R²²⁸ is preferably a C1-6 alkyl group.

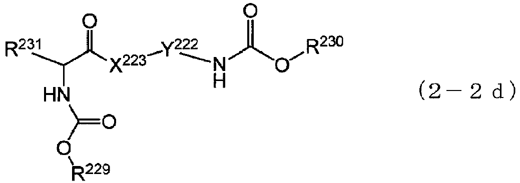

In the general formula (2-2d), although X²²³ is the same as X²¹¹, X²²³ is preferably an oxygen atom. Although R²²⁹ and R²³⁰ are each independently the same as R²², R²²⁹ and R²³⁰ are preferably phenyl groups. Y²²² is the same as Y²²¹. Although R²³¹ is the same as R²¹⁴, R²³¹ is preferably a C1-6 alkyl group or alkylthio group, and more preferably a C1-6 alkylthio group.

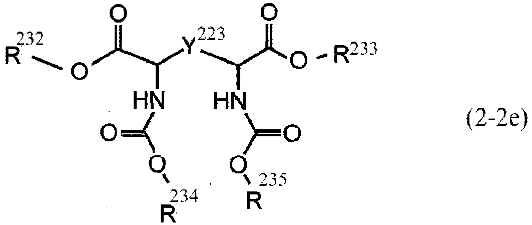

In the general formula (2-e), although R²³⁴ and R²³⁵ are each independently the same as R²², R²³⁴ and R²³⁵ are preferably phenyl groups. Y²²² is the same as Y²²¹. Although R²³² and R²³³ are the same as R²¹⁴, R²³² and R²³³ are preferably C1-6 alkyl groups.

### (R²²⁵)

Examples of the C1-10 divalent aliphatic hydrocarbon group as R²²⁵ include a methylene group, an ethylene group, a trimethylene group, a tetramethylene group, a pentamethylene group, and a hexamethylene group. Examples of the C6-10 divalent aromatic hydrocarbon group as R²²⁵ include a phenylene group and a naphthalene-diyl group.

### (Y²²¹)

Y²²¹ and Y²²² are each independently a C1-5 polyalkylene chain. Namely, Y²²¹ and Y²²² are divalent groups of the following general formula (II).

-(CH₂)ₙ₂₂₁- (II)

In the general formula (II), n221 is an integer of 1 to 5.

Examples of the C1-5 polyalkylene chain include a methylene group, an ethylene group, a trimethylene group, a tetramethylene group, and a pentamethylene group.

Specific preferable examples of the carbamate (2-2a), the carbamate (2-2b), the carbamate (2-2c) and the carbamate (2-2d) include C4-30 aliphatic dicarbamates, C8-30 alicyclic dicarbamates, and C8-30 dicarbamates having an aromatic group.

Specific examples of the C4-30 aliphatic dicarbamates include 1,5-pentamethylene di(carbamic acid methyl ester), 1,6-hexamethylene di(carbamic acid methyl ester), lysine ethyl ester di(carbamic acid methyl ester), 1,5-pentamethylene di(carbamic acid ethyl ester), 1,6-hexamethylene di(carbamic acid ethyl ester), lysine ethyl ester di(carbamic acid ethyl ester), 1,5-pentamethylene di(carbamic acid phenyl ester), 1,6-hexamethylene di(carbamic acid phenyl ester), lysine ethyl ester di(carbamic acid phenyl ester), and ethyl-2,6-bis((phenoxycarbonyl)amino)hexonate. Among these, 1,6-hexamethylene di(carbamic acid phenyl ester) is preferable.

Specific examples of the C8-30 alicyclic dicarbamates include isophorone di(carbamic acid methyl ester), 1,3-bis((carbamic acid methyl ester)methyl)-cyclohexane, 4,4'-dicyclohexylmethane di(carbamic acid methyl ester), hydrogenated tetramethylxylylene di(carbamic acid methyl ester), norbornene di(carbamic acid methyl ester), isophorone di(carbamic acid ethyl ester), 1,3-bis((carbamic acid ethyl ester)ethyl)-cyclohexane, 4,4'-dicyclohexylmethane di(carbamic acid ethyl ester), hydrogenated tetraethylxylylene di(carbamic acid ethyl ester), norbornene di(carbamic acid ethyl ester), isophorone di(carbamic acid phenyl ester), 1,3-bis((carbamic acid phenyl ester)phenyl)-cyclohexane, 4,4'-dicyclohexylmethane di(carbamic acid phenyl ester), hydrogenated tetraphenylxylylene di(carbamic acid phenyl ester), norbornene di(carbamic acid phenyl ester), and 3-(phenoxycarbonylamino-methyl)-3,5,5-trimethylcyclohexylcarbamic acid phenyl ester. Among these, 3-(phenoxycarbonylamino-methyl)-3,5,5-trimethylcyclohexylcarbamic acid phenyl ester is preferable.

Specific examples of C8-30 dicarbamates having an aromatic group include 4,4'-diphenylmethane di(carbamic acid methyl ester), 2,6-tolylene di(carbamic acid methyl ester), xylylene di(carbamic acid methyl ester), tetramethylxylylene di(carbamic acid methyl ester), naphthalene di(carbamic acid methyl ester), 4,4'-diphenylmethane di(carbamic acid ethyl ester), 2,6-tolylene di(carbamic acid ethyl ester), xylylene di(carbamic acid ethyl ester), tetraethylxylylene di(carbamic acid ethyl ester), naphthalene di(carbamic acid ethyl ester), 4,4'-diphenylmethane di(carbamic acid phenyl ester), 2,6-tolylene di(carbamic acid phenyl ester), xylylene di(carbamic acid phenyl ester), tetraphenylxylylene di(carbamic acid phenyl ester), and naphthalene di(carbamic acid dimethylphenyl ester).

In the case where the above-mentioned compound has structural isomers, the structural isomers are encompassed in the above-mentioned preferable examples of the carbamate (2).

### 3. Trifunctional or more-functional carbamate

When the carbamate (2) is a trifunctional carbamate in which n31 is 3 (that is, a compound having three carbamate groups in one molecule), preferable examples of the carbamate (2) include carbamates of the following general formula (2-3a) (hereinafter, may be abbreviated as "carbamates (2-3a)"), carbamates of the following general formula (2-3b) (hereinafter, may be abbreviated as "carbamates (2-3b)"), and carbamates of the following general formula (2-3c) (hereinafter, may be abbreviated as "carbamates (2-3c)").

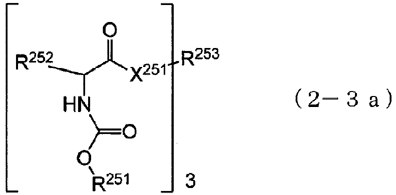

In the general formula (2-3a), X²⁵¹ is the same as X²¹¹ mentioned above. R²⁵¹ is the same as R²² mentioned above. R²⁵² is the same as R²¹⁴ mentioned above. R²⁵³ is a CI-10 trivalent aliphatic hydrocarbon group or a C6-10 trivalent aromatic hydrocarbon group.

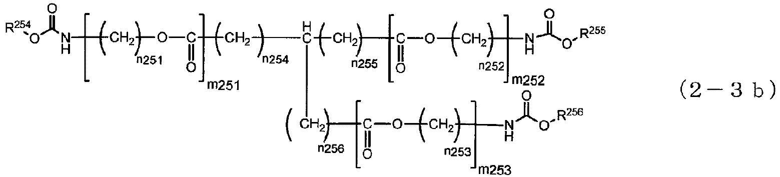

In the general formula (2-3b), n251, n252 and n253 are each independently an integer of 1 to 4. n254, n255 and n256 are each independently an integer of 0 to 5. It is preferable that at least one selected from the group consisting of n254, n255 and n256 be 0. Although m251, m252 and m253 are each independently 0 or 1, at least one selected from the group consisting of m251, m252 and m253 is 1. It is preferable that at least one selected from the group consisting of m251, m225 and m253 be 0. R²⁵⁴, R²⁵⁵ and R²⁵⁶ are each independently the same as R²², and preferably a phenyl group.

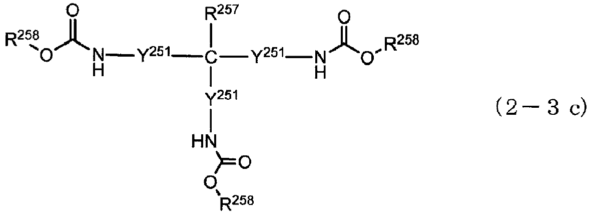

In the general formula (2-3c), plural Y²⁵¹ are each independently a single bond or a C1-20 divalent hydrocarbon group which may have at least one selected from the group consisting of an ester group and an ether group, and preferably a C1-6 alkylene group. Plural R²⁵⁸ are the same as R²², and preferably phenyl groups. Plural Y²⁵¹ and R²⁵⁸ may be identical to or different from each other. R²⁵⁷ is a hydrogen atom or a C1-12 monovalent hydrocarbon group, and preferably a hydrogen atom. The C1-20 divalent hydrocarbon group and the C1-20 hydrocarbon group may have a substituent.

### (R²⁵³)

R²⁵³ is a C1-10 trivalent aliphatic hydrocarbon group or a C6-10 trivalent aromatic hydrocarbon group.

Examples of the C1-10 trivalent aliphatic hydrocarbon group as R²⁵³ include a methanetriyl group, an ethanetriyl group, and a propanetriyl group. Examples of the C6-10 trivalent aromatic hydrocarbon group as R²⁵³ include a benzenetriyl group and a naphthalenetriyl group.

### (Y²⁵¹)

Preferable examples of Y²⁵¹ include C1-20 divalent aliphatic hydrocarbon groups, C6-20 divalent aromatic hydrocarbon groups, C2-20 divalent groups formed by bonding an aliphatic hydrocarbon group and an aliphatic hydrocarbon group via an ester group, C2-20 divalent groups formed by bonding an aliphatic hydrocarbon group and an aliphatic hydrocarbon group via an ether group, C7-20 divalent groups formed by bonding an aliphatic hydrocarbon group and an aromatic hydrocarbon group via an ester group, C7-20 divalent groups formed by bonding an aliphatic hydrocarbon group and an aromatic hydrocarbon group via an ether group, C14-20 divalent groups formed by bonding an aromatic hydrocarbon group and an aromatic hydrocarbon group via an ester group, and C14-20 divalent groups formed by bonding an aromatic hydrocarbon group and an aromatic hydrocarbon group via an ether group.

### (R²⁵⁷)

R²⁵⁷ is preferably a C1-10 aliphatic hydrocarbon group or a C6-10 aromatic hydrocarbon group. Examples of the C1-10 aliphatic hydrocarbon group and C6-10 aromatic hydrocarbon group as R²⁵⁷ include the same groups as those mentioned as R²¹⁴ and R²¹⁵.

Preferable examples of the carbamate (2-3b) include compounds of the following general formula (2-3b-1) (hereinafter, may be abbreviated as "compounds (2-3b-1)").

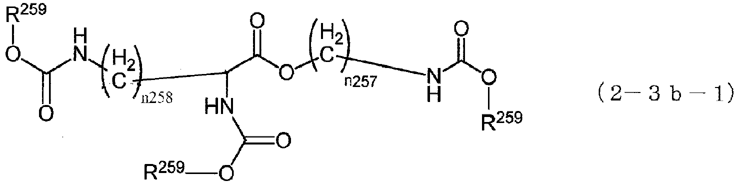

In the general formula (2-3b-1), plural R²⁵⁹ are the same as R²², and preferably phenyl groups. n257 and n258 are each independently an integer of 2 to 4.

Preferable examples of the compound (2-3b-1) include the following compounds.

In the general formula (2-3b-1), n257 is 2 and n258 is 4.

2,2-(Carbamic acid methyl ester)ethyl-2,6-di(carbamic acid methyl ester)hexanoate (R²⁵⁹ in the general formula (2-3b-1) is a methyl group).

2-(Carbamic acid ethyl ester)ethyl-2,6-di(carbamic acid ethyl ester)hexanoate (R²⁵⁹ in the general formula (2-3b-1) is an ethyl group).

2-(Carbamic acid butyl ester)ethyl-2,6-di(carbamic acid butyl ester)hexanoate (R²³⁹ in the general formula (2-3b-1) is a butyl group).

2-(Carbamic acid phenyl ester)ethyl-2,6-di(carbamic acid phenyl ester)hexanoate (R²⁵⁹ in the general formula (2-3b-1) is a phenyl group).

2-(Carbamic acid dimethylphenyl ester)ethyl-2,6-di(carbamic acid dimethylphenyl ester)hexanoate (R²⁵⁹ in the general formula (2-3b-1) is a dimethylphenyl group).

Preferable examples of the carbamate (2-3c) include compounds in which Y²⁵¹ is a C1-20 divalent aliphatic hydrocarbon group, and compounds in which Y²⁵¹ is a C6-20 divalent aromatic hydrocarbon group.

Specific examples of the compounds in which Y²⁵¹ is a C1-20 divalent aliphatic hydrocarbon group include 1,8-di(carbamic acid methyl ester)-4-(carbamic acid methyl ester)methyloctane, 1,8-di(carbamic acid ethyl ester) 4-(carbamic acid ethyl ester)methyloctane, 2-(carbamic acid ethyl ester)ethyl-2,5-di(carbamic acid ethyl ester)pentanoate, 2-(carbamic acid methyl ester)ethyl-2,5-di(carbamic acid methyl ester)pentanoate, 2-(carbamic acid methyl ester)ethyl-2,6-di(carbamic acid methyl ester)hexanoate, 2-(carbamic acid ethyl ester)ethyl-2,6-di(carbamic acid ethyl ester)hexanoate, bis(2-(carbamic acid ethyl ester)ethyl)-2-(carbamic acid ethyl ester)pentanedioate, bis(2-(carbamic acid methyl ester)ethyl)-2-(carbamic acid methyl ester)pentanedioate, bis(2-(carbamic acid butyl ester)ethyl)-2-(carbamic acid butyl ester)pentanedioate, 1,3,5-tri(carbamic acid methyl ester)benzene, and 1,3,5-tri(carbamic acid ethyl ester)benzene.

Specific examples of the compounds in which Y²⁵¹ is a C6-20 divalent aromatic hydrocarbon group include 1,8-di(carbamic acid phenyl ester) 4-(carbamic acid phenyl ester)methyloctane, 2-(carbamic acid phenyl ester)ethyl-2,5-di(carbamic acid phenyl ester)pentanoate, 2-(carbamic acid phenyl ester)ethyl-2,6-di(carbamic acid phenyl ester)hexanoate, bis(2-(carbamic acid phenyl)ethyl)-2-(carbamic acid phenyl)pentanedioate, and 1,3,5-tri(carbamic acid phenyl ester )benzene.

When the carbamate (2) is a polyfunctional carbamate in which n31 is 4 (that is, a compound having four carbamate groups in one molecule), preferable examples of the carbamate (2) include carbamates of the following general formula (2-4a) (hereinafter, may be abbreviated as "carbamate (2-4a)"), and carbamates of the following general formula (2-4b) (hereinafter, may be abbreviated as "carbamates (2-4b)").

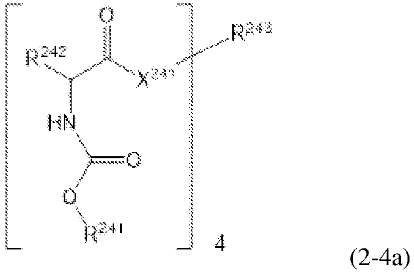

In the general formula (2-4a), X²⁴¹ is the same as X²¹¹ mentioned above. R²⁴¹ is the same as R²² mentioned above. R²⁴² is the same as R²¹⁴ mentioned above. R²⁴³ is a C1-10 tetravalent aliphatic hydrocarbon group or a C6-10 tetravalent aromatic hydrocarbon group.

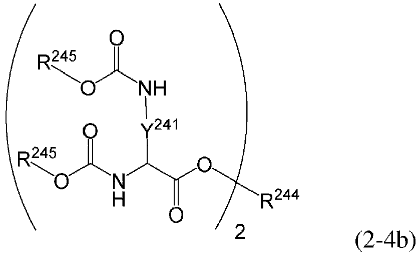

In the general formula (2-4b), Y²⁴¹ is a single bond or a C1-20 divalent hydrocarbon group which may have at least one selected from the group consisting of an ester group and an ether group. R²⁴⁴ is a single bond or a C1-20 divalent hydrocarbon group which may have at least one selected from the group consisting of an ester group and an ether group, and preferably a C2-6 alkylene group. Plural R²⁴⁵ are the same as R²² mentioned above, and preferably phenyl groups.

### (Y²⁴¹)

Preferable examples of Y²⁴¹ include C1-20 divalent aliphatic hydrocarbon groups, C6-20 divalent aromatic hydrocarbon groups, C2-20 divalent groups formed by boding an aliphatic hydrocarbon group and an aliphatic hydrocarbon group via an ester group, C2-20 divalent groups formed by boding an aliphatic hydrocarbon group and an aliphatic hydrocarbon group via an ether group, C7-20 divalent groups formed by boding an aliphatic hydrocarbon group and an aromatic hydrocarbon group via an ester group, C7-20 divalent groups formed by boding an aliphatic hydrocarbon group and an aromatic hydrocarbon group via an ether group, C14-20 divalent groups formed by boding an aromatic hydrocarbon group and an aromatic hydrocarbon group via an ester group, and C14-20 divalent groups formed by boding an aromatic hydrocarbon group and an aromatic hydrocarbon group via an ether group. Among these, Y²⁴¹ is preferably a C2-6 alkylene group.

Preferable examples of the carbamate (2-4b) include compounds of the following general formula (2-4b-1) (hereinafter, may be abbreviated as "compounds (2-4b-1)").

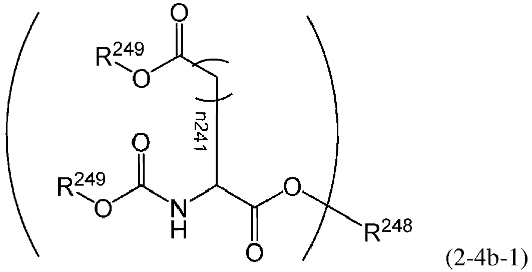

In the general formula (2-4b-1), R²⁴⁸ is the same as R²⁴⁴. Plural R²⁴⁹ are the same as R²². n241 is 3or 4.

Preferable examples of the compound (2-4b-1) include the following compounds.

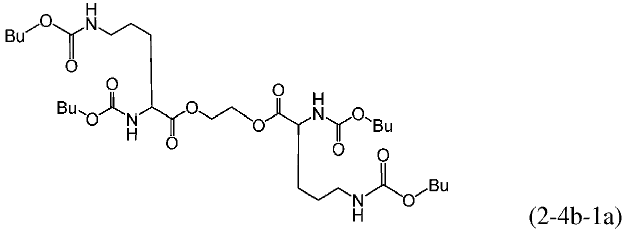

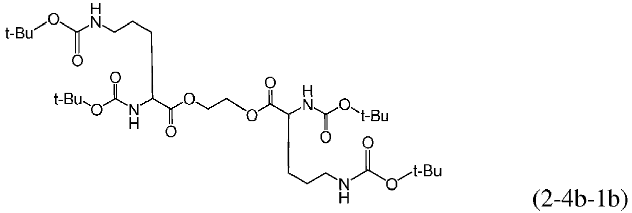

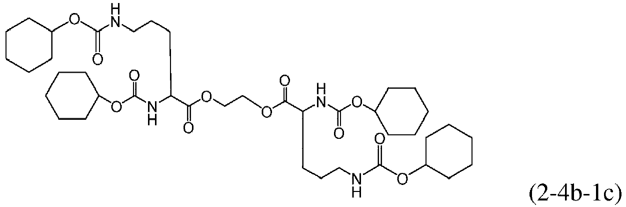

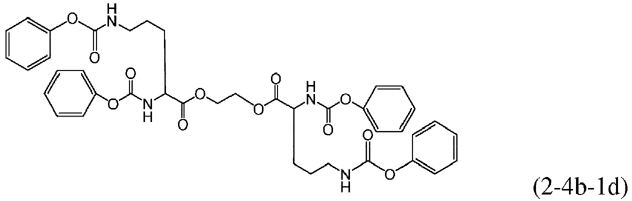

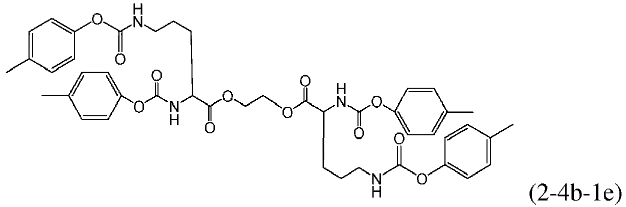

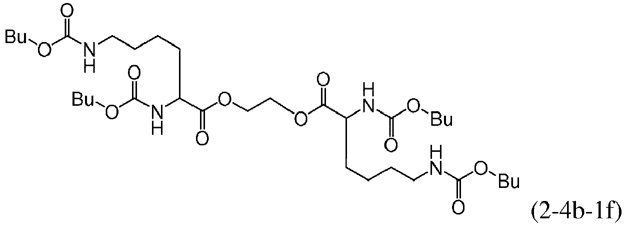

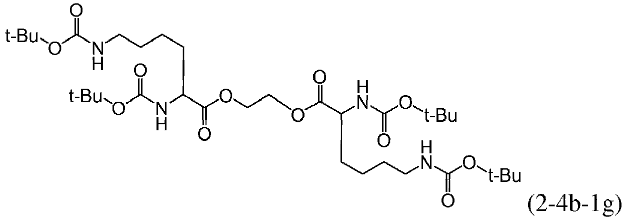

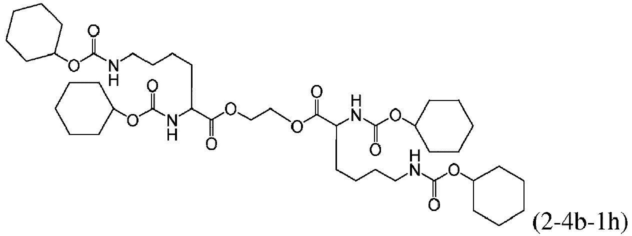

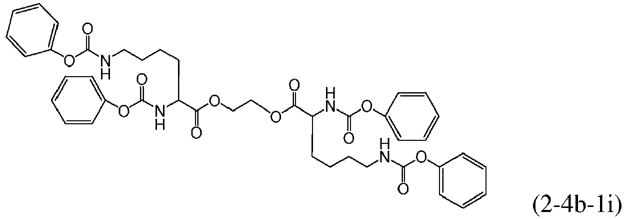

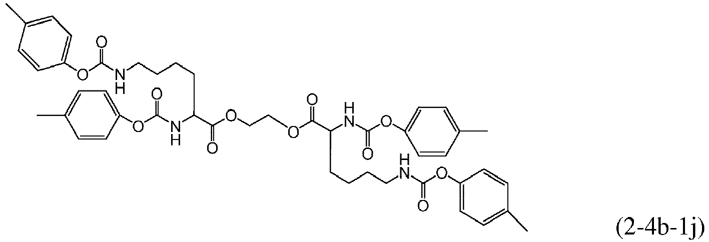

Among these, the carbamate (2) is preferably the carbamate (2-1a), the carbamate (2-1b), the carbamate (2-2a), the carbamate (2-2c), the carbamate (2-2d), the carbamate (2-2e), the carbamate (2-3b), the carbamate (2-3c), or the carbamate (2-4b).

### [Inert solvent]

An inert solvent available in the preparation method according to the present embodiment is not particularly limited, provided that the inert solvent is substantially inert under a reaction condition and has a standard boiling point lower than that of the compound (A), the standard boiling point being between standard boiling points of the resultant isocyanate and hydroxyl compound.

Examples of such an inert solvent include aliphatic compounds, alicyclic compounds, aromatic compounds which may have a substituent, unsubstituted hydrocarbons and mixtures thereof.

Additional examples thereof include compounds which may have an oxygen atom such as ethers, ketones, and esters, and compounds which may have a sulfur atom such as thioethers, sulfoxides, and sulfones.

Specific examples of the inert solvent include alkanes, aromatic hydrocarbons, alkyl-substituted aromatic hydrocarbons, aromatic compounds substituted with a nitro group or a halogen, polycyclic hydrocarbon compounds, alicyclic hydrocarbons, ketones, esters, ethers, thioethers, sulfoxides, sulfones, and silicon oils.

Examples of the alkanes include hexane, heptane, octane, nonane, decane, n-dodecane, n-hexadecane, n-octadecane, eicosane, and squalane.

Examples of the aromatic hydrocarbons and alkyl-substituted aromatic hydrocarbons include benzene, toluene, xylene, ethylbenzene, trimethylbenzene, triethylbenzene, cumene, diisopropylbenzene, dibutylbenzene, naphthalene, lower alkyl-substituted naphthalene, and dodecylbenzene. Xylene, trimethylbenzene and triethylbenzene are preferable.

Examples of the aromatic compounds substituted with a nitro group or a halogen include chlorbenzene, 4-methylbenzyl chloride, dichlorbenzene, bromobenzene, dibromobenzene, chlornaphthalene, bromonaphthalene, nitrobenzene, and nitronaphthalene. 4-Methylbenzyl chloride is preferable.

Examples of the polycyclic hydrocarbon compounds include diphenyl, substituted diphenyls, diphenylmethane, terphenyl, anthracene, phenanthrene, benzyltoluene, isomers of benzyltoluene, and triphenylmethane. Benzyltoluene is preferable.

Examples of the alicyclic hydrocarbons include cyclohexane and ethylcyclohexane.

Examples of the ketones include methylethylketone, acetophenone, and benzophenone. Benzophenone is preferable.

Examples of the esters include dibutylphthalate, dihexylphthalate, and dioctylphthalate.

Examples of the ethers and the thioethers include diphenyl ether, ethylene glycol monobutyl ether (may also be referred to as butyl cellosolve), and diphenyl sulfide. Ethylene glycol monobutyl ether is preferable.

Examples of the sulfoxides include dimethyl sulfoxide, and diphenyl sulfoxide. Examples of the sulfones include dimethyl sulfone, diethyl sulfone, diphenyl sulfone, and sulfolane.

Among these, the inert solvent is preferably an aromatic hydrocarbon, an alkyl-substituted aromatic hydrocarbon, or an aromatic compound substituted with a nitro group or a halogen such as chlorbenzene, dichlorbenzene, bromobenzene, dibromobenzene, chlornaphthalene, bromonaphthalene, nitrobenzene, or nitronaphthalene, and more preferably an alkyl-substituted aromatic hydrocarbon or an aromatic compound substituted with a halogen such as chlorobenzene or dichlorbenzene (preferably benzene), and even more preferably triethylbenzene or 4-methylbenzyl chloride.

### [Compound (A)]

The compound (A) available in the preparation method according to the present embodiment is at least one selected from the group consisting of compounds having a repeating unit of the following general formula (4) (repeating unit (4)) (polymers (4)), compounds of the following general formula (5) (compounds (5)), compounds of the following general formula (6) (compounds (6)), compounds of the following general formula (7) (compounds (7)), compounds of the following general formula (S1) (compounds (S1)), compounds of the following general formula (S2) (compounds (S2)), and compounds of the following general formula (S3) (compounds (S3)), compounds of the following general formula (9) (compounds (9)), compounds of the following general formula (10) (compounds (10)), and C9-35 chained or cyclic aliphatic hydrocarbons.

Compound (A) preferably has a polar group from the viewpoint of imparting appropriate polarity to ensure solubility. The polarity is an electrical bias that exists in the molecule and is created by an electric dipole moment. The polarity is generally imparted to an organic molecule by the presence of a heteroatom other than a carbon atom in the molecular structure. The number and the type of polar groups are not particularly limited provided that the above-mentioned viewpoint is satisfied.

It is preferable that the compound (A) have a molecular structure in which an intermolecular interaction with a substrate or a component produced in the preparing step is appropriate and be a component in which the molecule is stable and compatible. For example, a molecular structure having an aromatic ring is preferable from the viewpoint of the polarity and structure of the molecules sufficient for compatibility. Among them, it is also preferable to have a plurality of aromatic rings in the same molecular structure. The aromatic ring may be unsubstituted or may have a substituent. Although the number of aromatic rings in the same molecular structure is not particularly limited, the number is preferably 3 or more, and more preferably 4 or more. The use of the compound (A) having a preferable molecular structure makes it possible to obtain the target product in thermal decomposition in a high yield and to operate stably for a long period of time.

The compound (A) is preferably at least one selected from the group consisting of the polymer (4) and the compound (5).

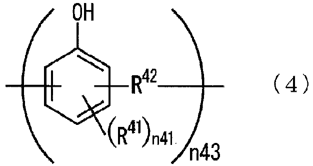

In the general formula (4), R⁴¹ is a monovalent hydrocarbon group. The hydrocarbon group may have either an ether bond or an ester bond, and may be substituted with a hydroxy group. n41 is 0 or an integer of 1 to 3. R⁴² is a divalent organic group. n43 is a repeating number, and is preferably 2 to 50.

The hydrocarbon group as R⁴¹ may be a C1-20 aliphatic hydrocarbon group or a C6-20 aromatic hydrocarbon group.

The aliphatic hydrocarbon group may be chained or cyclic. Examples of the chained aliphatic hydrocarbon group include linear alkyl groups and branched alkyl groups. The carbon number of the linear alkyl group is preferably 1 to 5. The carbon number of the branched alkyl group is preferably 3 to 10. The cyclic aliphatic hydrocarbon group (that is, alicyclic hydrocarbon group) may be monocyclic or polycyclic.

The organic group as R⁴² may be a C1-60 aliphatic hydrocarbon group, a C6-50 aromatic hydrocarbon group or a C7-60 group formed by bonding an aliphatic hydrocarbon group and an aromatic hydrocarbon group. The aliphatic hydrocarbon group and the aromatic hydrocarbon group may have a substituent. The aliphatic hydrocarbon group may have either an ether bond or an ester bond.

The repeating unit (4) is preferably a repeating unit of the following general formula (4-1) (repeating unit (4-1)) or a repeating unit of the following general formula (4-2) (repeating unit (4-2)), and more preferably a repeating unit of the following general formula (4-1-1) (repeating unit (4-1-1)) or a repeating unit of the following general formula (4-2-1) (repeating unit (4-1-2)).

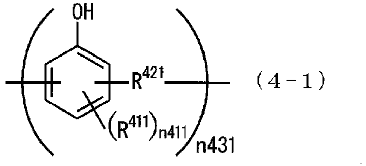

In the general formula (4-1), R⁴¹¹ is a monovalent hydrocarbon group. The monovalent hydrocarbon group may have either an ether bond or an ester bond, and may be substituted with a hydroxy group. n411 is 0 or an integer of 1 to 3. When n411 is 2 or 3, R⁴¹¹ may be identical to or different from each other. R⁴²¹ is a divalent aliphatic hydrocarbon group. The divalent aliphatic hydrocarbon group may have either an ether bond or an ester bond.

n431 is a repeating number, and is the same as n43.

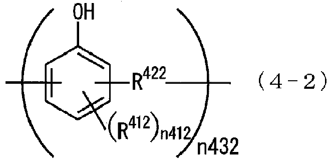

In the general formula (4-2), R⁴¹² is a monovalent hydrocarbon group. The monovalent hydrocarbon group may have either an ether bond or an ester bond. n412 is 0 or an integer of 1 to 3. R⁴²² is a divalent aromatic hydrocarbon group or a divalent group formed by bonding an aliphatic hydrocarbon group and an aromatic hydrocarbon group. The aliphatic hydrocarbon group may have either an ether bond or an ester bond.

n432 is a repeating number, and is the same as n43.

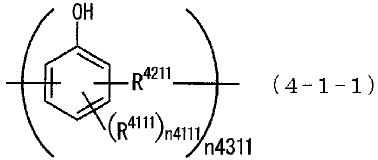

In the general formula (4-1-1), R⁴¹¹¹ is a C1-20 alkyl group which may be substituted with a hydroxy group or a C6-20 aryl group. n⁴¹¹¹ is 0 or an integer of 1 to 3. When n4111 is 2 or 3, R⁴¹¹¹ may be identical to or different from each other. R⁴²¹¹ is a divalent C1-20 alkylene group. The C1-20 alkylene group may have either an ether bond or an ester bond.

n4311 is a repeating number and is the same as n43.

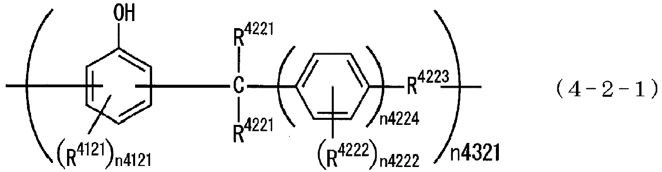

In the general formula (4-2-1), R⁴¹²¹ is a C1-20 alkyl group or a C6-20 aryl group. The C1-20 alkyl group may have either an ether bond or an ester bond. n4121 is 0 or an integer of 1 to 3.

Plural R⁴²²¹ are each independently a hydrogen atom or a C1-20 alkyl group.

R⁴²²² is a C1-20 alkyl group or a C6-20 aryl group. The C1-20 alkyl group may have either an ether bond or an ester bond. n4222 is 0 or an integer of 1 to 4.

n4224 is an integer of 1 or 2.

R⁴²²³ is a single bond or a divalent C1-20 aliphatic hydrocarbon group. The C1-20 aliphatic hydrocarbon group may have either an ether bond or an ester bond.

n4321 is a repeating number, and is the same as n43.

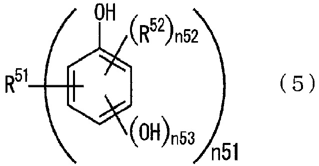

In the general formula (5), n51 is an integer of 1 to 4. R⁵¹ is a hydrogen atom or an n51-valent organic group. R⁵² is a monovalent hydrocarbon group. The hydrocarbon group may have either an ether bond or an ester bond. n52 is 0 or an integer of 1 to 4. n53 is 0 or 1.

The organic group as R⁵¹ may be a C1-60 hydrocarbon group. Examples of the hydrocarbon group include aliphatic hydrocarbon groups, aromatic hydrocarbon groups and groups formed by bonding an aliphatic hydrocarbon group and an aromatic hydrocarbon group.

The hydrocarbon group as R⁵¹ may have an ether bond, an ester bond, a carbonyl group, or a hetero ring. The hydrocarbon group may have a substituent.

The compound (5) is preferably a compound of the following general formula (5-1) (compound (5-1)) or a compound of the following general formula (5-2) (compound (5-2)), more preferably a compound of the following general formula (5-1-1) (compound (5-1-1)), a compound of the following general formula (5-1-2) (compound (5-1-2)), a compound of the following general formula (5-1-3) (compound(5-1-3)), a compound of the following general formula (5-2-1) (compound (5-2-1)), a compound of the following general formula (5-2-2) (compound (5-2-2)), or a compound of the following general formula (5-2-3) (compound (5-2-3)), and even more preferably a compound (5-1-3).

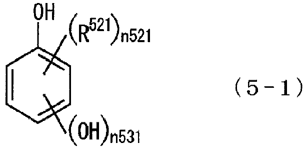

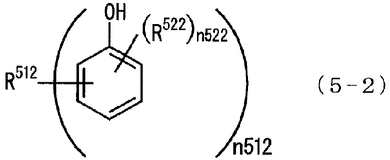

In the general formula (5-1), R⁵²¹ is a C1-20 (preferably C1-10) alkyl group which may be substituted with a C6-12 aryl group (preferably a phenyl group) or a C1-20 (preferably C1-6) alkoxycarbonyl group which may be substituted with a C6-12 aryl group (preferably a phenyl group). n521 is 0 or an integer of 1 to 4. n531 is 0 or 1, and more preferably 0.

In the general formula (5-2), n512 is an integer of 2 to 4. R⁵¹² is an n51-valent hydrocarbon group. Although the n51-divalent hydrocarbon group may have an ether bond, an ester bond, a carbonyl group, or a hetero ring, the n51-divalent hydrocarbon group is preferably a C1-6 alkylene group. R⁵²² is a monovalent hydrocarbon group. The monovalent hydrocarbon group may have either an ether bond or an ester bond. n522 is 0 or an integer of 1 to 4, and preferably 0.

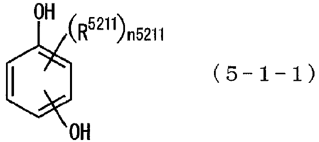

In the general formula (5-1-1), R⁵²¹¹ is a C1-20 alkyl group which may be substituted with a C6-12 aryl group. n5211 is 0 or an integer of 1 to 4.

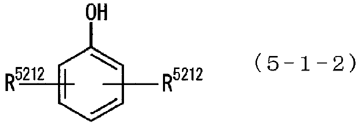

In the general formula (5-1-2), plural R⁵²¹² are each independently a C1-20 alkyl group which may be substituted with a C6-12 aryl group (preferably a phenyl group).

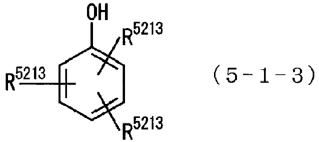

In the general formula (5-1-3), plural R⁵²¹³ are each independently a C1-20 alkyl group which may be substituted with a C6-12 aryl group (preferably a phenyl group), and preferably a benzyl group or an α-methylbenzyl group.

As the compound of the general formula (5-1-3), a compound of the following formula (5-1-3a) or (5-1-3b) is preferable.

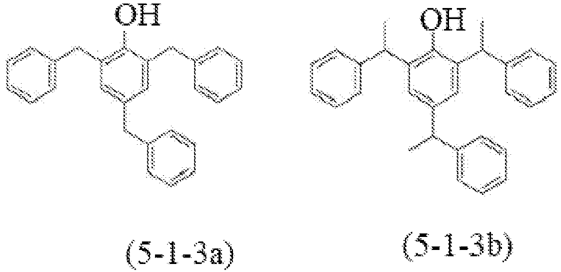

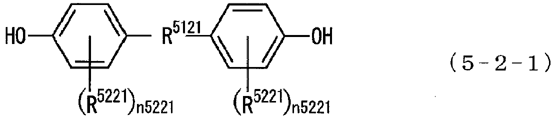

In the general formula (5-2-1), R⁵¹²¹ is a divalent hydrocarbon group (preferably a C1-20 alkylidene group). Plural R⁵²²¹ are each independently a C1-20 alkyl group. Plural n5221 are each independently 0 or an integer of 1 to 4.

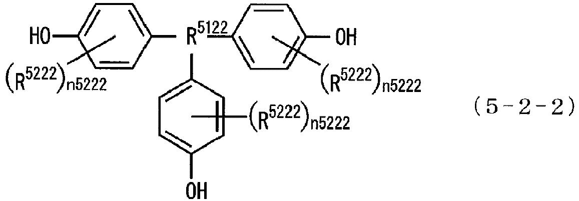

In the general formula (5-2-2), R⁵¹²² is a trivalent hydrocarbon group. Plural R⁵²²² are each independently a C1-20 alkyl group. Plural n5222 are each independently 0 or an integer of 1 to 4.

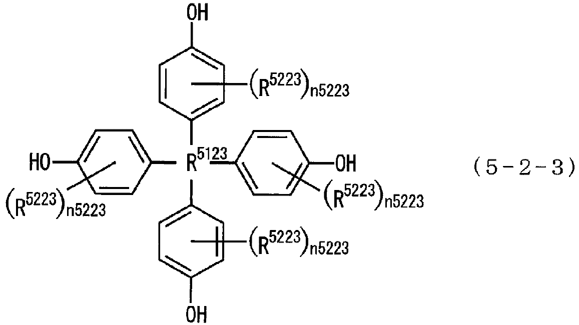

In the general formula (5-2-3), R³¹²³ is a tetravalent alkane group. Plural R⁵²²³ are each independently a C1-20 alkyl group. Plural n5223 are each independently 0 or an integer of 1 to 4.

R⁵¹²¹, R⁵¹²² or R⁵¹²³ may have an ether bond, an ester bond, a carbonyl group, or a hetero ring. R⁵¹²¹, R⁵¹²² or R⁵¹²³ may be a cyclic hydrocarbon group, a noncyclic hydrocarbon group or a group formed by binding at least one cyclic group with a noncyclic hydrocarbon group. The cyclic group may have a hetero ring structure or an oxo group. Examples of a hetero atom of the hetero ring include nitrogen, sulfur, and oxygen.

R⁵²²¹, R⁵²²², and R⁵²²³ are linear or branched.

Preferable examples of the polymer having the repeating unit (4-1-1) include phenolic novolac resins, ortho-cresolic novolac resins, phenolic resol resins, and cresolic resol resins, and phenolic novolac resins, phenolic resol resins, and cresolic resol resins are more preferable.

Preferable examples of the polymer having the repeating unit (4-2-1) include phenolaralkyl resins and biphenylaralkyl resins.

Preferable examples of the compound (5-1-1) include 2,5-di-tert-butylhydroquinone.

Preferable examples of the compound (5-1-2) include 2,4-diheptylphenol, 2,4-didodecylphenol, and 2,4-dipentylphenol.

Preferable examples of the compound (5-1-3) include stearyl 3-(3,5-di-tert-butyl-4-hydroxyphenyl)propionate (IRGANOX 1076).

Preferable examples of the compound (5-2-1) include 4,4'-(3,3,5-trimethylcyclohexylidene)bisphenol, bisphenol A, and bisphenol F.

Preferable examples of the compound (5-2-2) include α,α-bis(4-hydroxyphenyl)-4-(4-hydroxy-α,α-dimethylbenzyl)ethylbenzene, a compound of the following formula (5-2-2-1) and a compound of the following formula (5-2-2-2).

Preferable examples of the compound (5-2-3) include a compound of the following formula (5-2-3-1) (IRGANOX 1010).

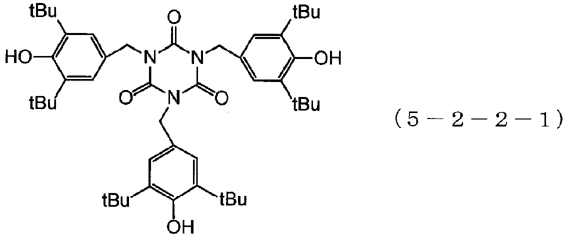

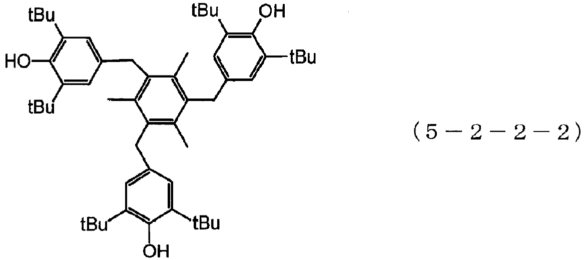

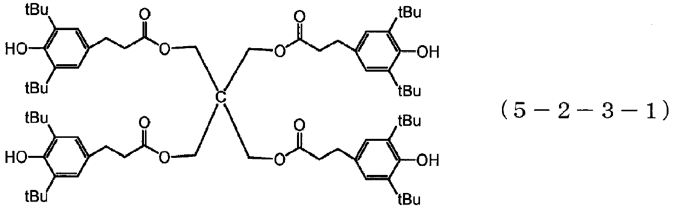

Alternatively, the compound (A) is preferably at least one selected from the group consisting of the compounds (6) and the compounds (7).

R⁶¹-(COO-R⁶²)ₙ₆₁ (6)

In the general formula (6), n61 is an integer of 1 to 3. R⁶¹ is an n61-valent C1-60 hydrocarbon group. The C1-60 hydrocarbon group may have either an ether bond or an ester bond. R⁶² is a C1-20 aliphatic hydrocarbon group or a C6-20 aromatic hydrocarbon group.

R⁷¹-(OCO-R⁷²)ₙ₇₁ (7)

In general formula (7), n71 is 2 or 3. R⁷¹ is an n71-valent C1-60 hydrocarbon group. The C1-60 hydrocarbon group may have either an ether bond or an ester bond. R⁷² is a C1-20 aliphatic hydrocarbon group or a C6-20 aromatic hydrocarbon group.

### (n61)

n61 is an integer of 1 to 3, preferably 2 or 3, and more preferably 3. When n61 is 1, R⁶¹ is a monovalent C1-60 hydrocarbon group. When n61 is 2, R⁶¹ is a divalent C1-60 hydrocarbon group. When n61 is 3, R⁶¹ is a trivalent C1-60 hydrocarbon group.

### (n71)

n71 is 2 or 3, and preferably 2. When n71 is 2, R⁷¹ is a divalent C1-60 hydrocarbon group. When n71 is 3, R⁷¹ is a trivalent C1-60 hydrocarbon group.

### (R⁶¹ and R⁷¹)

R⁶¹ is an n61-valent CI-60 hydrocarbon group. R⁷¹ is an n71-valent C1-60 hydrocarbon group. The hydrocarbon group as R⁶¹ or R⁷¹ may be an aliphatic hydrocarbon group or an aromatic hydrocarbon group. The carbon number of the hydrocarbon group is 1 to 60, preferably 2 to 60, more preferably 3 to 60, and even more preferably 3 to 56.

Examples of the hydrocarbon group as R⁶¹ and R⁷¹ include the same groups as those mentioned as R²¹ above.

The C1-60 hydrocarbon group may have either an ether bond or an ester bond. Namely, a carbon bond constituting the hydrocarbon group mentioned as R²¹ may be substituted with an ether bond or an ester bond.

Among these, when n61 is 1, that is R⁶¹ is monovalent, R⁶¹ is preferably a phenyl group which may have a C1-6 alkyl group or a C1-6 alkoxy group as a substituent. When n61 is 2 or n71 is 2, that is R⁶¹ or R⁷¹ is divalent, R⁶¹ or R⁷¹ is preferably a C3-10 alkylene group (trimethylene group), an oxybisethylene group, a 1-2-benzenediyl group or a group of the following general formula (III), and more preferably a C3-10 alkylene group or a 1-2-benzenediyl group. When n61 is 3 or n71 is 3, that is R⁶¹ or R⁷¹ is trivalent, R⁶¹ or R⁷¹ is preferably a 1-2,4-benzenetriyl group.

-[(CH₂)ₙ₆₄₁COO(CH₂)ₙ₆₄₂OCO]ₘ₆₁(CH₂)ₙ₆₄₃- (III)

In the general formula (III), n641, n642 and n643 are each independently an integer of 1 to 20. m61 is an integer of 1 to 4.

### (R⁶² and R⁷²)

R⁶² and R⁷² are each independently a C1-20 aliphatic hydrocarbon group (preferably alkyl group) or a C6-20 aromatic hydrocarbon group (preferably phenyl group).

When n61 is 1, R⁶² is preferably a C1-20, more preferably C1-6 aliphatic hydrocarbon group (preferably alkyl group).

When n61 is 2 or n71 is 2, the carbon number of an aliphatic hydrocarbon group as R⁶² or R⁷² is 1 to 20, preferably 2 to 18, more preferably 4 to 15, and even more preferably 6 to 12. The carbon number of an aromatic hydrocarbon group as R⁶² or R⁷² is 6 to 20, and preferably 6 to 12. Examples of the aliphatic hydrocarbon group and the aromatic hydrocarbon group as R⁶² and R⁷² include the same groups as those mentioned as R²² above. Among these, a C6-12 alkyl group (an octyl group, a 7-methyloctyl group, an isononyl group, an isodecyl group, an undecyl group, or a dodecyl group) or a phenyl group is preferable as R⁶² and R⁷².

Preferable examples of the compound (6) include compounds of the following general formula (6-1) (hereinafter, may be referred to as "compounds (6-1)") and compounds of the following general formula (6-2) (hereinafter, may be referred to as "compounds (6-2)").

R⁶¹¹-(COO-R⁶¹²)ₙ₆₁₁ (6-1)

R⁶²¹-(COO-R⁶²²)ₙ₆₂₁ (6-2)

In the general formula (6-1), n611 is 2 or 3. R⁶¹¹ is an n611-valent C1-60 aliphatic hydrocarbon group. The C1-60 aliphatic hydrocarbon group may have either an ether bond or an ester bond. R⁶¹² is the same as R⁶².

In the general formula (6-2), n621 is 2 or 3. R⁶²¹ is an n621-valent C6-60 aromatic hydrocarbon group. The C1-60 aromatic hydrocarbon group may have either an ether bond or an ester bond. R⁶²² is the same as R⁶² mentioned above.

Preferable examples of the compound (7) include compounds of the following general formula (7-1) (hereinafter, may be referred to as "compounds (7-1)").

R⁷¹¹-(OCO-R⁷¹²)ₙ₇₁₁ (7-1)

In the general formula (7-1), n711 is the same as n71 mentioned above. R⁷¹¹ is an n711-valent Cl-60 aliphatic hydrocarbon group. The C1-60 aliphatic hydrocarbon group may have either an ether bond or an ester bond. R⁷¹² is the same as R⁷² mentioned above.

### (R⁶¹¹ and R⁷¹¹)

R⁶¹¹ is an n611-valent C1-60 aliphatic hydrocarbon group. R⁷¹¹ is an n711-valent C1-60 aliphatic hydrocarbon group. The carbon number of the aliphatic hydrocarbon group is 1 to 60, preferably 2 to 60, more preferably 3 to 60, and even more preferably 3 to 56.

Examples of the aliphatic hydrocarbon group as R⁶¹¹ and R⁷¹¹ include the same groups as those mentioned as R²¹ above.

The C1-60 aliphatic hydrocarbon group may have either an ether bond or an ester bond. Namely, a carbon bond constituting the aliphatic hydrocarbon group mentioned as R²¹ above may be substituted with an ether bond or an ester bond.

Among these, when n611 is 2 or n711 is 2, that is, R⁶¹¹ or R⁷¹¹ is divalent, R⁶¹¹ or R⁷¹¹ is preferably a C3-10 alkylene group (trimethylene group), an oxybisethylene group, a 1-2-benzenediyl group or a group of the general formula (III), and more preferably a C3-10 alkylene group or a 1-2-benzenediyl group. When n611 is 3 or n711 is 3, that is, R⁶¹¹ or R⁷¹¹ is trivalent, R⁶¹¹ or R⁷¹¹ is preferably a 1-2,4-benzenetriyl group.

Preferable examples of the compound (6-1) include compounds of the following general formula (6-1-1) (hereinafter, may be referred to as "compounds (6-1-1)"). Preferable examples of the compound (6-2) include compounds of the following general formula (6-2-1) (hereinafter, may be referred to as "compounds (6-2-1)"). Among these, the compound (6-2-1) is more preferable.

R⁶¹³-OOC-Y⁶¹¹-COO-R⁶¹⁴ (6-1-1)

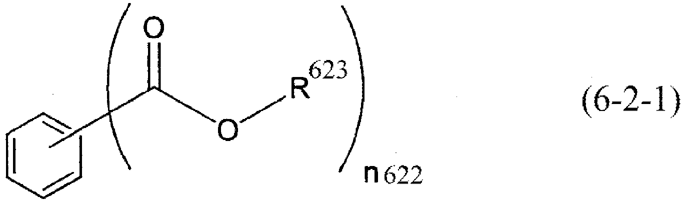

In the general formula (6-1-1), R⁶¹³ and R⁶¹⁴ may be identical to or different from each other and are each the same as R⁶¹² mentioned above. Y⁶¹¹ is a divalent C1-60 aliphatic hydrocarbon group. The C1-60 aliphatic hydrocarbon group may have either an ether bond or an ester bond.

In the general formula (6-2-1), R⁶²³ and n622 are each the same as R⁶²² and n621 mentioned above. When the number of a group having an ester bond (-COOR⁶²³) bonded with a benzene ring is two (that is, n622 is 2), the groups may be bonded at any of an ortho-position (bonded with carbon atoms at the 1^{st} position and the 2^{nd} position of the benzene ring), a meta-position (bonded with carbon atoms at the 1^{st} position and the 3^{rd} position of the benzene ring) or a para-position (bonded with carbon atoms at the 1^{st} position and 4^{th} position of the benzene ring), and, among these, preferably bonded at the ortho-position. When the number of a group having an ester bond (-COOR⁶²³) bonded with a benzene ring is three (that is, n622 is 3), the groups may be bonded at the positions of the 1^{st} position, the 2^{nd} position and the 3^{rd} position, the positions of the 1^{st} position, the 2^{nd} position and the 4^{th} position, or the positions of the 1^{st} position, the 3^{rd} position and the 5^{th} position, and, among them, are preferably bonded at the positions of the 1^{st} position, the 2^{nd} position and the 4^{th} position.

Preferable examples of the compound (7-1) include compounds of the following general formula (7-1-1) (hereinafter, may be referred to as "compounds (7-1-1)").

R⁷¹³-COO-Y⁷¹¹-OCO-R⁷¹⁴ (7-1-1)

In the general formula (7-1-1), R⁷¹³ and R⁷¹⁴ may be identical to or different from each other, and are each the same as R⁷² mentioned above. Y⁷¹¹ is a divalent C1-60 aliphatic hydrocarbon group. The C1-20 aliphatic hydrocarbon group may have either an ether bond or an ester bond.

### (Y⁶¹¹ and Y⁷¹¹)

Although the divalent C1-20 aliphatic hydrocarbon group as Y⁶¹¹ and Y⁷¹¹ may be chained or cyclic, the divalent C1-20 aliphatic hydrocarbon group is preferably chained. Although the chained aliphatic hydrocarbon group may be linear or branched, the chained aliphatic hydrocarbon group is preferably linear.

Examples of the linear aliphatic hydrocarbon group include a methylene group, an ethylene group, a trimethylene group, a tetramethylene group, a pentamethylene group, a hexamethylene group, a heptamethylene group, and an octamethylene group. A carbon bond constituting the linear aliphatic hydrocarbon group may be substituted with either an ether bond or an ester bond. Among them, a C3-10 alkylene group (trimethylene group), an oxybisethylene group, or a 1-2-benzenediyl group is preferable, and a C3-10 alkylene group or a 1-2-benzenediyl group is more preferable as Y⁶¹¹ and Y⁷¹¹. When substituted with an ether bond, Y⁶¹¹ and Y⁷¹¹ are preferably an oxyethylene group, an oxytetramethylene group or a repetition thereof. When substituted with an ester bond, Y⁶¹¹ and Y⁷¹¹ are preferably groups of the general formula (III).

Preferable examples of the compound (6-1-1) include diisononyl adipate, and compounds of the following general formula (6-1-1-1) (adipic acid-based polyester), and diisononyl adipate is more preferable.

C₈H₁₇O[CO(CH₂)₄COO(CH₂)₄O]ₙ₆₁₂CO(CH₂)₄COOC₈H₁₇ (6-1-1-1)

In the general formula (6-1-1-1), n612 is an integer of 1 to 4.

Preferable examples of the compound (6-2-1) include diethyl phthalate, di-n-octyl phthalate, bis(7-methyloctyl) phthalate, bis(2-ethylhexyl) phthalate, diisodecyl phthalate, diundecyl phthalate, diphenyl phthalate, and tris(2-ethylhexyl) trimellitate of the following formula (6-2-1a), and diethyl phthalate, diphenyl phthalate, di-n-octyl phthalate, or tris(2-ethylhexyl) trimellitate is more preferable, and tris(2-ethylhexyl) trimellitate is even more preferable.

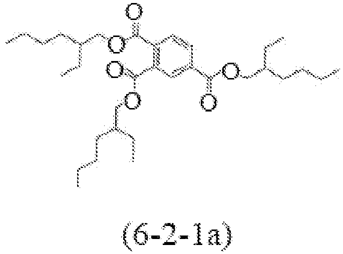

Preferable examples of the compound (7-1-1) include diethylene glycol dibenzoate (dibenzoic acid oxybisethylene ester).

Additional examples of the compound (6) include compounds of the following general formula (6-3) (hereinafter, may be referred to as "compounds (6-3)").

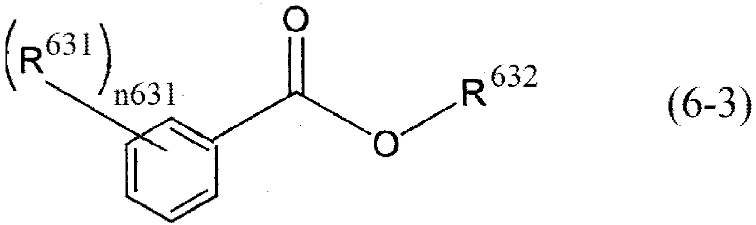

In the general formula (6-3), R⁶³¹ is a C1-6 alkyl group or a C1-6 alkoxy group, preferably a Cl-6 alkoxy group. n631 is 1 or 2, and preferably 2. R⁶³² is a C1-6 alkyl group.

Among them, the compound (A) is preferably the compound (6-2), more preferably the compound (6-2-1), even more preferably diethyl phthalate, diphenyl phthalate, di-n-octyl phthalate, or tris(2-ethylhexyl) trimellitate, and even more preferably tris(2-ethylhexyl) trimellitate.

Alternatively, the compound (A) is preferably at least one selected from the group consisting of compounds of the following general formula (S1) (hereinafter, may be referred to as "compounds (S1)"), compounds of the following general formula (S2) (hereinafter, may be referred to as "compounds (S2)"), and compounds of the following general formula (S3) (hereinafter, may be referred to as "compounds (S3)").

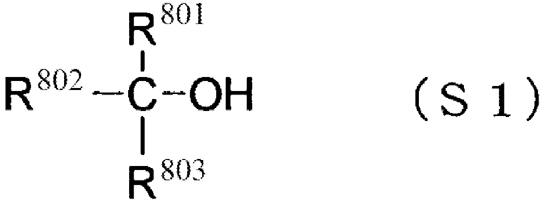

In the general formula (S1), R⁸⁰¹, R⁸⁰², and R⁸⁰³ are each independently a C1-60 saturated or unsaturated linear or branched hydrocarbon group. When R⁸⁰¹, R⁸⁰² or R⁸⁰³ has a methylene group, the methylene group may be substituted with an oxygen atom, an arylene group, a cycloalkylene group or an NH group. At least one CH group constituting R⁸⁰¹, R⁸⁰² or R⁸⁰³ may be substituted with a nitrogen atom. At least one hydrogen atom constituting R⁸⁰¹, R⁸⁰² or R⁸⁰³ may be substituted with a halogen atom or a hydroxy group. R⁸⁰¹, R⁸⁰² or R⁸⁰³ may be bonded together to form a monocycle or polycycle.

Among them, R⁸⁰¹, R⁸⁰² and R⁸⁰³ are each independently a phenyl group which may be substituted with a C1-6 alkyl group which may be substituted with a hydroxy group, or a C1-6 alkyl group.

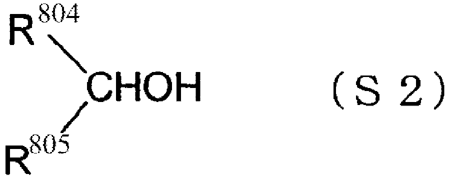

In the general formula (S2), R⁸⁰⁴ and R⁸⁰³ are each independently a C1-60 saturated or unsaturated linear or branched hydrocarbon group. When R⁸⁰⁴ or R⁸⁰⁵ has a methylene group, the methylene group may be substituted with an oxygen atom, an arylene group, a cycloalkylene group or an NH group. At least one CH group constituting R⁸⁰⁴ or R⁸⁰⁵ may be substituted with a nitrogen atom. At least one hydrogen atom constituting R⁸⁰⁴ or R⁸⁰⁵ may be substituted with a halogen atom or a hydroxy group. R¹⁰⁴ and R⁸⁰⁵ may be bonded together to form a monocycle or a polycycle.

Among them, R⁸⁰⁴ and R⁸⁰⁵ are each independently a C1-6 alkyl group, and the alkyl group may have an ether bond, and may be substituted with a phenyl group

R⁸⁰⁶-CH₂OH (S3)

In the general formula (S3), R⁸⁰⁶ is a C1-60 saturated or unsaturated linear or branched hydrocarbon group. When R⁸⁰⁶ has a methylene group, the methylene group may be substituted with an oxygen atom, an arylene group, a cycloalkylene group or an NH group. At least one CH group constituting R⁸⁰⁶ may be substituted with a nitrogen atom. At least one hydrogen atom constituting R⁸⁰⁶ may be substituted with a halogen atom or a hydroxy group, and the branched chains may be bonded together to form a ring.

The compound (S1) is a tertiary alcohol, the compound (S2) is a secondary alcohol, and the compound (S3) is a primary alcohol.

The compound (A) used in the preparation method according to the present embodiment may have at least one of one type selected from the group consisting of a hydroxy group bonded with a carbon atom to which one carbon atom is bonded in one molecule, a hydroxyl group bonded with a carbon atom to which two carbon atoms are bonded in one molecule, and a hydroxyl group bonded with a carbon atom to which three carbon atoms are bonded in one molecule, or may have at least one of two or more types selected therefrom.

In the present specification, when the compound (A) has plural hydroxy groups in one molecule, the compound (A) is classified into a primary alcohol, a secondary alcohol, or a tertiary alcohol, based on a hydroxyl group bonded with a carbon atom to which the largest number of carbon atoms are bonded. For example, when the compound (A) has both a hydroxyl group bonded with a carbon atom to which one carbon atom is bonded in one molecule and a hydroxyl group bonded with a carbon atom to which two carbon atoms are bonded in one molecule, the compound (A) is classified into the secondary alcohol.

The standard boiling point of the compound (A) is required to be higher than the standard boiling point of an isocyanate produced by thermal decomposition of a carbamate. Since the boiling point of the compound (A) tends to become high, a secondary alcohol or a tertiary alcohol is preferable rather than a primary alcohol as the compound (A), and a tertiary alcohol is more preferable.

In the preparation method according to the present embodiment, an isocyanate and a hydroxy compound are produced by thermal decomposition of a carbamate. The compound (A) may also be referred to as a hydroxy compound. However, the compound (A) differs from a hydroxy compound produced by thermal decomposition of a carbamate at least in terms of the standard boiling point of the compound (A) higher than the standard boiling point of an isocyanate produced by thermal decomposition of a carbamate.

The standard boiling point of the compound (A) is higher than the standard boiling point of a hydroxy compound produced by thermal decomposition of a carbamate by 10°C or more, for example, preferably by 30°C or more, and more preferably by 50°C or more. The standard boiling point of the compound (A) is higher than the standard boiling point of an isocyanate produced by thermal decomposition of a carbamate by 10°C or more, for example, preferably by 30°C or more, and more preferably by 50°C or more. When the standard boiling point of the compound (A) is within the above-mentioned range, an isocyanate can be produced continuously for a long time while extracting efficiently a high-boiling-point component produced by side reactions.

Specific examples of the compound of the general formula (S1) include compounds of the following formulae (S1-1) to (S1-14), and the compound of the following formula (S1-1) or the compound of the following formula (S1-8) is preferable.

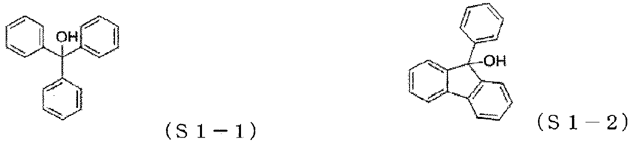

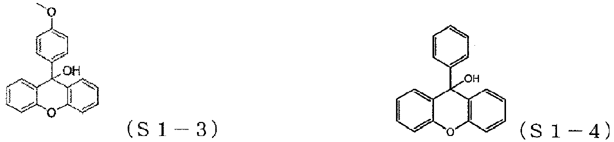

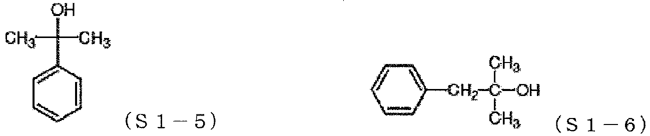

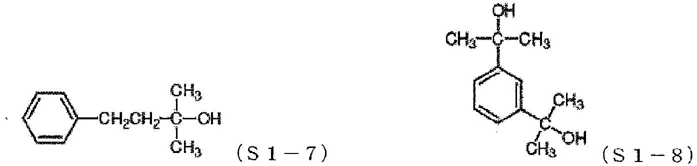

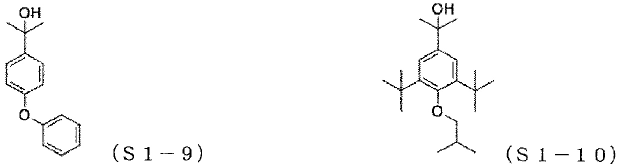

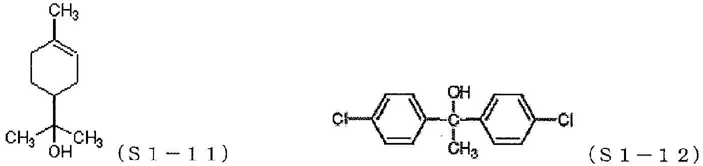

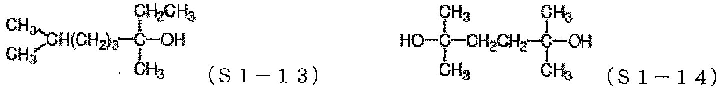

Specific examples of the compounds of the general formula (S2) include compounds of the following formulae (S2-1) to (S2-29), and the compound of the formula (S2-29) is preferable.

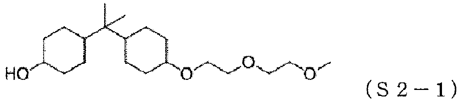

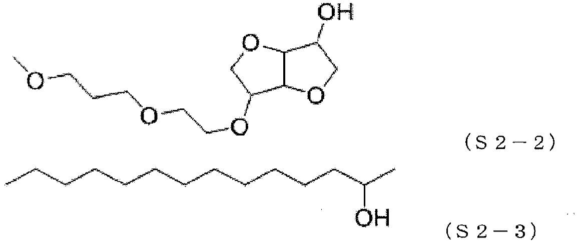

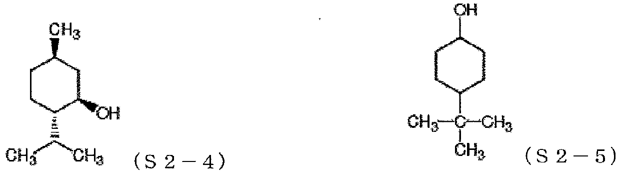

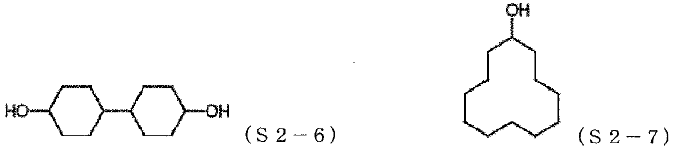

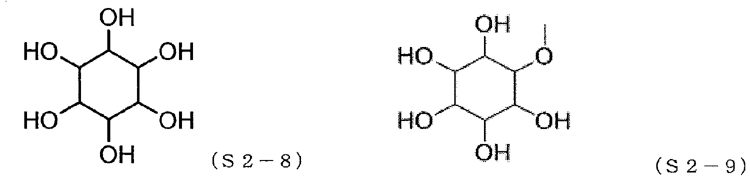

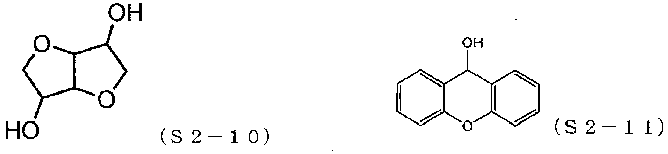

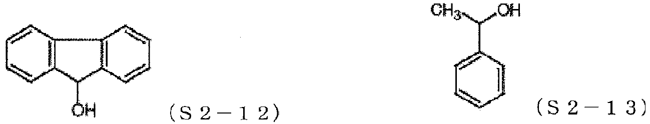

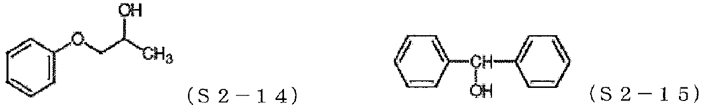

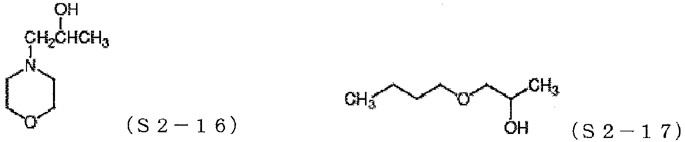

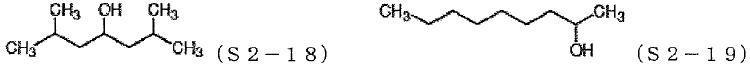

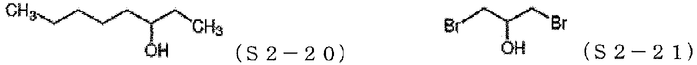

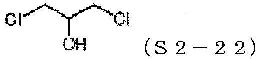

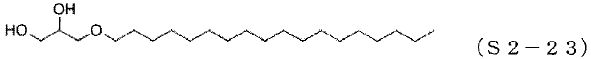

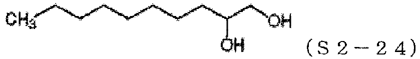

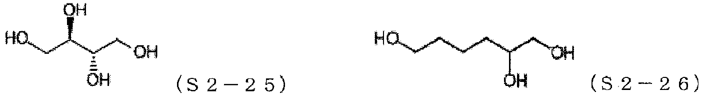

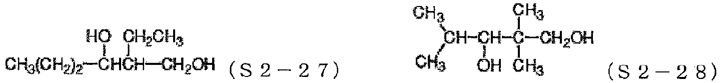

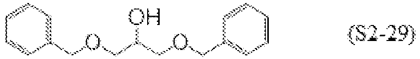

Specific examples of the compounds of the general formula (S3) include compounds of the following formulae (S3-1) to (S3-13).

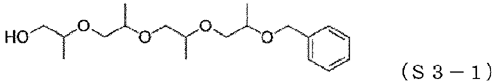

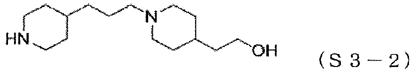

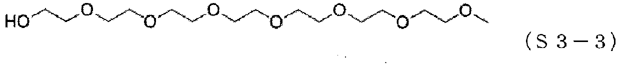

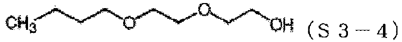

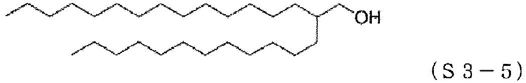

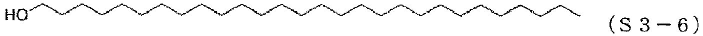

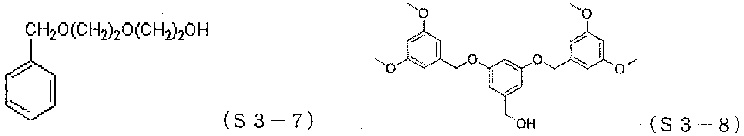

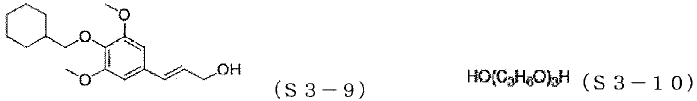

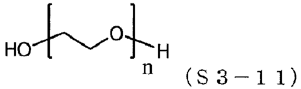

In the general formula (S31-1), n is an integer of 1 to 20.

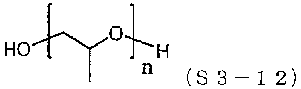

In the general formula (S3-12), n is an integer of 1 to 20.

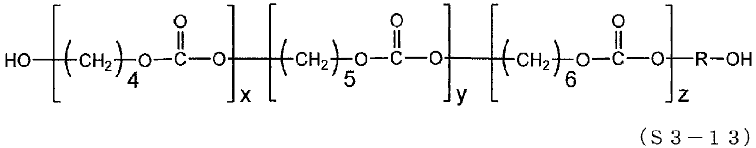

In the general formula (S3-13), x, y and z are each independently an integer of 0 to 20, the sum of x, y and z is an integer of 0 to 20, and R is -(CH₂)₄-, -(CH₂)₅-, or -(CH₂)₆-. The repeating unit of the following formula (S3-13-1), the repeating unit of the following formula (S3-13-2) and the repeating unit of the following formula (S3-13-3) may be contained randomly or in a block shape, the total number of the repeating unit of the following formula (S3-13-1) is x, the total number of the repeating unit of the following formula (S3-13-2) is y, and the total number of the repeating unit of the following formula (S3-13-3) is z.

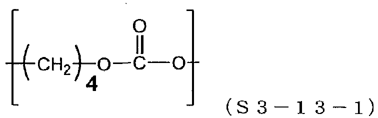

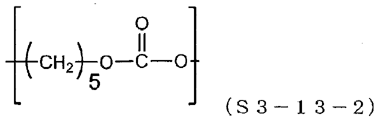

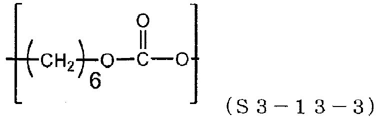

Alternatively, the compound (A) is preferably at least one selected from the group consisting of the compounds (9) and the compounds (10).

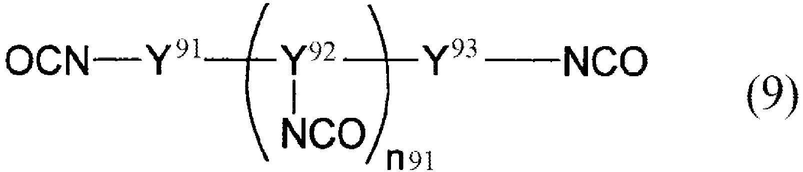

In the general formula (9), Y⁹¹ and Y⁹³ are each independently a C4-10 divalent hydrocarbon group having an alicyclic hydrocarbon group or an aromatic hydrocarbon group. Y⁹¹ and Y⁹³ may be identical to or different from each other, but are preferably identical to each other. Y⁹² is a C4-10 trivalent hydrocarbon group having an alicyclic hydrocarbon group or an aromatic hydrocarbon group, and at least one CH group constituting the aromatic hydrocarbon group may be substituted with a nitrogen atom or a carbonyl group. n91 is an integer of 0 to 5. When n91 is 0, "-(Y⁹²(-NCO))ₙ₉₁-" is a single bond, and the compound (9) is "OCN-Y⁹¹-Y⁹³-NCO".

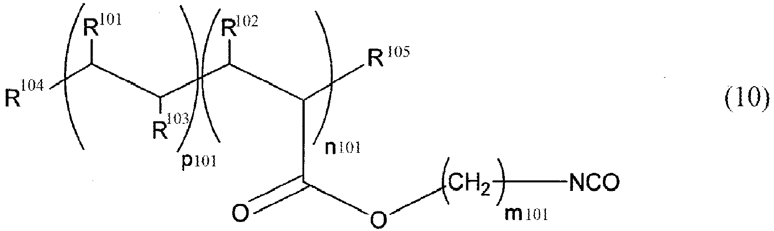

In the general formula (10), p101 is an integer of 0 to 90. n101 is an integer of 1 to 100. The sum of p101 and n101 is an integer of 10 to 100. m101 is an integer of 1 to 5. R¹⁰¹ and R¹⁰² are each independently a hydrogen atom or a C1-5 monovalent hydrocarbon group. R¹⁰³ is a C1-5 alkoxycarbonyl group or a C1-12 monovalent hydrocarbon group. R¹⁰⁴ and R¹⁰⁵ are each independently a monovalent organic group.

### (Y⁹¹ and Y⁹³)

The divalent hydrocarbon group as Y⁹¹ and Y⁹³ may consist of a chained aliphatic hydrocarbon group, an alicyclic hydrocarbon group or an aromatic hydrocarbon group, or may be a group formed by bonding either an alicyclic hydrocarbon group or an aromatic hydrocarbon group with a chained aliphatic hydrocarbon group, a group formed by bonding an alicyclic hydrocarbon group with an aromatic hydrocarbon group, or a repetition of these groups.

Although the divalent chained aliphatic hydrocarbon group as Y⁹¹ and Y⁹³ may be linear or branched, the divalent chained aliphatic hydrocarbon group is preferably linear. Examples of the linear aliphatic hydrocarbon group include a methylene group, an ethylene group, a trimethylene group, a tetramethylene group, a pentamethylene group, a hexamethylene group, a heptamethylene group, an octamethylene group, a nonamethylene group, and a decamethylene group, and a C1-10 alkylene group is preferable.

Examples of the divalent alicyclic hydrocarbon group as Y⁹¹ and Y⁹³ include a cyclopropylene group, a cyclotetramethylene group, a cyclopentamethylene group, a cyclohexylene group, a cyclohexamethylene group, a cycloheptamethylene group, a cyclooctamethylene group, a cyclononamethylene group, and a cyclodecamethylene group, and a cyclohexylene group is preferable.

Examples of the divalent aromatic hydrocarbon group as Y⁹¹ and Y⁹³ include a phenylene group, and a naphthalene-diyl group, and a phenylene group is preferable.

Among them, Y⁹¹ is preferably a group formed by bonding either an alicyclic hydrocarbon group or an aromatic hydrocarbon group with a chained aliphatic hydrocarbon group, a group formed by repetition thereof or a chained aliphatic hydrocarbon group.

Among them, Y⁹¹ and Y⁹³ are preferably alicyclic hydrocarbon groups, aromatic hydrocarbon groups, or groups formed by bonding either an alicyclic hydrocarbon group or an aromatic hydrocarbon group with a chained aliphatic hydrocarbon group, and more preferably Y⁹¹ is an alicyclic hydrocarbon group or an aromatic hydrocarbon group, and Y⁹³ is a group formed by bonding either an alicyclic hydrocarbon group or an aromatic hydrocarbon group with a chained aliphatic hydrocarbon group. Alternatively, Y⁹¹ and Y⁹³ are preferably chained aliphatic hydrocarbon groups (preferably C4-10 alkylene groups).

Preferable examples of Y⁹¹ and Y⁹³ include groups of the following general formula (IV) (groups (IV)).

-(CH₂)ₙ₉₂-Y⁹⁴- (IV)

In the general formula (IV), n92 is an integer of 0 to 5. When n92 is 0, "-(CH₂)ₙ₉₂-" is a single bond and the group (IV) is "-Y ⁹⁴-". Y⁹⁴ is a C4-10 divalent alicyclic hydrocarbon group or an aromatic hydrocarbon group.

n92 is an integer of 0 to 5, preferably an integer of 0 to 4, more preferably an integer of 0 to 3, and even more preferably an integer of 0 to 2. Namely, as the chained aliphatic hydrocarbon group (alkylene group) constituting the group formed by bonding either an alicyclic hydrocarbon group or an aromatic hydrocarbon group with a chained aliphatic hydrocarbon group, a single bond or a C1-4 chained alkylene group is preferable, a single bond or a C1-3 chained alkylene group is more preferable, and a single bond, a methylene group or an ethylene group is even more preferable.

Y⁹⁴ is a C4-10 divalent alicyclic hydrocarbon group or a C6-10 divalent aromatic hydrocarbon group. Examples of the C4-10 divalent alicyclic hydrocarbon group and the C6-10 divalent aromatic hydrocarbon group as Y⁹⁴ include the same groups as those mentioned as Y⁹¹ and Y⁹³ above. Among them, Y⁹⁴ is preferably a cyclopentamethylene group, a cyclohexylene group, a cyclohexamethylene group, a cycloheptamethylene group, a cyclooctamethylene group, a phenylene group or a naphthalene-diyl group, more preferably a cyclopentamethylene group, a cyclohexylene group, a cyclohexamethylene group or a phenylene group, and even more preferably a cyclohexylene group or a phenylene group.

### (Y⁹²)

Y⁹² is a C4-10 trivalent hydrocarbon group having an alicyclic hydrocarbon group or an aromatic hydrocarbon group. The trivalent hydrocarbon group as Y⁹² may consist of an alicyclic hydrocarbon group or an aromatic hydrocarbon group, or may be a group formed by bonding either an alicyclic hydrocarbon group or an aromatic hydrocarbon group with a chained aliphatic hydrocarbon group, or a group formed by bonding an alicyclic hydrocarbon group and an aromatic hydrocarbon group. At least one CH group constituting the aromatic hydrocarbon group may be substituted with a nitrogen atom or a carbonyl group, and more specifically one to six CH groups may be substituted with a nitrogen atom and/or a carbonyl group. Among them, Y⁹² is preferably a group formed by bonding either an alicyclic hydrocarbon group or an aromatic hydrocarbon group with a chained aliphatic hydrocarbon group.

Preferable examples of "-Y⁹²(-NCO)-" include groups of the following general formula (V1) or (V2) (group (V1) or group (V2)).

-(CH₂)ₙ₉₃₁-Y⁹⁵(-NCO)- (V1)

-Y⁹⁵(-(CH₂)ₙ₉₃₂-NCO)- (V2)

In the general formulae (V1) and (V2), n931 is an integer of 0 to 5, and n932 is an integer of 1 to 6. For example, when n931 is 0, "-(CH₂)ₙ₉₃-" is a single bond, and the group (V1) is "-Y⁹⁵(-NCO)-". Y⁹⁵ is a C4-10 divalent alicyclic hydrocarbon group or a C6-10 divalent aromatic hydrocarbon group. At least one CH group constituting the aromatic hydrocarbon group may be substituted with a nitrogen atom and/or a carbonyl group.

n931 is preferably an integer of 0 to 4, more preferably an integer of 0 to 3, and even more preferably an integer of 0 to 2. Namely, in the group (V1), as a chained aliphatic hydrocarbon group constituting the group formed by bonding either an alicyclic hydrocarbon group or an aromatic hydrocarbon group with the chained aliphatic hydrocarbon group, a single bond or a C1-4 chained alkylene group is preferable, a single bond or a C1-3 chained alkylene group is more preferable, and a single bond, a methylene group or an ethylene group is even more preferable.

In contrast, n932 is preferably an integer of 1 to 6, more preferably an integer of 4 to 6, and even more preferably 6.

Y⁹⁵ is a C4-10 trivalent alicyclic hydrocarbon group or a C6-10 trivalent aromatic hydrocarbon group. Examples of the trivalent alicyclic hydrocarbon group as Y⁹⁵ include a cyclobutane-triyl group, a cyclopentane-triyl group, a cyclohexanetriyl group, a cycloheptane-triyl group, a cyclooctanetriyl group, and a cyclodecane-triyl group. Examples of the trivalent aromatic hydrocarbon group as Y⁹⁸ include a benzene-triyl group and a naphthalenetriyl group. Examples of the group in which at least one CH group constituting the aromatic hydrocarbon group is substituted with a nitrogen atom and/or a carbonyl group include a 2,4,6-trioxohexahydro-1,3,5-triazine-1,3,5-triynyl group of the following formula.

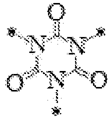

In the formula, ^{∗} indicates the bonding position with Y⁹¹, Y⁹³, or a carbon atom.

Among them, Y⁹⁵ is preferably a cyclopentane-triyl group, a cyclohexanetriyl group, a cycloheptane-triyl group, a cyclooctanetriyl group, a benzene-triyl group, a naphthalenetriyl group or a 2,4,6-trioxohexahydro-1,3,5-triazine-1,3,5-triynyl group, and more preferably a cyclopentane-triyl group, a cyclohexanetriyl group, a benzene-triyl group or a 2,4,6-trioxohexahydro-1,3,5-triazine-1,3,5-triynyl group.

The type of the aliphatic ring or the aromatic ring contained in Y⁹¹, Y⁹² and Y⁹³ may be identical to or different from each other, but is preferably identical to each other.

When Y⁹² is the group (V2), it is preferable that Y⁹¹ and Y⁹³ be each independently a chained aliphatic hydrocarbon group (C4-10 alkylene group).

### (R¹⁰¹ and R¹⁰²)

R¹⁰¹ and R¹⁰² are each independently a hydrogen atom or a C1-5 monovalent hydrocarbon group. R¹⁰¹ and R¹⁰² may be identical to or different from each other.

Examples of C1-5 monovalent hydrocarbon group as R¹⁰¹ and R¹⁰² include aliphatic hydrocarbon groups. Although the aliphatic hydrocarbon group may be linear, branched, or cyclic, the aliphatic hydrocarbon group is preferably linear. Examples of the linear aliphatic hydrocarbon group include a methyl group, an ethyl group, a propyl group, an n-butyl group, and an n-pentyl group.

Among them, R¹⁰¹ and R¹⁰² are preferably a hydrogen atom, a methyl group or an ethyl group, and more preferably a hydrogen atom or a methyl group.

### (R¹⁰³)

R¹⁰³ is a C1-5 alkoxycarbonyl group or a C1-12 monovalent hydrocarbon group.

Examples of the C1-5 alkoxycarbonyl group as R¹⁰³ include a methoxycarbonyl group, an ethoxycarbonyl group, a propoxycarbonyl group, an isopropoxycarbonyl group, a butoxycarbonyl group, a sec-butoxycarbonyl group, a tert-butoxycarbonyl group, a pentoxycarbonyl group, and a neopentoxycarbonyl group.

Examples of C1-12 monovalent hydrocarbon group as R¹⁰³ include aliphatic hydrocarbon groups, aromatic hydrocarbon groups and groups formed by bonding an aliphatic hydrocarbon group and an aromatic hydrocarbon group.

The aliphatic hydrocarbon group may be linear, branched, or cyclic. Examples of the linear aliphatic hydrocarbon group include the same groups as R¹⁰¹ and R¹⁰² mentioned above. Examples of the branched aliphatic hydrocarbon group include an isopropyl group, a sec-butyl group, an isobutyl group, a tert-butyl group, a neopentyl group, and an isohexyl group. Examples of the cyclic aliphatic hydrocarbon group (alicyclic hydrocarbon group) include a cyclopropyl group, a cyclobutyl group, a cyclopentyl group, a cyclohexyl group, a cycloheptyl group, and a cyclooctyl group.

Examples of the aromatic hydrocarbon group include a phenyl group, and a napthyl group.

Examples of the group formed by bonding an aliphatic hydrocarbon group and an aromatic hydrocarbon group include a benzyl group, and a phenylmethylene group.

Among them, R¹⁰³ is preferably a C1-3 alkoxycarbonyl group or an aromatic hydrocarbon group, and more preferably a methoxycarbonyl group, an ethoxycarbonyl group or a phenyl group.

### (R¹⁰⁴ and R¹⁰⁵)

R¹⁰⁴ and R¹⁰⁵ are each independently a monovalent organic group. R¹⁰⁴ and R¹⁰⁵ may be identical to or different from each other.

Examples of the monovalent organic group as R¹⁰⁴ and R¹⁰⁵ include monovalent groups mentioned as R²¹. Among them, a C1-20 monovalent aliphatic hydrocarbon group or a C6-20 monovalent aromatic hydrocarbon group is preferable. The CI-20 monovalent aliphatic hydrocarbon group and the C6-20 monovalent aromatic hydrocarbon group may have a substituent. Examples of the C1-20 monovalent aliphatic hydrocarbon group and the C6-20 monovalent aromatic hydrocarbon group include the same groups as those mentioned as R²¹ above.

### (p101 and n101)

p101 is an integer of 0 to 90, preferably an integer of 0 to 80, more preferably an integer of 0 to 60, and even more preferably an integer of 0 to 40.

n101 is an integer of 1 to 100, preferably an integer of 1 to 90, and more preferably an integer of 1 to 80.

The sum of p101 and n101 is an integer of 10 to 100, preferably an integer of 10 to 90, more preferably an integer of 20 to 80, and even more preferably an integer of 30 to 70.

### (m101)

m101 is an integer of 1 to 5, preferably an integer of 1 to 3, and more preferably 1 or 2.

Preferable examples of the compound (9) include compounds of the following general formula (9-1) (hereinafter, may be referred to as "compounds (9-1)").

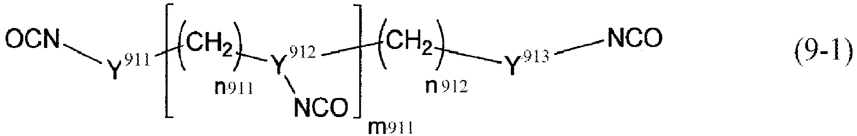

In the general formula (9-1), Y⁹¹¹ and Y⁹¹³ are each independently identical to Y⁹⁴ mentioned above. Y⁹¹² is identical to Y⁹⁵ mentioned above. n911 and n912 are each independently an integer of 1 to 5, and preferably 1. Although n911 and n912 may be identical to or different from each other, n911 and n912 are preferably identical to each other. m911 is an integer of 0 to 5. When m911 is 0, "-[(CH₂)ₙ₉₁₁-(Y⁹¹²(-NCO))-]" is a single bond, and the compound (9-1) is "OCN-Y⁹¹¹-(CH₂)ₙ₉₁₂-Y⁹¹³-NCO".

### (Y⁹¹¹ and Y⁹¹³)

As Y⁹¹¹ and Y⁹¹³, a cyclopentamethylene group, a cyclohexylene group, a cyclohexamethylene group, a cycloheptamethylene group, a cyclooctamethylene group, a phenylene group or a naphthalene-diyl group is preferable, a cyclopentamethylene group, a cyclohexylene group, a cyclohexamethylene group or a phenylene group is more preferable, and a cyclohexylene group or a phenylene group is even more preferable.

### (Y⁹¹²)

As Y⁹¹², a cyclopentane-triyl group, a cyclohexanetriyl group, a cycloheptane-triyl group, a cyclooctanetriyl group, a benzene-triyl group or a naphthalenetriyl group is preferable, and a cyclopentane-triyl group, a cyclohexanetriyl group or a benzene-triyl group is more preferable.

The type of the aliphatic rings or the aromatic rings contained in Y⁹¹¹, Y⁹¹² and Y⁹¹³ may be identical to or different from each other, but are preferably identical to each other.

### (n911 and n912)

n911 and n912 are each independently an integer of 1 to 5, preferably an integer of 1 to 4, more preferably an integer of 1 to 3, and even more preferably 1 or 2. Namely, as alkylene groups connecting Y⁹¹¹, Y⁹¹² and Y⁹¹³, C1-4 chained alkylene groups are preferable, C1-3 chained alkylene groups are more preferable, and a methylene group or an ethylene group is even more preferable.

Additional preferable examples of the compound (9) include compounds of the following formula (9-2) (hereinafter, may be referred to as "compounds (9-2)").

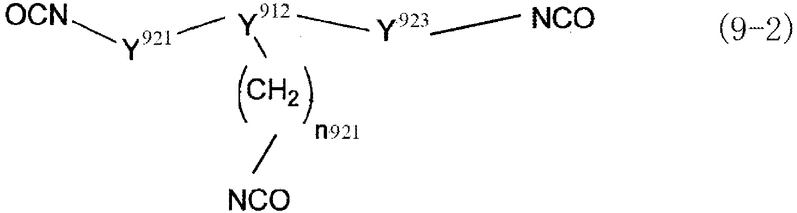

In the general formula (9-2), Y⁹²¹ and Y⁹²³ are each independently a C4-10 alkylene group.

Y⁹¹² is a 2,4,6-trioxohexahydro-1,3,5-triazine-1,3,5-triynyl group.

n921 is an integer of 1 to 6, and is preferably 6.

Preferable examples of the compound (10) include compounds of the following general formula (10-1) (compounds (10-1)).

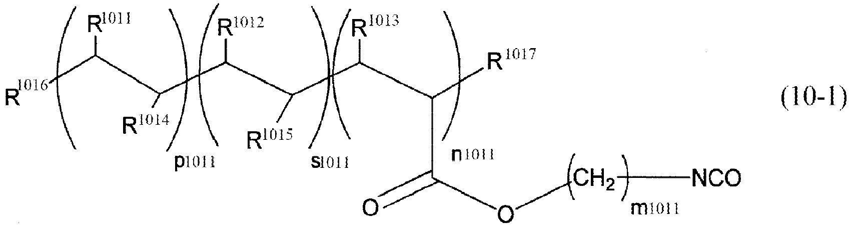

In the general formula (10-1), p1011 is an integer of 0 to 50, preferably an integer of 0 to 40, more preferably an integer of 0 to 30, and even more preferably an integer of 0 to 25. When p1011 is 0, an ethylene group having R¹⁰¹¹ and R¹⁰¹⁴ as side-chains becomes a single bond.
s1011 is an integer of 0 to 50, preferably an integer of 0 to 40, more preferably an integer of 0 to 30, and even more preferably an integer of 0 to 25. When s1011 is 0, an ethylene group having R¹⁰¹² and R¹⁰¹⁵ as side-chains becomes a single bond.
n1011 is the same as n101 mentioned above, and is preferably an integer of 1 to 90, more preferably an integer of 1 to 80, and even more preferably an integer of 1 to 60.

The sum of p1011, s1011 and n1011 is an integer of 10 to 100, preferably an integer of 10 to 90, more preferably an integer of 20 to 80, and even more preferably an integer of 30 to 70.
m1011 is the same as m101 mentioned above, and is preferably an integer of 1 to 3, and more preferably 1 or 2.

R¹⁰¹¹, R¹⁰¹² and R¹⁰¹³ are each the same as R¹⁰¹ and R¹⁰² mentioned above, and are preferably a hydrogen atom, a methyl group or an ethyl group, and more preferably a hydrogen atom or a methyl group. R¹⁰¹¹, R¹⁰¹² and R¹⁰¹³ may be identical to or different from each other.

R¹⁰¹⁴ and R¹⁰¹⁵ are each the same as R¹⁰³ mentioned above, and are preferably a C1-3 alkoxycarbonyl group or an aromatic hydrocarbon group, and more preferably a methoxycarbonyl group, an ethoxycarbonyl group or a phenyl group. R¹⁰¹⁴ and R¹⁰¹³ may be identical to or different from each other.

R¹⁰¹⁶ and R¹⁰¹⁷ are each the same as R¹⁰⁴ and R¹⁰⁵ mentioned above.

Specific examples of the preferable compound (9-1) include 4,4'-diphenylmethane diisocyanate, 4,4'-dicyclohexylmethane diisocyanate, compounds of the following general formula (9-1-1) (polymethylene polyphenyl polyisocyanate (polymeric MDI)), compounds of the following general formula (9-1-2), compounds of the following general formula (9-1-3) (polymeric hydrogenated MDI), and compounds of the following general formula (9-1-4), and 4,4'-diphenylmethane diisocyanate or 4,4'-dicyclohexylmethane diisocyanate is more preferable.

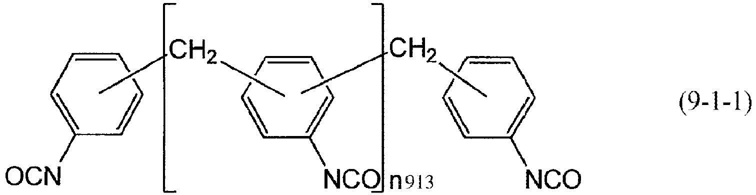

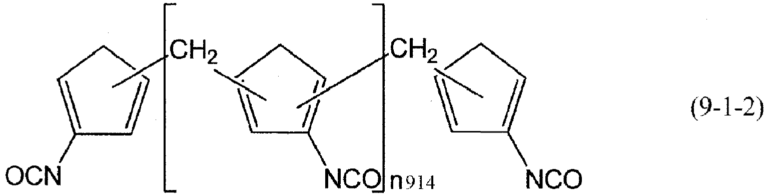

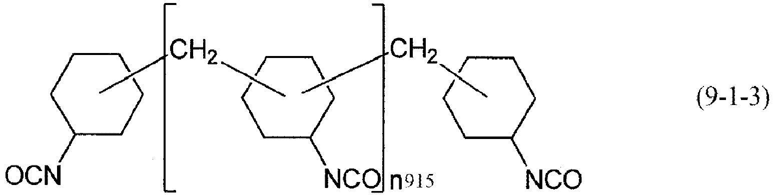

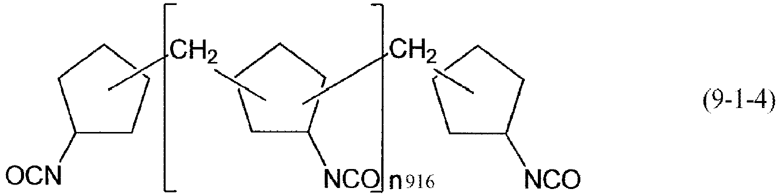

In the general formula (9-1-1), n913 is an integer of 1 to 5.

In the general formula (9-1-2), n914 is an integer of 1 to 5.

In the general formula (9-1-3), n915 is an integer of 1 to 5.

In the general formula (9-1-4), n916 is an integer of 1 to 5.

Preferable examples of the compound (9-2) include a compound of the following formula (9-2a).

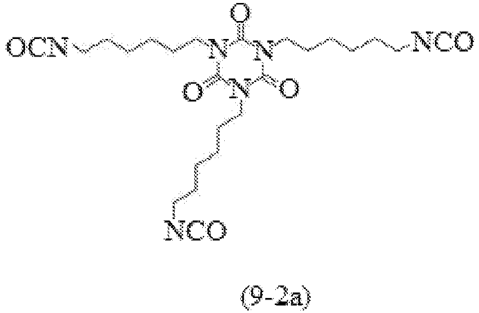

When p1011 and s1011 are 0, preferable examples of the compound (10-1) include compounds of the following general formula (10-1-1), and compounds of the following general formula (10-1-2).

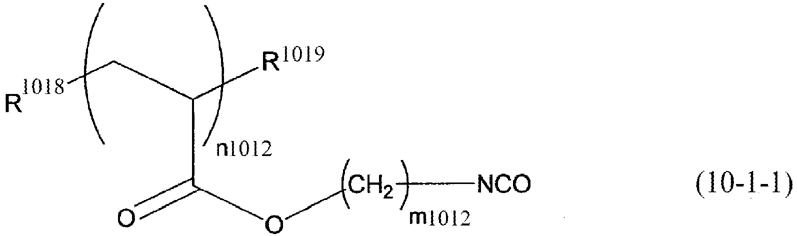

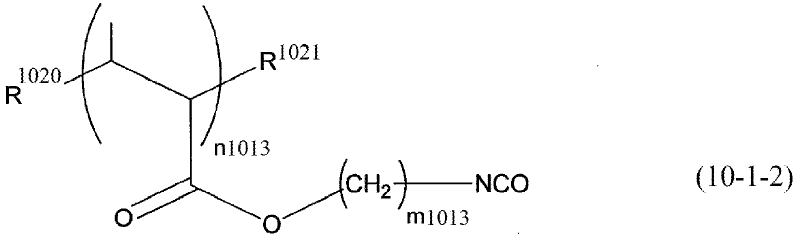

In the general formula (10-1-1), n1012 is an integer of 10 to 100, preferably an integer of 10 to 90, more preferably an integer of 20 to 80, and even more preferably an integer of 30 to 70. m1012 is the same as m101 mentioned above, and is preferably an integer of 1 to 3, and more preferably 1or 2. R¹⁰¹⁸ and R¹⁰¹⁹ are each the same as R¹⁰⁴ and R¹⁰⁵ mentioned above.

In the general formula (10-1-2), n1013 is an integer of 10 to 100, preferably an integer of 10 to 90, more preferably an integer of 20 to 80, and even more preferably an integer of 30 to 70. m1013 is the same as m101 mentioned above, and is preferably an integer of 1 to 3, and more preferably 1 or 2. R¹⁰²⁰ and R¹⁰²¹ are each the same as R¹⁰⁴ and R¹⁰⁵ mentioned above.

When R¹⁰¹⁴ is a methoxycarbonyl group and s1011 is 0, preferable examples of the compound (10-1) include compounds of the following general formula (10-1-3), compounds of the following general formula (10-1-4), and compounds of the following general formula (10-1-5).

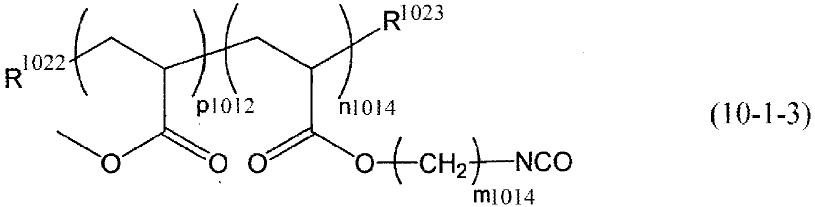

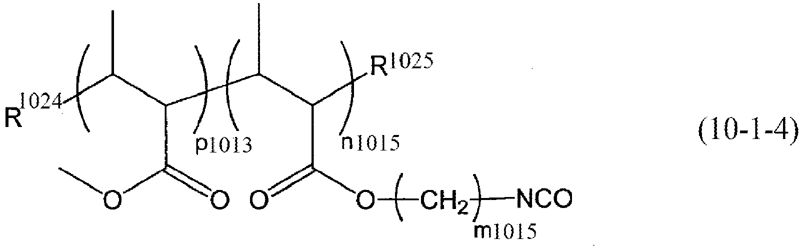

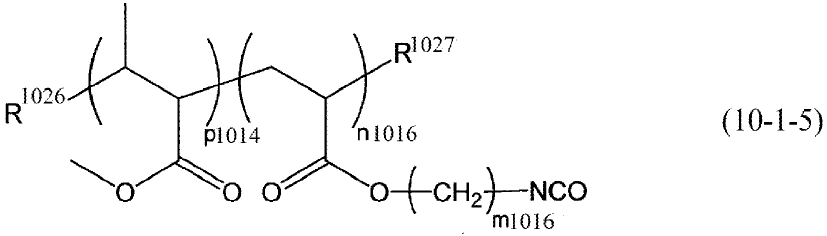

In the general formula (10-1-3), p1012 is an integer of 1 to 90. n1014 is an integer of 1 to 99. The sum of p1012 and n1014 is an integer of 10 to 100. m1014 is an integer of 1 to 5. R¹⁰²² and R¹⁰²³ are each the same as R¹⁰⁴ and R¹⁰⁵ mentioned above.

In the general formula (10-1-4), p1013 is an integer of 1 to 90. n1015 is an integer of 1 to 99. The sum of p1013 and n1015 is an integer of 10 to 100. m1015 is an integer of 1 to 5. R¹⁰²⁴ and R¹⁰²⁵ are each the same as R¹⁰⁴ and R¹⁰⁵ mentioned above.

In the general formula (10-1-5), p1014 is an integer of 1 to 90. n1016 is an integer of 1 to 99. The sum of p1014 and n1016 is an integer of 10 to 100. m1016 is an integer of 1 to 5. R¹⁰²⁶ and R¹⁰²⁷ are each the same as R¹⁰⁴ and R¹⁰⁵ mentioned above.

When R¹⁰¹⁵ is a phenyl group and p1011 is 0, preferable examples of the compound (10-1) include compounds of the following general formula (10-1-6), compounds of the following general formula (10-1-7), and compounds of the following general formula (10-1-8).

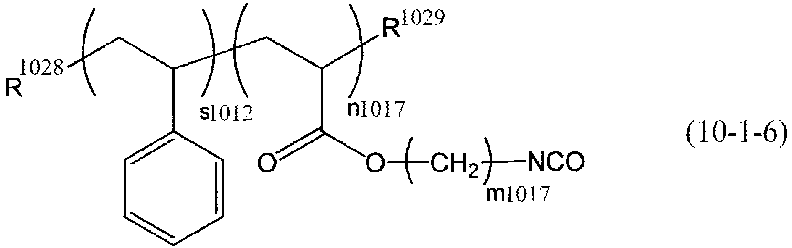

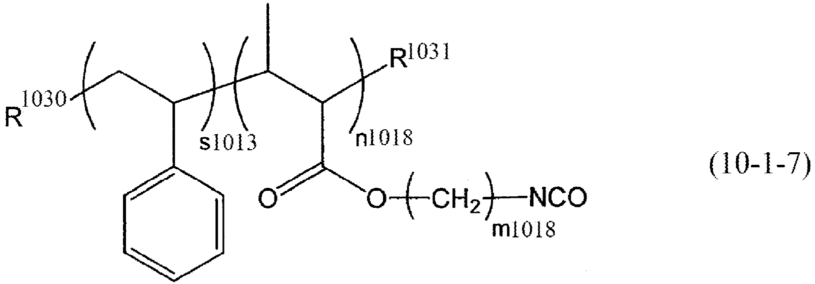

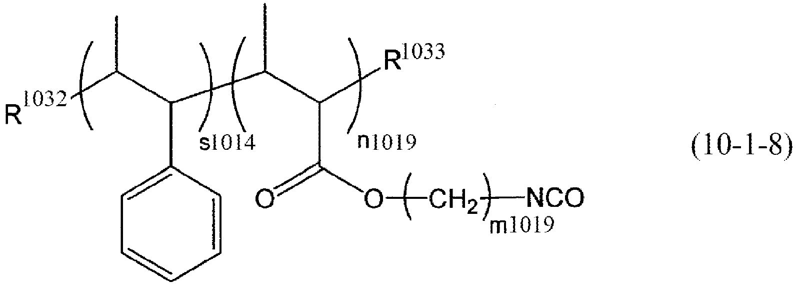

In the general formula (10-1-6), s1012 is an integer of 1 to 90. n1017 is an integer of 1 to 99. The sum of s1012 and n1017 is an integer of 10 to 100. m1017 is an integer of 1 to 5. R¹⁰²⁸ and R¹⁰²⁹ are each the same as R¹⁰⁴ and R¹⁰⁵ mentioned above.

In the general formula (10-1-7), s1013 is an integer of 1 to 90. n1018 is an integer of 1 to 99. The sum of s1013 and n1018 is an integer of 10 to 100. m1018 is an integer of 1 to 5. R¹⁰³⁰ and R¹⁰³¹ are each the same as R¹⁰⁴ and R¹⁰⁵ mentioned above.

In the general formula (10-1-8), s1014 is an integer of 1 to 90. n1019 is an integer of 1 to 99. The sum of s1014 and n1019 is an integer of 10 to 100. m1019 is an integer of 1 to 5. R¹⁰³² and R¹⁰³³ are each the same as R¹⁰⁴ and R¹⁰⁵ mentioned above.

When R¹⁰¹⁴ is a methoxycarbonyl group and R¹⁰¹⁵ is a phenyl group, preferable examples of the compound (10-1) include compounds of the following general formula (10-1-9a), compounds of the following general formula (10-1-9b), and compounds of the following general formula (10-1-9c).

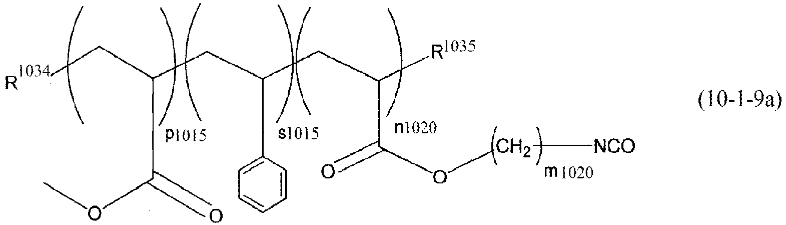

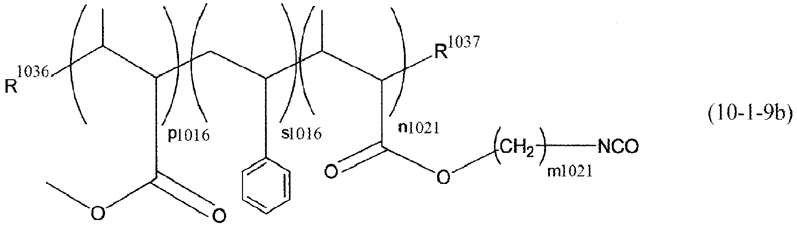

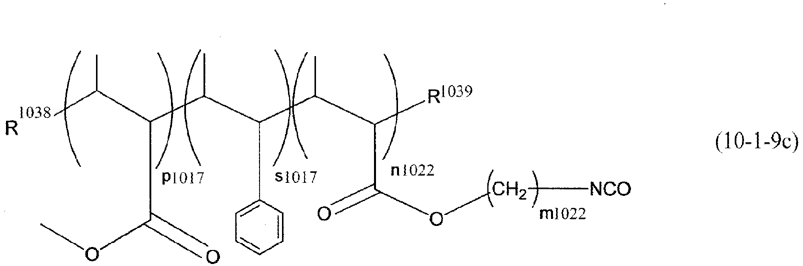

In the general formula (10-1-9a), p1015 is an integer of 1 to 50. s1015 is an integer of 1 to 50. n1020 is an integer of 1 to 98. The sum of p1015, s1015 and n1020 is an integer of 10 to 100. m1020 is an integer of 1 to 5. R¹⁰³⁴ and R¹⁰³⁵ are each the same as R¹⁰⁴ and R¹⁰⁵ mentioned above.

In the general formula (10-1-9b), p1016 is an integer of 1 to 50. s1016 is an integer of 1 to 50. n1021 is an integer of 1 to 98. The sum of p1016, s1016 and n1021 is an integer of 10 to 100. m1021 is an integer of 1 to 5. R¹⁰³⁶ and R¹⁰³⁷ are each the same as R¹⁰⁴ and R¹⁰⁵ mentioned above.

In the general formula (10-1-9c), p1017 is an integer of 1 to 50. s1017 is an integer of 1 to 50. n1022 is an integer of 1 to 98. The sum of p1017, s1017 and n1022 is an integer of 10 to 100. m1022 is an integer of 1 to 5. R¹⁰³⁸ and R¹⁰³⁹ are each the same as R¹⁰⁴ and R¹⁰⁵ mentioned above.

Alternatively, the compound (A) is preferably at least one selected from C9-35 chained or cyclic aliphatic hydrocarbons.

The carbon number of the chained aliphatic hydrocarbon is preferably 12 to 35, more preferably 12 to 30, and even more preferably 14 to 30. The chained aliphatic hydrocarbon may be a saturated aliphatic hydrocarbon or an unsaturated aliphatic hydrocarbon. Although the chained aliphatic hydrocarbon may be linear or branched, the chained aliphatic hydrocarbon preferably has a branched chain, and more preferably a branched chain composed of a C1-3 linear aliphatic hydrocarbon group. Examples of the C1-3 linear aliphatic hydrocarbon group include a methyl group, an ethyl group, and a propyl group.

Specific examples of the chained aliphatic hydrocarbon include 2,2,4,4,6-pentamethylheptane, 2-methyl-1-undecene, dodecane, 1-tridecene, tridecane, 1-tetradecene, tetradecane, 1-pentadecene, pentadecane, 1-hexadecene, hexadecane, 2,2,4,4,6,8,8-pentamethylnonane, 1-heptadecene, heptadecane, 1-octadecene, octadecane, 1-nonadecene, 2,6,10,1,4-tetramethylpentadecane, 1-eicosene, eicosane, heneicosane, docosane, tricosane, tetracosane, pentacosane, hexacosane, heptacosane, octacosane, nonacosane, squalene, and squalane, and squalane is preferable.

The cyclic aliphatic hydrocarbon (that is, alicyclic hydrocarbon) may be monocyclic, polycyclic, or condensed polycyclic. The carbon number of the alicyclic hydrocarbon is preferably 9 to 20, and more preferably 9 to 16. The alicyclic hydrocarbon may be formed by bonding a chained aliphatic hydrocarbon group to a ring, and a C1-6 linear aliphatic hydrocarbon group is preferable as the chained aliphatic hydrocarbon group bonded to a ring. Examples of the C1-6 linear aliphatic hydrocarbon group include a methyl group, an ethyl group, a propyl group, a butyl group, a pentyl group, and a hexyl group.

Specific examples of the alicyclic hydrocarbon include butylcyclohexane, 1-2,4-trimethylcyclohexane, decahydronaphthalene, adamantane, tricyclodecane, methyldecarine, tricyclo[6.2.1.02,7]undeca-4-ene, tetracyclododecane, bicyclohexyl, dicyclopentadiene, α-pinene, and β-pinene.

Among them, the compound (A) is preferably at least one selected from the group consisting of the compound of the formula (5-1-3a), the compound of the formula (5-1-3b), the compound of the formula (6-2-1a) and the compound of the formula (9-2a), more preferably the compound of the formula (5-1-3a) or the compound of the formula (5-1-3b), and even more preferably the compound of the formula (5-1-3a).

### [Carbonic acid ester]

Preferable examples of a carbonic acid ester available to prepare a carbamate include compounds of the following general formula (3) (hereinafter, may be referred to as "compounds (3)").

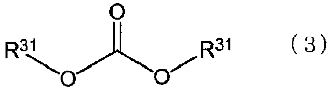

In the general formula (3), plural R³¹ are each independently a C1-20 aliphatic hydrocarbon group or a C6-20 aromatic hydrocarbon group. The plural R³¹ may be identical to or different from each other. Among them, the plural R³¹ are preferably identical to each other.

### (R³¹)

Examples of the C1-20 aliphatic hydrocarbon group and the C6-20 aromatic hydrocarbon group as R³¹ include the same groups as R²² mentioned above.

Preferable examples of the compound (3) include diaryl carbonates of the following general formula (3 - 1) (hereinafter, may be referred to as "diaryl carbonates (3-1)").

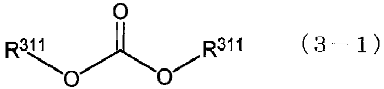

In the general formula (3-1), plural R³¹¹ are each independently a C6-20 aromatic hydrocarbon group.

### (R³¹¹)

In the general formula (3-1), R³¹¹ is a C6-20 aromatic hydrocarbon group, preferably a C6-12 aromatic hydrocarbon group, and more preferably a C6-8 aromatic hydrocarbon group. Specific examples of such R⁸¹¹ include the C6-20 aromatic hydrocarbon groups mentioned as R²² above.

Preferable examples of the diaryl carbonate (3-1) include diaryl carbonates in which R³¹¹ is a C6-8 aromatic hydrocarbon group. Specific examples of such a diaryl carbonate (3-1) include diphenyl carbonate, di(methylphenyl)carbonate (each isomer), di(diethylphenyl)carbonate (each isomer), and di(methylethylphenyl)carbonate (each isomer).

The carbonic acid ester may contain a metal atom. The amount of the metal atom relative to the mass of the carbonic acid ester is preferably 0.001 ppm by mass to 100,000 ppm by mass, more preferably 0.001 ppm by mass to 50,000 ppm by mass, and even more preferably 0.002 ppm by mass to 30,000 ppm by mass.

The metal atom may be present as a metal ion or a simple substance of the metal atom. Among them, the metal atom is preferably a divalent to tetravalent metal atom, and more preferably at least one metal selected from the group consisting of iron, cobalt, nickel, zinc, tin, copper and titanium.

As the preparation method of the carbonic acid ester, a conventionally-known method may be adopted. Particularly, a method disclosed in International Patent Application Publication No. 2009/139061 (Reference Document 1) in which an organic tin compound having a tin-oxygen-carbon bond and carbon dioxide are reacted to prepare an aliphatic carbonic acid ester, and then an aromatic carbonic acid ester (that is, diaryl carbonate) is prepared from the aliphatic carbonic acid ester and an aromatic hydroxy compound is preferable. The carbonic acid ester may be prepared using a preparation device disclosed in International Patent Application Publication No. 2009/139061 (Reference Document 1), for example.

### [Amine compound]

An amine compound in which an amino group is present instead of a carbamate group of a carbamate to be subjected to thermal decomposition is used to prepare the carbamate. Namely, an amine compound in which an amino group (-NH₂) is present instead of a carbamate group of the carbamate of the general formula (2), the carbamate of the general formula (2-1a), the carbamate of the general formula (2-1b), the carbamate of the general formula (2-2a), the carbamate of the general formula (2-2b), the carbamate of the general formula (2-2c), the carbamate of the general formula (2-2d), the carbamate of the general formula (2-2e), the carbamate of the general formula (2-3a), the carbamate of the general formula (2-3b), the carbamate of the general formula (2-3c), the carbamate of the general formula (2-4a) or the carbamate of the general formula (2-4b) is preferable.

### [Isocyanate]

An isocyanate obtained in the preparation method according to the present embodiment is formed by substituting a carbamate group of the carbamate subjected to thermal decomposition with an isocyanate group. Among them, an isocyanate in which an isocyanate group (-NCO) is present instead of a carbamate group of the carbamate of the general formula (2), the carbamate of the general formula (2-1a), the carbamate of the general formula (2-1b), the carbamate of the general formula (2-2a), the carbamate of the general formula (2-2b), the carbamate of the general formula (2-2c), the carbamate of the general formula (2-2d), the carbamate of the general formula (2-2e), the carbamate of the general formula (2-3a), the carbamate of the general formula (2-3b), the carbamate of the general formula (2-3c), the carbamate of the general formula (2-4a), or the carbamate of the general formula (2-4b) is preferable. Namely, a compound of the following general formula (2)', a compound of the following general formula (2-1a)', a compound of the following general formula (2-1b)', a compound of the following general formula (2-2a)', a compound of the following general formula (2-2b)', a compound of the following general formula (2-2c)', a compound of the following general formula (2-2d)', a compound of the following general formula (2-2e)', a compound of the following general formula (2-3a)', a compound of the following general formula (2-3b)', a compound of the following general formula (2-3c)', a compound of the following general formula (2-4a)' or a compound of the following general formula (2-4b)' is preferable.

R²¹-(NCO)ₙ₂₁ (2)'

R²¹¹-NCO (2-1a)'

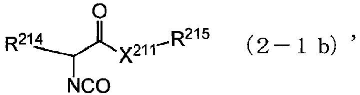

R²²¹-(NCO)₂ (2-2a)'

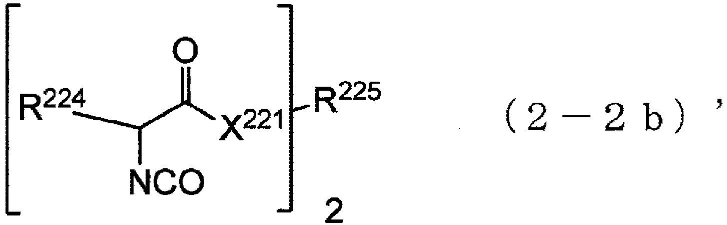

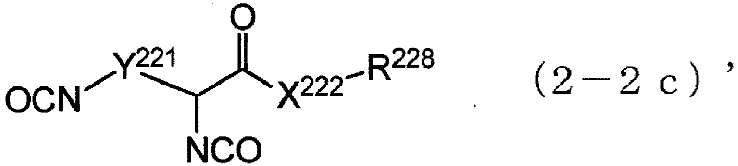

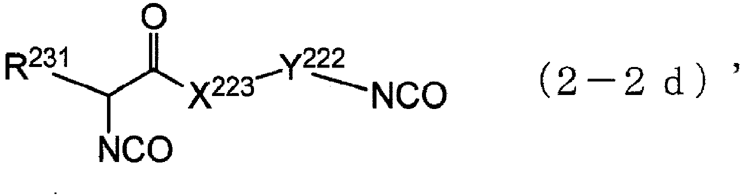

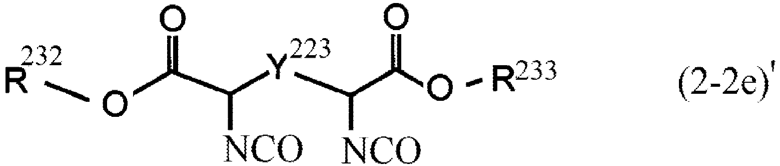

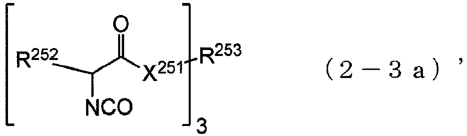

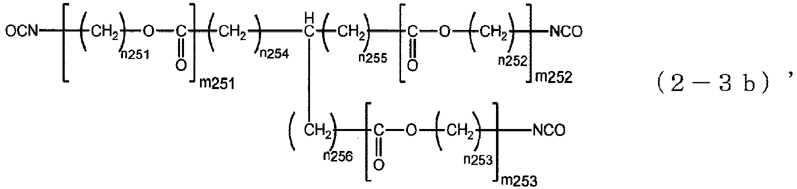

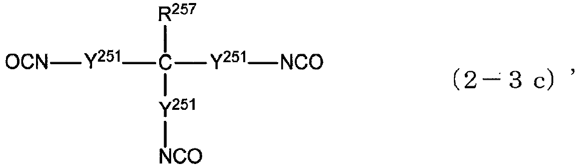

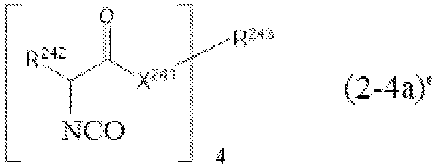

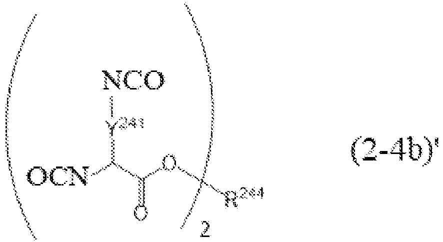

In the general formula (2)', n21 and R²¹ are the same as n21 and R²¹ in the general formula (2), respectively.

In the general formula (2-1a)', R²¹¹ is the same as R²¹¹ in the general formula (2-1a).

In the general formula (2-1b)', X²¹¹, R²¹⁴ and R²¹⁵ are the same as X²¹¹, R²¹⁴ and R²¹⁵ in the general formula (2-1b), respectively.

In the general formula (2-2a)', R²²¹ is the same as R²²¹ in the general formula (2-2a).

In the general formula (2-2b)', X²²¹, R²²⁴ and R²²⁵ are the same as X²²¹, R²²⁴ and R²²⁵ in the general formula (2-2b), respectively.

In the general formula (2-2c)', X²²², Y²²¹ and R²²⁸ are the same as X²²², Y²²¹ and R²²⁸ in the general formula (2-2c), respectively.

In the general formula (2-2d)', X²²³, Y²²² and R²³¹ are the same as X²²³, Y²²² and R²³¹ in the general formula (2-2d), respectively.

In the general formula (2-2e)', Y²²³, R²³² and R²³³ are the same as Y²²³, R²³² and R²³³ in the general formula (2-2e), respectively.

In the general formula (2-3a)', X²⁵¹, R²⁵² and R²⁵³ are the same as X²⁵¹, R²⁵² and R²⁵³ in the general formula (2-3a), respectively.

In the general formula general formula (2-3b)', n251, n252, n253, n254, n255, n256, m251, m252 and m253 are the same as n251, n252, n253, n254, n255, n256, m251, m252 and m253 in the general formula (2-3b), respectively.

In the general formula (2-3c)', R²⁵⁷ and plural Y²⁵¹ are the same as R²⁵⁷ and Y²⁵¹ in the general formula (2-3c), respectively.

In the general formula (2-4a)', X²⁴¹, R²⁴² and R²⁴³ are the same as X²⁴¹, R²⁴² and R²⁴³ in the general formula (2-4a), respectively.

In the general formula (2-4b)', Y²⁴¹ and R²⁴⁴ are the same as Y²⁴¹ and R²⁴⁴ in the general formula (2-4b), respectively.

### Examples

Hereinafter, the present embodiment will be described in more detail with reference to specific examples and comparative examples, but the present embodiment is not limited to the following examples provided that the gist of the present embodiment is not exceeded.

### Example 1-1

### 1. Preparation of mixture liquid

30 kg of 3-(phenoxycarbonylamino-methyl)-3,5,5-trimethylcyclohexylcarbamic acid phenyl ester, 40 kg of benzophenone and 30 kg of a phenolic novolac resin were mixed in a stirring tank heated at 120°C under nitrogen at atmospheric pressure to obtain a uniform mixture liquid.

### 2. Thermal decomposition of carbamate

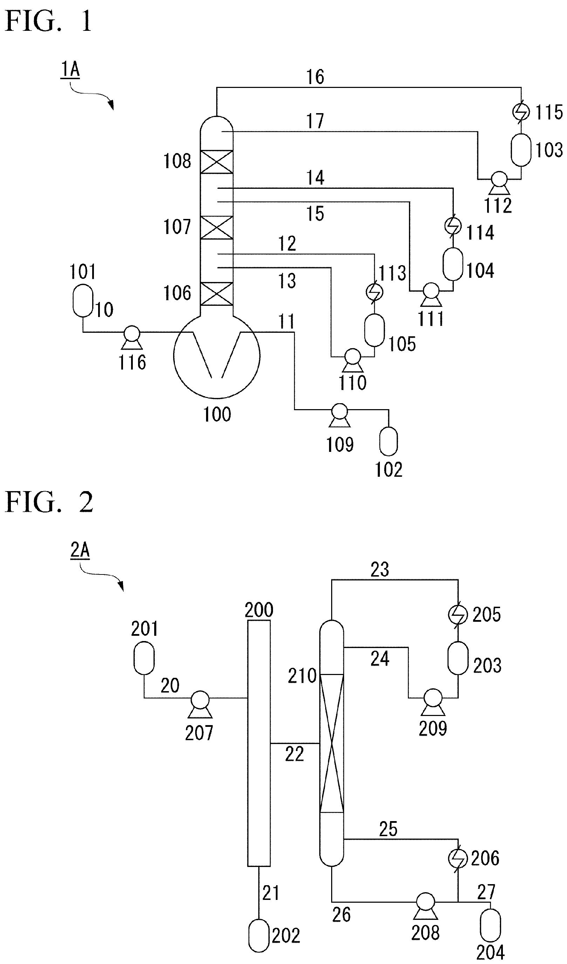

The mixture liquid prepared in the step 1 was charged in a storage tank 101 of an isocyanate preparation device 1A shown in Fig. 1. A mixture liquid composed of a phenolic novolac resin and benzophenone was charged into a reactor 100 equipped with a heat medium jacket to form a state in which the benzophenone was refluxed through a line 16, a condenser 115, a storage tank 103, a liquid feed pump 112, and a line 17 provided on the upper part of a packed bed 108 while maintaining the temperature of the heat medium passing through the heat medium jacket at 270°C and adjusting the internal pressure.

The mixture liquid was supplied from the storage tank 101 to the reactor 100 via a line 10 and a liquid feed pump 116 at 1 kg/hr to allow thermal decomposition of 3-(phenoxycarbonylamino-methyl)-3,5,5-trimethylcyclohexyl carbamic acid phenyl ester to proceed. A mixture liquid containing phenol produced by thermal decomposition and benzophenone was collected in the storage tank 103 via the line 16 and the condenser 115 provided on the upper part of the packed bed 108. In contrast, a mixture liquid containing isophorone diisocyanate produced by thermal decomposition, benzophenone, and the phenolic novolac resin was collected in a storage tank 104 via a line 14 and a condenser 114 provided on the upper part of a packed bed 107 to form a reflux state through a liquid feed pump 111 and a line15. The mixed liquid containing benzophenone as the main component was collected in a storage tank 105 via a line 12 and a condenser 113 provided on the upper part of a packed bed 106 during operation and a reflux state was formed via a pump 110 and a line 13. Furthermore, the reaction liquid was extracted from the bottom of the reactor 100 via a line 11 to be collected in a storage tank 102 via a liquid feed pump 109 such that the liquid surface inside the reactor 100 was constant. The yield of isophorone diisocyanate collected in the storage tank 104 was 69%. The above operation could be continuously performed for 200 hours.

### Example 1-2

### 1. Preparation of mixture liquid

20 kg of the compound of the formula (2-1b-2) (compound (2-1b-2)), 30 kg of p-xylene and 50 kg of 2,5-di-tert-butylhydroquinone were mixed in a stirring tank heated at 120°C under nitrogen at atmospheric pressure to obtain a uniform mixture liquid.

### 2. Thermal decomposition of carbamate

The mixture liquid prepared in the step 1 was charged in a storage tank 201 of an isocyanate preparation device 2A shown in Fig. 2. A p-xylene was charged into a distillation column 210 to form a state in which p-xylene was refluxed via a line 23, a condenser 205, a storage tank 203, a liquid feed pump 209, and a line 24 provided in the upper part of the distillation column 210 while maintaining the temperature of a reboiler 206 at 200°C and adjusting the internal pressure.

The mixture liquid was supplied from the storage tank 201 to a falling film type reactor 200 preheated at 250°C via a line 20 and a liquid feed pump 207 at 1 kg/hr to allow thermal decomposition of the compound (2-1b-2) to proceed. Gaseous components containing ethanol and methyl 2-isocyanatopropionate and p-xylene produced by thermal decomposition were supplied to the distillation column 210 via a line 22. In contrast, 2,5-di-tert-butylhydroquinone containing a by-product was collected in a storage tank 202 from the bottom of the falling film type reactor via a line 21. The gaseous components collected via the line 22 were separated by distillation in the distillation column 210 to collect a mixture liquid containing ethanol and p-xylene in a storage tank 203 via a line 23 and a condenser 205. In contrast, a mixture liquid containing methyl 2-isocyanatopropionate, p-xylene and a small amount of 2,5-di-tert-butylhydroquinone was collected in a storage tank 204 from the bottom of the distillation column 210 via a line 26, a liquid feed pump 208, and a line 27, and a portion of the liquid extracted from the bottom of the column was heated by a reboiler 206 and returned to the bottom of the column via a line 25. The yield of methyl 2-isocyanatopropionate collected in the storage tank 204 was 92%. The above operation could be continuously performed for 200 hours.

### Comparative Example 1-1

### 1. Preparation of mixture liquid

60 kg of 3-(phenoxycarbonylamino-methyl)-3,5,5-trimethylcyclohexylcarbamic acid phenyl ester and 80 kg of benzophenone were mixed in a stirring tank heated at 120°C under nitrogen at atmospheric pressure to obtain a uniform mixture liquid.

### 2. Thermal decomposition of carbamate

A mixture liquid containing isophorone diisocyanate produced by thermal decomposition and benzophenone was collected in a storage tank 104 by conducting thermal decomposition by the same method as that in the step 2 "Thermal decomposition of carbamate" in Example 1-1 except that the mixture liquid prepared in the step 1 was charged in a storage tank 101 of the isocyanate preparation device 1A shown in Fig. 1, benzophenone was charged in a reactor 100 equipped with a heat medium jacket to form a state in which benzophenone was refluxed, and the mixture liquid was supplied from the storage tank 101 via a line 10 to the reactor 100 at 0.4 kg/hr. The yield of isophorone diisocyanate collected in the storage tank 104 was 15%. When the above operation was continued for 2 days, the line 11 was blocked and the continuation of the operation became difficult.

### Comparative Example 1-2

### 1. Preparation of mixture liquid

40 kg of the compound (2-1b-2) and 60 kg of p-xylene were mixed in a stirring tank heated at 120°C under nitrogen at atmospheric pressure to obtain a uniform mixture liquid.

### 2. Thermal decomposition of carbamate

A mixture liquid containing methyl 2-isocyanatopropionate and p-xylene was collected in a storage tank 204 via a line 27 by conducting thermal decomposition by the same method as that in the step 2 "Thermal decomposition of carbamate" in Example 1-2 except that the mixture liquid prepared in the step 1 was charged in a storage tank 201 of the isocyanate preparation device 2A shown in Fig. 2, p-xylene was charged in a distillation column 210 to form a state in which p-xylene was refluxed, and the mixture liquid was supplied from the storage tank 201 via a line 20 to a falling film type reactor 200 preheated at 250°C at 0.3 kg/hr to allow a thermal decomposition of the compound (2-1b-2) to proceed. The yield of methyl 2-isocyanatopropionate collected in the storage tank 204 was 28%. When the above operation was continued for 2 days, the line 21 was blocked and the continuation of the operation became difficult.

### Example 2-1

### 1. Preparation of mixture liquid

30 kg of 1,6-hexamethylene di(carbamic acid phenyl ester) (carbamate), 10 kg of n-dodecane (inert solvent) and 60 kg of diethyl phthalate (compound A) were mixed in a stirring tank heated at 120°C under nitrogen at atmospheric pressure to obtain a uniform mixture liquid.

### 2. Thermal decomposition of carbamate

The mixture liquid prepared in the step 1 was charged in a storage tank 101 of the isocyanate preparation device 1A shown in Fig. 1. Diethyl phthalate and n-dodecane were charged in a reactor 100 equipped with a heat medium jacket to form a state in which n-dodecane was refluxed via a line 16, a condenser 115, a storage tank 103 and a line 17 provided on the upper part of a packed bed 108 while maintaining the temperature of a heat medium passing through the heat medium jacket at 270°C and adjusting the internal pressure.

The mixture liquid was supplied to the reactor 100 from a storage tank 101 via a line 10 at 1 kg/hr to allow a thermal decomposition of 1,6-hexamethylene di(carbamic acid phenyl ester) to proceed. A mixture liquid containing phenol produced by thermal decomposition and n-dodecane was collected in the storage tank 103 via a line 16 and a condenser 115 provided on the upper part of the packed bed 108. In contrast, a mixture liquid containing hexamethylene diisocyanate produced by thermal decomposition, n-dodecane and diethyl phthalate was collected in a storage tank 104 via a line 14 and a condenser 114 provided on the upper part of a packed bed 107. Furthermore, the reaction liquid was extracted from the bottom of the reactor 100 via a line 11 to be collected in a storage tank 102 such that the liquid surface inside the reactor 100 was constant. The yield of hexamethylene diisocyanate collected in the storage tank 104 was 58%. The above operation could be continuously performed for 200 hours.

### Example 2-2

### 1. Preparation of mixture liquid

10 kg of 1,6-hexamethylene di(carbamic acid phenyl ester) (carbamate), 20 kg of triethylbenzene (inert solvent) and 70 kg of tris(2-ethylhexyl) trimellitate were mixed in a stirring tank heated at 120°C under nitrogen at atmospheric pressure to obtain a uniform mixture liquid.

### 2. Thermal decomposition of carbamate

The mixture liquid prepared in the step 1 was charged in a storage tank 201 of the isocyanate preparation device 2A shown in Fig. 2. Triethylbenzene was charged in a distillation column 210 to form a state in which triethylbenzene was refluxed via a line 23, a condenser 205, a storage tank 203 and a line 24 provided on the upper part of the distillation column 210 while maintaining the temperature of a reboiler 206 at 200°C and adjusting the internal pressure.

The mixture liquid was supplied at 1 kg/hr from the storage tank 201 via a line 20 to a falling film type reactor 200 preheated at 250°C to allow a thermal decomposition of 1,6-hexamethylene di(carbamic acid phenyl ester) to proceed. Gaseous components containing phenol, hexamethylene diisocyanate and triethylbenzene produced by thermal decomposition were supplied to the distillation column 210 via a line 22. In contrast, tris(2-ethylhexyl) trimellitate containing a by-product was collected from the bottom of the falling film type reactor via a line 21 in a storage tank 202. The gaseous components collected via the line 22 were separated by distillation in the distillation column 210, and the mixture liquid containing phenol and triethylbenzene was collected in a storage tank 203 via a line 23 and a condenser 205. In contrast, the mixture liquid containing hexamethylene diisocyanate, triethylbenzene and a small amount of tris(2-ethylhexyl) trimellitate was collected via a line 27 in a storage tank 204. The yield of hexamethylene diisocyanate collected in the storage tank 204 was 88%. The above operation could be continuously performed for 200 hours.

### Example 2-3

### 1. Preparation of mixture liquid

10 kg of the compound (2-1b-2) (carbamate), 20 kg of triethylbenzene (inert solvent) and 70 kg of tris(2-ethylhexyl) trimellitate (compound A) were mixed in a stirring tank heated at 120°C under nitrogen at atmospheric pressure to obtain a uniform mixture liquid.

### 2. Thermal decomposition of carbamate

The mixture liquid prepared in the step 1 was charged in a storage tank 201 of the isocyanate preparation device 2A shown in Fig. 2. Triethylbenzene was charged in a distillation column 210 to form a state in which triethylbenzene was refluxed via a line 23, a condenser 205, a storage tank 203 and a line 24 provided on the upper part of the distillation column 210 while maintaining the temperature of a reboiler 206 at 200°C and adjusting the internal pressure.

The mixture liquid was supplied at 1 kg/hr from the storage tank 201 via a line 20 to a falling film type reactor 200 preheated at 250°C to allow a thermal decomposition of the compound (2-1b-2) to proceed. Gaseous components containing phenol, methyl 2-isocyanatopropionate and triethylbenzene produced by thermal decomposition were supplied to the distillation column 210 via a line 22. In contrast, tris(2-ethylhexyl) trimellitate containing a by-product was collected from the bottom of the falling film type reactor via a line 21 in a storage tank 202. The gaseous components collected via the line 22 were separated by distillation in the distillation column 210, and a mixture liquid containing phenol and triethylbenzene was collected in the storage tank 203 via the line 23 and condenser 205. In contrast, a mixture liquid containing methyl 2-isocyanatopropionate, triethylbenzene and a small amount of tris(2-ethylhexyl) trimellitate was collected in a storage tank 204 via a line 27. The yield of methyl 2-isocyanatopropionate collected in the storage tank 204 was 88%. The above operation could be continuously performed for 200 hours.

### Comparative Example 2-1

### 1. Preparation of mixture liquid

60 kg of 1,6-hexamethylene di(carbamic acid phenyl ester) and 30 kg of n-dodecane were mixed in a stirring tank heated at 120°C under nitrogen at atmospheric pressure to obtain a uniform mixture liquid.

### 2. Thermal decomposition of carbamate

A mixture liquid containing hexamethylene diisocyanate produced by thermal decomposition and n-dodecane was collected in a storage tank 104 by conducting thermal decomposition by the same method as that in the step 2 "Thermal decomposition of carbamate" in Example 2-1 except that the mixture liquid prepared in the step 1 was charged in a storage tank 101 of the isocyanate preparation device 1A shown in Fig. 1, n-dodecane was charged in a reactor 100 equipped with a heat medium jacket to form a state in which n-dodecane was refluxed, and the mixture liquid was supplied at 0.4 kg/hr from the storage tank 101 via a line 10 to the reactor 100. The yield of hexamethylene diisocyanate collected in the storage tank 104 was 18%. When the above operation was continued for 2 days, the line 11 was blocked and the continuation of the operation became difficult.

### Comparative Example 2-2

### 1. Preparation of mixture liquid

10 kg of 1,6-hexamethylene di(carbamic acid phenyl ester) and 20 kg of triethylbenzene were mixed in a stirring tank heated at 120°C under nitrogen at atmospheric pressure to obtain a uniform mixture liquid.

### 2. Thermal decomposition of carbamate

A mixture liquid containing hexamethylene diisocyanate and triethylbenzene was collected in a storage tank 204 via a line 27 by conducting thermal decomposition by the same method as that in the step 2 "Thermal decomposition of carbamate" in Example 2-2 except that the mixture liquid prepared in the step 1 was charged in a storage tank 201 of the isocyanate preparation device 2A shown in Fig. 2, triethylbenzene was charged in a distillation column 210 to form a state in which triethylbenzene was refluxed, and the mixture liquid was supplied at 0.3 kg/hr from the storage tank 201 via a line 20 to a falling film type reactor 200 preheated at 250°C to allow a thermal decomposition of 1,6-hexamethylene di(carbamic acid phenyl ester) to proceed. The yield of hexamethylene diisocyanate collected in the storage tank 204 was 23%. When the above operation was continued for 2 days, the line 21 was blocked and the continuation of the operation became difficult.

### Comparative Example 2-3

### 1. Preparation of mixture liquid

30 kg of the compound (2-1b-2) and 60 kg of triethylbenzene were mixed in a stirring tank heated at 120°C under nitrogen at atmospheric pressure to obtain a uniform mixture liquid.

### 2. Thermal decomposition of carbamate

A mixture liquid containing methyl 2-isocyanatopropionate and triethylbenzene was collected in a storage tank 204 via a line 27 by conducting thermal decomposition by the same method as that in the step 2 "Thermal decomposition of carbamate" in Example 2-2 except that the mixture liquid prepared in the step 1 was charged in a storage tank 201 of the isocyanate preparation device 2A shown in Fig. 2, triethylbenzene was charged in a distillation column 210 to form a state in which triethylbenzene was refluxed, and the mixture liquid was supplied at 0.3 kg/hr from the storage tank 201 via a line 20 to a falling film type reactor 200 preheated at 250°C to allow a thermal decomposition of the compound of the formula (E-3) to proceed. The yield of methyl 2-isocyanatopropionate collected in the storage tank 204 was 20%. When the above operation was continued for 2 days, the line 21 was blocked and the continuation of the operation became difficult.

### Example 3-1

### (Step of preparing mixture liquid)

20 kg of 3-(phenoxycarbonylamino-methyl)-3,5,5-trimethylcyclohexylcarbamic acid phenyl ester (carbamate), 20 kg of triethylbenzene (inert solvent) and 60 kg of 1,3-bis(α-hydroxyisopropyl)benzene (compound A) were mixed in a stirring tank heated at 120°C under nitrogen at atmospheric pressure to obtain a uniform solution.

### (Thermal decomposition of carbamate)

The mixture liquid obtained in the "step of preparing mixture liquid" was charged in a storage tank 101 of the isocyanate preparation device 1A shown in Fig. 1. 1,3-bis(α-hydroxyisopropyl)benzene and triethylbenzene were charged in a reactor 100 equipped with a heat medium jacket to form a state in which triethylbenzene was refluxed via a line 16, a condenser 115, a storage tank 103, and a line 17 provided on the upper part of a packed bed 108 while maintaining the temperature of a heat medium passing through the heat medium jacket at 270°C and adjusting the internal pressure.

The mixture liquid was supplied at 1 kg/hr from the storage tank 101 via a line 10 to the reactor 100 to allow a thermal decomposition of 3-(phenoxycarbonylamino-methyl)-3,5,5-trimethylcyclohexyl carbamic acid phenyl ester to proceed. A mixture liquid containing phenol produced by thermal decomposition and triethylbenzene was collected in a storage tank 103 via a line 16 and a condenser 115 provided on the upper part of a packed bed 108, and a mixture liquid containing isophorone diisocyanate produced by thermal decomposition and triethylbenzene was collected in a storage tank 104 via a line 14 and a condenser 114 provided on the upper part of a packed bed 107. In contrast, the reaction liquid was extracted from the bottom of the reactor 100 via a line 11 to be collected in a storage tank 102 such that the liquid surface inside the reactor 100 was constant. The yield of isophorone diisocyanate collected in the storage tank 104 was 80%. The above operation could be continuously performed for 200 hours.

### Example 3-2

### (Step of preparing mixture liquid)

30 kg of N,N'-(4,4'-methanediyl-diphenyl)-dicarbamic acid diphenyl ester (carbamate), 30 kg of benzyltoluene (inert solvent) and 40 kg of triphenylmethanol (compound A) were mixed in a stirring tank heated at 150°C under nitrogen at atmospheric pressure to obtain a uniform solution.

### (Thermal decomposition of carbamate)

The mixture liquid obtained in the "step of preparing mixture liquid" was charged in a storage tank 201 of the isocyanate preparation device 2A shown in Fig. 2. Benzyltoluene was charged in a distillation column 210 to form a state in which benzyltoluene was refluxed via a line 23, a condenser 205, a storage tank 203, and a line 24 provided on the upper part of the distillation column 210 while maintaining the temperature of a reboiler 206 at 200°C and adjusting the internal pressure.

The mixture liquid was supplied at 1 kg/hr from the storage tank 201 via a line 20 to a falling film type reactor 200 preheated at 250°C to allow a thermal decomposition of N,N'-(4,4'-methanediyl-diphenyl)-dicarbamic acid diphenyl ester to proceed. Gaseous components containing phenol, 4,4'-diphenylmethane diisocyanate and benzyltoluene produced by thermal decomposition were supplied to the distillation column 210 via a line 22. In contrast, triphenylmethanol containing a by-product was collected from the bottom of the falling film type reactor via a line 21 in a storage tank 202. The gaseous components collected via the line 22 were separated by distillation in the distillation column 210, and a mixture liquid containing phenol and benzyltoluene was collected in a storage tank 203 via a line 23 and a condenser 205. In contrast, a mixture liquid containing 4,4'-diphenylmethane diisocyanate and benzyltoluene was collected in a storage tank 204 via a line 27. The yield of 4,4'-diphenylmethane diisocyanate collected in the storage tank 204 was 79%. The above operation could be continuously performed for 200 hours.

### Example 3-3

### (Step of preparing mixture liquid)

20 kg of the compound of the formula (2-1b-3) (compound (2-1b-3)) (carbamate), 20 kg of triethylbenzene (inert solvent) and 60 kg of triphenylmethanol (compound A) were mixed in a stirring tank heated at 150°C under nitrogen at atmospheric pressure to obtain a uniform solution.

### (Thermal decomposition of carbamate)

The mixture liquid obtained in the "step of preparing mixture liquid" was charged in a storage tank 201 of the isocyanate preparation device 2A shown in Fig. 2. Triethylbenzene was charged in a distillation column 210 to form a state in which triethylbenzene was refluxed via a line 23, a condenser 205, a storage tank 203, and a line 24 provided on the upper part of the distillation column 210 while maintaining the temperature of a reboiler 206 at 200°C and adjusting the internal pressure.

The mixture liquid was supplied at 1 kg/hr from the storage tank 201 via a line 20 to a falling film type reactor 200 preheated at 250°C to allow a thermal decomposition of the compound (2-1b-3) to proceed. Gas containing methyl 2-isocyanatopropionate produced by thermal decomposition and triethylbenzene was supplied to the distillation column 210 via a line 22. In contrast, triphenylmethanol containing a by-product was collected in a storage tank 202 from the bottom of the falling film type reactor via a line 21. The gaseous component collected via the line 22 was separated by distillation in the distillation column 210, and a mixture liquid containing phenol and triethylbenzene was collected in a storage tank 203 via a line 23 and a condenser 205. In contrast, a mixture liquid containing methyl 2-isocyanatopropionate and triethylbenzene was collected in a storage tank 204 via a line 27. The yield of methyl 2-isocyanatopropionate collected in the storage tank 204 was 75%. The above operation could be continuously performed for 200 hours.

### Comparative Example 3-1

### (Step of preparing mixture liquid)

50 kg of 3-(phenoxycarbonylamino-methyl)-3,5,5-trimethylcyclohexylcarbamic acid phenyl ester and 50 kg of triethylbenzene were mixed in a stirring tank heated at 120°C under nitrogen at atmospheric pressure to obtain a uniform solution.

### (Thermal decomposition of carbamate)

A mixture liquid containing isophorone diisocyanate produced by thermal decomposition and triethylbenzene was collected in a storage tank 104 by conducting "thermal decomposition of carbamate" in a manner as conducted in Example 3-1 except that the mixture liquid obtained in the "step of preparing mixture liquid" was charged in a storage tank 101 of the isocyanate preparation device 1A shown in Fig. 1, triethylbenzene was charged in a reactor 100 equipped with a heat medium jacket to form a state in which triethylbenzene was refluxed, and the mixture liquid was supplied at 0.4 kg/hr from the storage tank 101 via a line 10 to a reactor 100. The yield of isophorone diisocyanate collected in the storage tank 104 was 15%. When the above operation was continued for 2 days, the line 11 was blocked and the continuation of the operation became difficult.

### Comparative Example 3-2

### (Step of preparing mixture liquid)

30 kg of N,N'-(4,4'-methanediyl-diphenyl)-dicarbamic acid diphenyl ester and 30kg of benzyltoluene were mixed in a stirring tank heated at 150°C under nitrogen at atmospheric pressure to obtain a uniform solution.

### (Thermal decomposition of carbamate)

A mixture liquid containing 4,4'-diphenylmethane diisocyanate and benzyltoluene was collected in a storage tank 204 via a line 27 by conducting "thermal decomposition of carbamate" in a manner as conducted in Example 3-2 except that the mixture liquid obtained in the "step of preparing mixture liquid" was charged in a storage tank 201 of the isocyanate preparation device 2A shown in Fig. 2, benzyltoluene was charged in a distillation column 210 to form a state in which benzyltoluene was refluxed, and the mixture liquid was supplied at 0.3 kg/hr from the storage tank 201 via a line 20 to a falling film type reactor 200 preheated at 250°C to allow a thermal decomposition of N,N'-(4,4'-methanediyl-diphenyl)-dicarbamic acid diphenyl ester to proceed. The yield of 4,4'-diphenylmethane diisocyanate collected in the storage tank 204 was 15%. When the above operation was continued for 2 days, the line 21 was blocked and the continuation of the operation became difficult.

### Comparative Example 3-3

### (Step of preparing mixture liquid)

50 kg of the compound (2-1b-3) and 50 kg of triethylbenzene were mixed in a stirring tank heated at 150°C under nitrogen at atmospheric pressure to obtain a uniform solution.

### (Thermal decomposition of carbamate)

A mixture liquid containing methyl 2-isocyanatopropionate and triethylbenzene was collected in a storage tank 204 via a line 27 by conducting "thermal decomposition of carbamate" in a manner as conducted in Example 3-3 except that the mixture liquid obtained in the "step of preparing mixture liquid" was charged in a storage tank 201 of the isocyanate preparation device 2A shown in Fig. 2, triethylbenzene was charged in a distillation column 210 to form a state in which triethylbenzene was refluxed, and the mixture liquid was supplied at 0.3 kg/hr from the storage tank 201 via a line 20 to a falling film type reactor 200 preheated at 250°C to allow a thermal decomposition of the compound (2-1b-3) to proceed. The yield of methyl 2-isocyanatopropionate collected in the storage tank 204 was 15%. When the above operation was continued for 2 days, the line 21 was blocked and the continuation of the operation became difficult.

### Example 4-1

### 1. Preparation of mixture liquid

30 kg of 1,6-hexamethylene di(carbamic acid phenyl ester) (carbamate), 10 kg of 4-methylbenzyl chloride (inert solvent), and 60 kg of 4,4'-diphenylmethane diisocyanate (compound A) were mixed in a stirring tank heated at 120°C under nitrogen at atmospheric pressure to obtain a uniform mixture liquid.

### 2. Thermal decomposition of carbamate

The mixture liquid prepared in the step 1 was charged in a storage tank 101 of the isocyanate preparation device 1A shown in Fig. 1. 4,4'-Diphenylmethane diisocyanate and 4-methylbenzyl chloride were charged in a reactor 100 equipped with a heat medium jacket to form a state in which 4-methylbenzyl chloride was refluxed via a line 16, a condenser 115, a storage tank 103 and a line 17 provided on the upper part of a packed bed 108, while maintaining the temperature of a heat medium passing through the heat medium jacket at 270°C and adjusting the internal pressure.

The mixture liquid was supplied at 1 kg/hr from the storage tank 101 via a line 10 to the reactor 100 to allow a thermal decomposition of 1,6-hexamethylene di(carbamic acid phenyl ester) to proceed. A mixture liquid containing phenol produced by thermal decomposition and 4-methylbenzyl chloride was collected in the storage tank 103 via a line 16 and a condenser 115 provided on the upper part of a packed bed 108. In contrast, a mixture liquid containing hexamethylene diisocyanate produced by thermal decomposition and 4-methylbenzyl chloride was collected in a storage tank 104 via a line 14 and a condenser 114 provided on the upper part of a packed bed 107. Furthermore, the reaction liquid was extracted from the bottom of the reactor 100 via a line 11 to be collected in a storage tank 102 such that the liquid surface inside the reactor 100 was constant. The yield of hexamethylene diisocyanate collected in the storage tank 104 was 88%. The above operation could be continuously performed for 200 hours.

### Example 4-2

### 1. Preparation of mixture liquid

20 kg of the compound of the formula (2-1b-1) (compound (2-1b-1)) (carbamate), 20 kg of trimethylbenzene (inert solvent) and 60 kg of 4,4'-dicyclohexylmethane diisocyanate (compound A) were mixed in a stirring tank heated at 120°C under nitrogen at atmospheric pressure to obtain a uniform mixture liquid.

### 2. Thermal decomposition of carbamate

The mixture liquid prepared in the step 1 was charged in a storage tank 201 of the isocyanate preparation device 2A shown in Fig. 2. Triethylbenzene was charged in a distillation column 210 to form a state in which triethylbenzene was refluxed via a line 23, a condenser 205, a storage tank 203 and a line 24 provided on the upper part of a distillation column 210 while maintaining the temperature of a reboiler 206 at 200°C and adjusting the internal pressure.

The mixture liquid was supplied at 1 kg/hr from the storage tank 201 via a line 20 to a falling film type reactor 200 preheated at 250°C to allow a thermal decomposition of the compound (2-1b-1) to proceed. Gaseous components containing phenol, methyl 2-isocyanato-4-methylvalerate and triethylbenzene produced by thermal decomposition were supplied to a distillation column 210 via a line 22. In contrast, 4,4'-dicyclohexylmethane diisocyanate containing a by-product was collected from the bottom of the falling film type reactor via a line 21 in a storage tank 202. The gaseous components collected via the line 22 were separated by distillation in the distillation column 210 and a mixture liquid containing phenol and triethylbenzene was collected in a storage tank 203 via the line 23 and the condenser 205. In contrast, a mixture liquid containing methyl 2-isocyanato-4-methylvalerate, triethylbenzene and a small amount of 4,4'-dicyclohexylmethane diisocyanate was collected in a storage tank 204 via a line 27. The yield of methyl 2-isocyanato-4-methylvalerate collected in the storage tank 204 was 88%. The above operation could be continuously performed for 200 hours.

### Comparative Example 4-1

### 1. Preparation of mixture liquid

60 kg of 1,6-hexamethylene di(carbamic acid phenyl ester) and 20 kg of 4-methylbenzyl chloride were mixed in a stirring tank heated at 120°C under nitrogen at atmospheric pressure to obtain a uniform mixture liquid.

### 2. Thermal decomposition of carbamate

A mixture liquid containing hexamethylene diisocyanate produced by thermal decomposition and 4-methylbenzyl chloride was collected in a storage tank 104 by conducting thermal decomposition by the same method as that in the step 2 "Thermal decomposition of carbamate" in Example 4-1 except that the mixture liquid prepared in the step 1 was charged in a storage tank 101 of the isocyanate preparation device 1A shown in Fig. 1, 4-methylbenzyl chloride was charged in a reactor 100 equipped with a heat medium jacket to form a state in which 4-methylbenzyl chloride was refluxed, and the mixture liquid was supplied at 0.4 kg/hr from the storage tank 101 via a line 10 to the reactor 100. The yield of hexamethylene diisocyanate collected in the storage tank 104 was 20%. When the above operation was continued for 2 days, the line 11 was blocked and the continuation of the operation became difficult.

### Comparative Example 4-2

### 1. Preparation of mixture liquid

30 kg of the compound (2-1b-1) and 60 kg of triethylbenzene were mixed in a stirring tank heated at 120°C under nitrogen at atmospheric pressure to obtain a uniform mixture liquid.

### 2. Thermal decomposition of carbamate

A mixture liquid containing methyl 2-isocyanato-4-methylvalerate and triethylbenzene was collected in a storage tank 204 via a line 27 by conducting thermal decomposition by the same method as that in the step 2 "Thermal decomposition of carbamate" in Example 4-2 except that the mixture liquid prepared in the step 1 was charged in a storage tank 201 of the isocyanate preparation device 2A shown in Fig. 2, triethylbenzene was charged in a distillation column 210 to form a state in which triethylbenzene was refluxed, and the mixture liquid was supplied at 0.3 kg/hr from the storage tank 201 via a line 20 to a falling film type reactor 200 preheated at 250°C to allow a thermal decomposition of the compound (2-1b-1) to proceed. The yield of methyl 2-isocyanato-4-methylvalerate collected in the storage tank 204 was 20%. When the above operation was continued for 2 days, the line 21 was blocked and the continuation of the operation became difficult.

### Example 5-1

### 1. Preparation of mixture liquid

10 kg of the carbamate of the formula (2-1b-4) (carbamate (2-1b-4)), 50 kg of butyl cellosolve (inert solvent) and 40 kg of squalane (compound A) were mixed in a stirring tank heated at 120°C under nitrogen at atmospheric pressure to obtain a uniform mixture liquid

### 2. Thermal decomposition of carbamate

The mixture liquid prepared in the step 1 was charged in a storage tank 101 of the isocyanate preparation device 1A shown in Fig. 1. Squalane and butyl cellosolve were charged in a reactor 100 equipped with a heat medium jacket to form a state in which butyl cellosolve was refluxed via a line 16, a condenser 115, a storage tank 103 and a line 17 provided on the upper part of a packed bed 108, while maintaining the temperature of a heat medium passing through the heat medium jacket at 270°C and adjusting the internal pressure.

The mixture liquid was supplied at 1 kg/hr from the storage tank 101 via a line 10 to the reactor 100 to allow a thermal decomposition of the carbamate (2-1b-4) to proceed. A mixture liquid containing phenol produced by thermal decomposition and butyl cellosolve was collected in a storage tank 103 via a line 16 and a condenser 115 provided on the upper part of a packed bed 108. In contrast, a mixture liquid containing methyl 2-isocyanato-4-(methylthio)butyrate produced by thermal decomposition and butyl cellosolve was collected in a storage tank 104 via a line 14 and a condenser 114 provided on the upper part of a packed bed 107. Furthermore, the reaction liquid was extracted from the bottom of the reactor 100 via a line 11 to be collected in a storage tank 102 such that the liquid surface inside the reactor 100 was constant. The yield of methyl 2-isocyanato-4-(methylthio)butyrate collected in the storage tank 104 was 62%. The above operation could be continuously performed for 200 hours.

### Example 5-2

### 1. Preparation of mixture liquid

20 kg of 1,6-hexamethylene di(carbamic acid phenyl ester) (carbamate), 50 kg of triethylbenzene (inert solvent), and 30 kg of squalene (compound A) were mixed in a stirring tank heated at 120°C under nitrogen at atmospheric pressure to obtain a uniform mixture liquid.

### 2. Thermal decomposition of carbamate

The mixture liquid prepared in the step 1 was charged in a storage tank 201 of the isocyanate preparation device 2A shown in Fig. 2. Triethylbenzene was charged in a distillation column 210 to form a state in which triethylbenzene was refluxed via a line 23, a condenser 205, a storage tank 203 and a line 24 provided on the upper part of the distillation column 210, while maintaining the temperature of a reboiler 206 at 200°C and adjusting the internal pressure.

The mixture liquid was supplied at 1 kg/hr from the storage tank 201 via a line 20 to a falling film type reactor 200 preheated at 250°C to allow a thermal decomposition of 1,6-hexamethylene di(carbamic acid phenyl ester) to proceed. Gaseous components containing phenol, hexamethylene diisocyanate and triethylbenzene produced by thermal decomposition were supplied to a distillation column 210 via a line 22. In contrast, squalene containing a by-product was collected from the bottom of the falling film type reactor via a line 21 in a storage tank 202. The gaseous components collected via the line 22 were separated by distillation in the distillation column 210, and a mixture liquid containing phenol and triethylbenzene was collected in the storage tank 203 via the line 23 and the condenser 205. In contrast, a mixture liquid containing hexamethylene diisocyanate, triethylbenzene and a small amount of squalene was collected in a storage tank 204 via a line 27. The yield of hexamethylene diisocyanate collected in the storage tank 204 was 74%. The above operation could be continuously performed for 200 hours.

### Comparative Example 5-1

### 1. Preparation of mixture liquid

20 kg of the carbamate (2-1b-4) and 100 kg of butyl cellosolve were mixed in a stirring tank heated at 120°C under nitrogen at atmospheric pressure to obtain a uniform mixture liquid.

### 2. Thermal decomposition of carbamate

A mixture liquid containing methyl 2-isocyanato-4-(methylthio)butyrate produced by thermal decomposition and butyl cellosolve was collected in a storage tank 104 by conducting thermal decomposition by the same method as that in the step 2 "Thermal decomposition of carbamate" in Example 5-1 except that the mixture liquid prepared in the step 1 was charged in a storage tank 101 of the isocyanate preparation device 1A shown in Fig. 1, butyl cellosolve was charged in a reactor 100 equipped with a heat medium jacket to form a state in which butyl cellosolve was refluxed, and the mixture liquid was supplied at 0.4 kg/hr from the storage tank 101 via a line 10 to the reactor 100. The yield of methyl 2-isocyanato-4-(methylthio)butyrate collected in the storage tank 104 was 32%. When the above operation was continued for 2 days, the line 11 was blocked and the continuation of the operation became difficult.

### Comparative Example 5-2

### 1. Preparation of mixture liquid

20 kg of 1,6-hexamethylene di(carbamic acid phenyl ester) and 50 kg of triethylbenzene were mixed in a stirring tank heated at 120°C under nitrogen at atmospheric pressure to obtain a uniform mixture liquid.

### 2. Thermal decomposition of carbamate

A mixture liquid containing hexamethylene diisocyanate and triethylbenzene was collected in a storage tank 204 via a line 27 by conducting thermal decomposition by the same method as that in the step 2 "Thermal decomposition of carbamate" in Example 5-2 except that the mixture liquid prepared in the step 1 was charged in a storage tank 201 of the isocyanate preparation device 2A shown in Fig. 2, triethylbenzene was charged in a distillation column 210 to form a state in which triethylbenzene was refluxed, and the mixture liquid was supplied at 0.3 kg/hr from the storage tank 201 via a line 20 to a falling film type reactor 200 preheated at 250°C to allow a thermal decomposition of 1,6-hexamethylene di(carbamic acid phenyl ester) to proceed. The yield of hexamethylene diisocyanate collected in the storage tank 204 was 35%. When the above operation was continued for 2 days, the line 21 was blocked and the continuation of the operation became difficult.

### Examples 1A to 15A

Each isocyanate was prepared by conducting thermal decomposition of a carbamate by the same method as Example 1-1, except that each carbamate shown in the following tables was used.

**Table 1**

| Ex. | Carbamate | Resultant isocyanate | Thermal decomposition yield (%) |
|---|---|---|---|
| 1A | 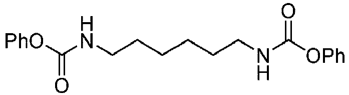 | 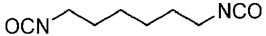 | 76 |
| 2A | 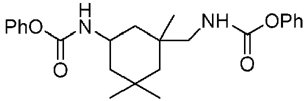 | 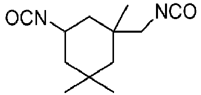 | 69 |
| 3A | 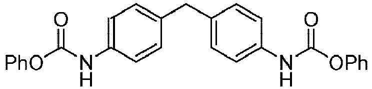 | 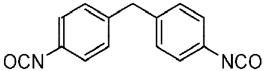 | 74 |
| 4A | 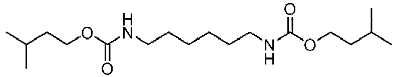 | 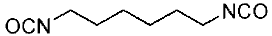 | 70 |
| 5A | 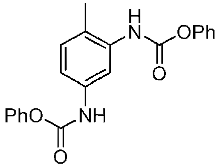 | 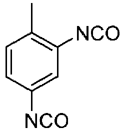 | 75 |
| 6A | 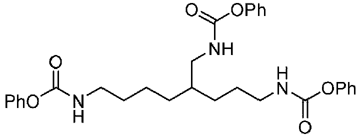 | 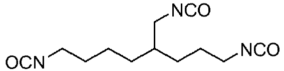 | 70 |
| 7A | 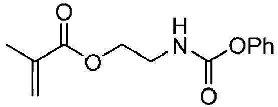 | 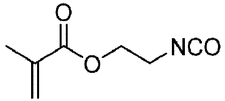 | 71 |
| 8A | 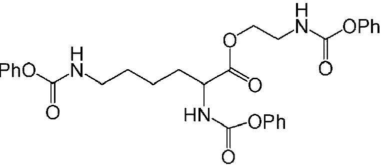 | 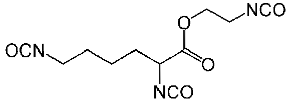 | 70 |

| | | | |
|---|---|---|---|
| (Ex: Example) | | | |

**Table 2**

| Ex. | Carbamate | Resultant isocyanate | Thermal decomposition yield (%) |
|---|---|---|---|
| 9A | 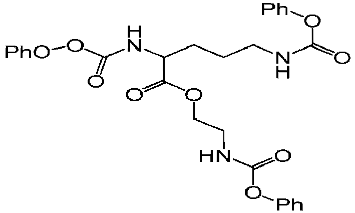 | 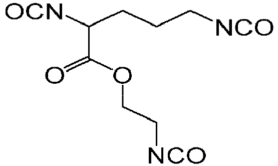 | 73 |
| 10A | 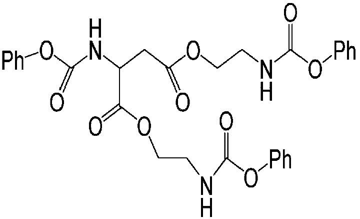 | 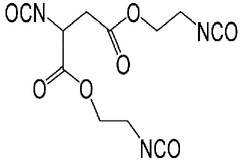 | 68 |
| 11A | 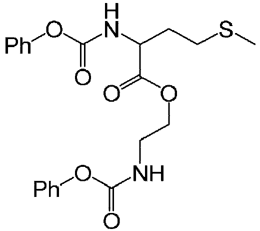 | 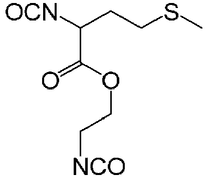 | 73 |
| 12A | 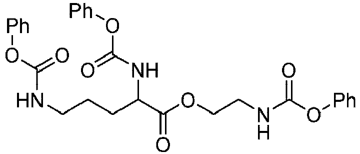 | 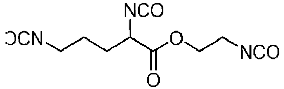 | 71 |
| 13A | 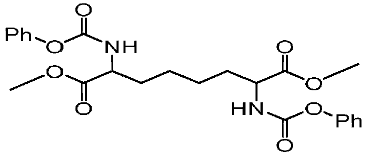 | 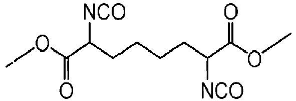 | 74 |

**Table 3**

| Ex. | Carbamate | Resultant isocyanate | Thermal decomposition yield (%) |
|---|---|---|---|
| 14A | 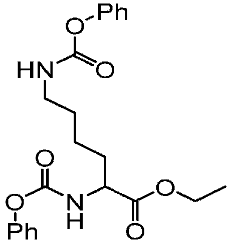 | 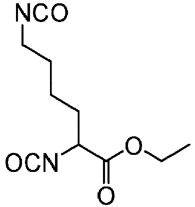 | 73 |
| 15A | 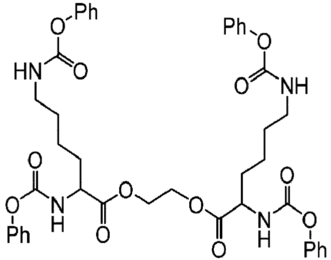 | 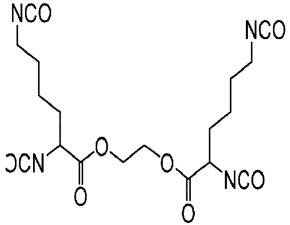 | 57 |

| | | | |
|---|---|---|---|
| (Ex: Example) | | | |

### Examples 1B to 15B

Each isocyanate was prepared by thermal decomposition of carbamate as conducted in Example 1-2 except that a phenolic novolac resin was used as the compound A, benzophenone was used as an inert solvent, and each carbamate shown in the following tables was used.

**Table 4**

| Ex. | Carbamate | Resultant isocyanate | Thermal decomposition yield (%) |
|---|---|---|---|
| 1B | 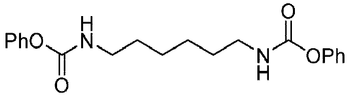 | 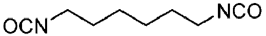 | 88 |
| 2B | 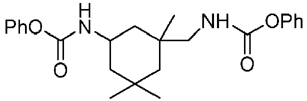 | 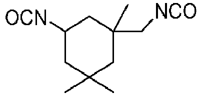 | 81 |
| 3B | 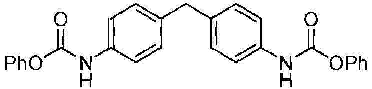 | 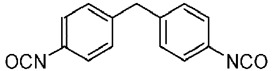 | 86 |
| 4B | 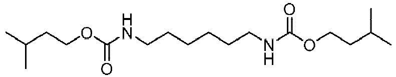 | 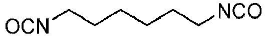 | 84 |
| 5B | 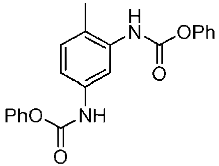 | 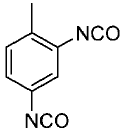 | 86 |
| 6B | 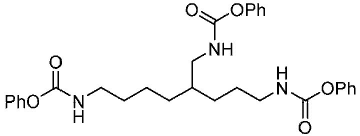 | 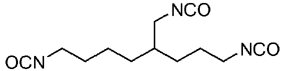 | 84 |
| 7B | 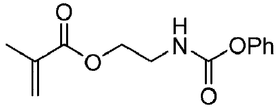 | 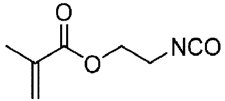 | 82 |
| 8B | 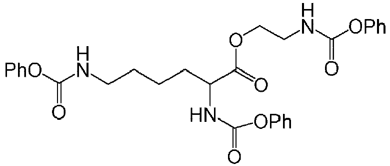 | 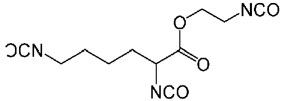 | 83 |

| | | | |
|---|---|---|---|
| (Ex: Example) | | | |

**Table 5**

| Ex. | Carbamate | Resultant isocyanate | Thermal decomposition yield (%) |
|---|---|---|---|
| 9B | 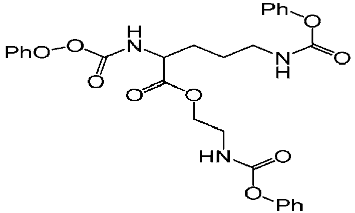 | 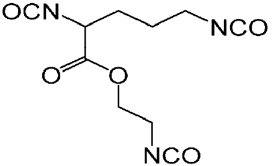 | 84 |
| 10B | 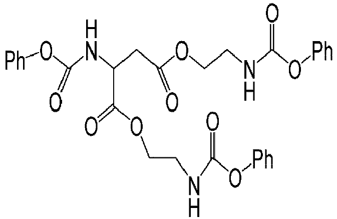 | 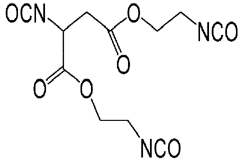 | 79 |
| 11B | 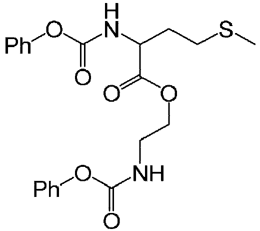 | 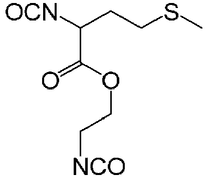 | 85 |
| 12B | 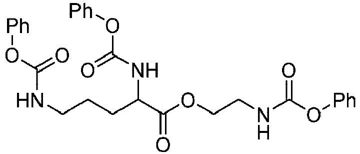 | 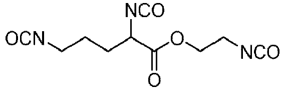 | 84 |
| 13B | 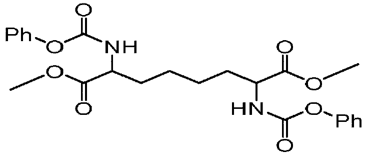 | 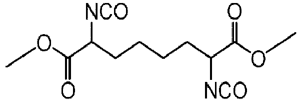 | 86 |

| | | | |
|---|---|---|---|
| (Ex: Example) | | | |

**Table 6**

| Ex. | Carbamate | Resultant isocyanate | Thermal decomposition yield (%) |
|---|---|---|---|
| 14B | 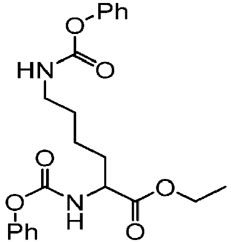 | 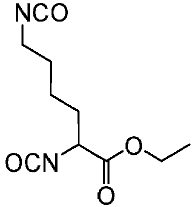 | 84 |
| 15B | 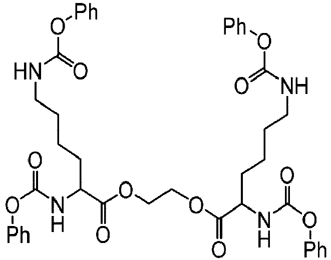 | 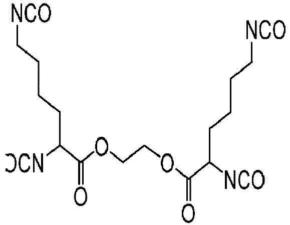 | 68 |

| | | | |
|---|---|---|---|
| (Ex: Example) | | | |

### Examples 17A to 31A

Each isocyanate was prepared by thermal decomposition of carbamate as conducted in Example 1-1 except that a phenolic resol resin was used as the compound A and each carbamate shown in the following tables was used.

**Table 7**

| Ex. | Carbamate | Resultant isocyanate | Thermal decomposition yield (%) |
|---|---|---|---|
| 17 A | 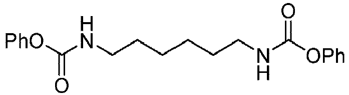 | 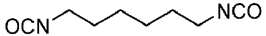 | 77 |
| 18 A | 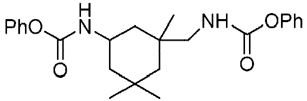 | 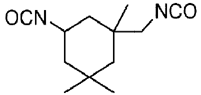 | 68 |
| 19 A | 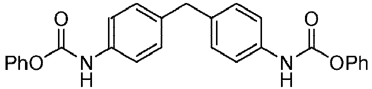 | 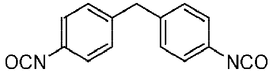 | 73 |
| 20 A | 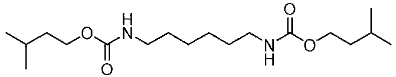 | 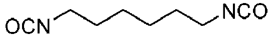 | 71 |
| 21 A | 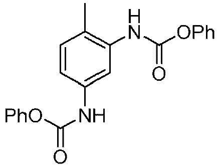 | 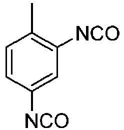 | 74 |
| 22 A | 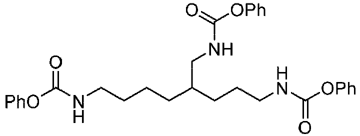 | 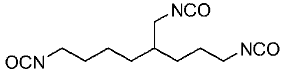 | 71 |
| 23 A | 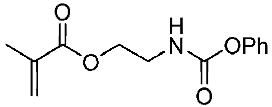 | 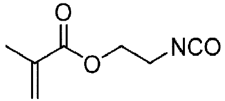 | 72 |
| 24 A | 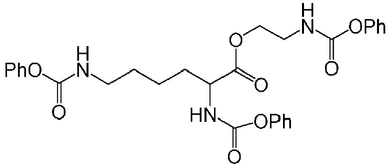 | 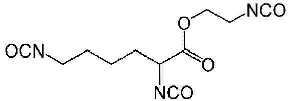 | 71 |

| | | | |
|---|---|---|---|
| (Ex: Example) | | | |

**Table 8**

| Ex. | Carbamate | Resultant isocyanate | Thermal decomposition yield (%) |
|---|---|---|---|
| 25 A | 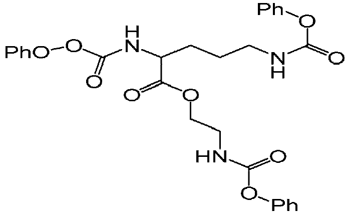 | 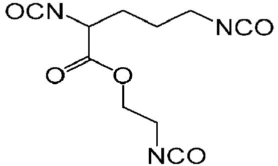 | 72 |
| 26 A | 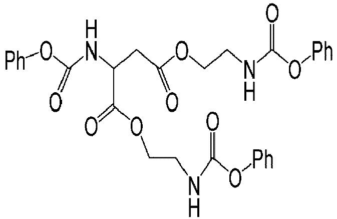 | 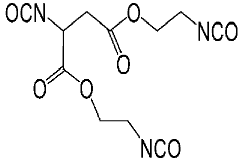 | 69 |
| 27 A | 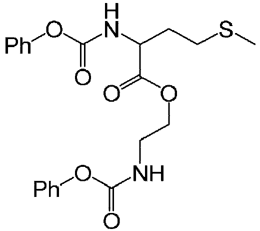 | 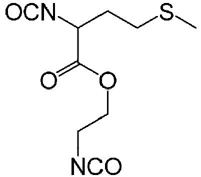 | 73 |
| 28 A | 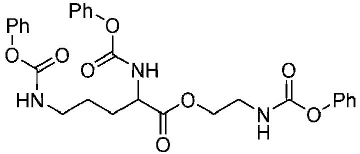 | 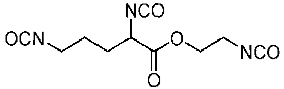 | 72 |
| 29 A | 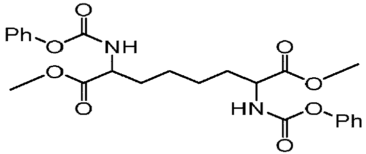 | 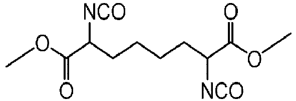 | 73 |

| | | | |
|---|---|---|---|
| (Ex: Example) | | | |

**Table 9**

| Ex. | Carbamate | Resultant isocyanate | Thermal decomposition yield (%) |
|---|---|---|---|
| 30 A | 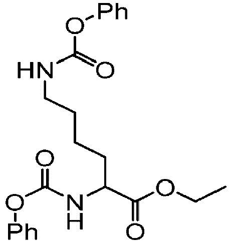 | 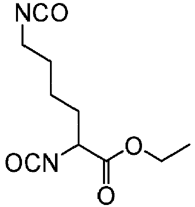 | 74 |
| 31 A | 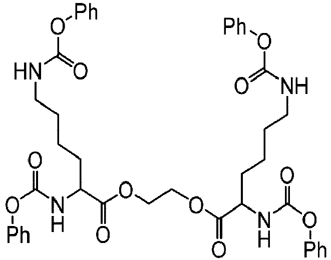 | 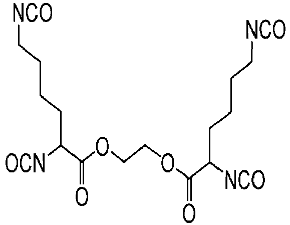 | 57 |

| | | | |
|---|---|---|---|
| (Ex: Example) | | | |

### Examples 17B to 31B

Each isocyanate was prepared by thermal decomposition of carbamate as conducted in Example 1-2 except that a phenolic resol resin was used as the compound A, benzophenone was used as an inert solvent, and each carbamate shown in the following tables was used.

**Table 10**

| Ex. | Carbamate | Resultant isocyanate | Thermal decomposition yield (%) |
|---|---|---|---|
| 17 B | 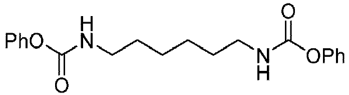 | 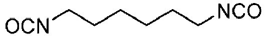 | 89 |
| 18 B | 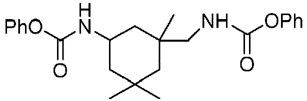 | 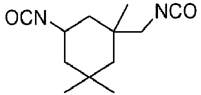 | 83 |
| 19 B | 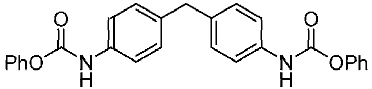 | 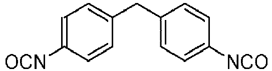 | 87 |
| 20 B | 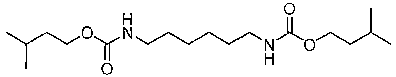 | 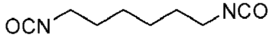 | 84 |
| 21 B | 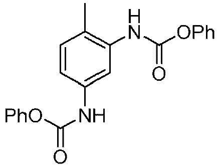 | 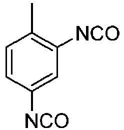 | 87 |
| 22 B | 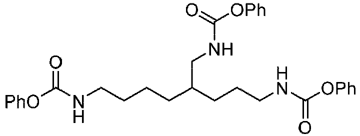 | 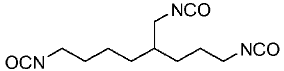 | 85 |
| 23 B | 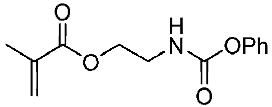 | 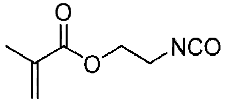 | 83 |
| 24 B | 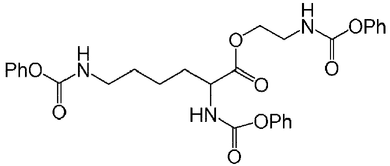 | 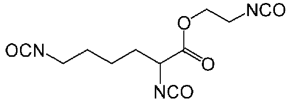 | 84 |

| | | | |
|---|---|---|---|
| (Ex: Example) | | | |

**Table 11**

| Ex. | Carbamate | Resultant isocyanate | Thermal decomposition yield (%) |
|---|---|---|---|
| 25 B | 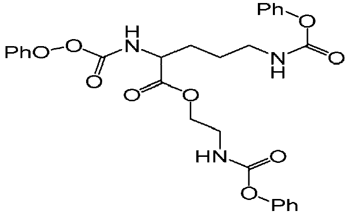 | 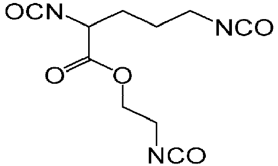 | 85 |
| 26 B | 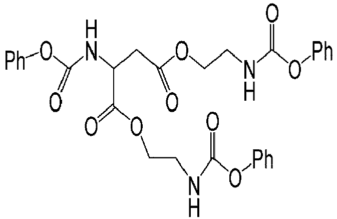 | 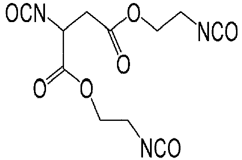 | 80 |
| 27 B | 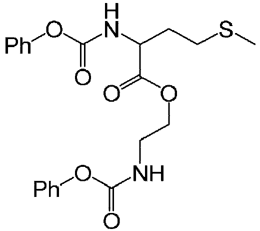 | 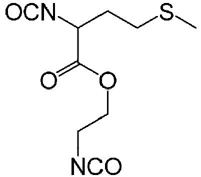 | 87 |
| 28 B | 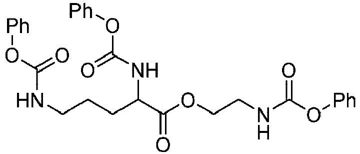 | 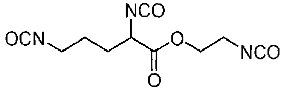 | 87 |
| 29 B | 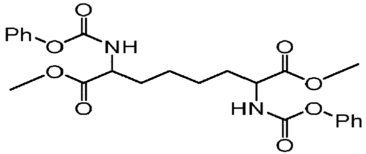 | 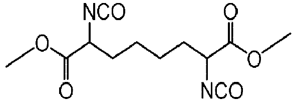 | 87 |

| | | | |
|---|---|---|---|
| (Ex: Example) | | | |

**Table 12**

| Ex. | Carbamate | Resultant isocyanate | Thermal decomposition yield (%) |
|---|---|---|---|
| 30 B | 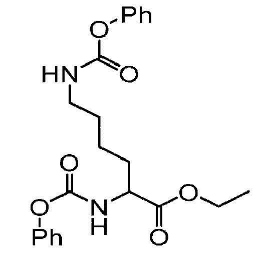 | 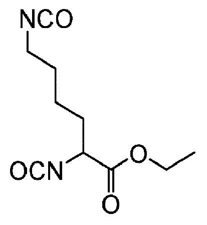 | 85 |
| 31 B | 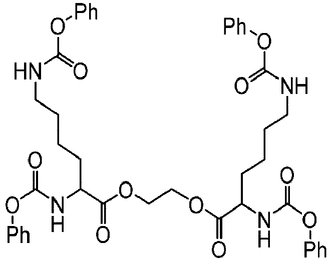 | 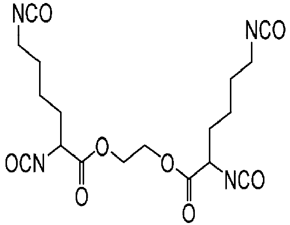 | 69 |

| | | | |
|---|---|---|---|
| (Ex: Example) | | | |

### Examples 33A to 47A

Each isocyanate was prepared by thermal decomposition of carbamate as conducted in Example 1-1 except that a cresolic resol resin was used as the compound A and each carbamate shown in the following tables was used.

**Table 13**

| Ex. | Carbamate | Resultant isocyanate | Thermal decomposition yield (%) |
|---|---|---|---|
| 33 A | 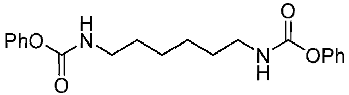 | 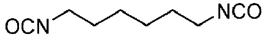 | 78 |
| 34 A | 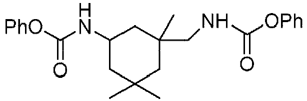 | 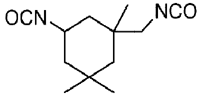 | 69 |
| 35 A | 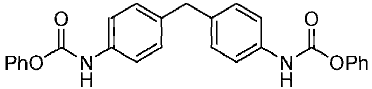 | 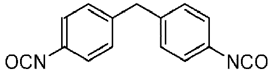 | 73 |
| 36 A | 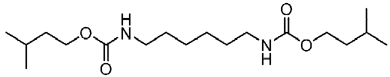 | 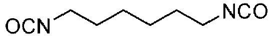 | 72 |
| 37 A | 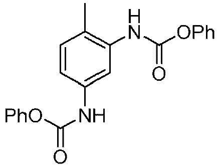 | 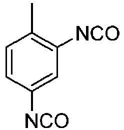 | 74 |
| 38 A | 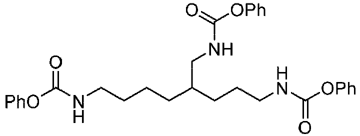 | 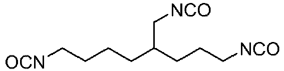 | 72 |
| 39 A | 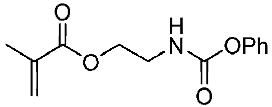 | 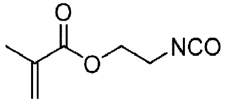 | 73 |
| 40 A | 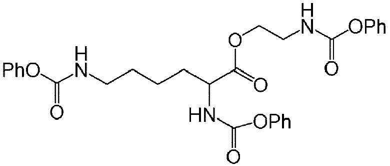 | 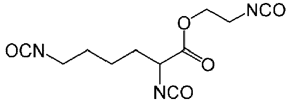 | 71 |

| | | | |
|---|---|---|---|
| (Ex: Example) | | | |

**Table 14**

| Ex. | Carbamate | Resultant isocyanate | Thermal decomposition yield (%) |
|---|---|---|---|
| 41 A | 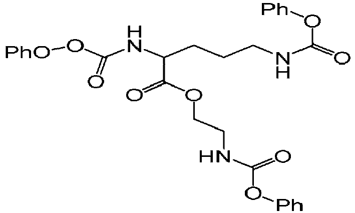 | 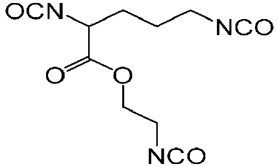 | 72 |
| 42 A | 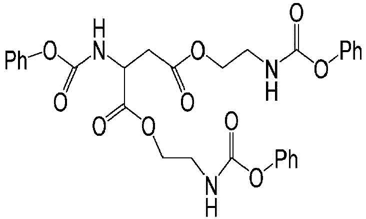 | 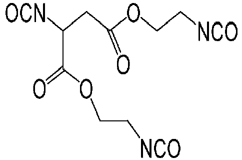 | 70 |
| 43 A | 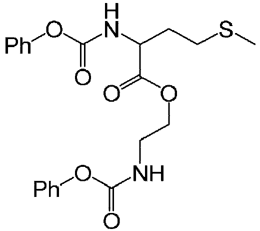 | 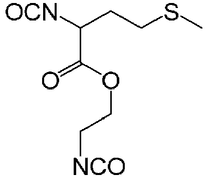 | 73 |
| 44 A | 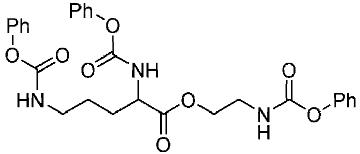 | 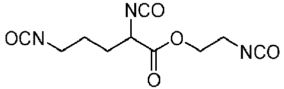 | 73 |
| 45 A | 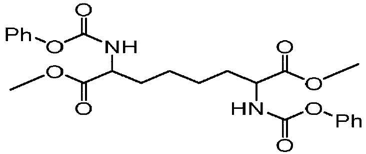 | 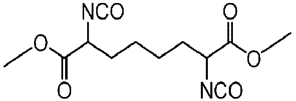 | 73 |

| | | | |
|---|---|---|---|
| (Ex: Example) | | | |

**Table 15**

| Ex. | Carbamate | Resultant isocyanate | Thermal decomposition yield (%) |
|---|---|---|---|
| 46 A | 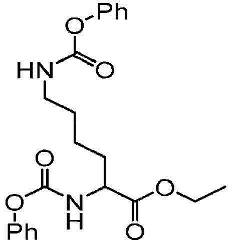 | 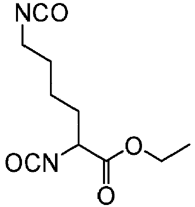 | 74 |
| 47 A | 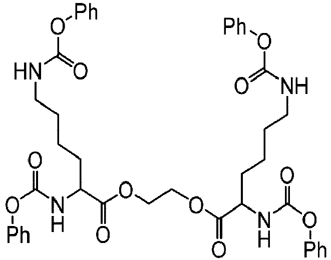 | 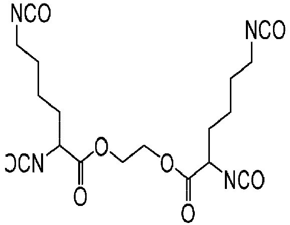 | 58 |

| | | | |
|---|---|---|---|
| (Ex: Example) | | | |

### Examples 33B to 47B

Each isocyanate was prepared by thermal decomposition of carbamate as conducted in Example 1-2 except that a cresolic resol resin was used as the compound A, benzophenone was used as an inert solvent and each carbamate shown in the following tables was used.

**Table 16**

| Ex. | Carbamate | Resultant isocyanate | Thermal decomposition yield (%) |
|---|---|---|---|
| 33 B | 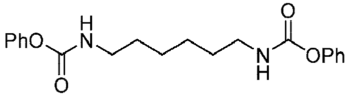 | 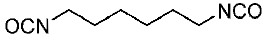 | 88 |
| 34 B | 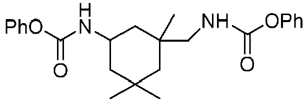 | 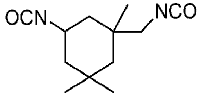 | 83 |
| 35 B | 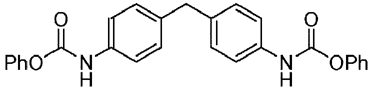 | 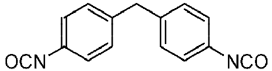 | 86 |
| 36 B | 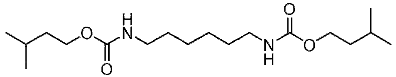 | 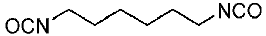 | 83 |
| 37 B | 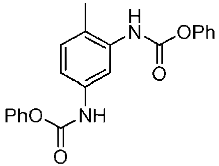 | 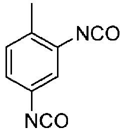 | 86 |
| 38 B | 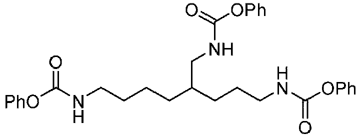 | 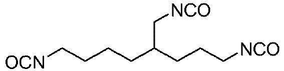 | 84 |
| 39 B | 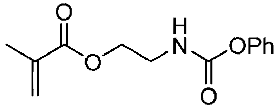 | 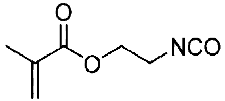 | 83 |
| 40 B | 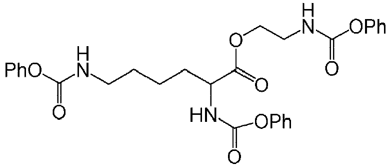 | 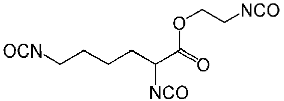 | 83 |

| | | | |
|---|---|---|---|
| (Ex: Example) | | | |

**Table 17**

| Ex. | Carbamate | Resultant isocyanate | Thermal decomposition yield (%) |
|---|---|---|---|
| 41 B | 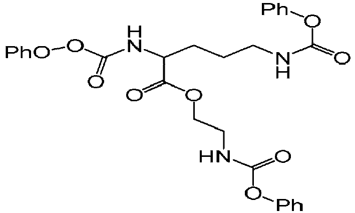 | 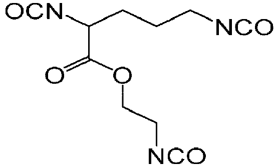 | 84 |
| 42 B | 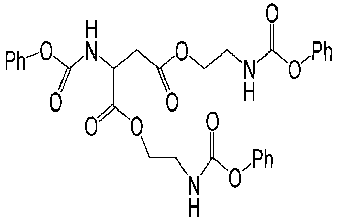 | 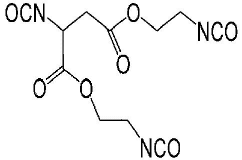 | 84 |
| 43 B | 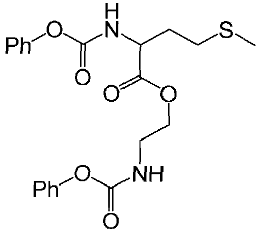 | 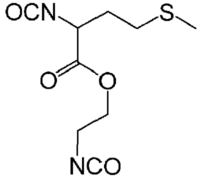 | 86 |
| 44 B | 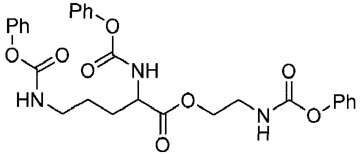 | 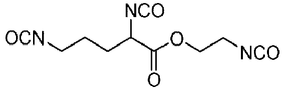 | 85 |
| 45 B | 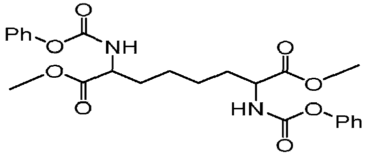 | 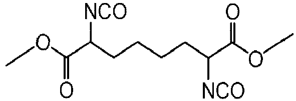 | 84 |

| | | | |
|---|---|---|---|
| (Ex: Example) | | | |

**Table 18**

| Ex. | Carbamate | Resultant isocyanate | Thermal decomposition yield (%) |
|---|---|---|---|
| 46 B | 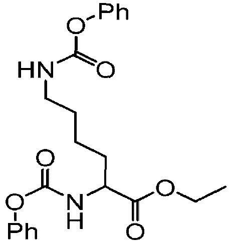 | 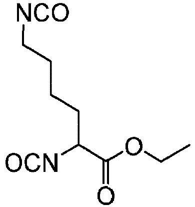 | 84 |
| 47 B | 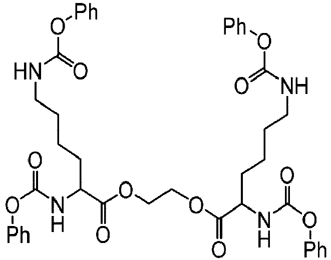 | 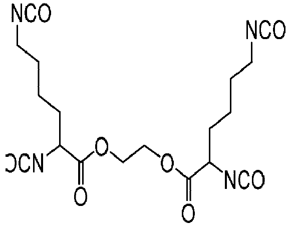 | 67 |

| | | | |
|---|---|---|---|
| (Ex: Example) | | | |

### Examples 49A to 62A

Each isocyanate was prepared by thermal decomposition of carbamate as conducted in Example 1-1 except that a compound of the following formula (5-1d) was used as the compound A and each carbamate shown in the following tables was used.

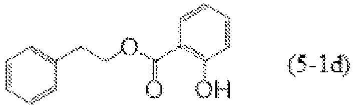

**Table 19**

| Ex. | Carbamate | Resultant isocyanate | Thermal decomposition yield (%) |
|---|---|---|---|
| 49 A | 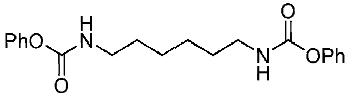 | 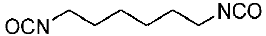 | 77 |
| 50 A | 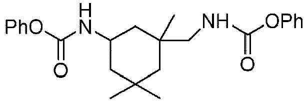 | 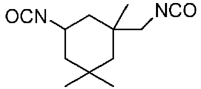 | 68 |
| 51 A | 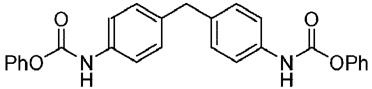 | 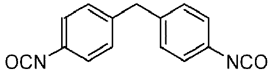 | 73 |
| 52 A | 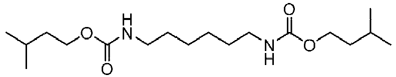 | 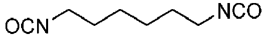 | 71 |
| 53 A | 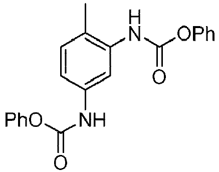 | 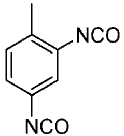 | 73 |
| 54 A | 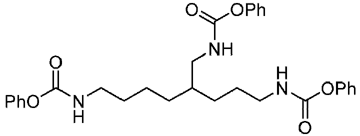 | 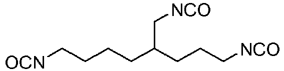 | 72 |
| 55 A | 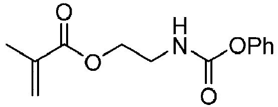 | 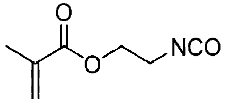 | 71 |
| 56 A | 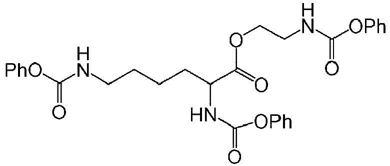 | 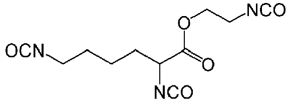 | 71 |

| | | | |
|---|---|---|---|
| (Ex: Example) | | | |

**Table 20**

| Ex. | Carbamate | Resultant isocyanate | Thermal decomposition yield (%) |
|---|---|---|---|
| 57 A | 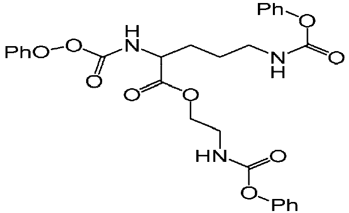 | 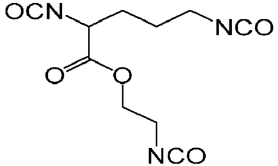 | 71 |
| 58 A | 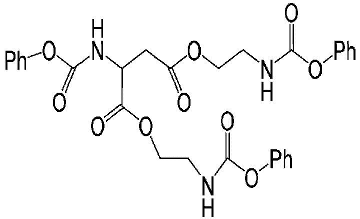 | 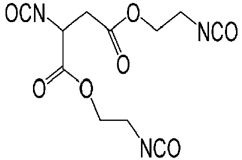 | 68 |
| 59 A | 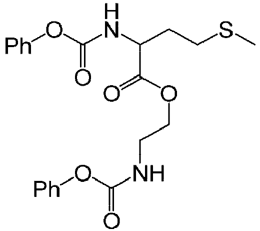 | 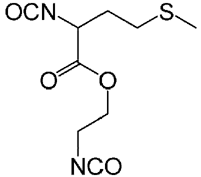 | 74 |
| 60 A | 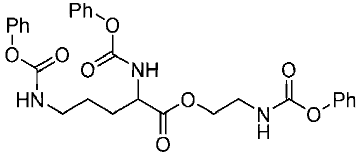 | 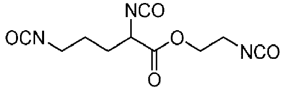 | 73 |
| 61 A | 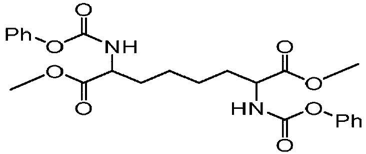 | 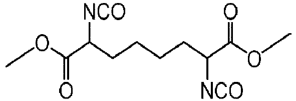 | 72 |

| | | | |
|---|---|---|---|
| (Ex: Example) | | | |

**Table 21**

| Ex. | Carbamate | Resultant isocyanate | Thermal decomposition yield (%) |
|---|---|---|---|
| 62 A | 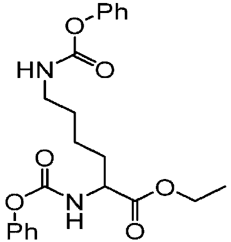 | 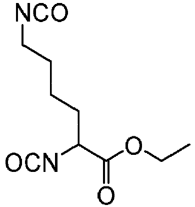 | 73 |

| | | | |
|---|---|---|---|
| (Ex: Example) | | | |

### Examples 49B to 62B

Each isocyanate was prepared by thermal decomposition of carbamate as conducted in Example 1-2 except that the compound of the formula (5-1d) was used as the compound A, benzophenone was used as an inert solvent and each carbamate shown in the following tables was used.

**Table 22**

| Ex. | Carbamate | Resultant isocyanate | Thermal decomposition yield (%) |
|---|---|---|---|
| 49 B | 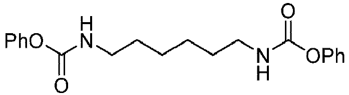 | 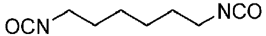 | 86 |
| 50 B | 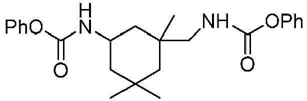 | 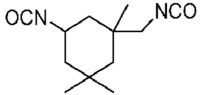 | 81 |
| 51 B | 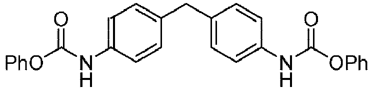 | 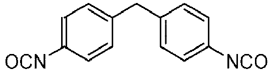 | 85 |
| 52 B | 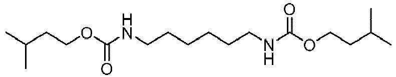 | 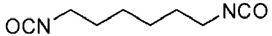 | 83 |
| 53 B | 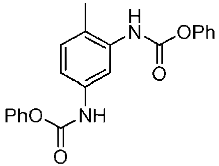 | 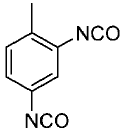 | 85 |
| 54 B | 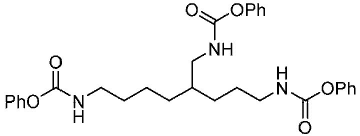 | 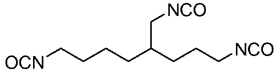 | 84 |
| 55 B | 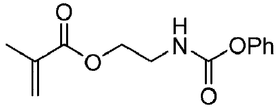 | 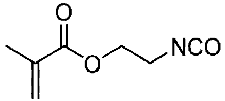 | 82 |
| 56 B | 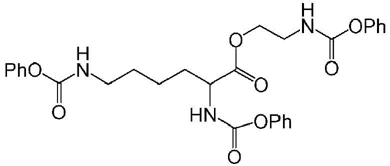 | 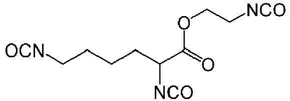 | 85 |

| | | | |
|---|---|---|---|
| (Ex: Example) | | | |

**Table 23**

| Ex. | Carbamate | Resultant isocyanate | Thermal decomposition yield (%) |
|---|---|---|---|
| 57 B | 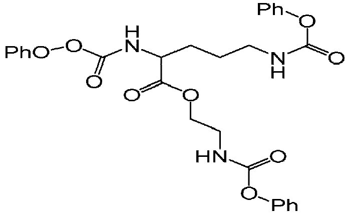 | 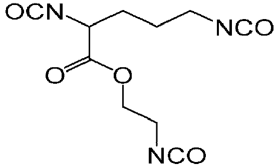 | 84 |
| 58 B | 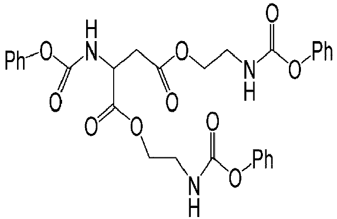 | 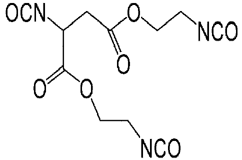 | 84 |
| 59 B | 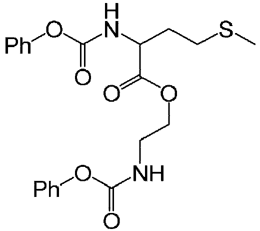 | 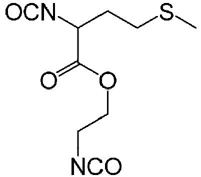 | 86 |
| 60 B | 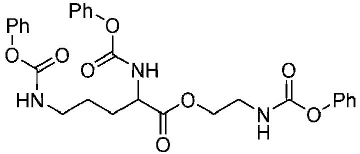 | 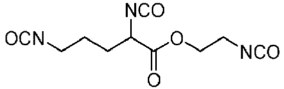 | 85 |
| 61 B | 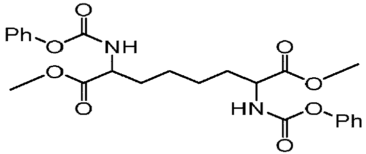 | 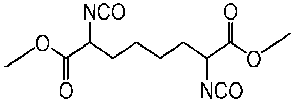 | 86 |

| | | | |
|---|---|---|---|
| (Ex: Example) | | | |

**Table 24**

| Ex. | Carbamate | Resultant isocyanate | Thermal decomposition yield (%) |
|---|---|---|---|
| 62 B | 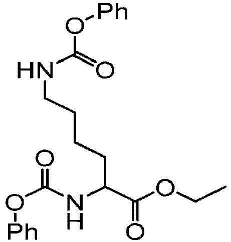 | 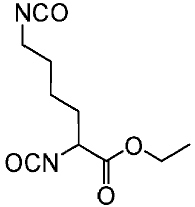 | 84 |

| | | | |
|---|---|---|---|
| (Ex: Example) | | | |

### Examples 63A to 76A

Each isocyanate was prepared by thermal decomposition of carbamate as conducted in Example 1-1 except that a compound of the following formula (5-2-1a) was used as the compound A and each carbamate shown in the following tables was used.

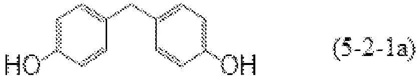

**Table 25**

| Ex. | Carbamate | Resultant isocyanate | Thermal decomposition yield (%) |
|---|---|---|---|
| 63 A | 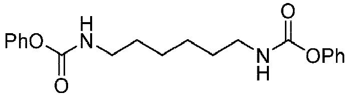 | 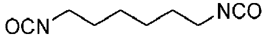 | 77 |
| 64 A | 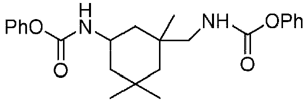 | 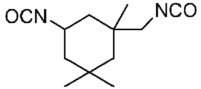 | 69 |
| 65 A | 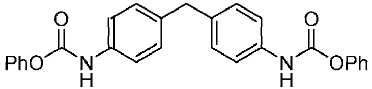 | 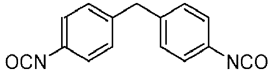 | 74 |
| 66 A | 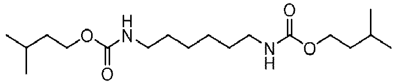 | 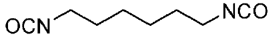 | 72 |
| 67 A | 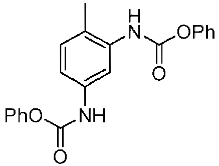 | 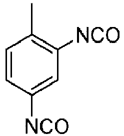 | 74 |
| 68 A | 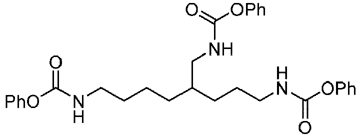 | 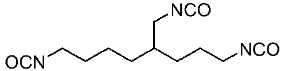 | 73 |
| 69 A | 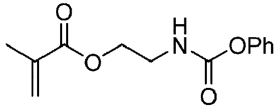 | 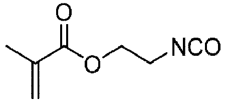 | 72 |
| 70 A | 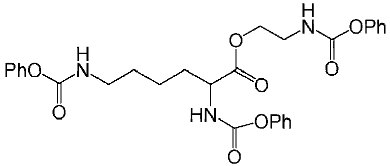 | 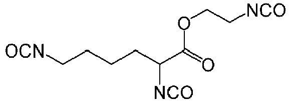 | 72 |

| | | | |
|---|---|---|---|
| (Ex: Example) | | | |

**Table 26**

| Ex. | Carbamate | Resultant isocyanate | Thermal decomposition yield (%) |
|---|---|---|---|
| 71 A | 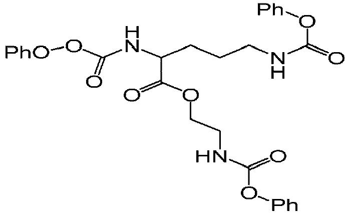 | 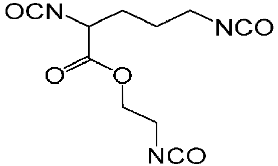 | 71 |
| 72 A | 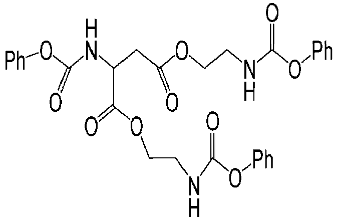 | 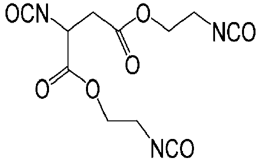 | 69 |
| 73 A | 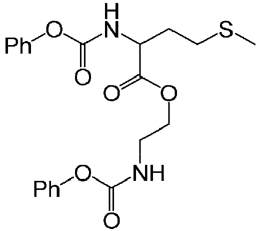 | 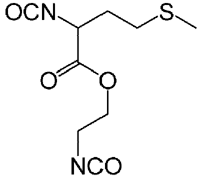 | 73 |
| 74 A | 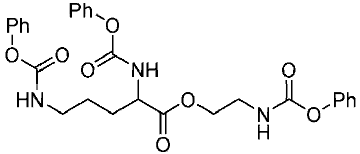 | 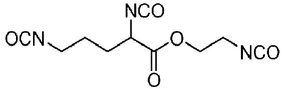 | 72 |
| 75 A | 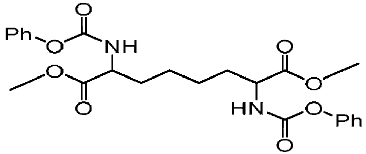 | 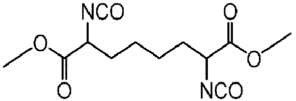 | 73 |

| | | | |
|---|---|---|---|
| (Ex: Example) | | | |

**Table 27**

| Ex. | Carbamate | Resultant isocyanate | Thermal decomposition yield (%) |
|---|---|---|---|
| 76 A | 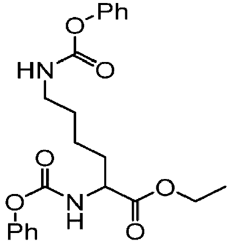 | 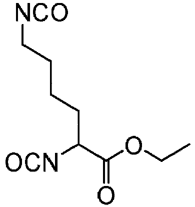 | 73 |

| | | | |
|---|---|---|---|
| (Ex: Example) | | | |

### Examples 63B to 76B

Each isocyanate was prepared by thermal decomposition of carbamate as conducted in Example 1-2 except that the compound of the formula (5-2-1a) was used as the compound A, benzophenone was used as an inert solvent and each carbamate shown in the following tables was used.

**Table 28**

| Ex. | Carbamate | Resultant isocyanate | Thermal decomposition yield (%) |
|---|---|---|---|
| 63 B | 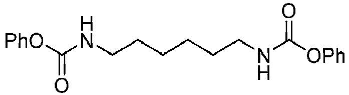 | 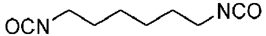 | 85 |
| 64 B | 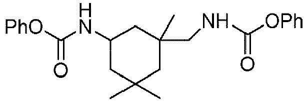 | 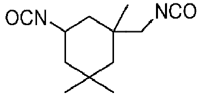 | 80 |
| 65 B | 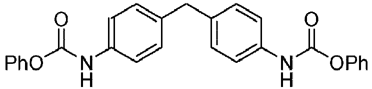 | 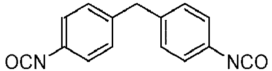 | 85 |
| 66 B | 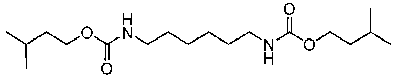 | 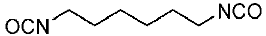 | 83 |
| 67 B | 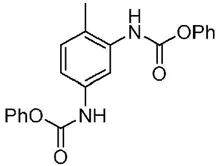 | 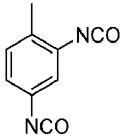 | 83 |
| 68 B | 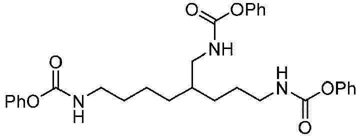 | 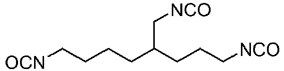 | 84 |
| 69 B | 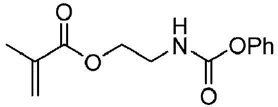 | 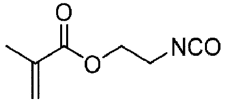 | 82 |
| 70 B | 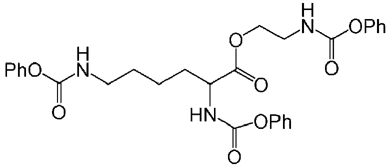 | 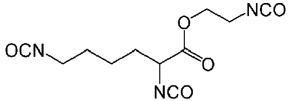 | 83 |

| | | | |
|---|---|---|---|
| (Ex: Example) | | | |

**Table 29**

| Ex. | Carbamate | Resultant isocyanate | Thermal decomposition yield (%) |
|---|---|---|---|
| 71 B | 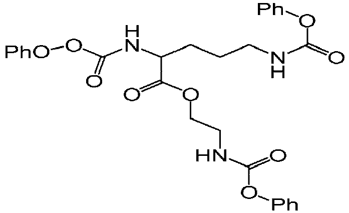 | 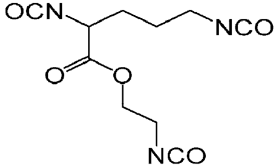 | 82 |
| 72 B | 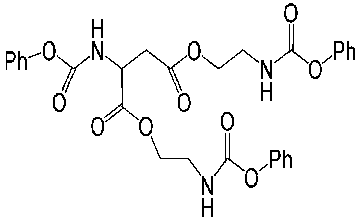 | 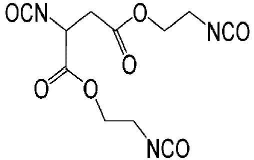 | 82 |
| 73 B | 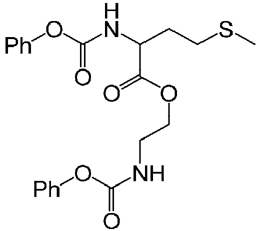 | 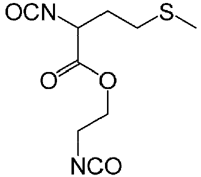 | 85 |
| 74 B | 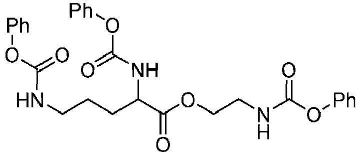 | 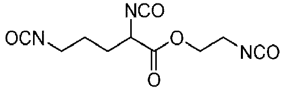 | 84 |
| 75 B | 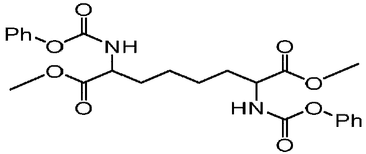 | 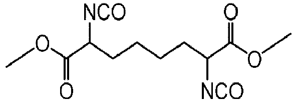 | 84 |

| | | | |
|---|---|---|---|
| (Ex: Example) | | | |

**Table 30**

| Ex. | Carbamate | Resultant isocyanate | Thermal decomposition yield (%) |
|---|---|---|---|
| 76 B | 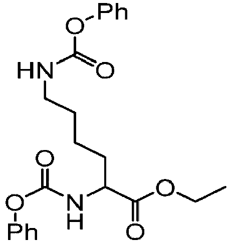 | 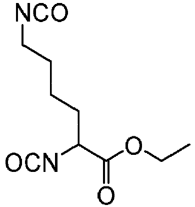 | 85 |

| | | | |
|---|---|---|---|
| (Ex: Example) | | | |

### Examples 77A to 90A

Each isocyanate was prepared by thermal decomposition of carbamate as conducted in Example 1-1 except that a compound of the following formula (5-2-1b) was used as the compound A and each carbamate shown in the following tables was used.

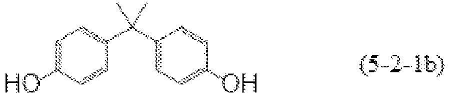

**Table 31**

| Ex. | Carbamate | Resultant isocyanate | Thermal decomposition yield (%) |
|---|---|---|---|
| 77 A | 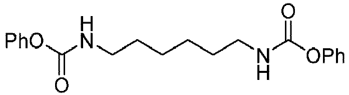 | 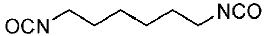 | 78 |
| 78 A | 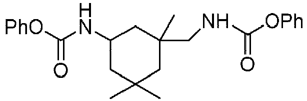 | 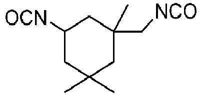 | 70 |
| 79 A | 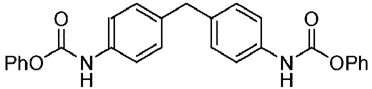 | 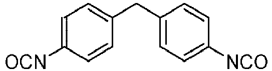 | 73 |
| 80 A | 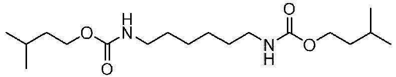 | 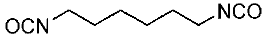 | 73 |
| 81 A | 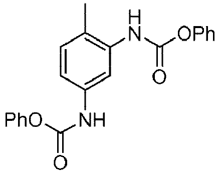 | 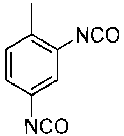 | 73 |
| 82 A | 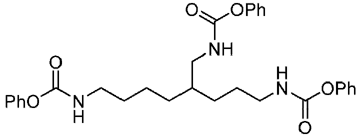 | 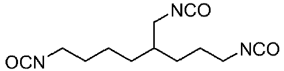 | 74 |
| 83 A | 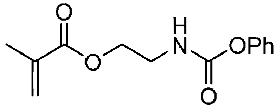 | 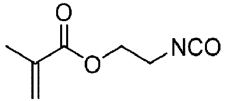 | 73 |
| 84 A | 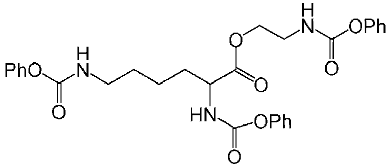 | 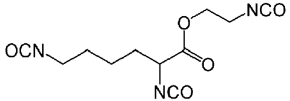 | 71 |

| | | | |
|---|---|---|---|
| (Ex: Example) | | | |

**Table 32**

| Ex. | Carbamate | Resultant isocyanate | Thermal decomposition yield (%) |
|---|---|---|---|
| 85 A | 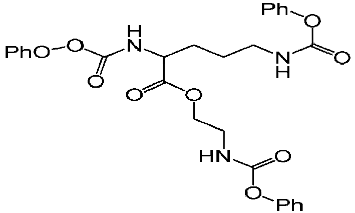 | 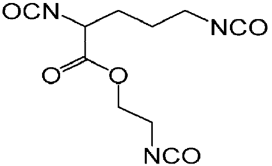 | 71 |
| 86 A | 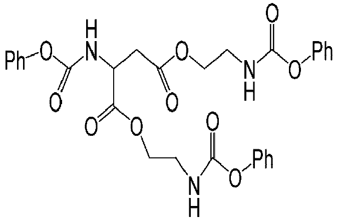 | 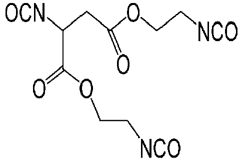 | 68 |
| 87 A | 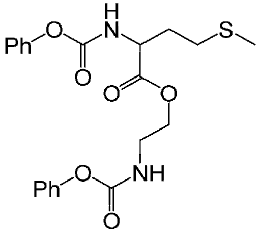 | 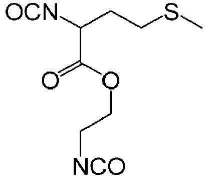 | 72 |
| 88 A | 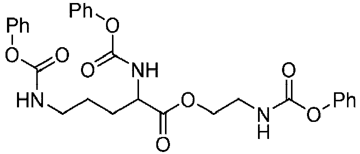 | 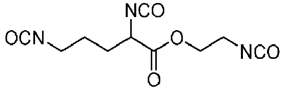 | 71 |
| 89 A | 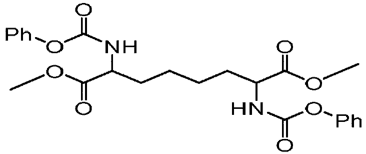 | 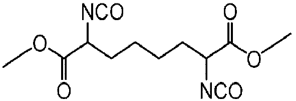 | 72 |

| | | | |
|---|---|---|---|
| (Ex: Example) | | | |

**Table 33**

| Ex. | Carbamate | Resultant isocyanate | Thermal decomposition yield (%) |
|---|---|---|---|
| 90 A | 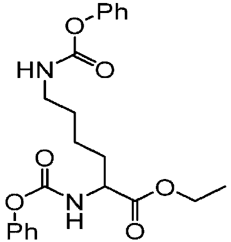 | 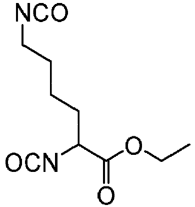 | 73 |

### Examples 77B to 90B

Each isocyanate was prepared by thermal decomposition of carbamate as conducted in Example 1-2 except that the compound of the formula (5-2-1b) was used as the compound A, benzophenone was used as an inert solvent and each carbamate shown in the following tables was used.

**Table 34**

| Ex. | Carbamate | Resultant isocyanate | Thermal decomposition yield (%) |
|---|---|---|---|
| 77 B | 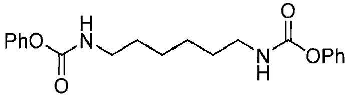 | 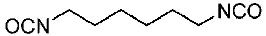 | 85 |
| 78 B | 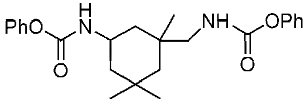 | 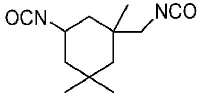 | 80 |
| 79 B | 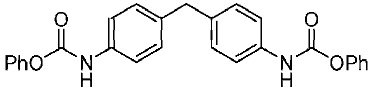 | 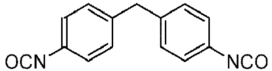 | 85 |
| 80 B | 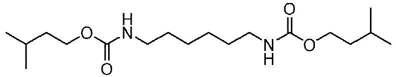 | 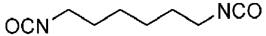 | 83 |
| 81 B | 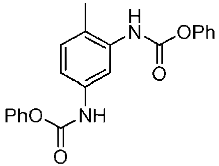 | 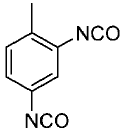 | 83 |
| 82 B | 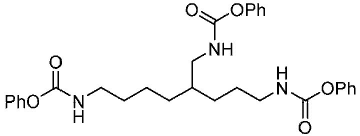 | 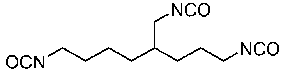 | 84 |
| 83 B | 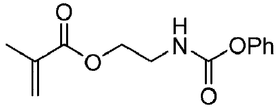 | 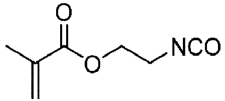 | 82 |
| 84 B | 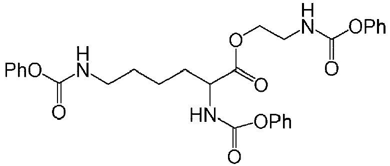 | 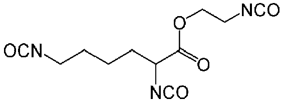 | 83 |

| | | | |
|---|---|---|---|
| (Ex: Example) | | | |

**Table 35**

| Ex. | Carbamate | Resultant isocyanate | Thermal decomposition yield (%) |
|---|---|---|---|
| 85 B | 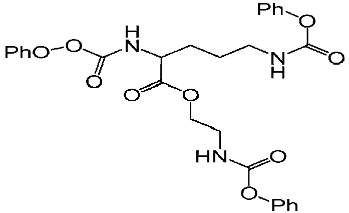 | 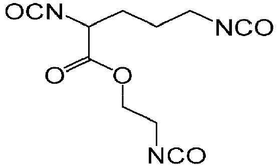 | 82 |
| 86 B | 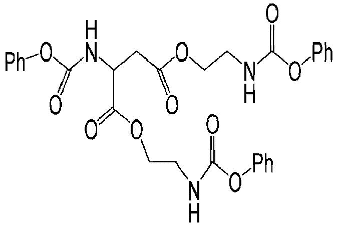 | 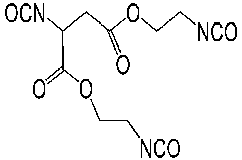 | 82 |
| 87 B | 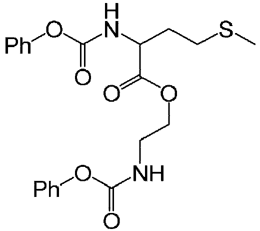 | 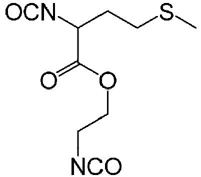 | 85 |
| 88 B | 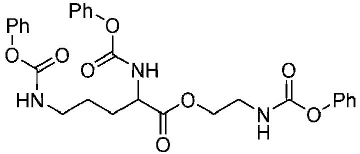 | 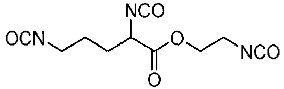 | 84 |
| 89 B | 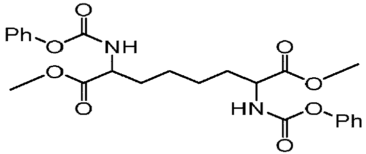 | 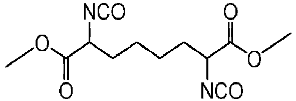 | 84 |

| | | | |
|---|---|---|---|
| (Ex: Example) | | | |

**Table 36**

| Ex. | Carbamate | Resultant isocyanate | Thermal decomposition yield (%) |
|---|---|---|---|
| 90 B | 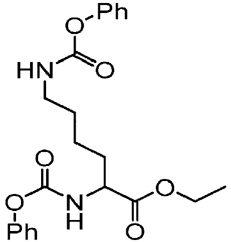 | 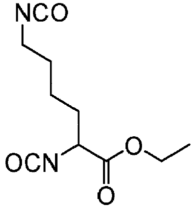 | 85 |

| | | | |
|---|---|---|---|
| (Ex: Example) | | | |

### Examples 91A to 104A

Each isocyanate was prepared by thermal decomposition of carbamate as conducted in Example 1-1 except that a compound of the following formula (5-1-2a) was used as the compound A and each carbamate shown in the following tables was used.

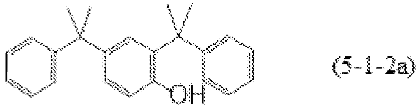

**Table 37**

| Ex. | Carbamate | Resultant isocyanate | Thermal decomposition yield (%) |
|---|---|---|---|
| 91 A | 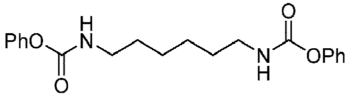 | 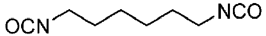 | 77 |
| 92 A | 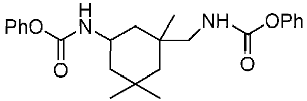 | 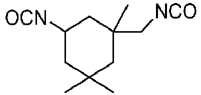 | 69 |
| 93 A | 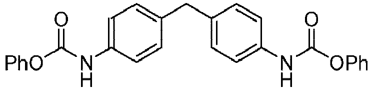 | 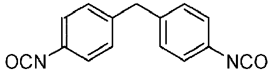 | 72 |
| 94 A | 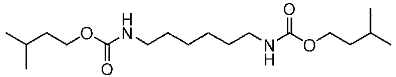 | 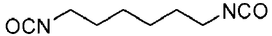 | 73 |
| 95 A | 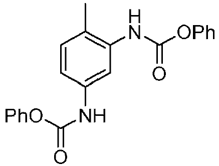 | 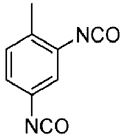 | 73 |
| 96 A | 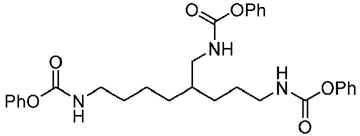 | 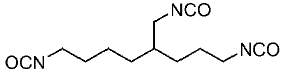 | 72 |
| 97 A | 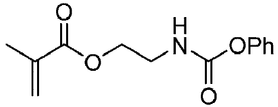 | 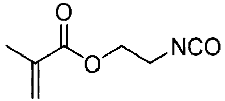 | 71 |
| 98 A | 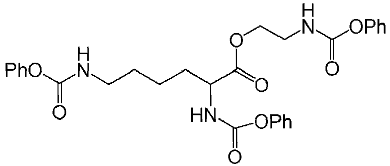 | 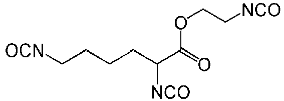 | 71 |

**Table 38**

| Ex. | Carbamate | Resultant isocyanate | Thermal decomposition yield (%) |
|---|---|---|---|
| 99 A | 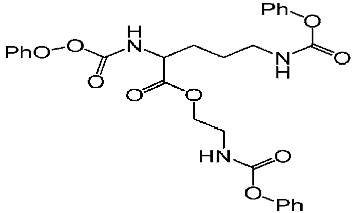 | 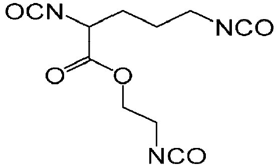 | 72 |
| 100 A | 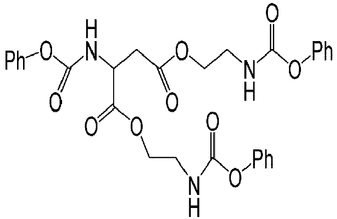 | 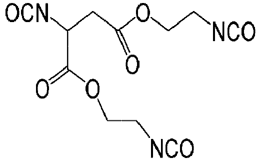 | 69 |
| 101 A | 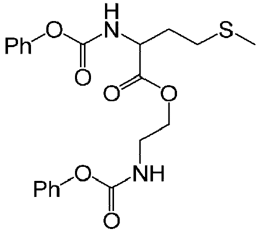 | 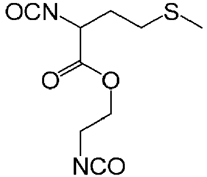 | 72 |
| 102 A | 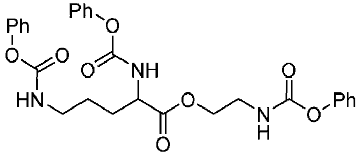 | 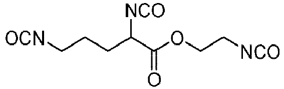 | 70 |
| 103 A | 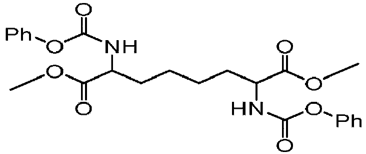 | 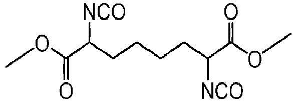 | 71 |

| | | | |
|---|---|---|---|
| (Ex: Example) | | | |

**Table 39**

| Ex. | Carbamate | Resultant isocyanate | Thermal decomposition yield (%) |
|---|---|---|---|
| 104 A | 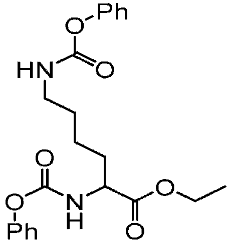 | 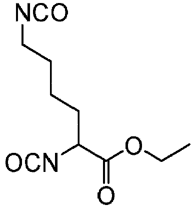 | 73 |

| | | | |
|---|---|---|---|
| (Ex: Example) | | | |

### Examples 91B to 104B

Each isocyanate was prepared by thermal decomposition of carbamate as conducted in Example 1-2 except that the compound of the formula (5-1-2b) was used as the compound A, benzophenone was used as an inert solvent and each carbamate shown in the following tables was used.

**Table 40**

| Ex. | Carbamate | Resultant isocyanate | Thermal decomposition yield (%) |
|---|---|---|---|
| 91B | 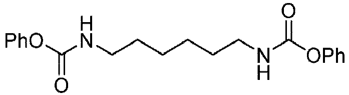 | 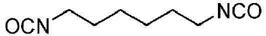 | 86 |
| 92B | 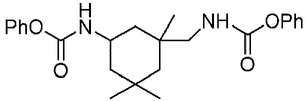 | 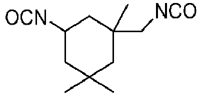 | 80 |
| 93B | 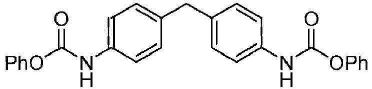 | 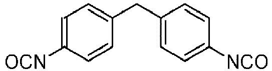 | 86 |
| 94B | 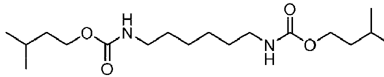 | 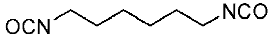 | 83 |
| 95B | 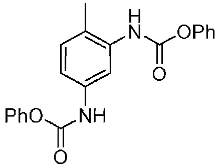 | 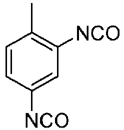 | 84 |
| 96B | 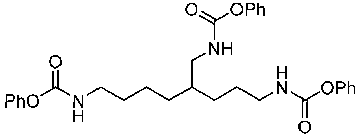 | 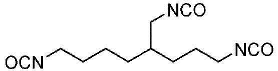 | 85 |
| 97B | 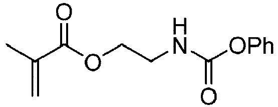 | 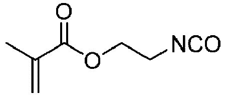 | 83 |
| 98B | 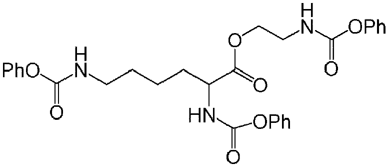 | 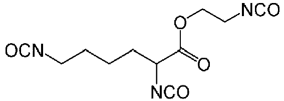 | 84 |

**Table 41**

| Ex. | Carbamate | Resultant isocyanate | Thermal decomposition yield (%) |
|---|---|---|---|
| 99B | 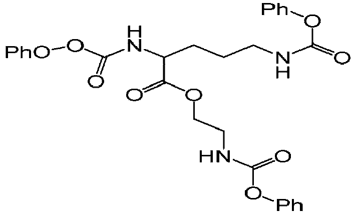 | 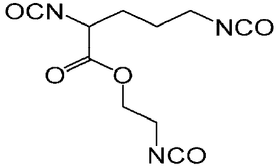 | 82 |
| 100B | 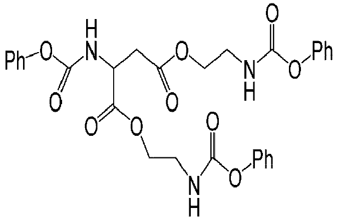 | 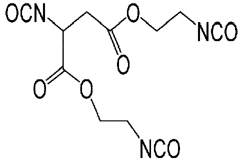 | 83 |
| 101B | 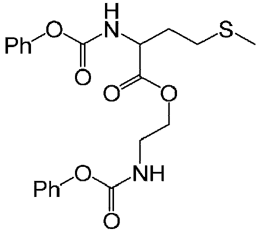 | 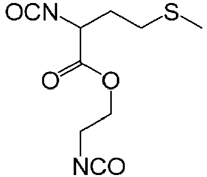 | 85 |
| 102B | 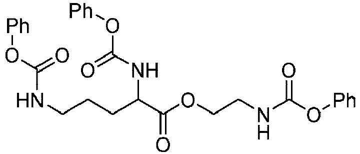 | 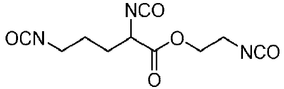 | 85 |
| 103B | 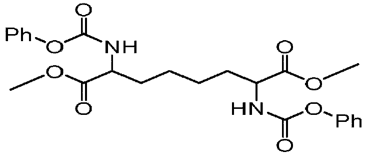 | 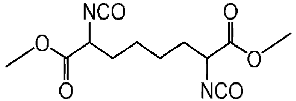 | 84 |

| | | | |
|---|---|---|---|
| (Ex: Example) | | | |

**Table 42**

| Ex. | Carbamate | Resultant isocyanate | Thermal decomposition yield (%) |
|---|---|---|---|
| 104B | 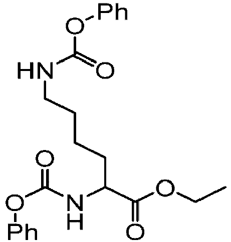 | 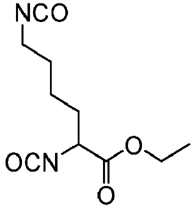 | 86 |

| | | | |
|---|---|---|---|
| (Ex: Example) | | | |

### Examples 105A to 118A

Each isocyanate was prepared by thermal decomposition of carbamate as conducted in Example 1-1 except that the compounds of the following formula (5-1a) were used as the compound A and each carbamate shown in the following tables was used.

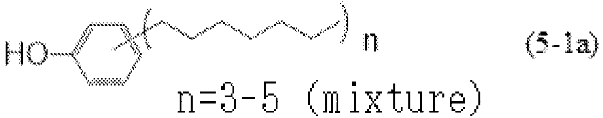

**Table 43**

| Ex. | Carbamate | Resultant isocyanate | Thermal decomposition yield (%) |
|---|---|---|---|
| 105A | 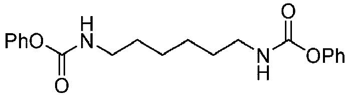 | 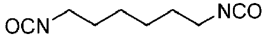 | 78 |
| 106A | 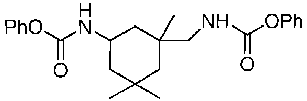 | 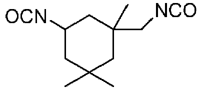 | 70 |
| 107A | 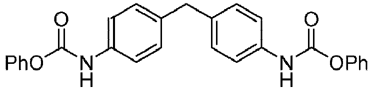 | 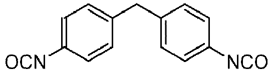 | 73 |
| 108A | 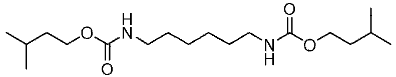 | 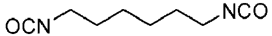 | 74 |
| 109A | 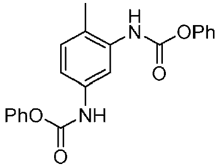 | 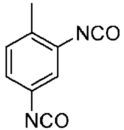 | 73 |
| 110A | 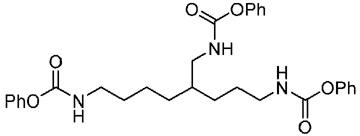 | 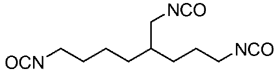 | 73 |
| 111A | 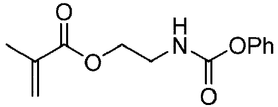 | 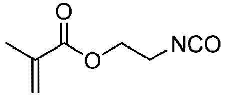 | 72 |
| 112A | 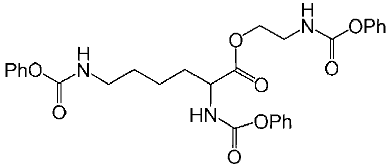 | 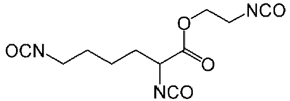 | 72 |

| | | | |
|---|---|---|---|
| (Ex: Example) | | | |

**Table 44**

| Ex. | Carbamate | Resultant isocyanate | Thermal decomposition yield (%) |
|---|---|---|---|
| 113A | 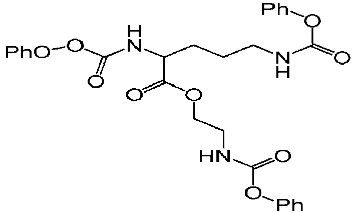 | 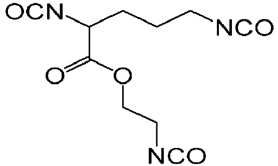 | 73 |
| 114A | 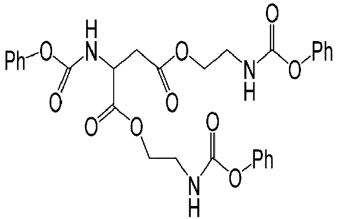 | 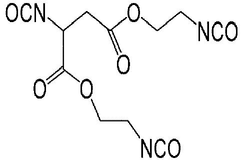 | 69 |
| 115A | 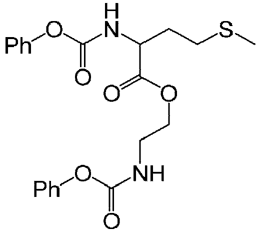 | 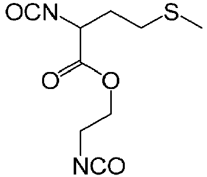 | 72 |
| 116A | 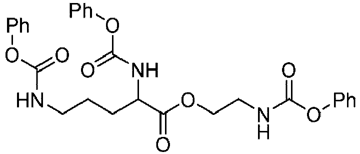 | 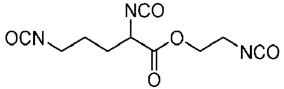 | 71 |
| 117A | 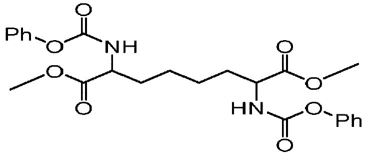 | 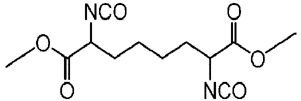 | 71 |

| | | | |
|---|---|---|---|
| (Ex: Example) | | | |

**Table 45**

| Ex. | Carbamate | Resultant isocyanate | Thermal decomposition yield (%) |
|---|---|---|---|
| 118A | 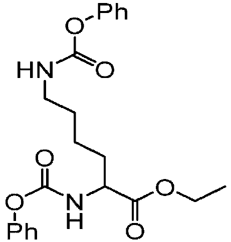 | 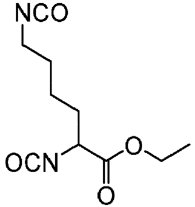 | 74 |

| | | | |
|---|---|---|---|
| (Ex: Example) | | | |

### Examples 105B to 118B

Each isocyanate was prepared by thermal decomposition of carbamate as conducted in Example 1-2 except that the compounds of the formula (5-1a) were used as the compound A, benzophenone was used an inert solvent, and each carbamate shown in the following tables was used.

**Table 46**

| Ex. | Carbamate | Resultant isocyanate | Thermal decomposition yield (%) |
|---|---|---|---|
| 105B | 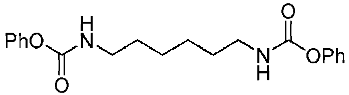 | 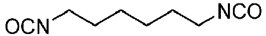 | 83 |
| 106B | 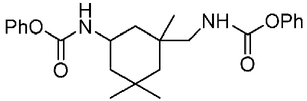 | 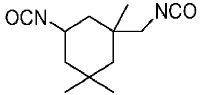 | 79 |
| 107B | 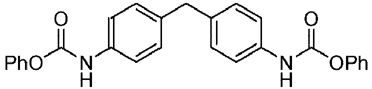 | 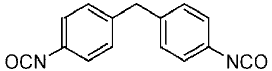 | 83 |
| 108B | 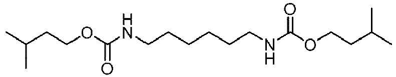 | 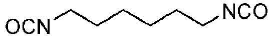 | 82 |
| 109B | 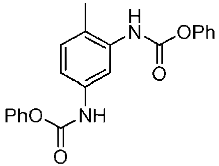 | 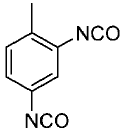 | 82 |
| 110B | 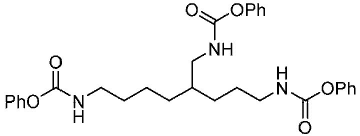 | 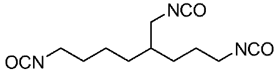 | 83 |
| 111B | 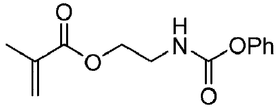 | 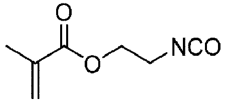 | 81 |
| 112B | 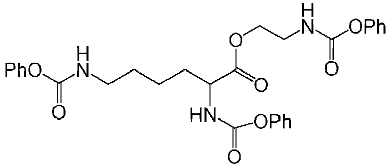 | 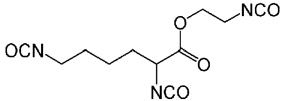 | 82 |

| | | | |
|---|---|---|---|
| (Ex: Example) | | | |

**Table 47**

| Ex. | Carbamate | Resultant isocyanate | Thermal decomposition yield (%) |
|---|---|---|---|
| 113B | 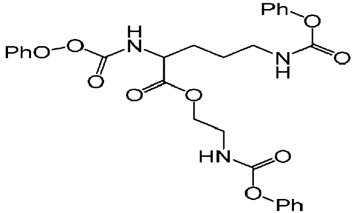 | 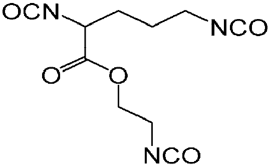 | 82 |
| 114B | 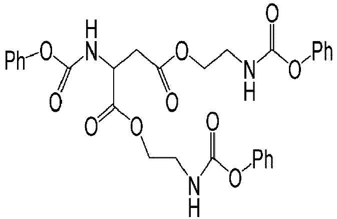 | 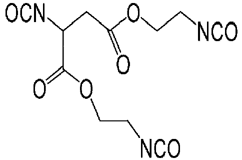 | 81 |
| 115B | 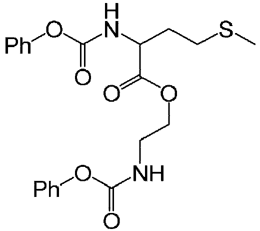 | 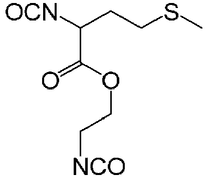 | 84 |
| 116B | 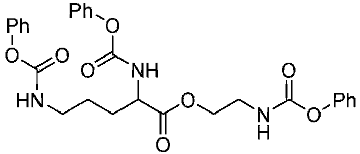 | 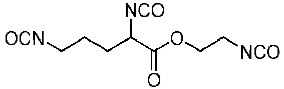 | 84 |
| 117B | 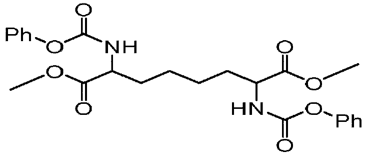 | 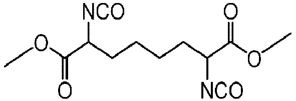 | 84 |

| | | | |
|---|---|---|---|
| (Ex: Example) | | | |

**Table 48**

| Ex. | Carbamate | Resultant isocyanate | Thermal decomposition yield (%) |
|---|---|---|---|
| 118B | 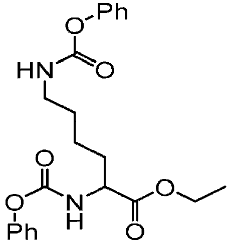 | 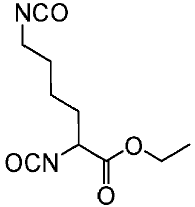 | 84 |

| | | | |
|---|---|---|---|
| (Ex: Example) | | | |

### Examples 119A to 132A

Each isocyanate was prepared by thermal decomposition of carbamate as conducted in Example 1-1 except that the compounds of the following formula (5-1b) were used as the compound A, and each carbamate shown in the following tables was used.

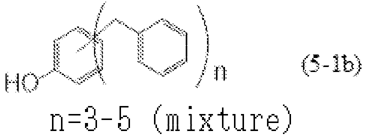

**Table 49**

| Ex. | Carbamate | Resultant isocyanate | Thermal decomposition yield (%) |
|---|---|---|---|
| 119 A | 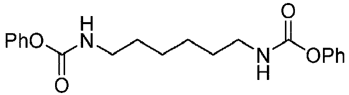 | 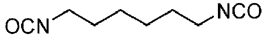 | 76 |
| 120 A | 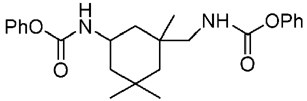 | 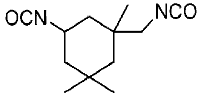 | 69 |
| 121 A | 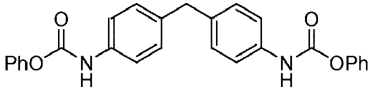 | 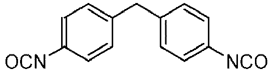 | 71 |
| 122 A | 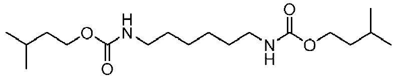 | 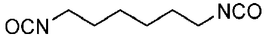 | 72 |
| 123 A | 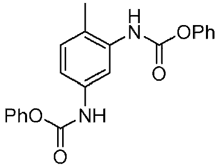 | 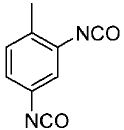 | 73 |
| 124 A | 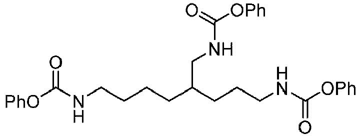 | 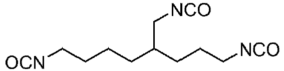 | 71 |
| 125 A | 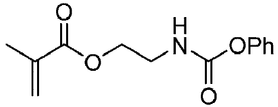 | 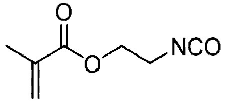 | 70 |
| 126 A | 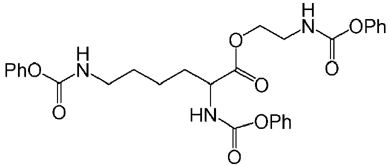 | 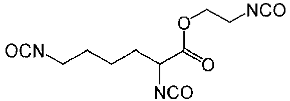 | 71 |

| | | | |
|---|---|---|---|
| (Ex: Example) | | | |

**Table 50**

| Ex. | Carbamate | Resultant isocyanate | Thermal decomposition yield (%) |
|---|---|---|---|
| 127 A | 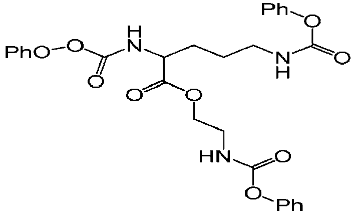 | 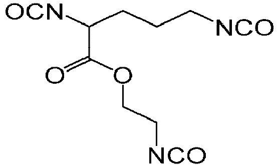 | 71 |
| 128 A | 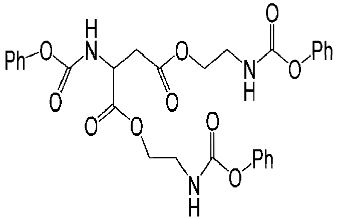 | 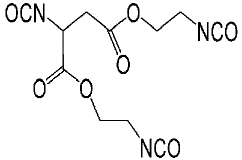 | 67 |
| 129 A | 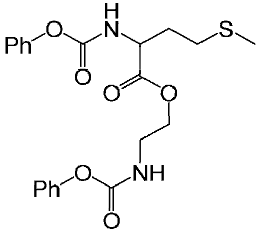 | 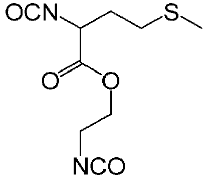 | 70 |
| 130 A | 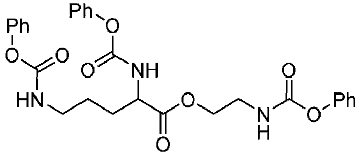 | 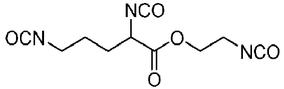 | 70 |
| 131 A | 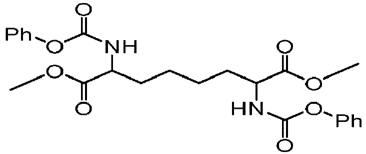 | 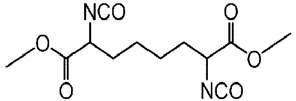 | 70 |

| | | | |
|---|---|---|---|
| (Ex: Example) | | | |

**Table 51**

| Ex. | Carbamate | Resultant isocyanate | Thermal decomposition yield (%) |
|---|---|---|---|
| 132 A | 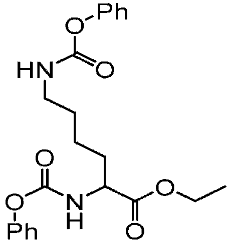 | 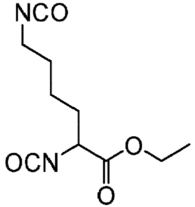 | 71 |

| | | | |
|---|---|---|---|
| (Ex: Example) | | | |

### Examples 119B to 132B

Each isocyanate was prepared by thermal decomposition of carbamate as conducted in Example 1-2 except that the compounds of formula (5-1b) were used as the compound A, benzophenone was used as an inert solvent and each carbamate shown in the following tables was used.

**Table 52**

| Ex. | Carbamate | Resultant isocyanate | Thermal decomposition yield (%) |
|---|---|---|---|
| 119 B | 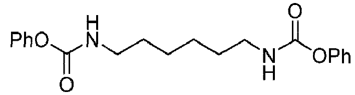 | 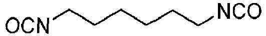 | 82 |
| 12C B | 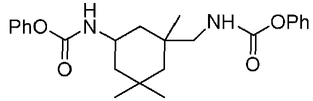 | 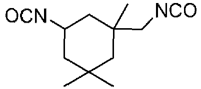 | 79 |
| 121 B | 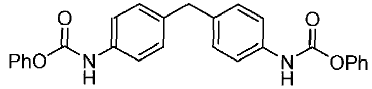 | 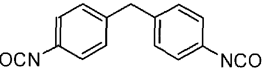 | 82 |
| 122 B | 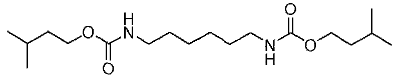 | 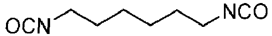 | 82 |
| 123 B | 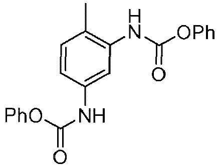 | 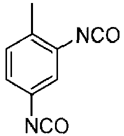 | 81 |
| 124 B | 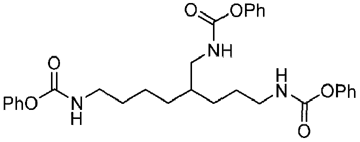 | 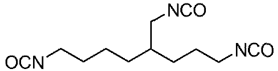 | 82 |
| 125 B | 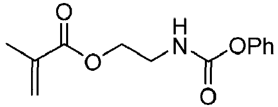 | 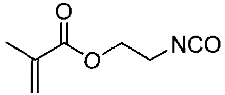 | 82 |
| 126 B | 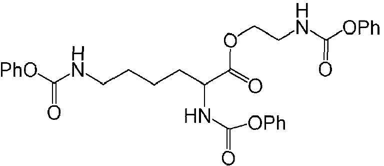 | 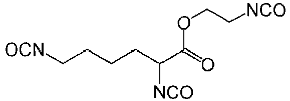 | 81 |

**Table 53**

| Ex. | Carbamate | Resultant isocyanate | Thermal decomposition yield (%) |
|---|---|---|---|
| 127 B | 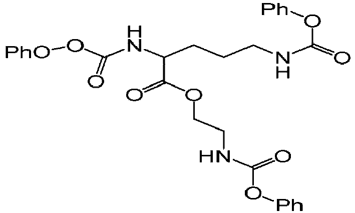 | 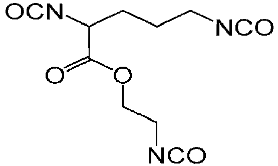 | 82 |
| 128 B | 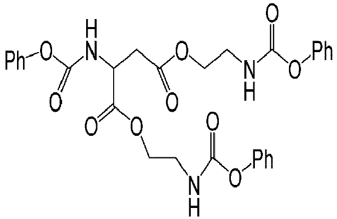 | 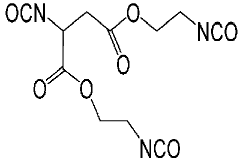 | 79 |
| 129 B | 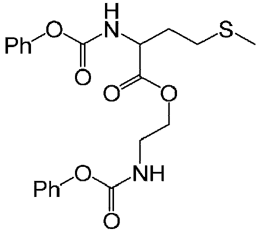 | 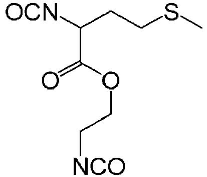 | 83 |
| 130 B | 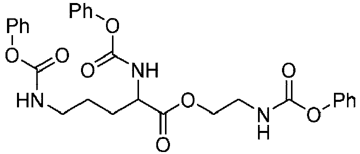 | 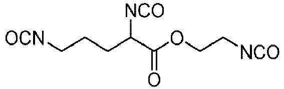 | 82 |
| 131 B | 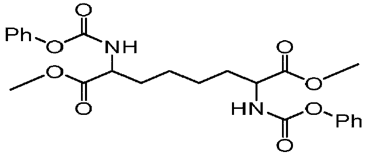 | 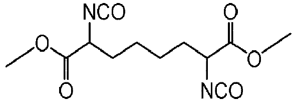 | 83 |

| | | | |
|---|---|---|---|
| (Ex: Example) | | | |

**Table 54**

| Ex. | Carbamate | Resultant isocyanate | Thermal decomposition yield (%) |
|---|---|---|---|
| 132 B | 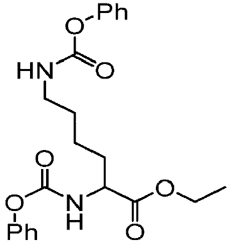 | 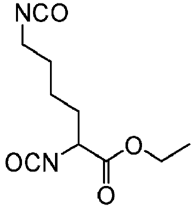 | 83 |

| | | | |
|---|---|---|---|
| (Ex: Example) | | | |

### Examples 133A to 146A

Each isocyanate was prepared by thermal decomposition of carbamate as conducted in Example 1-1 except that the compounds of the following formula (5-1c) were used as the compound A and each carbamate shown in the following tables was used.

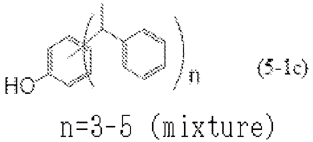

**Table 55**

| Ex. | Carbamate | Resultant isocyanate | Thermal decomposition yield (%) |
|---|---|---|---|
| 133 A | 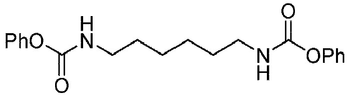 | 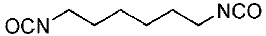 | 76 |
| 134 A | 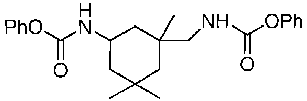 | 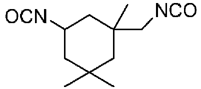 | 68 |
| 135 A | 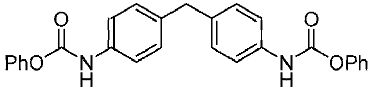 | 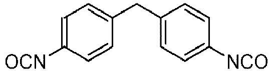 | 71 |
| 136 A | 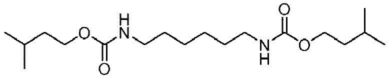 | 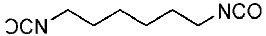 | 71 |
| 137 A | 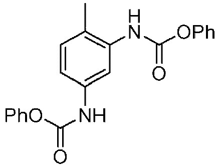 | 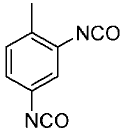 | 72 |
| 138 A | 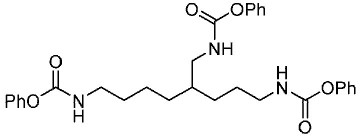 | 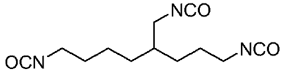 | 70 |
| 139 A | 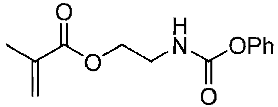 | 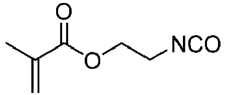 | 70 |
| 140 A | 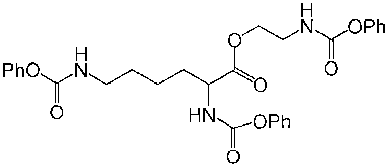 | 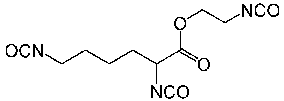 | 70 |

| | | | |
|---|---|---|---|
| (Ex: Example) | | | |

**Table 56**

| Ex. | Carbamate | Resultant isocyanate | Thermal decomposition yield (%) |
|---|---|---|---|
| 141 A | 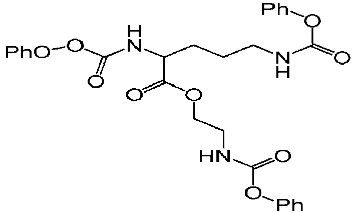 | 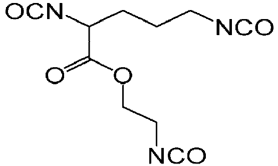 | 71 |
| 142 A | 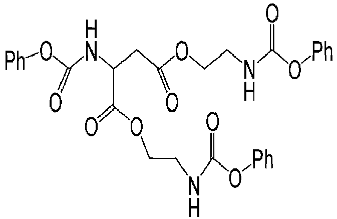 | 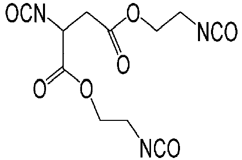 | 68 |
| 143 A | 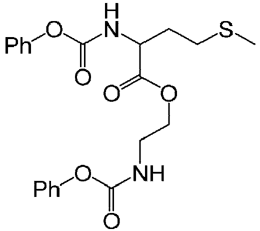 | 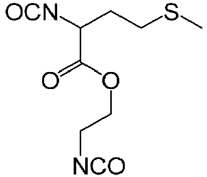 | 70 |
| 144 A | 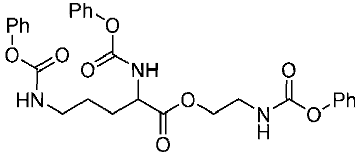 | 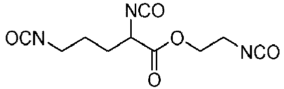 | 71 |
| 145 A | 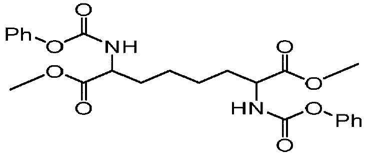 | 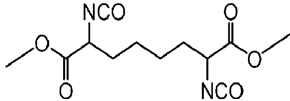 | 70 |

| | | | |
|---|---|---|---|
| (Ex: Example) | | | |

**Table 57**

| Ex. | Carbamate | Resultant isocyanate | Thermal decomposition yield (%) |
|---|---|---|---|
| 146 A | 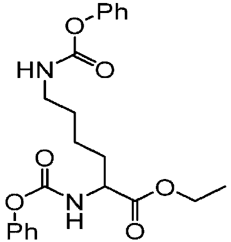 | 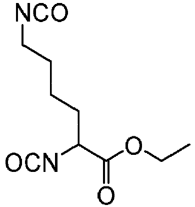 | 71 |

| | | | |
|---|---|---|---|
| (Ex: Example) | | | |

### Examples 133B to 146B

Each isocyanate was prepared by thermal decomposition of carbamate as conducted in Example 1-2 except that the compounds of the formula (5-1c) were used as the compound A, benzophenone was used as an inert solvent and each carbamate shown in the following tables was used.

**Table 58**

| Ex. | Carbamate | Resultant isocyanate | Thermal decomposition yield (%) |
|---|---|---|---|
| 133 B | 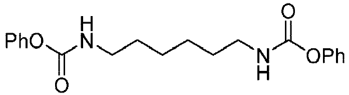 | 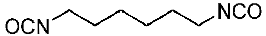 | 81 |
| 134 B | 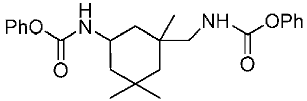 | 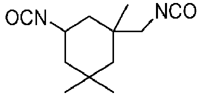 | 78 |
| 135 B | 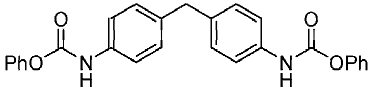 | 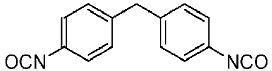 | 82 |
| 136 B | 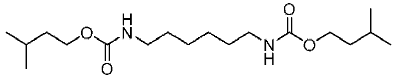 | 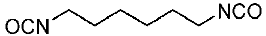 | 81 |
| 137 B | 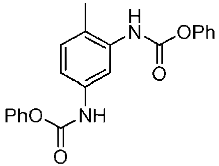 | 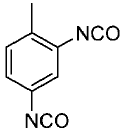 | 81 |
| 138 B | 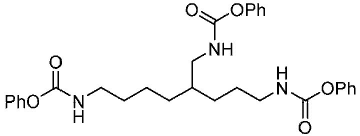 | 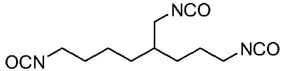 | 81 |
| 139 B | 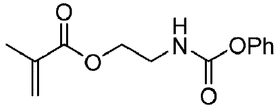 | 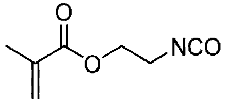 | 81 |
| 140 B | 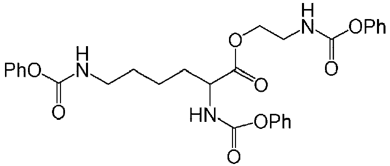 | 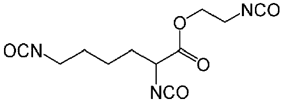 | 80 |

| | | | |
|---|---|---|---|
| (Ex: Example) | | | |

**Table 59**

| Ex. | Carbamate | Resultant isocyanate | Thermal decomposition yield (%) |
|---|---|---|---|
| 141 B | 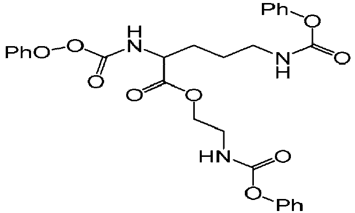 | 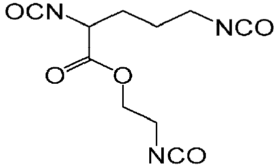 | 81 |
| 142 B | 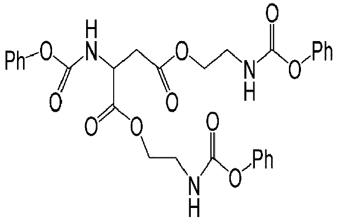 | 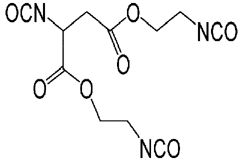 | 78 |
| 143 B | 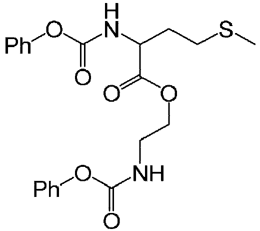 | 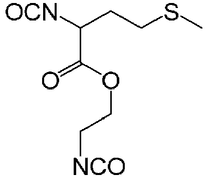 | 82 |
| 144 B | 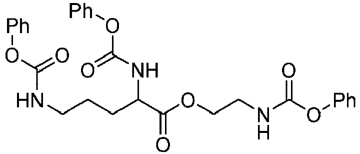 | 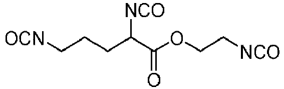 | 81 |
| 145 B | 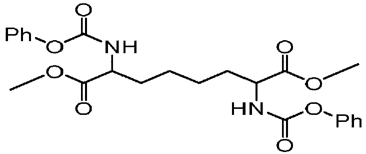 | 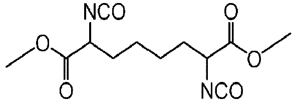 | 82 |

| | | | |
|---|---|---|---|
| (Ex: Example) | | | |

**Table 60**

| Ex. | Carbamate | Resultant isocyanate | Thermal decomposition yield (%) |
|---|---|---|---|
| 146 B | 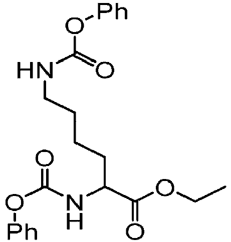 | 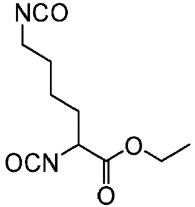 | 82 |

| | | | |
|---|---|---|---|
| (Ex: Example) | | | |

### Examples 147A to 160A

Each isocyanate was prepared by thermal decomposition of carbamate as conducted in Example 2-1 except that a compound of the following formula (6-2-1b) was used as the compound A, benzophenone was used as an inert solvent and each carbamate shown in the following tables was used.

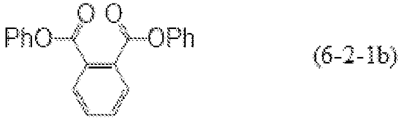

**Table 61**

| Ex. | Carbamate | Resultant isocyanate | Thermal decomposition yield (%) |
|---|---|---|---|
| 147 A | 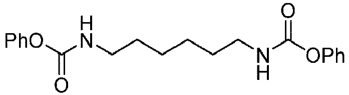 | 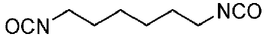 | 79 |
| 148 A | 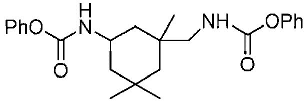 | 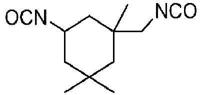 | 71 |
| 149 A | 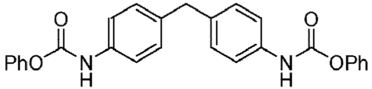 | 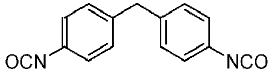 | 74 |
| 150 A | 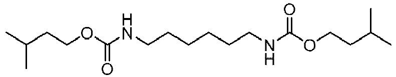 | 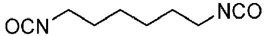 | 74 |
| 151 A | 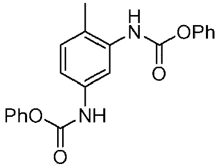 | 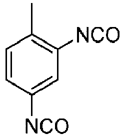 | 75 |
| 152 A | 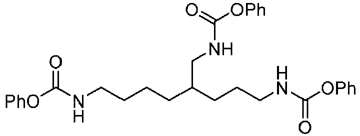 | 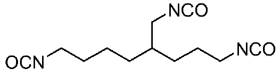 | 74 |
| 153 A | 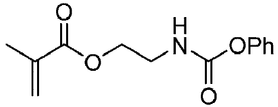 | 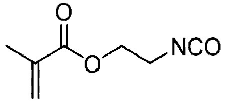 | 73 |
| 154 A | 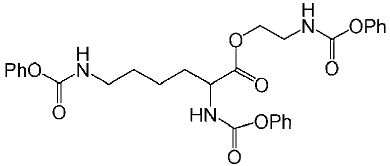 | 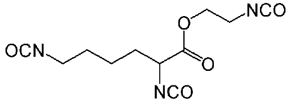 | 72 |

**Table 62**

| Ex. | Carbamate | Resultant isocyanate | Thermal decomposition yield (%) |
|---|---|---|---|
| 155 A | 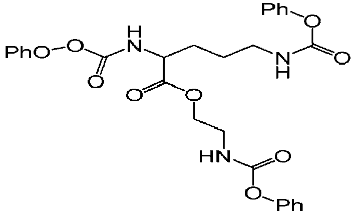 | 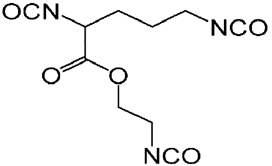 | 71 |
| 156 A | 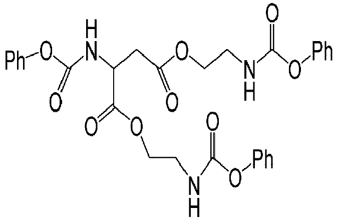 | 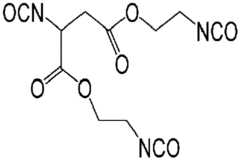 | 70 |
| 157 A | 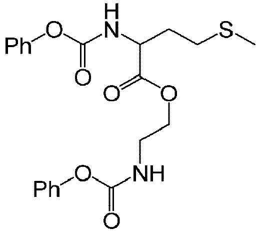 | 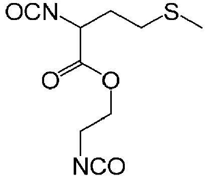 | 71 |
| 158 A | 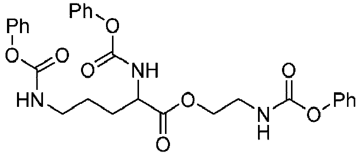 | 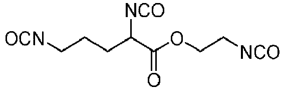 | 72 |
| 159 A | 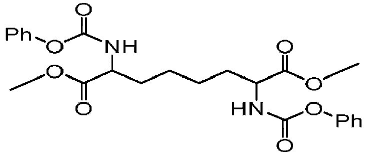 | 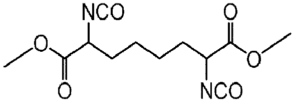 | 73 |

| | | | |
|---|---|---|---|
| (Ex: Example) | | | |

**Table 63**

| Ex. | Carbamate | Resultant isocyanate | Thermal decomposition yield (%) |
|---|---|---|---|
| 160 A | 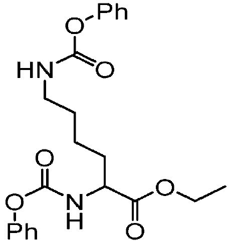 | 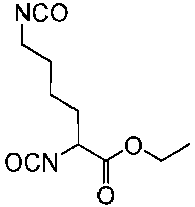 | 73 |

| | | | |
|---|---|---|---|
| (Ex: Example) | | | |

### Examples 147B to 160B

Each isocyanate was prepared by thermal decomposition of carbamate as conducted in Example 2-2 except that the compound of the formula (6-2-1b) was used as the compound A, benzophenone was used as an inert solvent and each carbamate shown in the following tables was used.

**Table 64**

| Ex. | Carbamate | Resultant isocyanate | Thermal decomposition yield (%) |
|---|---|---|---|
| 147 B | 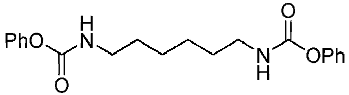 | 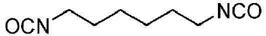 | 89 |
| 148 B | 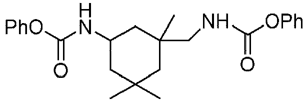 | 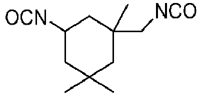 | 84 |
| 149 B | 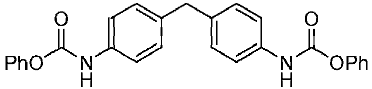 | 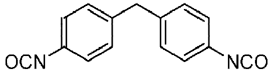 | 87 |
| 150 B | 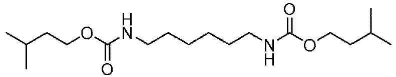 | 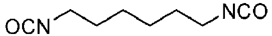 | 85 |
| 151 B | 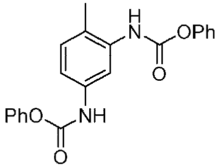 | 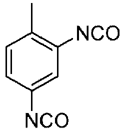 | 86 |
| 152 B | 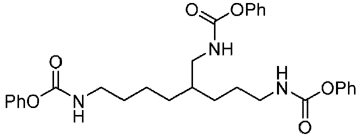 | 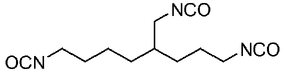 | 85 |
| 153 B | 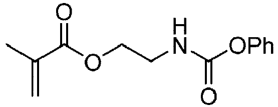 | 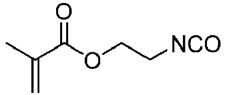 | 83 |
| 154 B | 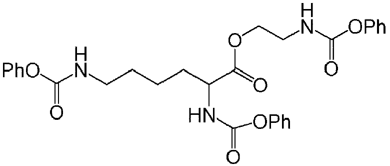 | 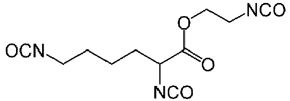 | 84 |

| | | | |
|---|---|---|---|
| (Ex: Example) | | | |

**Table 65**

| Ex. | Carbamate | Resultant isocyanate | Thermal decomposition yield (%) |
|---|---|---|---|
| 155 B | 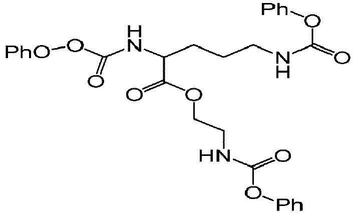 | 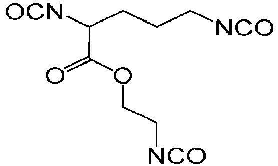 | 84 |
| 156 B | 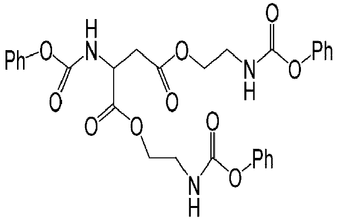 | 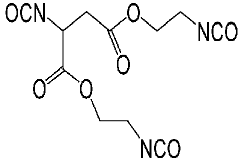 | 81 |
| 157 B | 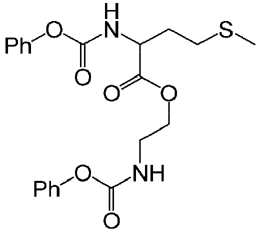 | 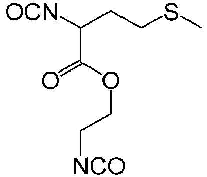 | 86 |
| 158 B | 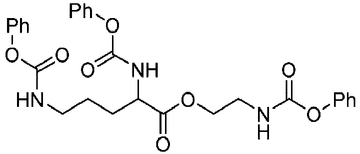 | 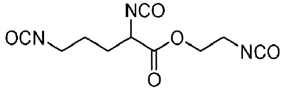 | 86 |
| 159 B | 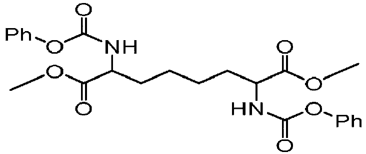 | 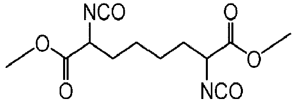 | 85 |

| | | | |
|---|---|---|---|
| (Ex: Example) | | | |

**Table 66**

| Ex. | Carbamate | Resultant isocyanate | Thermal decomposition yield (%) |
|---|---|---|---|
| 160 B | 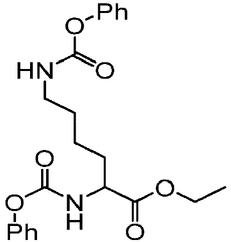 | 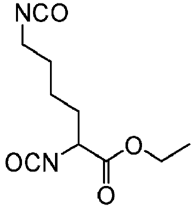 | 84 |

| | | | |
|---|---|---|---|
| (Ex: Example) | | | |

### Examples 161A to 174A

Each isocyanate was prepared by thermal decomposition of carbamate as conducted in Example 2-1 except that a compound of the following formula (6-2-1c) was used as the compound A, benzophenone was used as an inert solvent and each carbamate shown in the following tables was used.

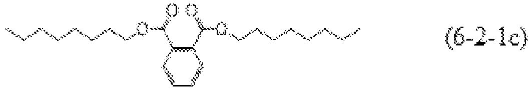

**Table 67**

| Ex. | Carbamate | Resultant isocyanate | Thermal decomposition yield (%) |
|---|---|---|---|
| 161 A | 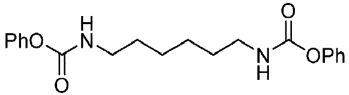 | 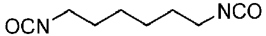 | 78 |
| 162 A | 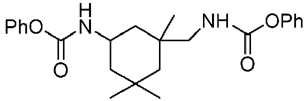 | 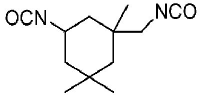 | 72 |
| 163 A | 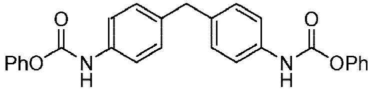 | 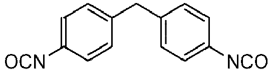 | 73 |
| 164 A | 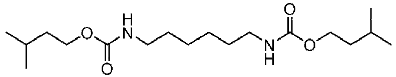 | 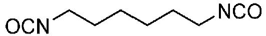 | 72 |
| 165 A | 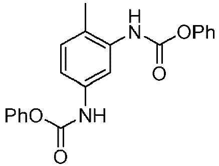 | 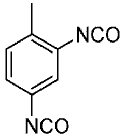 | 76 |
| 166 A | 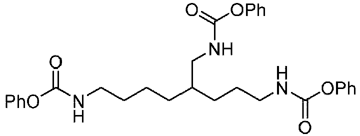 | 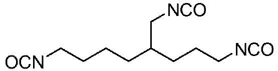 | 73 |
| 167 A | 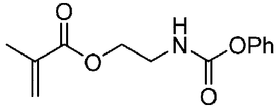 | 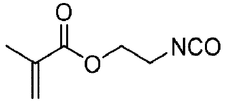 | 73 |
| 168 A | 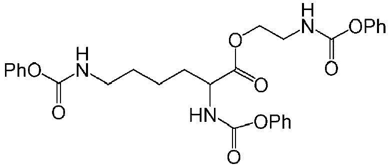 | 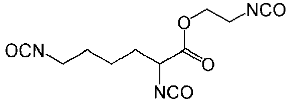 | 72 |

| | | | |
|---|---|---|---|
| (Ex: Example) | | | |

**Table 68**

| Ex. | Carbamate | Resultant isocyanate | Thermal decomposition yield (%) |
|---|---|---|---|
| 169 A | 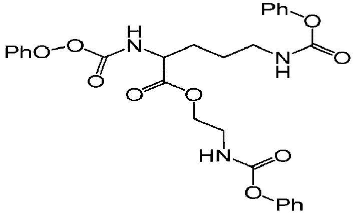 | 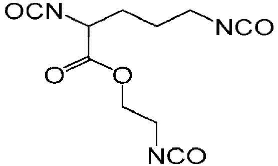 | 71 |
| 170 A | 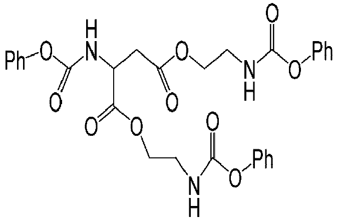 | 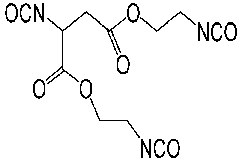 | 71 |
| 171 A | 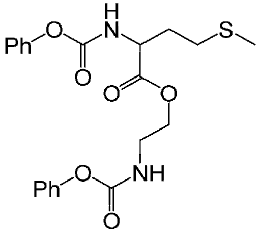 | 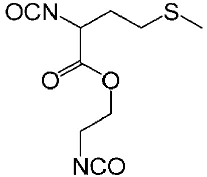 | 72 |
| 172 A | 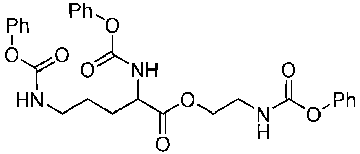 | 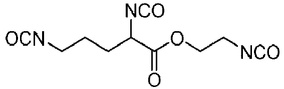 | 73 |
| 173 A | 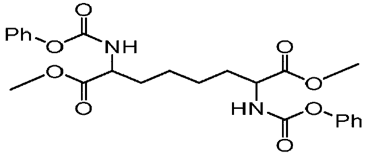 | 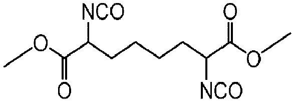 | 72 |

| | | | |
|---|---|---|---|
| (Ex: Example) | | | |

**Table 69**

| Ex. | Carbamate | Resultant isocyanate | Thermal decomposition yield (%) |
|---|---|---|---|
| 174 A | 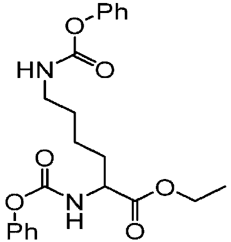 | 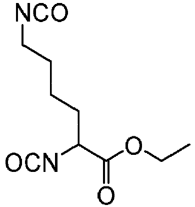 | 72 |

| | | | |
|---|---|---|---|
| (Ex: Example) | | | |

### Examples 161B to 174B

Each isocyanate was prepared by thermal decomposition of carbamate as conducted in Example 2-2 except that the compound of the formula (6-2-1c) was used as the compound A, benzophenone was used as an inert solvent and each carbamate shown in the following tables was used.

**Table 70**

| Ex. | Carbamate | Resultant isocyanate | Thermal decomposition yield (%) |
|---|---|---|---|
| 161 B | 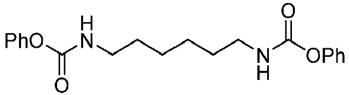 | 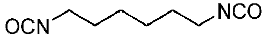 | 89 |
| 162 B | 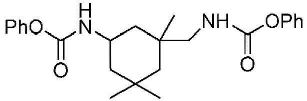 | 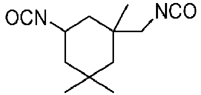 | 85 |
| 163 B | 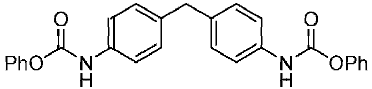 | 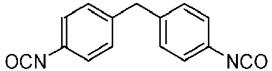 | 86 |
| 164 B | 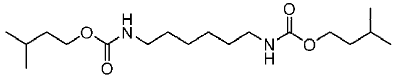 | 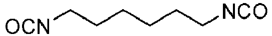 | 85 |
| 165 B | 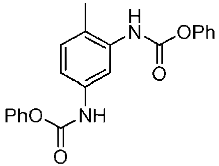 | 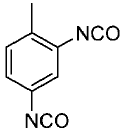 | 85 |
| 166 B | 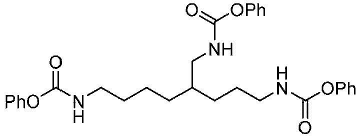 | 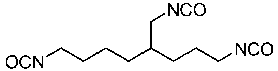 | 86 |
| 167 B | 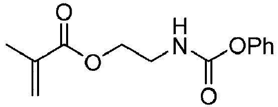 | 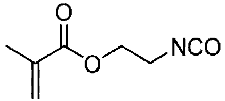 | 84 |
| 168 B | 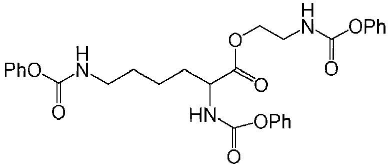 | 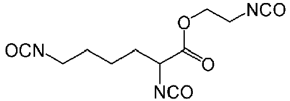 | 85 |

| | | | |
|---|---|---|---|
| (Ex: Example) | | | |

**Table 71**

| Ex. | Carbamate | Resultant isocyanate | Thermal decomposition yield (%) |
|---|---|---|---|
| 169 B | 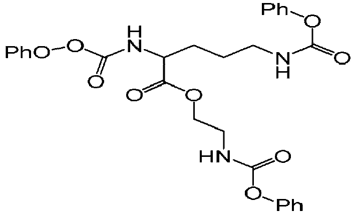 | 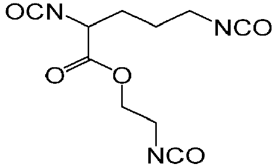 | 85 |
| 170 B | 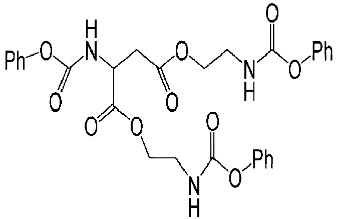 | 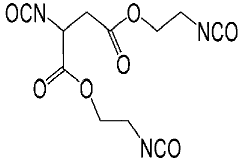 | 82 |
| 171 B | 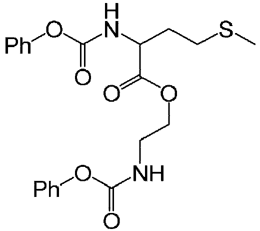 | 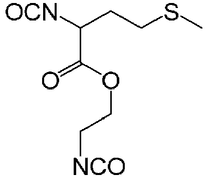 | 86 |
| 172 B | 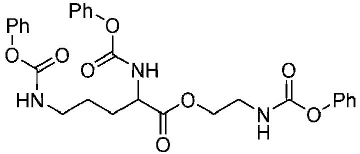 | 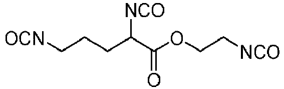 | 85 |
| 173 B | 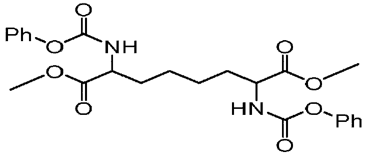 | 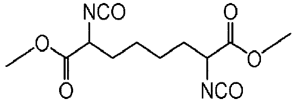 | 85 |

| | | | |
|---|---|---|---|
| (Ex: Example) | | | |

**Table 72**

| Ex. | Carbamate | Resultant isocyanate | Thermal decomposition yield (%) |
|---|---|---|---|
| 174 B | 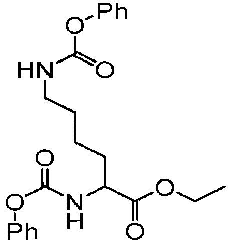 | 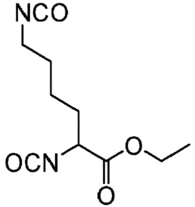 | 84 |

| | | | |
|---|---|---|---|
| (Ex: Example) | | | |

### Examples 175A to 188A

Each isocyanate was prepared by thermal decomposition of carbamate as conducted in Example 2-1 except that a compound of the following formula (6-3a) was used as the compound A, benzophenone was used as an inert solvent and each carbamate shown in the following tables was used.

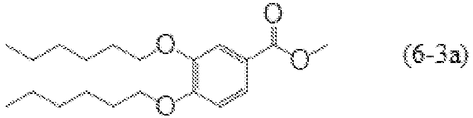

**Table 73**

| Ex. | Carbamate | Resultant isocyanate | Thermal decomposition yield (%) |
|---|---|---|---|
| 175 A | 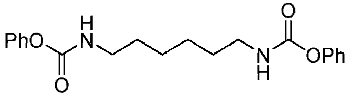 | 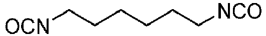 | 79 |
| 176 A | 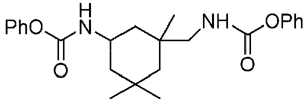 | 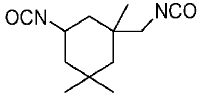 | 72 |
| 177 A | 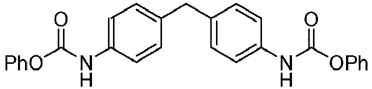 | 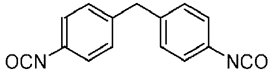 | 72 |
| 178 A | 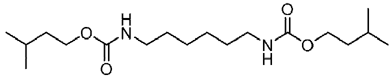 | 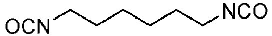 | 73 |
| 179 A | 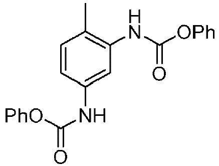 | 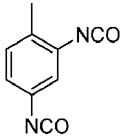 | 75 |
| 180 A | 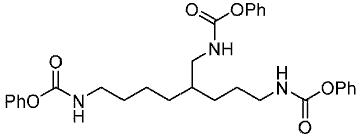 | 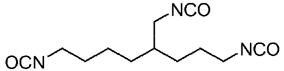 | 72 |
| 181 A | 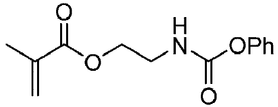 | 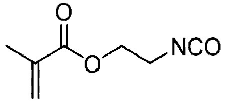 | 72 |
| 182 A | 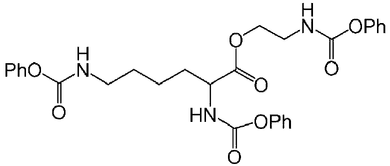 | 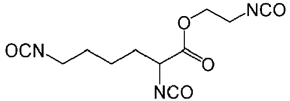 | 71 |

| | | | |
|---|---|---|---|
| (Ex: Example) | | | |

**Table 74**

| Ex. | Carbamate | Resultant isocyanate | Thermal decomposition yield (%) |
|---|---|---|---|
| 183 A | 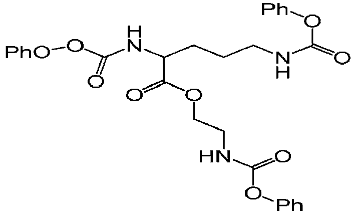 | 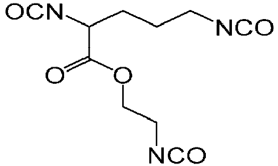 | 72 |
| 184 A | 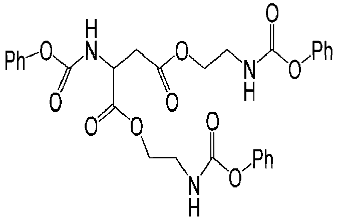 | 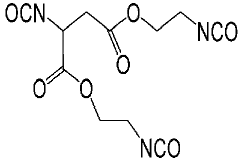 | 73 |
| 185 A | 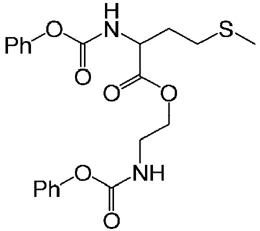 | 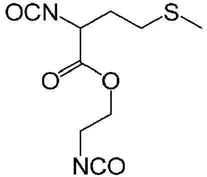 | 72 |
| 186 A | 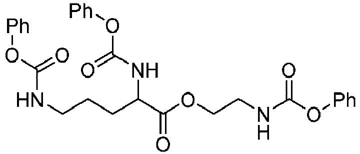 | 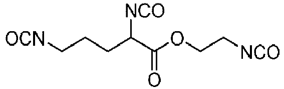 | 72 |
| 187 A | 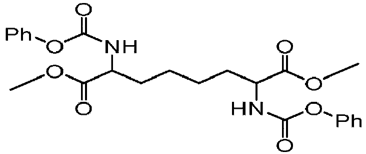 | 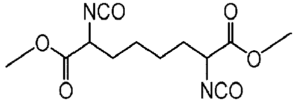 | 73 |

| | | | |
|---|---|---|---|
| (Ex: Example) | | | |

**Table 75**

| Ex. | Carbamate | Resultant isocyanate | Thermal decomposition yield (%) |
|---|---|---|---|
| 188 A | 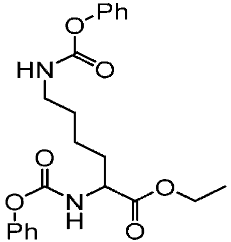 | 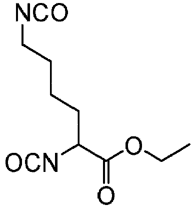 | 73 |

| | | | |
|---|---|---|---|
| (Ex: Example) | | | |

### Examples 175B to 188B

Each isocyanate was prepared by thermal decomposition of carbamate as conducted in Example 2-2 except that the compound of the formula (6-3a) was used as the compound A, benzophenone was used as an inert solvent and each carbamate shown in the following tables was used.

**Table 76**

| Ex. | Carbamate | Resultant isocyanate | Thermal decomposition yield (%) |
|---|---|---|---|
| 175 B | 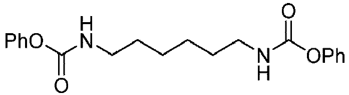 | 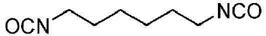 | 88 |
| 176 B | 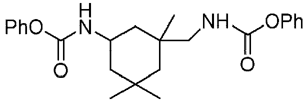 | 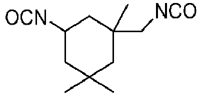 | 84 |
| 177 B | 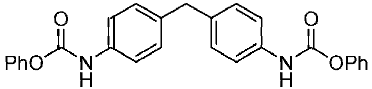 | 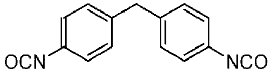 | 86 |
| 178 B | 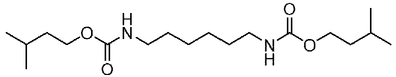 | 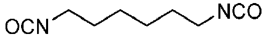 | 85 |
| 179 B | 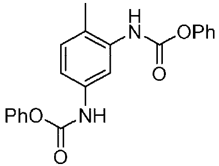 | 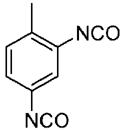 | 86 |
| 180 B | 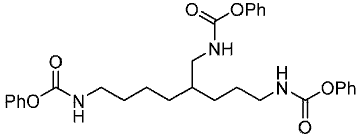 | 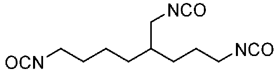 | 85 |
| 181 B | 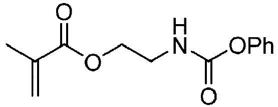 | 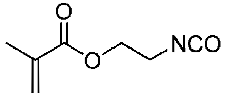 | 83 |
| 182 B | 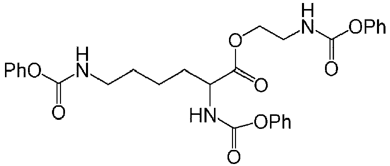 | 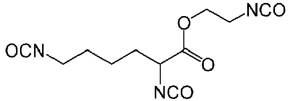 | 84 |

| | | | |
|---|---|---|---|
| (Ex: Example) | | | |

**Table 77**

| Ex. | Carbamate | Resultant isocyanate | Thermal decomposition yield (%) |
|---|---|---|---|
| 183 B | 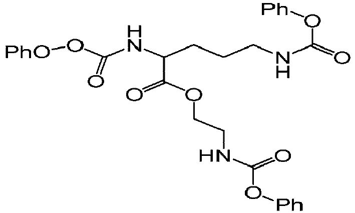 | 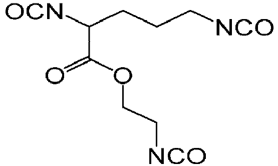 | 84 |
| 184 B | 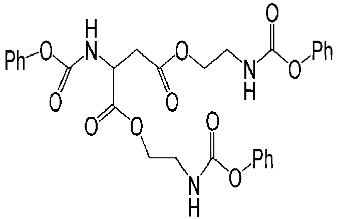 | 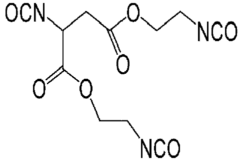 | 81 |
| 185 B | 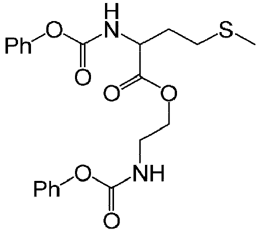 | 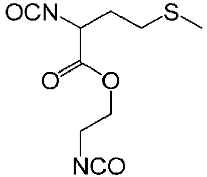 | 86 |
| 186 B | 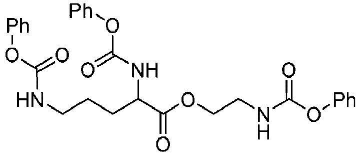 | 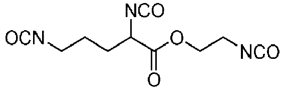 | 86 |
| 187 B | 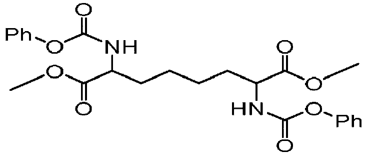 | 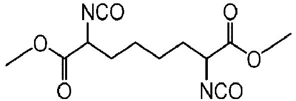 | 85 |

| | | | |
|---|---|---|---|
| (Ex: Example) | | | |

**Table 78**

| Ex. | Carbamate | Resultant isocyanate | Thermal decomposition yield (%) |
|---|---|---|---|
| 188 B | 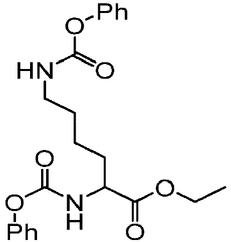 | 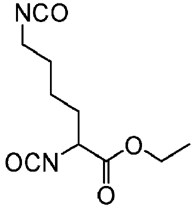 | 84 |

| | | | |
|---|---|---|---|
| (Ex: Example) | | | |

### Examples 189A to 202A

Each isocyanate was prepared by thermal decomposition of carbamate as conducted in Example 2-1 except that tris(2-ethylhexyl) trimellitate of the following formula (6-2-1a) was used as the compound A, triethylbenzene was used as an inert solvent and each carbamate shown in the following tables was used.

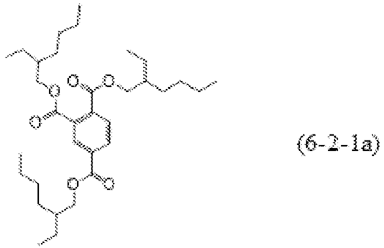

**Table 79**

| Ex. | Carbamate | Resultant isocyanate | Thermal decomposition yield (%) |
|---|---|---|---|
| 189 A | 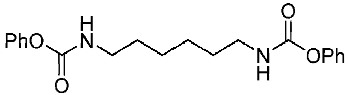 | 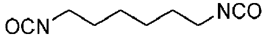 | 78 |
| 190 A | 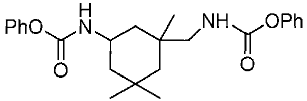 | 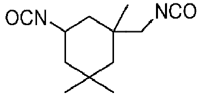 | 71 |
| 191 A | 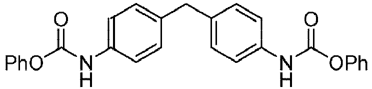 | 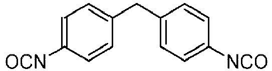 | 72 |
| 192 A | 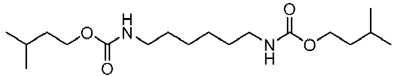 | 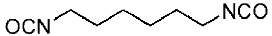 | 73 |
| 193 A | 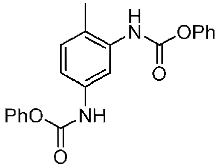 | 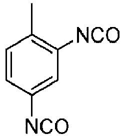 | 74 |
| 194 A | 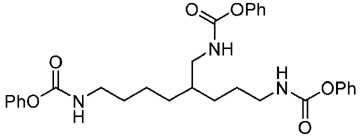 | 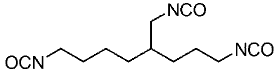 | 72 |
| 195 A | 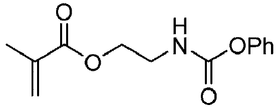 | 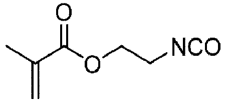 | 72 |
| 196 A | 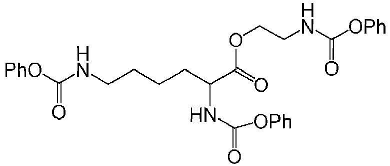 | 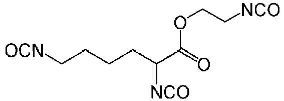 | 72 |

| | | | |
|---|---|---|---|
| (Ex: Example) | | | |

**Table 80**

| Ex. | Carbamate | Resultant isocyanate | Thermal decomposition yield (%) |
|---|---|---|---|
| 197 A | 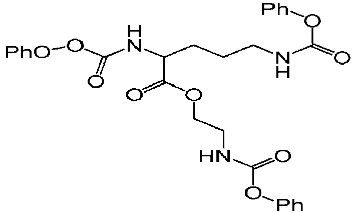 | 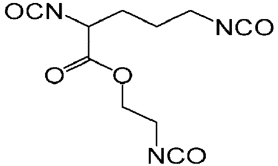 | 71 |
| 198 A | 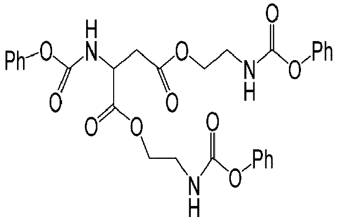 | 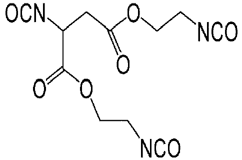 | 72 |
| 199 A | 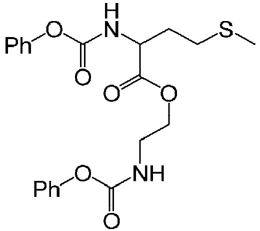 | 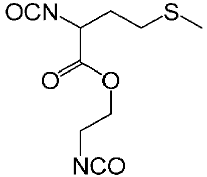 | 72 |
| 200 A | 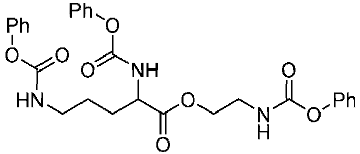 | 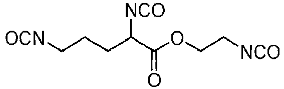 | 71 |
| 201 A | 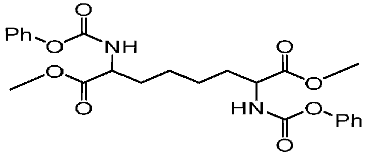 | 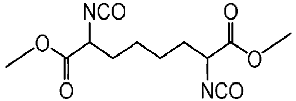 | 72 |

| | | | |
|---|---|---|---|
| (Ex: Example) | | | |

**Table 81**

| Ex. | Carbamate | Resultant isocyanate | Thermal decomposition yield (%) |
|---|---|---|---|
| 202 A | 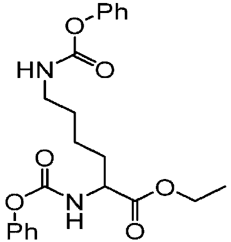 | 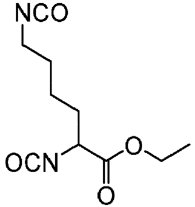 | 73 |

| | | | |
|---|---|---|---|
| (Ex: Example) | | | |

### Examples 189B to 202B

Each isocyanate was prepared by thermal decomposition of carbamate as conducted in Example 2-2 except that each carbamate shown in the following tables was used.

**Table 82**

| Ex. | Carbamate | Resultant isocyanate | Thermal decomposition yield (%) |
|---|---|---|---|
| 189 B | 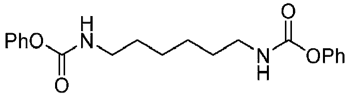 | 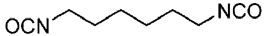 | 89 |
| 190 B | 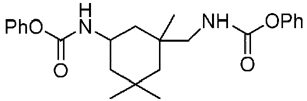 | 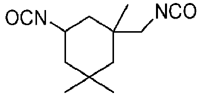 | 85 |
| 191 B | 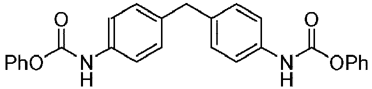 | 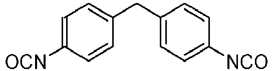 | 86 |
| 192 B | 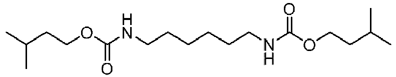 | 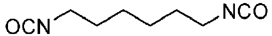 | 85 |
| 193 B | 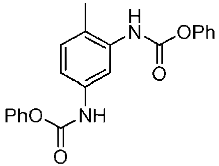 | 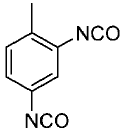 | 87 |
| 194 B | 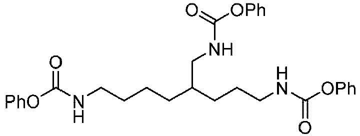 | 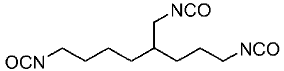 | 86 |
| 195 B | 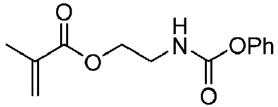 | 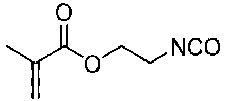 | 84 |
| 196 B | 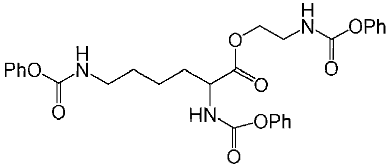 | 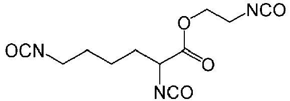 | 85 |

| | | | |
|---|---|---|---|
| (Ex: Example) | | | |

**Table 83**

| Ex. | Carbamate | Resultant isocyanate | Thermal decomposition yield (%) |
|---|---|---|---|
| 197 B | 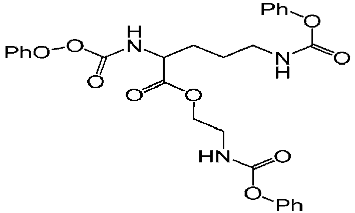 | 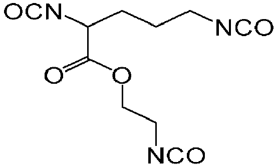 | 84 |
| 198 B | 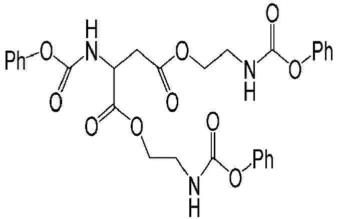 | 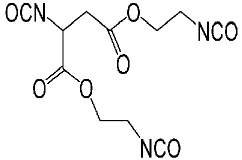 | 83 |
| 199 B | 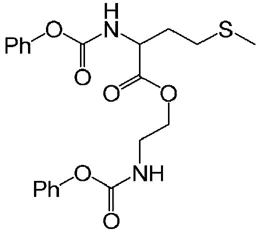 | 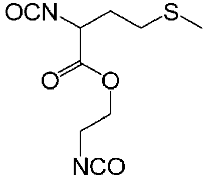 | 86 |
| 200 B | 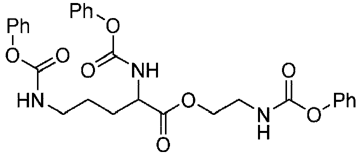 | 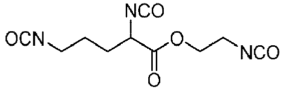 | 86 |
| 201 B | 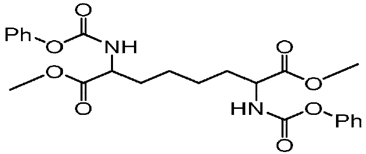 | 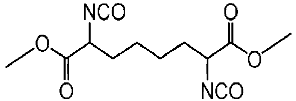 | 85 |

| | | | |
|---|---|---|---|
| (Ex: Example) | | | |

**Table 84**

| Ex. | Carbamate | Resultant isocyanate | Thermal decomposition yield (%) |
|---|---|---|---|
| 202 B | 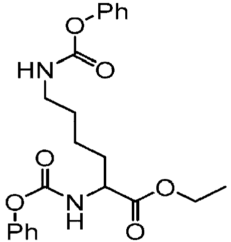 | 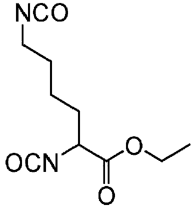 | 84 |

| | | | |
|---|---|---|---|
| (Ex: Example) | | | |

### Examples 203A to 214A

Each isocyanate was prepared by thermal decomposition of carbamate as conducted in Example 2-1 except that diisononyl adipate of the following formula (6-1-1a) was used as the compound A, triethylbenzene was used as an inert solvent, and each carbamate shown in the following tables was used.

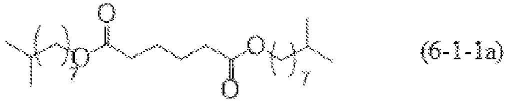

**Table 85**

| Ex. | Carbamate | Resultant isocyanate | Thermal decomposition yield (%) |
|---|---|---|---|
| 203 A | 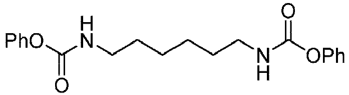 | 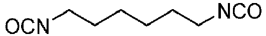 | 77 |
| 204 A | 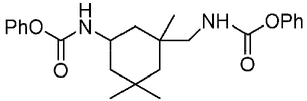 | 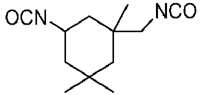 | 71 |
| 205 A | 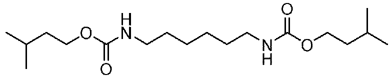 | 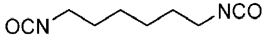 | 73 |
| 206 A | 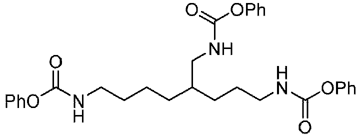 | 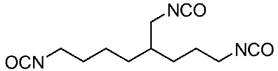 | 71 |
| 207 A | 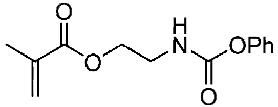 | 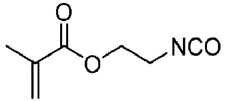 | 71 |
| 208 A | 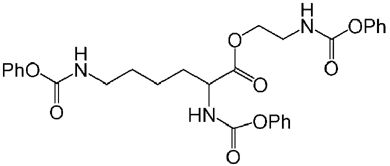 | 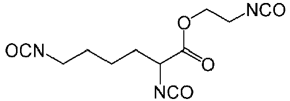 | 71 |

### (Ex: Example)

**Table 86**

| Ex. | Carbamate | Resultant isocyanate | Thermal decomposition yield (%) |
|---|---|---|---|
| 209 A | 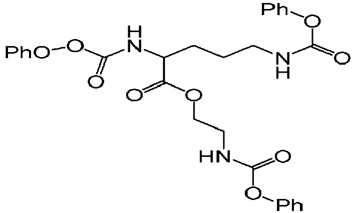 | 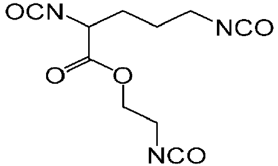 | 72 |
| 210 A | 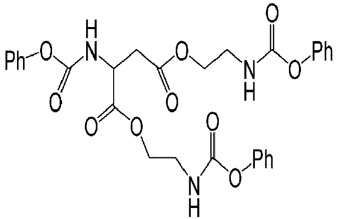 | 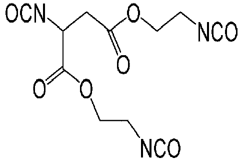 | 72 |
| 211 A | 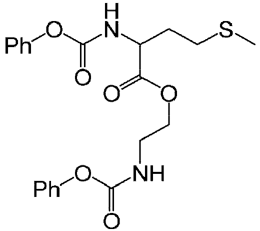 | 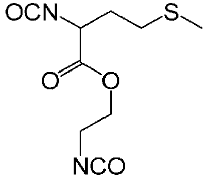 | 72 |
| 212 A | 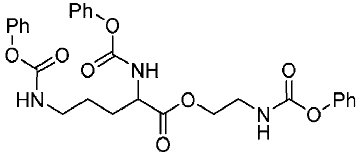 | 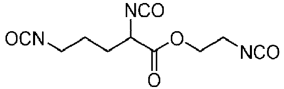 | 72 |
| 213 A | 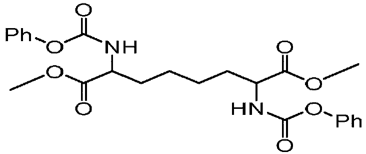 | 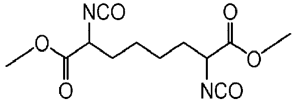 | 73 |

### (Ex: Example)

**Table 87**

| Ex. | Carbamate | Resultant isocyanate | Thermal decomposition yield (%) |
|---|---|---|---|
| 214 A | 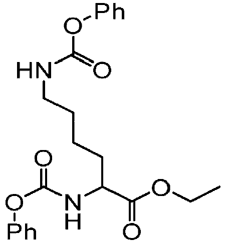 | 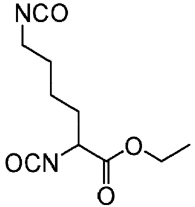 | 73 |

| | | | |
|---|---|---|---|
| (Ex: Example) | | | |

### Examples 203B to 214B

Each isocyanate was prepared by thermal decomposition of carbamate as conducted in Example 2-2 except that diisononyl adipate of the formula (6-1-1a) was used as the compound A and each carbamate shown in the following tables was used.

**Table 88**

| Ex. | Carbamate | Resultant isocyanate | Thermal decomposition yield (%) |
|---|---|---|---|
| 203 B | 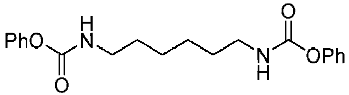 | 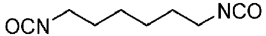 | 82 |
| 204 B | 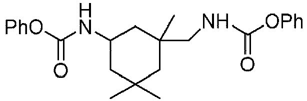 | 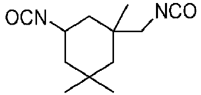 | 79 |
| 205 B | 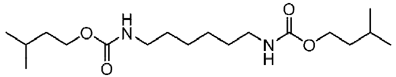 | 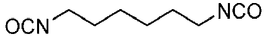 | 80 |
| 206 B | 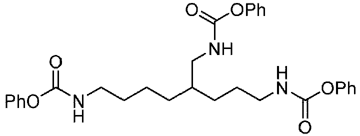 | 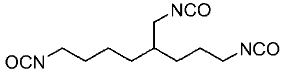 | 81 |
| 207 B | 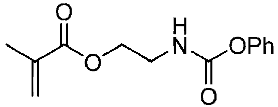 | 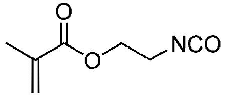 | 80 |
| 208 B | 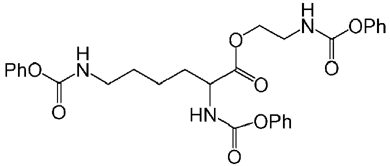 | 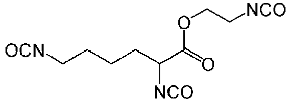 | 80 |

| | | | |
|---|---|---|---|
| (Ex: Example) | | | |

**Table 89**

| Ex. | Carbamate | Resultant isocyanate | Thermal decomposition yield (%) |
|---|---|---|---|
| 209 B | 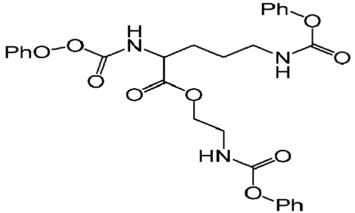 | 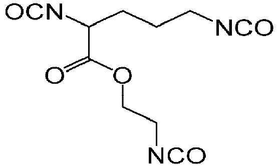 | 80 |
| 210 B | 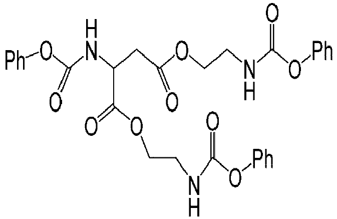 | 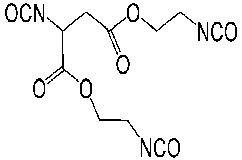 | 78 |
| 211 B | 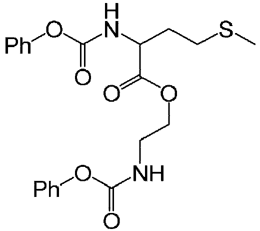 | 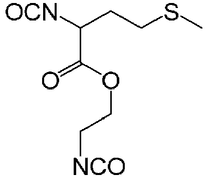 | 81 |
| 212 B | 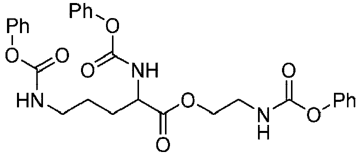 | 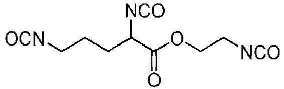 | 80 |
| 213 B | 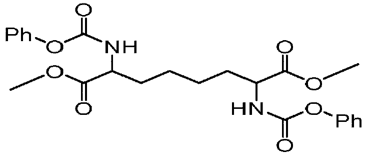 | 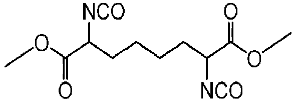 | 81 |

| | | | |
|---|---|---|---|
| (Ex: Example) | | | |

**Table 90**

| Ex. | Carbamate | Resultant isocyanate | Thermal decomposition yield (%) |
|---|---|---|---|
| 214 B | 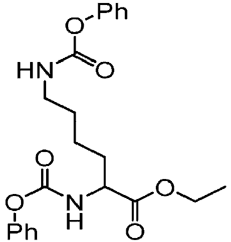 | 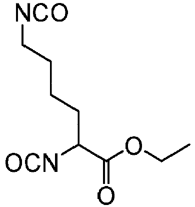 | 82 |

| | | | |
|---|---|---|---|
| (Ex: Example) | | | |

### Examples 215A to 228A

Each isocyanate was prepared by thermal decomposition of carbamate as conducted in Example 3-1 except that triphenylmethanol was used as the compound A, benzyltoluene was used as an inert solvent, and each carbamate shown in the following tables was used.

**Table 91**

| Ex. | Carbamate | Resultant isocyanate | Thermal decomposition yield (%) |
|---|---|---|---|
| 215 A | 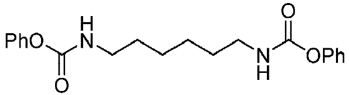 | 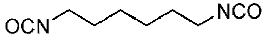 | 78 |
| 216 A | 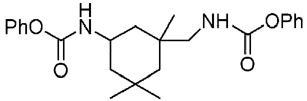 | 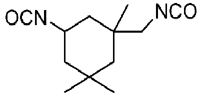 | 73 |
| 217 A | 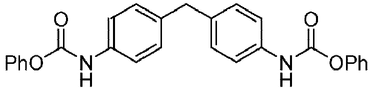 | 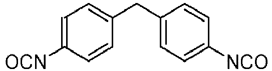 | 72 |
| 218 A | 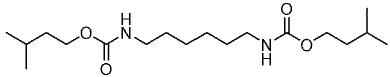 | 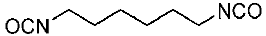 | 72 |
| 219 A | 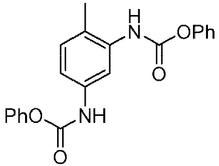 | 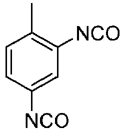 | 74 |
| 220 A | 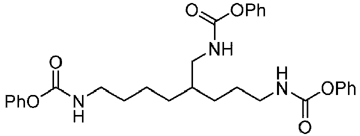 | 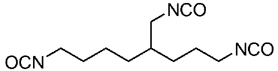 | 73 |
| 221 A | 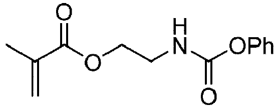 | 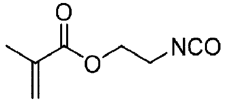 | 72 |
| 222 A | 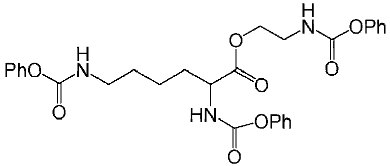 | 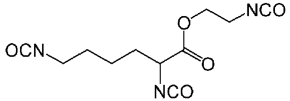 | 72 |

| | | | |
|---|---|---|---|
| (Ex: Example) | | | |

**Table 92**

| Ex. | Carbamate | Resultant isocyanate | Thermal decomposition yield (%) |
|---|---|---|---|
| 223 A | 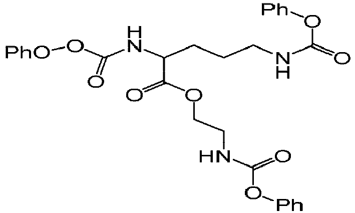 | 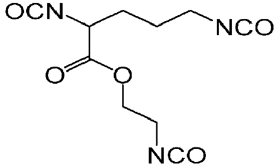 | 71 |
| 224 A | 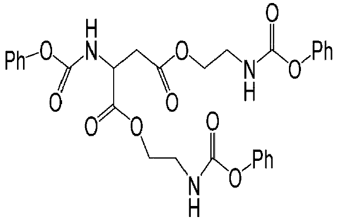 | 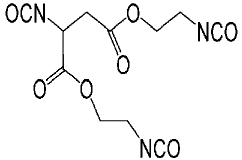 | 72 |
| 225 A | 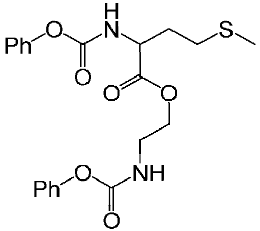 | 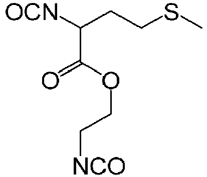 | 72 |
| 226 A | 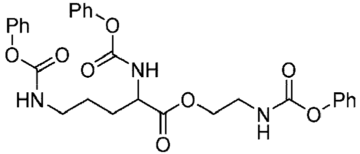 | 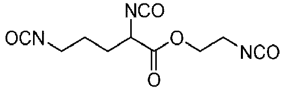 | 72 |
| 227 A | 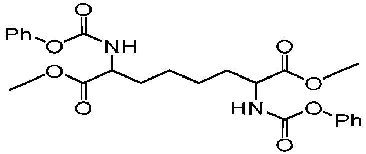 | 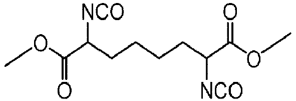 | 72 |

| | | | |
|---|---|---|---|
| (Ex: Example) | | | |

**Table 93**

| Ex. | Carbamate | Resultant isocyanate | Thermal decomposition yield (%) |
|---|---|---|---|
| 228 A | 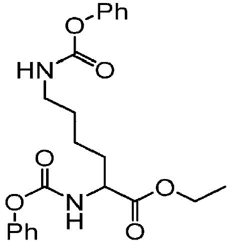 | 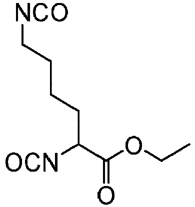 | 73 |

| | | | |
|---|---|---|---|
| (Ex: Example) | | | |

### Examples 215B to 228B

Each isocyanate was prepared by thermal decomposition of carbamate as conducted in Example 3-2 except that each carbamate shown in the following tables was used.

**Table 94**

| Ex. | Carbamate | Resultant isocyanate | Thermal decomposition yield (%) |
|---|---|---|---|
| 215 B | 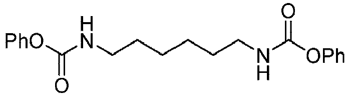 | 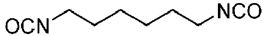 | 84 |
| 216 B | 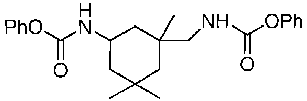 | 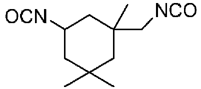 | 80 |
| 217 B | 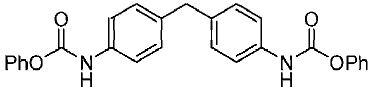 | 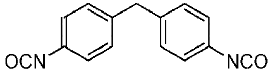 | 84 |
| 218 B | 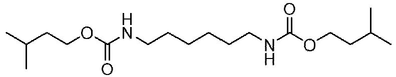 | 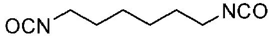 | 83 |
| 219 B | 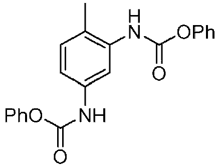 | 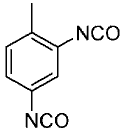 | 83 |
| 220 B | 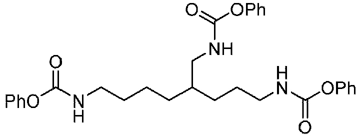 | 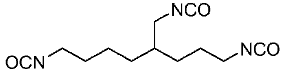 | 84 |
| 221 B | 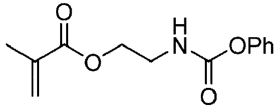 | 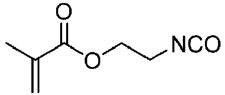 | 82 |
| 222 B | 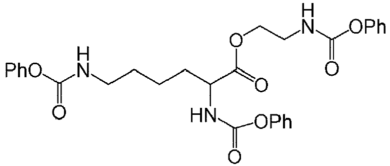 | 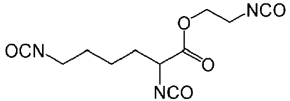 | 83 |

| | | | |
|---|---|---|---|
| (Ex: Example) | | | |

**Table 95**

| Ex. | Carbamate | Resultant isocyanate | Thermal decomposition yield (%) |
|---|---|---|---|
| 223 B | 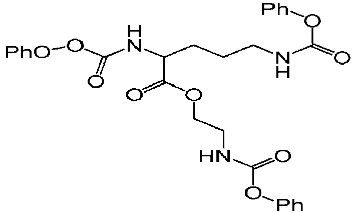 | 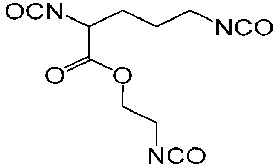 | 83 |
| 224 B | 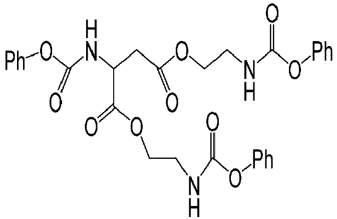 | 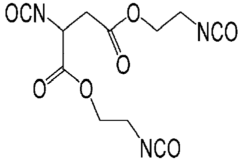 | 82 |
| 225 B | 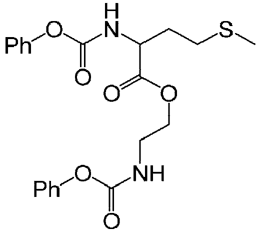 | 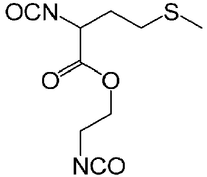 | 85 |
| 226 B | 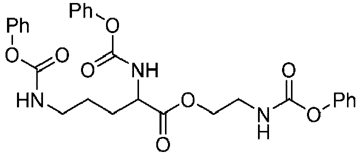 | 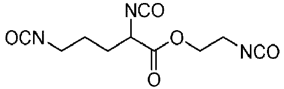 | 84 |
| 227 B | 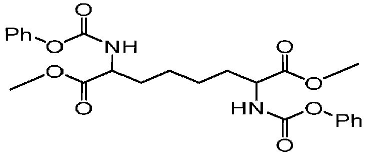 | 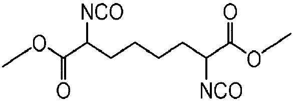 | 85 |

| | | | |
|---|---|---|---|
| (Ex: Example) | | | |

**Table 96**

| Ex. | Carbamate | Resultant isocyanate | Thermal decomposition yield (%) |
|---|---|---|---|
| 228 B | 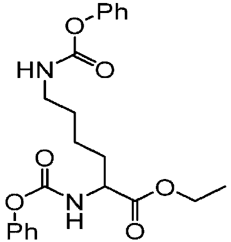 | 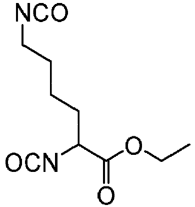 | 84 |

| | | | |
|---|---|---|---|
| (Ex: Example) | | | |

### Examples 229A to 242A

Each isocyanate was prepared by thermal decomposition of carbamate as conducted in Example 3-1 except that a compound of the following formula (S2-29) was used as the compound A, benzyltoluene was used as an inert solvent, and each carbamate shown in the following tables was used.

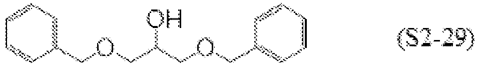

**Table 97**

| Ex. | Carbamate | Resultant isocyanate | Thermal decomposition yield (%) |
|---|---|---|---|
| 229 A | 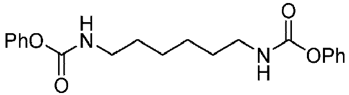 | 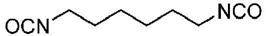 | 77 |
| 230 A | 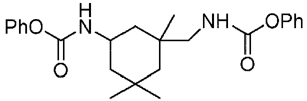 | 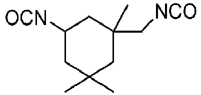 | 73 |
| 231 A | 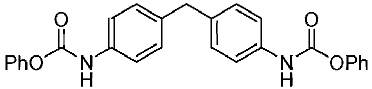 | 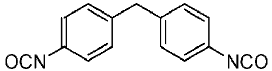 | 72 |
| 232 A | 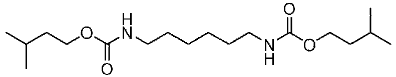 | 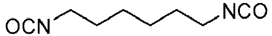 | 71 |
| 233 A | 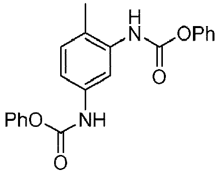 | 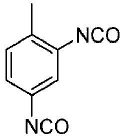 | 73 |
| 234 A | 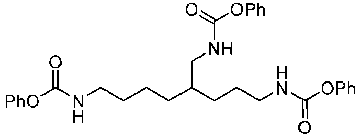 | 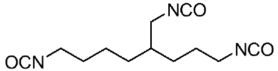 | 72 |
| 235 A | 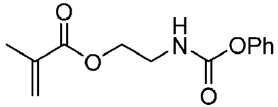 | 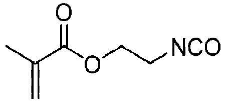 | 72 |
| 236 A | 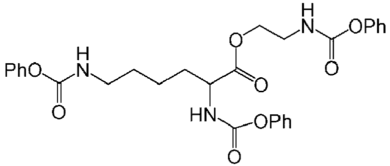 | 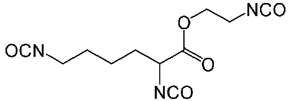 | 72 |

| | | | |
|---|---|---|---|
| (Ex: Example) | | | |

**Table 98**

| Ex. | Carbamate | Resultant isocyanate | Thermal decomposition yield (%) |
|---|---|---|---|
| 237 A | 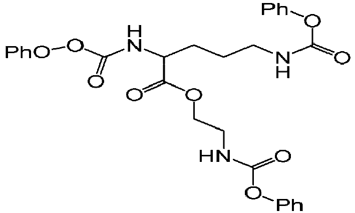 | 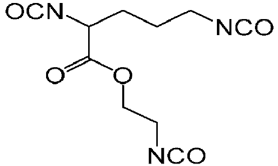 | 71 |
| 238 A | 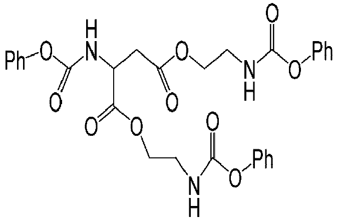 | 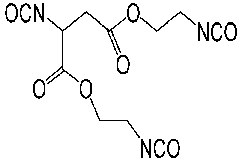 | 71 |
| 239 A | 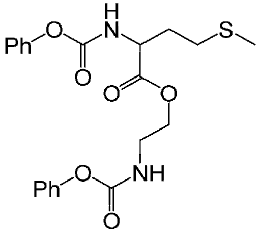 | 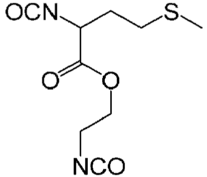 | 72 |
| 240 A | 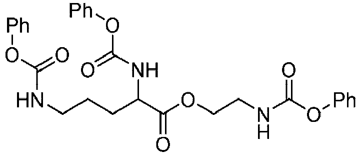 | 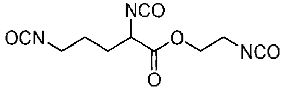 | 71 |
| 241 A | 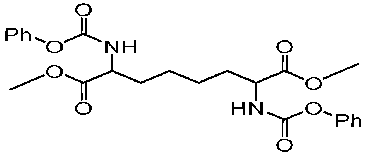 | 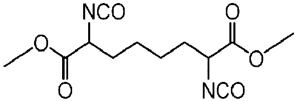 | 71 |

| | | | |
|---|---|---|---|
| (Ex: Example) | | | |

**Table 99**

| Ex. | Carbamate | Resultant isocyanate | Thermal decomposition yield (%) |
|---|---|---|---|
| 242 A | 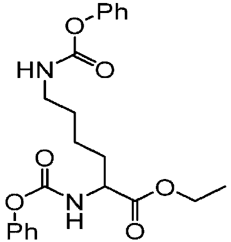 | 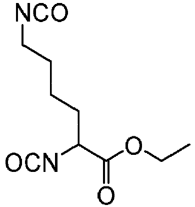 | 72 |

| | | | |
|---|---|---|---|
| (Ex: Example) | | | |

### Examples 229B to 242B

Each isocyanate was prepared by thermal decomposition of carbamate as conducted in Example 3-2 except that the compound of the formula (S2-29) was used as the compound A and each carbamate shown in the following tables was used.

**Table 100**

| Ex. | Carbamate | Resultant isocyanate | Thermal decomposition yield (%) |
|---|---|---|---|
| 229 B | 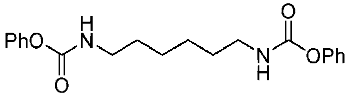 | 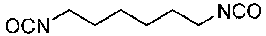 | 84 |
| 230 B | 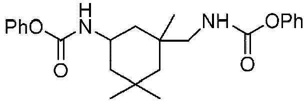 | 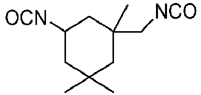 | 80 |
| 231 B | 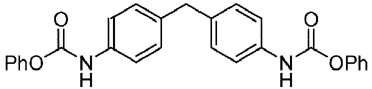 | 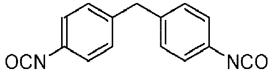 | 84 |
| 232 B | 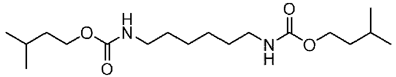 | 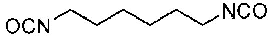 | 82 |
| 233 B | 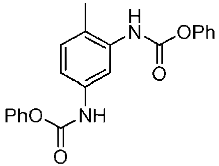 | 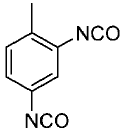 | 83 |
| 234 B | 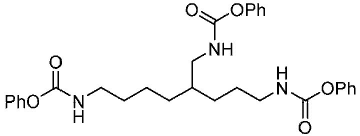 | 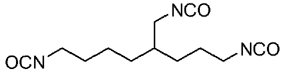 | 84 |
| 235 B | 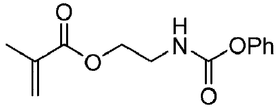 | 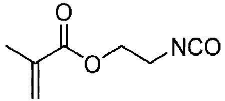 | 82 |
| 236 B | 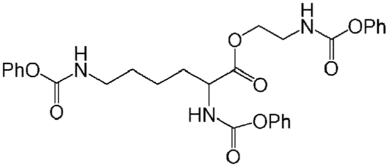 | 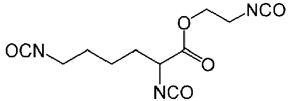 | 83 |

| | | | |
|---|---|---|---|
| (Ex: Example) | | | |

**Table 101**

| Ex. | Carbamate | Resultant isocyanate | Thermal decomposition yield (%) |
|---|---|---|---|
| 237 B | 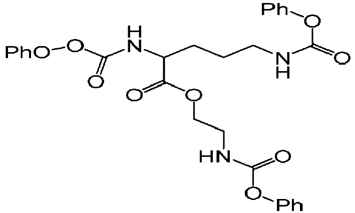 | 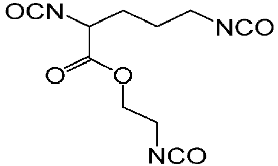 | 82 |
| 238 B | 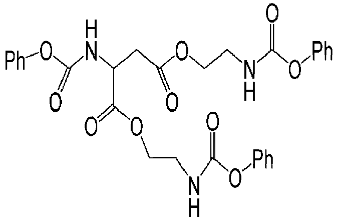 | 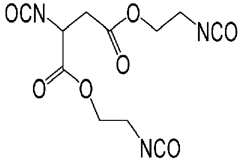 | 82 |
| 239 B | 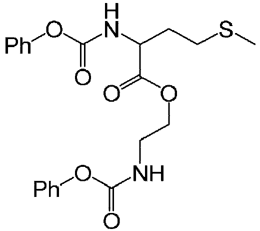 | 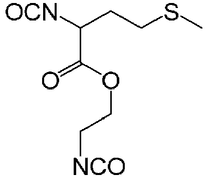 | 84 |
| 240 B | 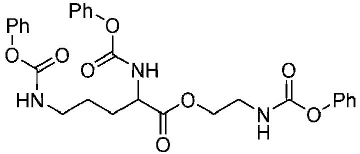 | 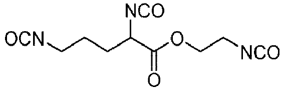 | 84 |
| 241 B | 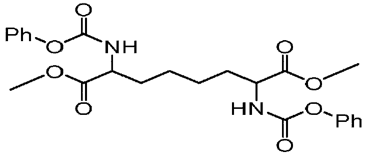 | 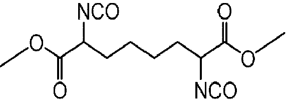 | 85 |

| | | | |
|---|---|---|---|
| (Ex: Example) | | | |

**Table 102**

| Ex. | Carbamate | Resultant isocyanate | Thermal decomposition yield (%) |
|---|---|---|---|
| 242 B | 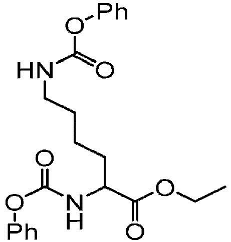 | 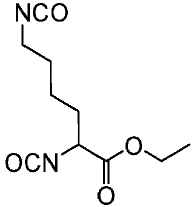 | 83 |

| | | | |
|---|---|---|---|
| (Ex: Example) | | | |

### Examples 243A to 256A

Each isocyanate was prepared by thermal decomposition of carbamate as conducted in Example 4-1 except that 4,4'-dicyclohexylmethane diisocyanate was used as the compound A, benzyltoluene was used as an inert solvent, and each carbamate shown in the following tables was used.

**Table 103**

| Ex. | Carbamate | Resultant isocyanate | Thermal decomposition yield (%) |
|---|---|---|---|
| 243 A | 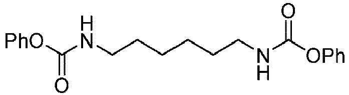 | 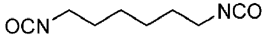 | 77 |
| 244 A | 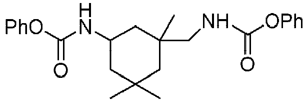 | 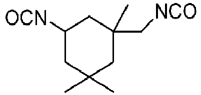 | 74 |
| 245 A | 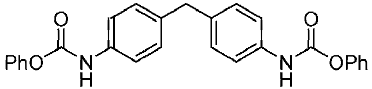 | 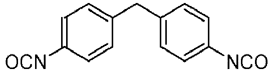 | 73 |
| 246 A | 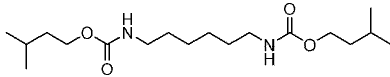 | 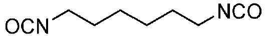 | 72 |
| 247 A | 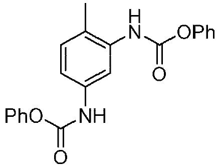 | 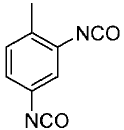 | 73 |
| 248 A | 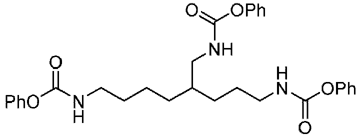 | 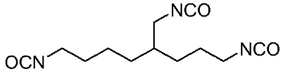 | 73 |
| 249 A | 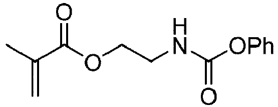 | 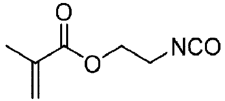 | 71 |
| 250 A | 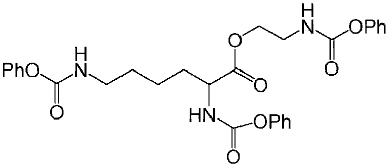 | 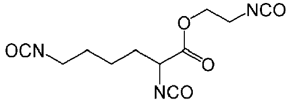 | 72 |

| | | | |
|---|---|---|---|
| (Ex: Example) | | | |

**Table 104**

| Ex. | Carbamate | Resultant isocyanate | Thermal decomposition yield (%) |
|---|---|---|---|
| 251 A | 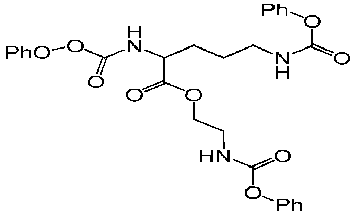 | 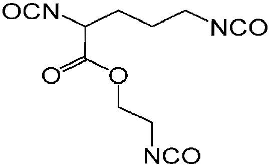 | 72 |
| 252 A | 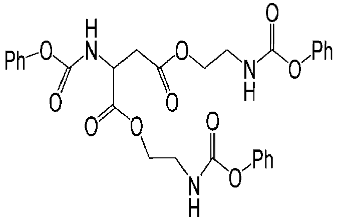 | 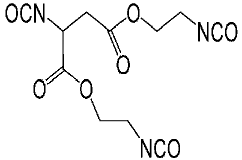 | 72 |
| 253 A | 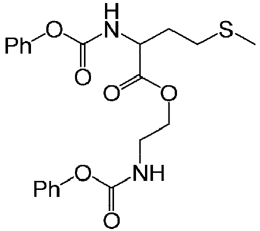 | 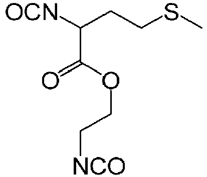 | 72 |
| 254 A | 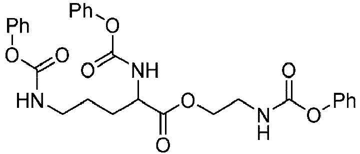 | 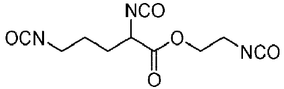 | 71 |
| 255 A | 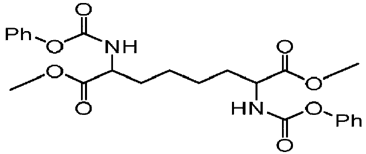 | 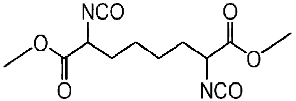 | 72 |

| | | | |
|---|---|---|---|
| (Ex: Example) | | | |

**Table 105**

| Ex. | Carbamate | Resultant isocyanate | Thermal decomposition yield (%) |
|---|---|---|---|
| 256 A | 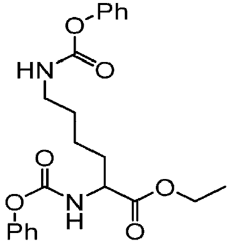 | 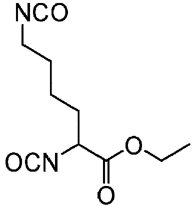 | 72 |

| | | | |
|---|---|---|---|
| (Ex: Example) | | | |

### Examples 243B to 256B

Each isocyanate was prepared by thermal decomposition of carbamate as conducted in Example 4-2 except that benzyltoluene was used as an inert solvent and each carbamate shown in the following tables was used.

**Table 106**

| Ex. | Carbamate | Resultant isocyanate | Thermal decomposition yield (%) |
|---|---|---|---|
| 243 B | 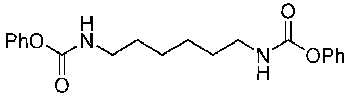 | 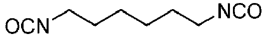 | 84 |
| 244 B | 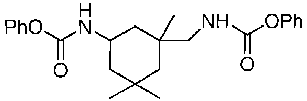 | 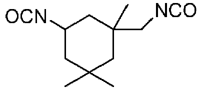 | 81 |
| 245 B | 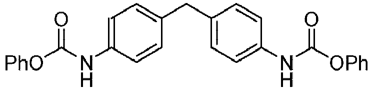 | 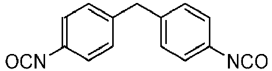 | 85 |
| 246 B | 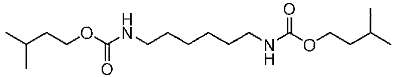 | 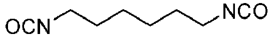 | 83 |
| 247 B | 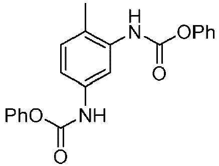 | 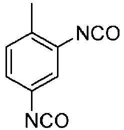 | 84 |
| 248 B | 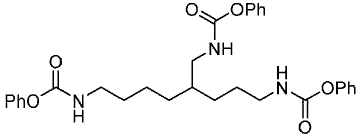 | 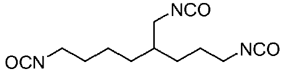 | 85 |
| 249 B | 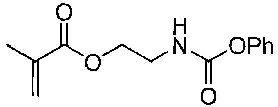 | 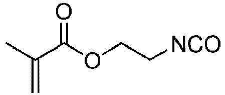 | 82 |
| 250 B | 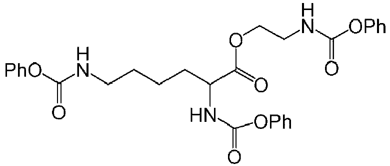 | 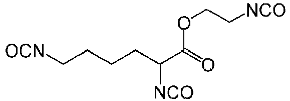 | 84 |

| | | | |
|---|---|---|---|
| (Ex: Example) | | | |

**Table 107**

| Ex. | Carbamate | Resultant isocyanate | Thermal decomposition yield (%) |
|---|---|---|---|
| 251 B | 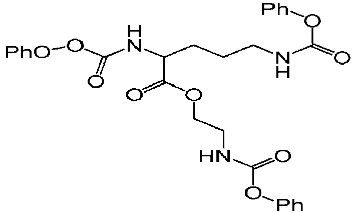 | 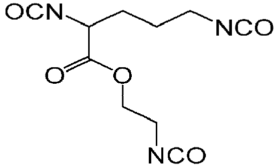 | 83 |
| 252 B | 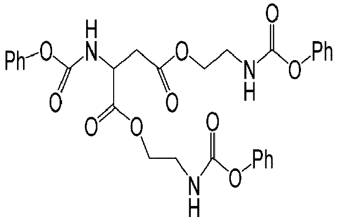 | 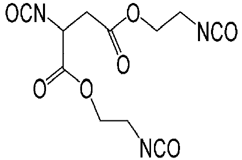 | 83 |
| 253 B | 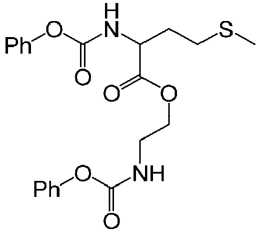 | 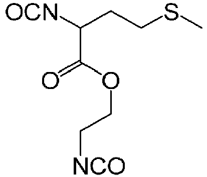 | 84 |
| 254 B | 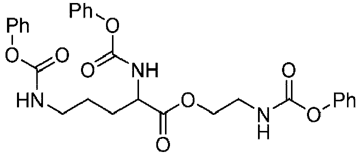 | 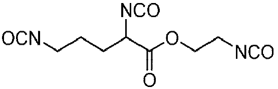 | 83 |
| 255 B | 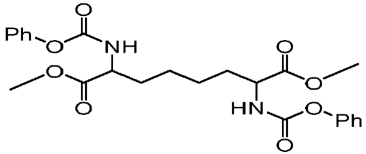 | 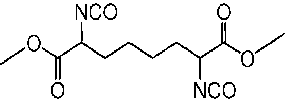 | 85 |

| | | | |
|---|---|---|---|
| (Ex: Example) | | | |

**Table 108**

| Ex. | Carbamate | Resultant isocyanate | Thermal decomposition yield (%) |
|---|---|---|---|
| 256 B | 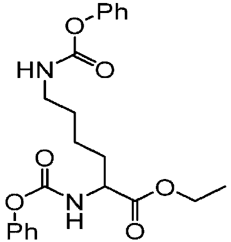 | 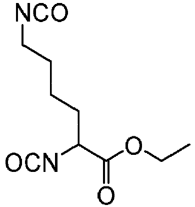 | 84 |

| | | | |
|---|---|---|---|
| (Ex: Example) | | | |

### Examples 257A to 271A

Each isocyanate was prepared by thermal decomposition of carbamate as conducted in Example 4-1 except that a compound of the following formula (9-2a) was used as the compound A, benzyltoluene was used as an inert solvent and each carbamate shown in the following tables was used.

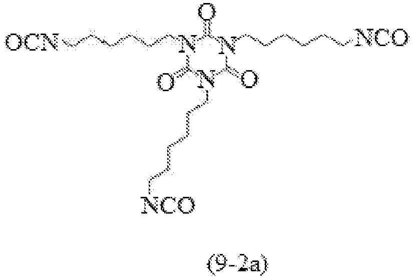

**Table 109**

| Ex. | Carbamate | Resultant isocyanate | Thermal decomposition yield (%) |
|---|---|---|---|
| 257 A | 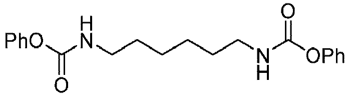 | 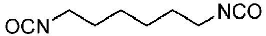 | 78 |
| 258 A | 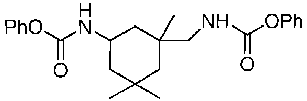 | 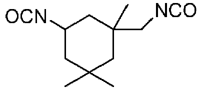 | 75 |
| 259 A | 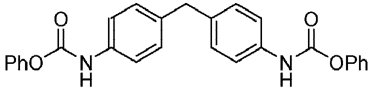 | 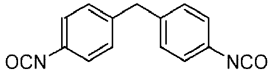 | 74 |
| 260 A | 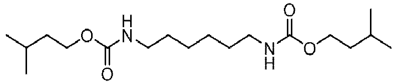 | 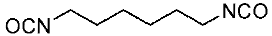 | 73 |
| 261 A | 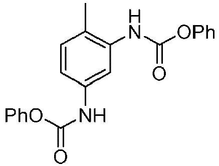 | 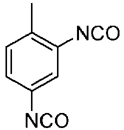 | 73 |
| 262 A | 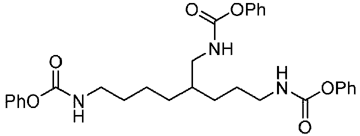 | 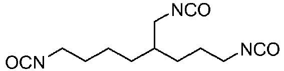 | 73 |
| 263 A | 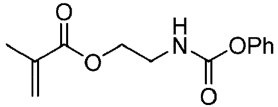 | 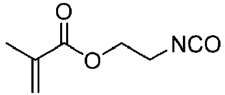 | 72 |
| 264 A | 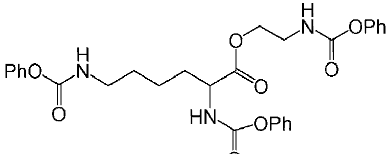 | 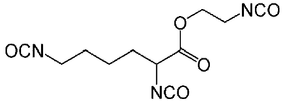 | 72 |

| | | | |
|---|---|---|---|
| (Ex: Example) | | | |

**Table 110**

| Ex. | Carbamate | Resultant isocyanate | Thermal decomposition yield (%) |
|---|---|---|---|
| 265 A | 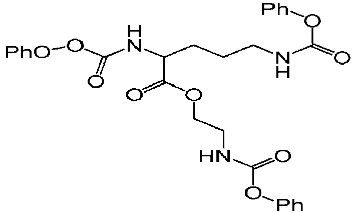 | 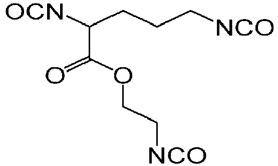 | 72 |
| 266 A | 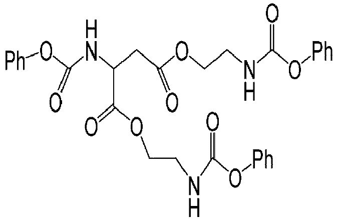 | 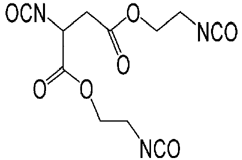 | 73 |
| 267 A | 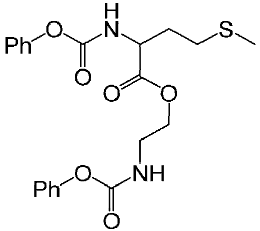 | 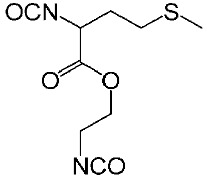 | 73 |
| 268 A | 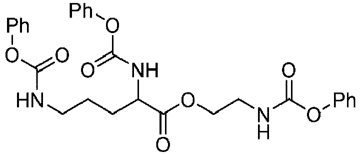 | 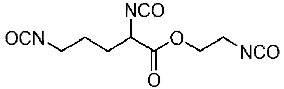 | 72 |
| 269 A | 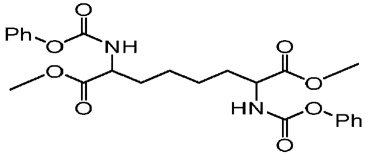 | 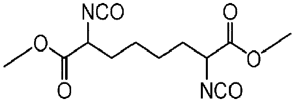 | 73 |

| | | | |
|---|---|---|---|
| (Ex: Example) | | | |

**Table 111**

| Ex. | Carbamate | Resultant isocyanate | Thermal decomposition yield (%) |
|---|---|---|---|
| 270 A | 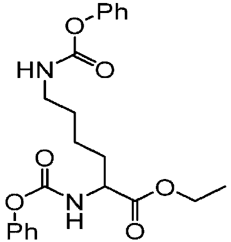 | 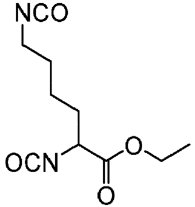 | 72 |
| 271 A | 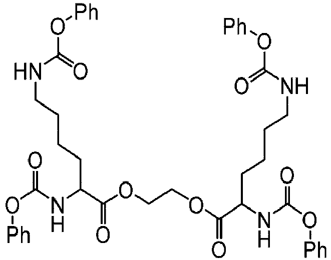 | 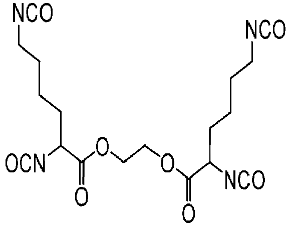 | 56 |

| | | | |
|---|---|---|---|
| (Ex: Example) | | | |

### Examples 257B to 271B

Each isocyanate was prepared by thermal decomposition of carbamate as conducted in Example 4-2 except that the compound of the formula (9-2a) was used as the compound A, benzyltoluene was used as an inert solvent and each carbamate shown in the following tables was used.

**Table 112**

| Ex. | Carbamate | Resultant isocyanate | Thermal decomposition yield (%) |
|---|---|---|---|
| 257 B | 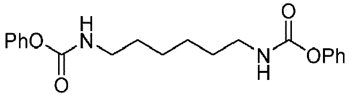 | 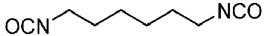 | 86 |
| 258 B | 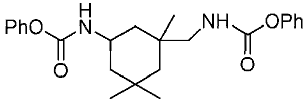 | 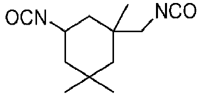 | 84 |
| 259 B | 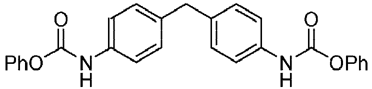 | 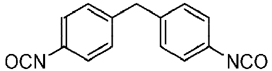 | 85 |
| 260 B | 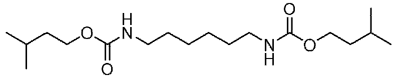 | 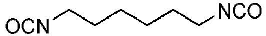 | 84 |
| 261 B | 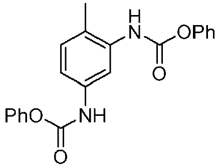 | 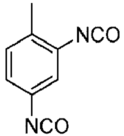 | 84 |
| 262 B | 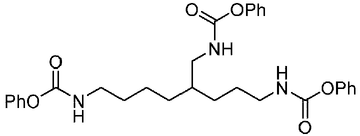 | 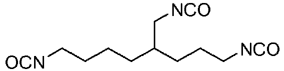 | 86 |
| 263 B | 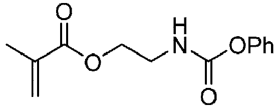 | 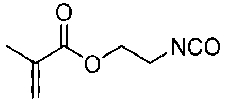 | 85 |
| 264 B | 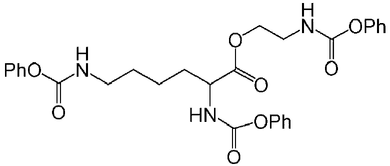 | 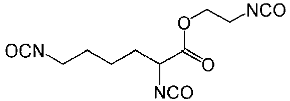 | 83 |

| | | | |
|---|---|---|---|
| (Ex: Example) | | | |

**Table 113**

| Ex. | Carbamate | Resultant isocyanate | Thermal decomposition yield (%) |
|---|---|---|---|
| 265 B | 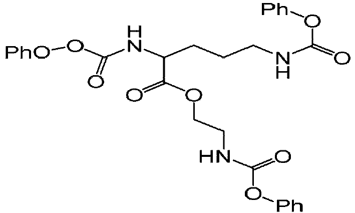 | 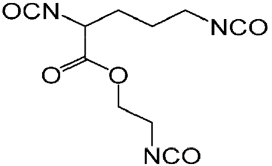 | 84 |
| 266 B | 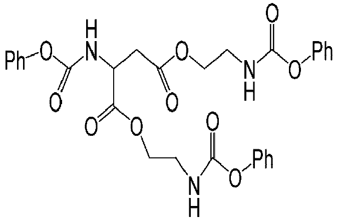 | 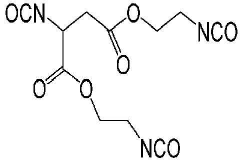 | 84 |
| 267 B | 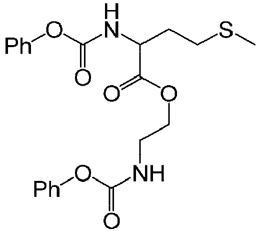 | 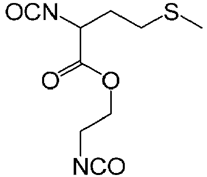 | 83 |
| 268 B | 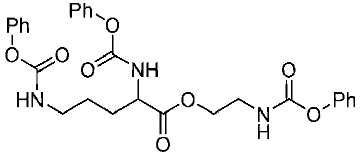 | 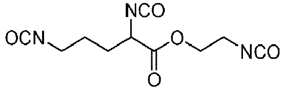 | 84 |
| 269 B | 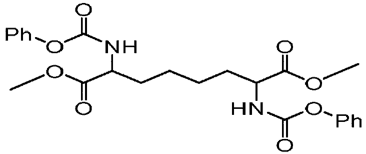 | 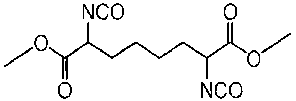 | 86 |

| | | | |
|---|---|---|---|
| (Ex: Example) | | | |

**Table 114**

| Ex. | Carbamate | Resultant isocyanate | Thermal decomposition yield (%) |
|---|---|---|---|
| 270 B | 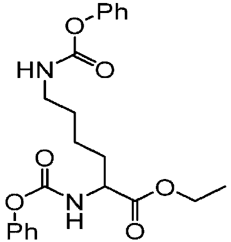 | 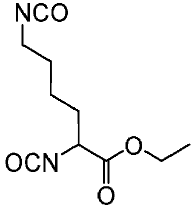 | 84 |
| 271 B | 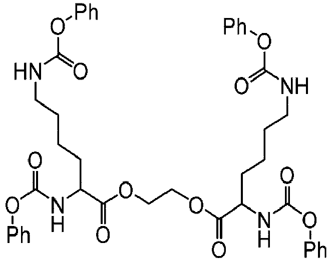 | 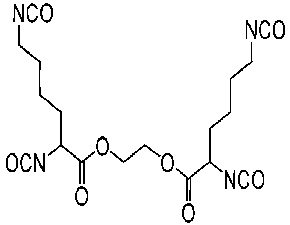 | 60 |

| | | | |
|---|---|---|---|
| (Ex: Example) | | | |

### Examples 273A to 287A

Each isocyanate was prepared by thermal decomposition of carbamate as conducted in Example 4-1 except that the compounds of the following formula (9-1-1a) were used as the compound A, benzyltoluene was used as an inert solvent and each carbamate shown in the following tables was used.

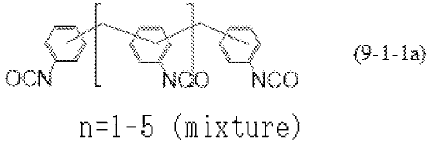

**Table 115**

| Ex. | Carbamate | Resultant isocyanate | Thermal decomposition yield (%) |
|---|---|---|---|
| 273 A | 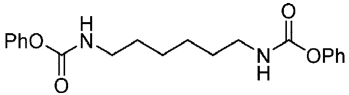 | 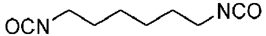 | 79 |
| 274 A | 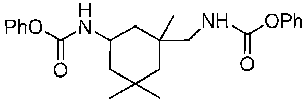 | 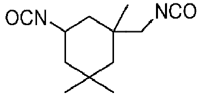 | 76 |
| 275 A | 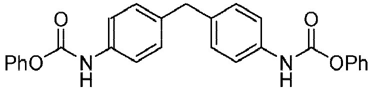 | 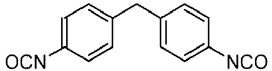 | 73 |
| 276 A | 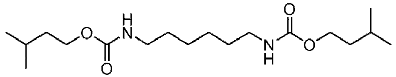 | 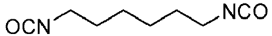 | 72 |
| 277 A | 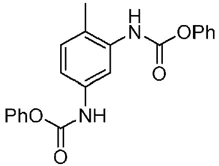 | 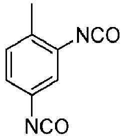 | 73 |
| 278 A | 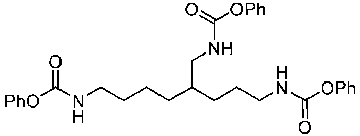 | 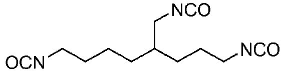 | 74 |
| 279 A | 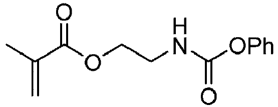 | 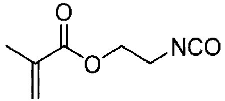 | 73 |
| 280 A | 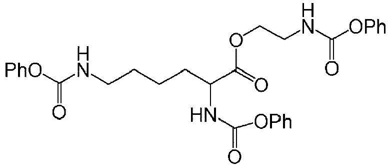 | 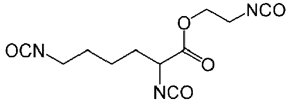 | 72 |

| | | | |
|---|---|---|---|
| (Ex: Example) | | | |

**Table 116**

| Ex. | Carbamate | Resultant isocyanate | Thermal decomposition yield (%) |
|---|---|---|---|
| 281 A | 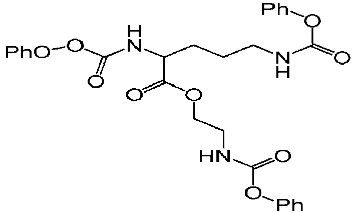 | 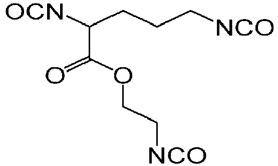 | 72 |
| 282 A | 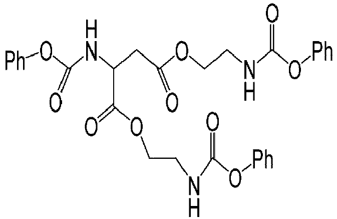 | 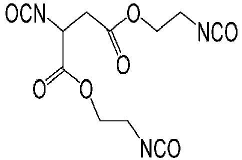 | 74 |
| 283 A | 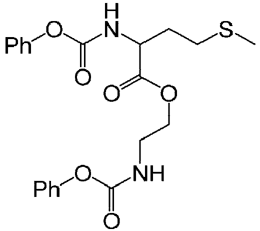 | 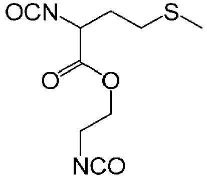 | 73 |
| 284 A | 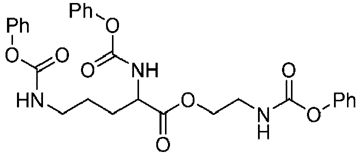 | 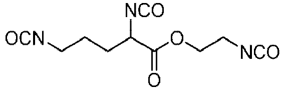 | 72 |
| 285 A | 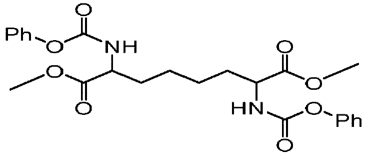 | 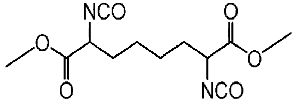 | 73 |

| | | | |
|---|---|---|---|
| (Ex: Example) | | | |

**Table 117**

| Ex. | Carbamate | Resultant isocyanate | Thermal decomposition yield (%) |
|---|---|---|---|
| 286 A | 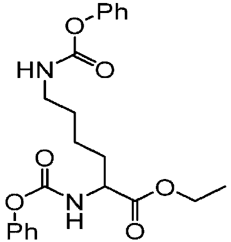 | 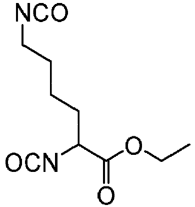 | 72 |
| 287 A | 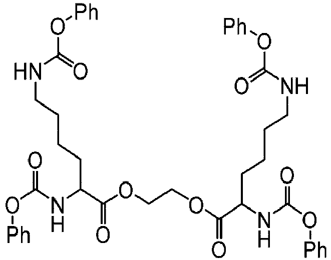 | 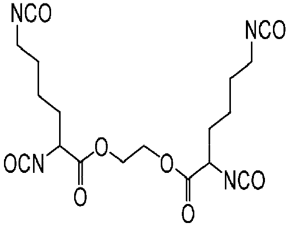 | 48 |

| | | | |
|---|---|---|---|
| (Ex: Example) | | | |

### Examples 273B to 287B

Each isocyanate was prepared by thermal decomposition of carbamate as conducted in Example 4-2 except that the compounds of the formula (9-1-1a) were used as the compound A, benzyltoluene was used as an inert solvent and each carbamate shown in the following tables was used.

**Table 118**

| Ex. | Carbamate | Resultant isocyanate | Thermal decomposition yield (%) |
|---|---|---|---|
| 273 B | 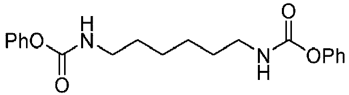 | 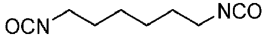 | 87 |
| 274 B | 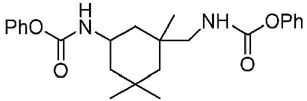 | 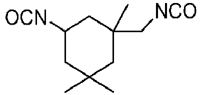 | 85 |
| 275 B | 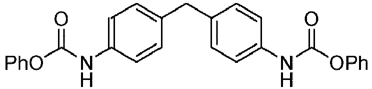 | 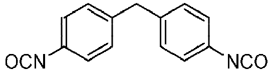 | 85 |
| 276 B | 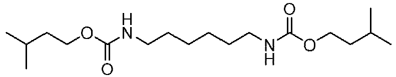 | 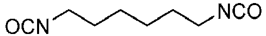 | 86 |
| 277 B | 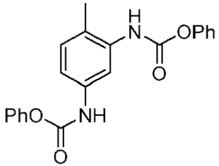 | 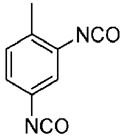 | 85 |
| 278 B | 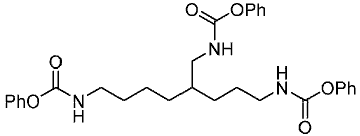 | 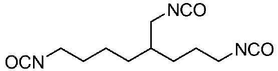 | 86 |
| 279 B | 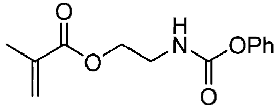 | 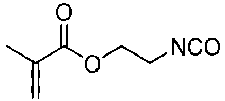 | 85 |
| 280 B | 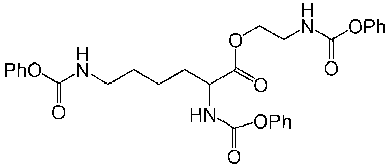 | 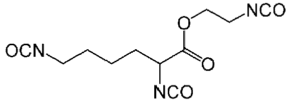 | 83 |

| | | | |
|---|---|---|---|
| (Ex: Example) | | | |

**Table 119**

| Ex. | Carbamate | Resultant isocyanate | Thermal decomposition yield (%) |
|---|---|---|---|
| 281 B | 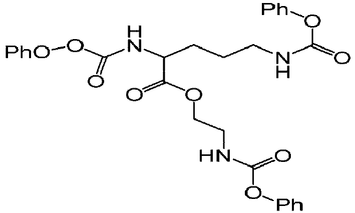 | 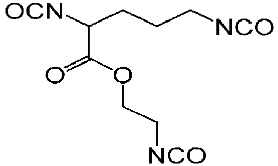 | 84 |
| 282 B | 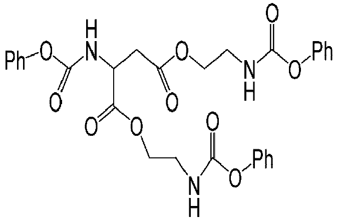 | 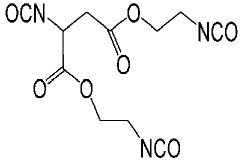 | 84 |
| 283 B | 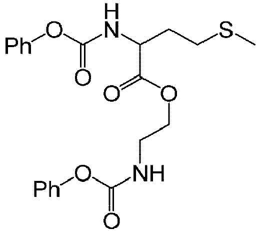 | 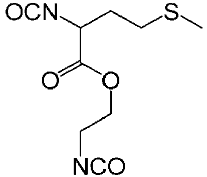 | 83 |
| 284 B | 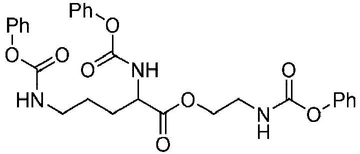 | 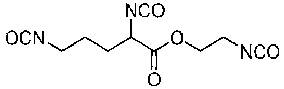 | 84 |
| 285 B | 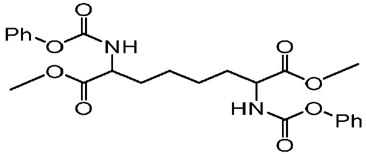 | 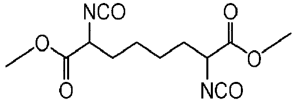 | 85 |

| | | | |
|---|---|---|---|
| (Ex: Example) | | | |

**Table 120**

| Ex. | Carbamate | Resultant isocyanate | Thermal decomposition yield (%) |
|---|---|---|---|
| 286 B | 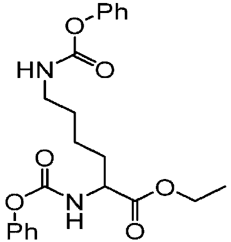 | 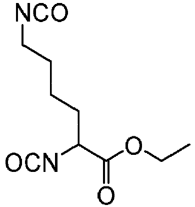 | 84 |
| 287 B | 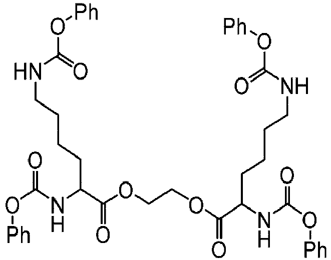 | 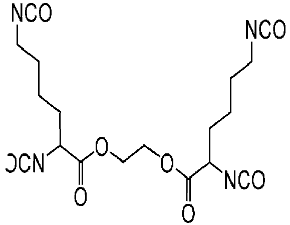 | 60 |

| | | | |
|---|---|---|---|
| (Ex: Example) | | | |

### Examples 289A to 297A

Each isocyanate was prepared by thermal decomposition of carbamate as conducted in Example 5-1 except that each carbamate shown in the following tables was used.

**Table 121**

| Ex. | Carbamate | Resultant isocyanate | Thermal decomposition yield (%) |
|---|---|---|---|
| 289 A | 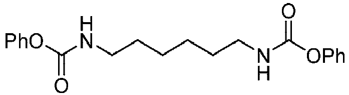 | 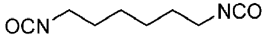 | 72 |
| 290 A | 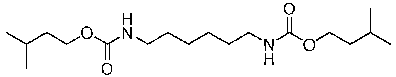 | 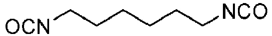 | 66 |
| 291 A | 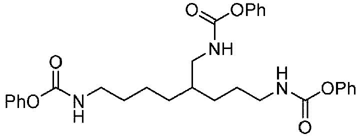 | 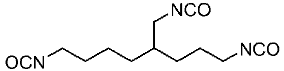 | 67 |
| 292 A | 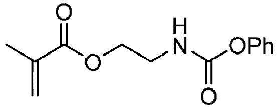 | 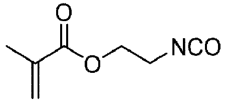 | 66 |
| 293 A | 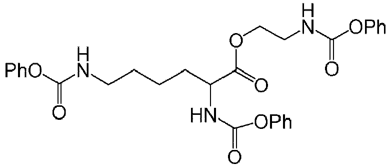 | 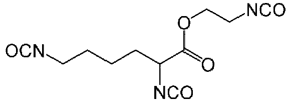 | 64 |

| | | | |
|---|---|---|---|
| (Ex: Example) | | | |

**Table 122**

| Ex. | Carbamate | Resultant isocyanate | Thermal decomposition yield (%) |
|---|---|---|---|
| 294 A | 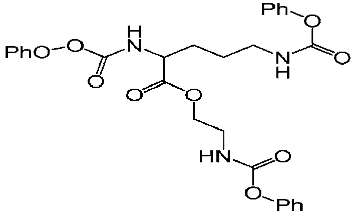 | 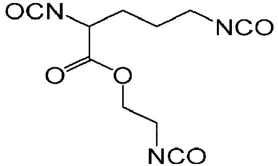 | 64 |
| 295 A | 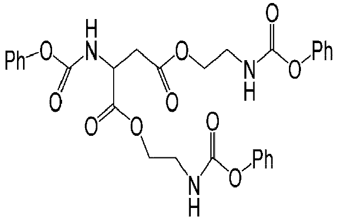 | 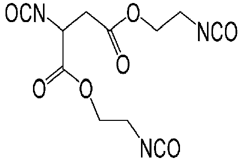 | 63 |
| 296 A | 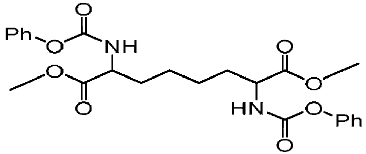 | 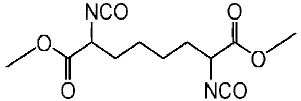 | 66 |
| 297 A | 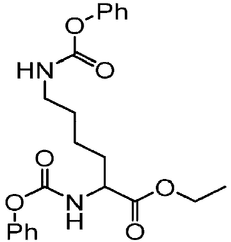 | 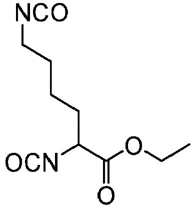 | 66 |

| | | | |
|---|---|---|---|
| (Ex: Example) | | | |

### Examples 289B to 297B

Each isocyanate was prepared by thermal decomposition of carbamate as conducted in Example 5-2 except that butyl cellosolve was used as an inert solvent and each carbamate shown in the following tables was used.

**Table 123**

| Ex. | Carbamate | Resultant isocyanate | Thermal decomposition yield (%) |
|---|---|---|---|
| 289 B | 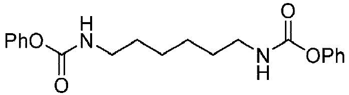 | 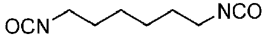 | 80 |
| 290 B | 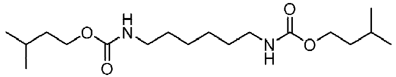 | 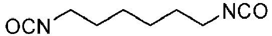 | 76 |
| 291 B | 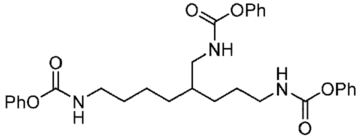 | 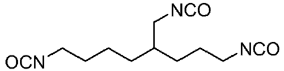 | 77 |
| 292 B | 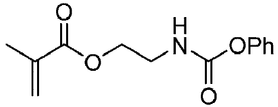 | 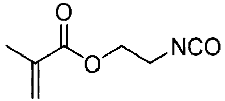 | 75 |
| 293 B | 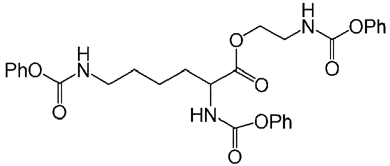 | 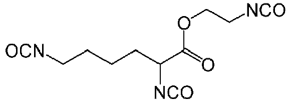 | 73 |

| | | | |
|---|---|---|---|
| (Ex: Example) | | | |

**Table 124**

| Ex. | Carbamate | Resultant isocyanate | Thermal decomposition yield (%) |
|---|---|---|---|
| 294 B | 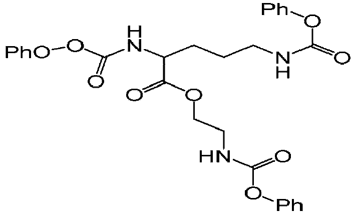 | 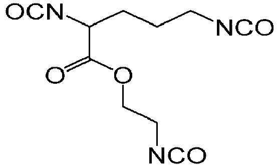 | 72 |
| 295 B | 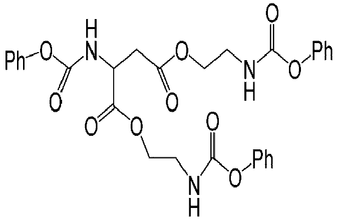 | 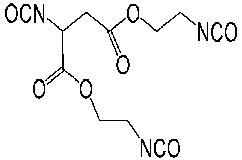 | 72 |
| 296 B | 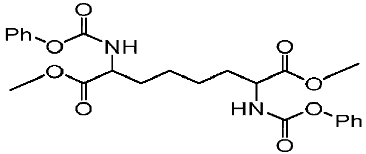 | 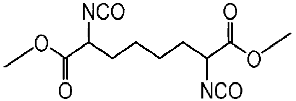 | 73 |
| 297 B | 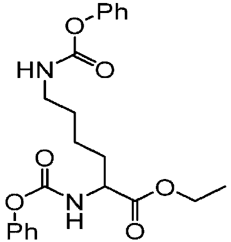 | 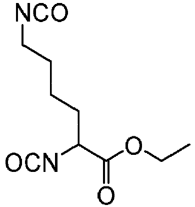 | 74 |

| | | | |
|---|---|---|---|
| (Ex: Example) | | | |

### Examples 298A to 306A

Each isocyanate was prepared by thermal decomposition of carbamate as conducted in Example 5-1 except that a compound of the following formula (11a) was used as the compound A and each carbamate shown in the following tables was used.

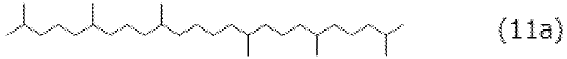

**Table 125**

| Ex. | Carbamate | Resultant isocyanate | Thermal decomposition yield (%) |
|---|---|---|---|
| 298 A | 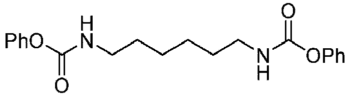 | 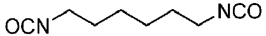 | 72 |
| 299 A | 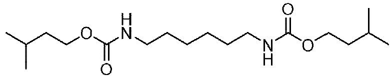 | 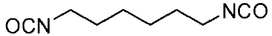 | 66 |
| 300 A | 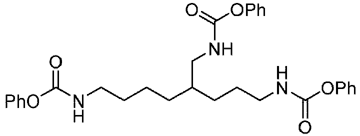 | 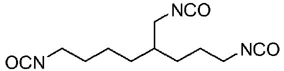 | 67 |
| 301 A | 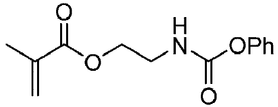 | 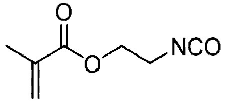 | 66 |
| 302 A | 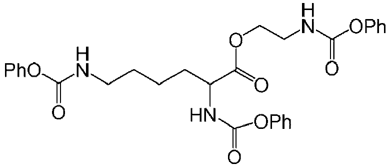 | 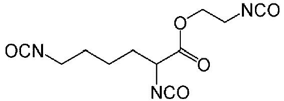 | 64 |

| | | | |
|---|---|---|---|
| (Ex: Example) | | | |

**Table 126**

| Ex. | Carbamate | Resultant isocyanate | Thermal decomposition yield (%) |
|---|---|---|---|
| 303 A | 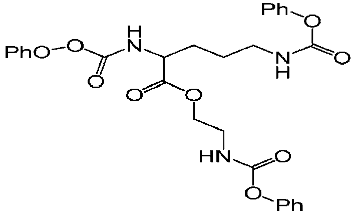 | 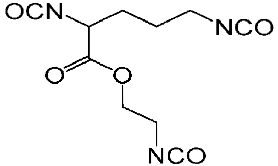 | 64 |
| 304 A | 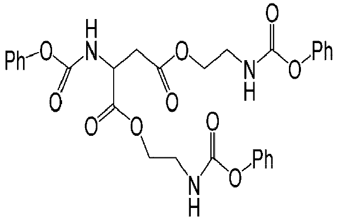 | 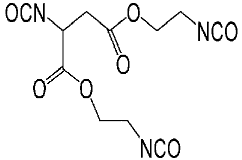 | 63 |
| 305 A | 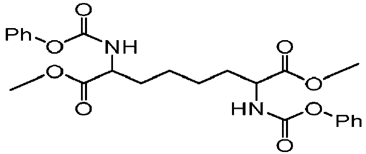 | 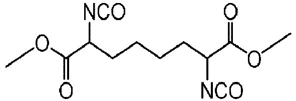 | 66 |
| 306 A | 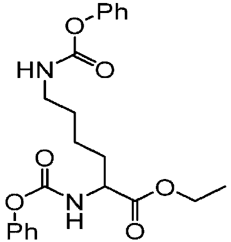 | 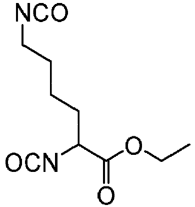 | 66 |

| | | | |
|---|---|---|---|
| (Ex: Example) | | | |

### Examples 298B to 306B

Each isocyanate was prepared by thermal decomposition of carbamate as conducted in Example 5-2 except that the compound of the formula (11a) was used as the compound A, butyl cellosolve was used as an inert solvent and each carbamate shown in the following tables was used.

**Table 127**

| Ex. | Carbamate | Resultant isocyanate | Thermal decomposition yield (%) |
|---|---|---|---|
| 298 B | 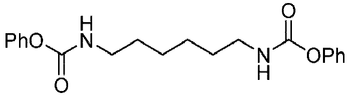 | 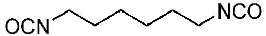 | 79 |
| 299 B | 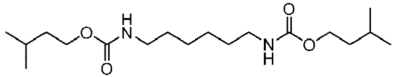 | 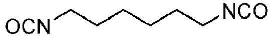 | 75 |
| 300 B | 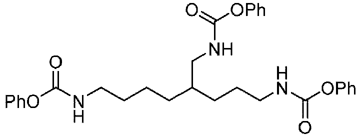 | 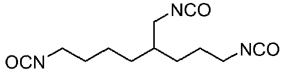 | 76 |
| 301 B | 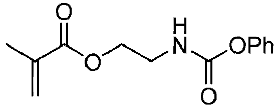 | 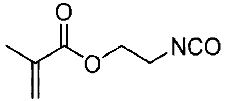 | 70 |
| 302 B | 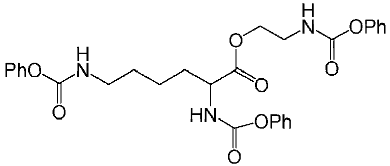 | 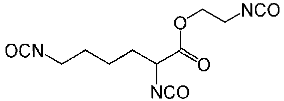 | 70 |

| | | | |
|---|---|---|---|
| (Ex: Example) | | | |

**Table 128**

| Ex. | Carbamate | Resultant isocyanate | Thermal decomposition yield (%) |
|---|---|---|---|
| 303 B | 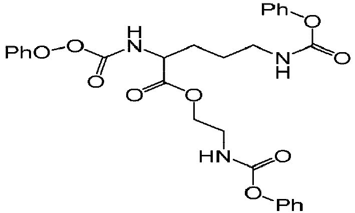 | 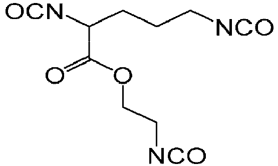 | 68 |
| 304 B | 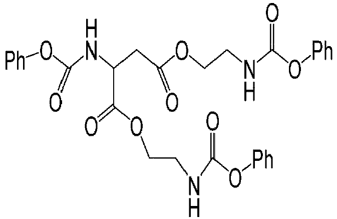 | 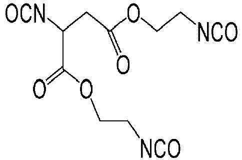 | 67 |
| 305 B | 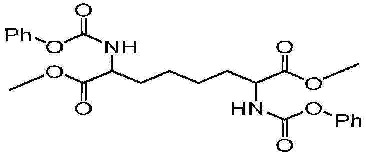 | 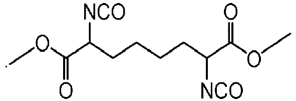 | 70 |
| 306 B | 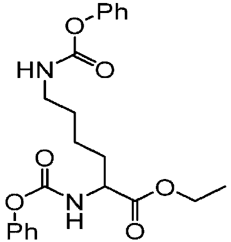 | 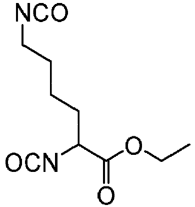 | 71 |

| | | | |
|---|---|---|---|
| (Ex: Example) | | | |

### INDUSTRIAL APPLICABILITY

The preparation method of an isocyanate according to the above-mentioned aspects makes it possible to prepare an isocyanate continuously while suppressing side reactions.

### EXPLANATION OF REFERENCE NUMERALS

100: Reactor
101, 102, 103, 104, 105: Storage tank
106, 107, 108: Packed bed
109, 110, 111, 112, 116: Liquid feed pump
113, 114, 115: Condenser
10, 11, 12, 13, 14, 15, 16, 17: Line
1A: Isocyanate preparation device
200: Falling film type reactor
201, 202, 203, 204: Storage tank
205: Condenser
206: Reboiler
207, 208, 209: Liquid feed pump
210: Distillation column
20, 21, 22, 23, 24, 25, 26, 27: Line
2A: Isocyanate preparation device

## Claims

1. A preparation method of an isocyanate in which the isocyanate is prepared by thermal decomposition of a carbamate, the preparation method comprising:
a thermal decomposition step in which a mixture liquid comprising a carbamate and at least one compound (A) is introduced continuously into a thermal decomposition reactor to allow a thermal decomposition reaction of the carbamate to proceed;
a low-boiling-point decomposition product collecting step in which a low-boiling-point decomposition product having a standard boiling point lower than a standard boiling point of the compound (A) is extracted continuously from the thermal decomposition reactor in a gaseous state; and
a high-boiling-point component collecting step in which a liquid-phase component which is not collected in a gaseous state in the low-boiling-point decomposition product collecting step is extracted continuously from the thermal decomposition reactor as a high-boiling-point component,
wherein
the compound (A) is selected from the group consisting of polymers having a repeating unit of general formula (4), compounds of general formula (5), compounds of general formula (6), compounds of general formula (7), compounds of general formula (S1), compounds of general formula (S2), compounds of general formula (S3), compounds of general formula (9), compounds of general formula (10), and C9-35 chained or cyclic aliphatic hydrocarbons, 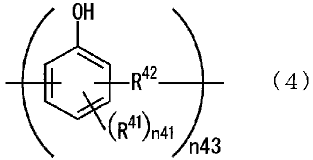
in the general formula (4), R⁴¹ is a monovalent hydrocarbon group, the hydrocarbon group may have either an ether bond or an ester bond, n41 is 0 or an integer of 1 to 3, R⁴² is a divalent organic group, and n43 is an integer of 2 to 50, 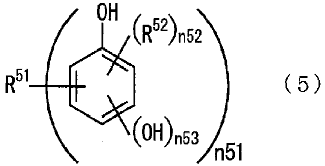
in the general formula (5), n51 is an integer of 1 to 4, R⁵¹ is a hydrogen atom or an n51-valent organic group, R⁵² is a monovalent hydrocarbon group, the hydrocarbon group may have either an ether bond or an ester bond, n52 is 0 or an integer of 1 to 4, and n53 is 0 or 1,
R⁶¹-(COO-R⁶²)ₙ₆₁ (6)
in the general formula (6), n61 is an integer of 1 to 3, R⁶¹ is an n61-valent C1-60 hydrocarbon group, the C1-60 hydrocarbon group may have either an ether bond or an ester bond, R⁶² is a C1-20 aliphatic hydrocarbon group or a C6-20 aromatic hydrocarbon group,
R⁷¹-(OCO-R⁷²)ₙ₇₁ (7)
in the general formula (7), n71 is 2 or 3, R⁷¹ is an n71-valent C1-60 hydrocarbon group, the C1-60 hydrocarbon group may have either an ether bond or an ester bond, R⁷² is a C1-20 aliphatic hydrocarbon group or a C6-20 aromatic hydrocarbon group, 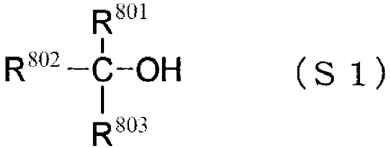
in the general formula (S1), R⁸⁰¹, R⁸⁰² and R⁸⁰³ are each independently a C1-60 saturated or unsaturated linear or branched hydrocarbon group, when R⁸⁰¹, R⁸⁰² or R⁸⁰³ has a methylene group, the methylene group may be substituted with an oxygen atom, an arylene group, a cycloalkylene group or an NH group, at least one CH group constituting R⁸⁰¹, R⁸⁰² or R⁸⁰³ may be substituted with a nitrogen atom, at least one hydrogen atom constituting R⁸⁰¹, R⁸⁰² or R⁸⁰³ may be substituted with a halogen atom or a hydroxy group, R⁸⁰¹, R⁸⁰² or R⁸⁰³ may be bonded together to form a monocycle or a polycycle, 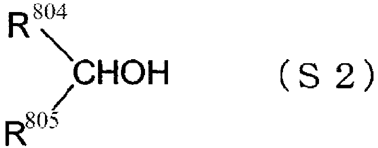
in the general formula (S2), R⁸⁰⁴ and R⁸⁰⁵ are each independently a C1-60 saturated or unsaturated linear or branched hydrocarbon group, when R⁸⁰⁴ or R⁸⁰⁵ has a methylene group, the methylene group may be substituted with an oxygen atom, an arylene group, a cycloalkylene group or an NH group, at least one CH group constituting R⁸⁰⁴ or R⁸⁰⁵ may be substituted with a nitrogen atom, at least one hydrogen atom constituting R⁸⁰⁴ or R⁸⁰⁵ may be substituted with a halogen atom or a hydroxy group, and R⁸⁰⁴ or R⁸⁰⁵ may be bonded together to form a monocycle or a polycycle,
R ⁸⁰⁶-CH₂OH (S3)
in the general formula (S3), R⁸⁰⁶ is a C1-60 saturated or unsaturated linear or branched hydrocarbon group, when R⁸⁰⁶ has a methylene group, the methylene group may be substituted with an oxygen atom, an arylene group, a cycloalkylene group or an NH group, at least one CH group constituting R⁸⁰⁶ may be substituted with a nitrogen atom, at least one hydrogen atom constituting R⁸⁰⁶ may be substituted with a halogen atom or a hydroxy group, branched chains may be bonded together to form a ring, 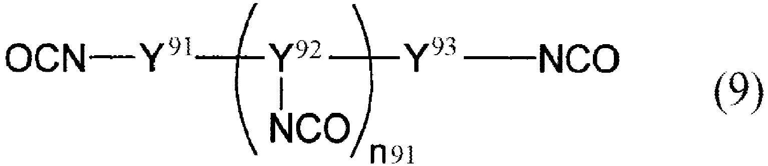
in the general formula (9), Y⁹¹ and Y⁹³ are each independently a C4-10 divalent hydrocarbon group having an alicyclic hydrocarbon group or an aromatic hydrocarbon group, Y⁹² is a C4-10 trivalent hydrocarbon group having an alicyclic hydrocarbon group or an aromatic hydrocarbon group, and n91 is an integer of 0 to 5, and 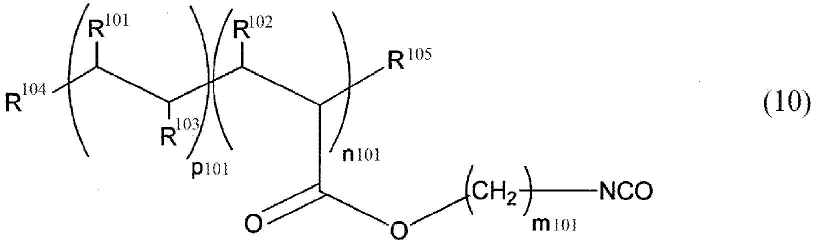
in the general formula (10), p101 is an integer of 0 to 90, n101 is an integer of 1 to 100, a sum of p101 and n101 is an integer of 10 to 100, m101 is an integer of 1 to 5, R¹⁰¹ and R¹⁰² are each independently a hydrogen atom or a C1-5 monovalent hydrocarbon group, R¹⁰³ is a C1-5 alkoxycarbonyl group or a C1-12 monovalent hydrocarbon group and R¹⁰⁴ and R¹⁰⁵ are each independently a monovalent organic group.

2. The preparation method of an isocyanate according to claim 1, wherein the compound (A) is selected from the group consisting of the polymers having a repeating unit of the general formula (4) and the compounds of the general formula (5).

3. The preparation method of an isocyanate according to claim 2, wherein the compound (A) is selected from the group consisting of polymers having either a repeating unit of general formula (4-1) or a repeating unit of general formula (4-2), compounds of general formula (5-1) and compounds of general formula (5-2), 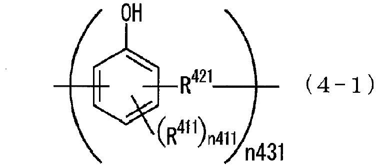 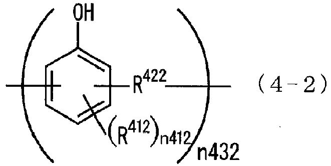
in the general formula (4-1), R⁴¹¹ is a monovalent hydrocarbon group, the monovalent hydrocarbon group may have either an ether bond or an ester bond, and may be substituted with a hydroxy group, n411 is 0 or an integer of 1 to 3, when n411 is 2 or 3, R⁴¹¹ may be identical to or different from each other, R⁴²¹ is a divalent aliphatic hydrocarbon group, the divalent aliphatic hydrocarbon group may have either an ether bond or an ester bond, and n431 is an integer of 2 to 50,
in the general formula (4-2), R⁴¹² is a monovalent hydrocarbon group, the monovalent hydrocarbon group may have either an ether bond or an ester bond, n412 is 0 or an integer of 1 to 3, R⁴²² is a divalent aromatic hydrocarbon group or a divalent group formed by bonding an aliphatic hydrocarbon group and an aromatic hydrocarbon group, the aliphatic hydrocarbon group may have either an ether bond or an ester bond, and n432 is an integer of 2 to 50, 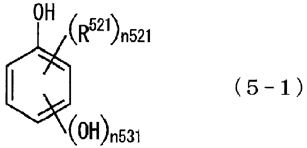 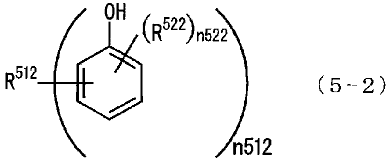
in the general formula (5-1), R⁵²¹ is a C1-20 alkyl group which may be substituted with a C6-12 aryl group or a C1-20 alkoxycarbonyl group which may be substituted with a C6-12 aryl group, n521 is 0 or an integer of 1 to 4, and n531 is 0 or 1, and
in the general formula (5-2), n512 is an integer of 2 to 4, R⁵¹² is an n512-divalent hydrocarbon group, the n512-divalent hydrocarbon group may have an ether bond, an ester bond, a carbonyl group, or a hetero ring, R⁵²² is a monovalent hydrocarbon group, the monovalent hydrocarbon group may have either an ether bond or an ester bond, and n522 is 0 or an integer of 1 to 4.

4. The preparation method of an isocyanate according to claim 1, wherein the compound (A) is selected from the group consisting of the compounds of the general formula (6) and the compounds of the general formula (7).

5. The preparation method of an isocyanate according to claim 7, wherein the compound (A) is selected from the group consisting of compounds of general formula (6-1), compounds of general formula (6-2), and compounds of general formula (7-1),
R⁶¹¹-(COO-R⁶¹²)ₙ₆₁₁ (6-1)
R⁶²¹-(COO-R⁶²²)ₙ₆₂₁ (6-2)
in the general formula (6-1), n611 is 2 or 3, R⁶¹¹ is an n611-valent C1-60 aliphatic hydrocarbon group, the C1-60 aliphatic hydrocarbon group may have either an ether bond or an ester bond, and R⁶¹² is a C1-20 aliphatic hydrocarbon group or a C6-20 aromatic hydrocarbon group,
in the general formula (6-2), n621 is 2 or 3, R⁶²¹ is an n621-valent C6-60 aromatic hydrocarbon group, the C6-60 aromatic hydrocarbon group may have either an ether bond or an ester bond, and R⁶²² is a C1-20 aliphatic hydrocarbon group or a C6-20 aromatic hydrocarbon group, and
R⁷¹¹-(OCO-R⁷¹²)ₙ₇₁₁ (7-1)
in the general formula (7-1), n711 is 2 or 3, R⁷¹¹ is an n711-valent C1-60 aliphatic hydrocarbon group, the C1-60 aliphatic hydrocarbon group may have either an ether bond or an ester bond, and R⁷¹² is a C1-20 aliphatic hydrocarbon group or a C6-20 aromatic hydrocarbon group.

6. The preparation method of an isocyanate according to claim 8, wherein the compound (A) is selected from the group consisting of compounds of general formula (6-1-1), compounds of general formula (6-2-1), and compounds of general formula (7-1-1), R
⁶¹³-OCO-Y⁶¹¹-COO-R⁶¹⁴ (6-1-1) 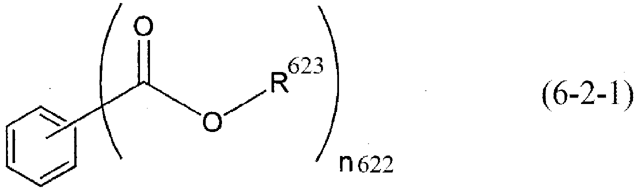
in the general formula (6-1-1), R⁶¹³ and R⁶¹⁴ are each independently a C1-20 aliphatic hydrocarbon group or a C6-20 aromatic hydrocarbon group, Y⁶¹¹ is a divalent C1-60 aliphatic hydrocarbon group, and the C1-60 aliphatic hydrocarbon group may have either an ether bond or an ester bond,
in the general formula (6-2-1), R⁶²³ is a C1-20 aliphatic hydrocarbon group or a C6-20 aromatic hydrocarbon group, and n622 is 2 or 3, and
R⁷¹³-COO-Y⁷¹¹-OCO-R⁷¹⁴ (7-1-1)
in the general formula (7-1-1), R⁷¹³ and R⁷¹⁴ are each independently a C1-20 aliphatic hydrocarbon group or a C6-20 aromatic hydrocarbon group, Y⁷¹¹ is a divalent C1-60 aliphatic hydrocarbon group, and the C1-20 aliphatic hydrocarbon group may have either an ether bond or an ester bond.

7. The preparation method of an isocyanate according to claim 1, wherein the compound (A) is selected from the group consisting of the compounds of the general formula (S1), the compounds of the general formula (S2) and the compounds of the general formula (S3).

8. The preparation method of an isocyanate according to claim 7, wherein the compound (A) is a compound of the general formula (S1).

9. The preparation method of an isocyanate according to claim 1, wherein the compound (A) is selected from the group consisting of the compounds of the general formula (9) and the compounds of the general formula (10).

10. The preparation method of an isocyanate according to claim 9, wherein the compound (A) is selected from the group consisting of compounds of general formula (9-1) and compounds of general formula (10-1), 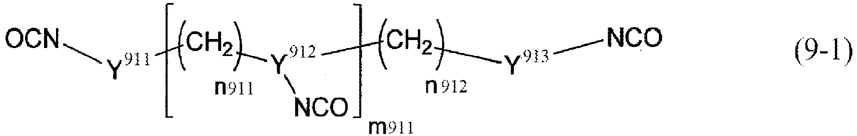
in the general formula (9-1), Y⁹¹¹ and Y⁹¹³ are each independently a C4-10 divalent alicyclic hydrocarbon group or a C6-10 divalent aromatic hydrocarbon group, Y⁹¹² is a C4-10 trivalent alicyclic hydrocarbon group or a C6-10 divalent aromatic hydrocarbon group, n911 and n912 are each independently an integer of 1 to 5, and m911 is an integer of 0 to 5, and 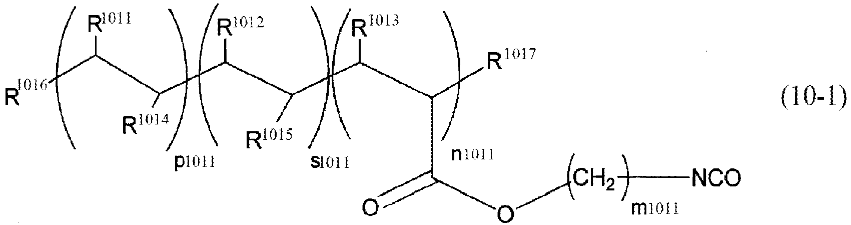
in the general formula (10-1), p1011 is an integer of 0 to 50, s1011 is an integer of 0 to 50, n1011 is an integer of 1 to 100, a sum of p1011, s1011 and n1011 is an integer of 10 to 100, m1011 is an integer of 1 to 5, R¹⁰¹¹, R¹⁰¹² and R¹⁰¹³ are each independently a hydrogen atom or a C1-5 monovalent hydrocarbon group, R¹⁰¹⁴ and R¹⁰¹⁵ are each independently a C1-5 alkoxycarbonyl group or a C1-12 monovalent hydrocarbon group, and R¹⁰¹⁶ and R¹⁰¹⁷ are each independently a monovalent organic group.

11. The preparation method of an isocyanate according to claim 9, wherein the compound (A) is a compound of formula (9-2), 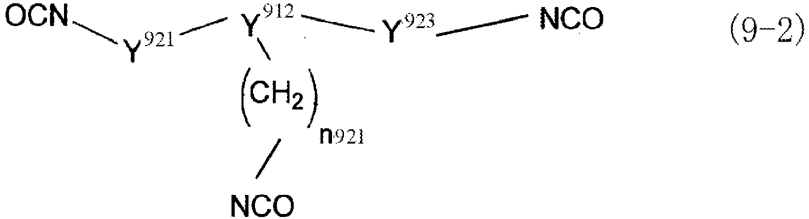 in the general formula (9-2), Y⁹²¹ and Y⁹²³ are each independently a C4-10 alkylene group, Y⁹¹² is a 2,4,6-trioxohexahydro-1,3,5-triazine-1,3,5-triynyl group, and n921 is an integer of 1 to 6.

12. The preparation method of an isocyanate according to claim 1, wherein the compound (A) is a C9-35 chained or cyclic aliphatic hydrocarbon.

13. The preparation method of an isocyanate according to claim 12, wherein the chained aliphatic hydrocarbon is a chained aliphatic hydrocarbon having a branched chain consisting of a C1-3 linear aliphatic hydrocarbon group.

14. The preparation method of an isocyanate according to claim 12 or 13, wherein a carbon number of the chained aliphatic hydrocarbon is 12 to 30.

15. The preparation method of an isocyanate according to any one of claims 1 to 14, wherein the mixture liquid further comprises an inert solvent,
in the low-boiling-point decomposition product collecting step, the low-boiling-point decomposition product and the inert solvent are extracted continuously from the thermal decomposition reactor in a gaseous state, and
the inert solvent is substantially inert under thermal decomposition reaction conditions, and a standard boiling point of the inert solvent is lower than the standard boiling point of the compound (A) and is between standard boiling points of the isocyanate and a hydroxy compound produced by thermal decomposition.

16. The preparation method of an isocyanate according to any one of claim 1 to 15, wherein the carbamate is a compound of general formula (2), 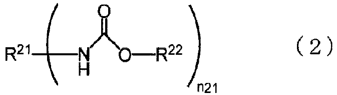 in the general formula (2), n21 is an integer of 1 or more, R²¹ is an n21-valent organic group, R²² is a remaining group formed by removing one hydroxyl group from a hydroxy compound.

17. The preparation method of an isocyanate according to claim 16, wherein in the general formula (2), n21 is 2 or 3 and R²² is a C6-20 aromatic group.

18. The preparation method of an isocyanate according to any one of claims 1 to 17, wherein the thermal decomposition reactor is a tubular reactor.

19. The preparation method of an isocyanate according to any one of claims 1 to 18,
wherein the low-boiling-point decomposition product comprises the isocyanate, and
the preparation method further comprises: a separation step in which the low-boiling-point decomposition product is supplied to a distillation column in a gaseous state, and the isocyanate is separated in the distillation column.

20. The preparation method of an isocyanate according to any one of claims 1 to 19,
wherein a carrier agent which is substantially inert in a gaseous state under thermal decomposition reaction conditions is introduced into the thermal decomposition reactor and a gaseous component is discharged from the thermal decomposition reactor.
